(19) Europäisches Patentamt
European Patent Office
Office européen des brevets

(11) EP 2 853 542 A1

(12) **EUROPEAN PATENT APPLICATION**

(43) Date of publication:
**01.04.2015 Bulletin 2015/14**

(21) Application number: **14193184.0**

(22) Date of filing: **23.11.2011**

(51) Int Cl.:
***C07K 16/22*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **24.11.2010 US 416844 P**

(62) Document number(s) of the earlier application(s) in accordance with Art. 76 EPC:
**11785457.0 / 2 643 351**

(27) Previously filed application:
**23.11.2011 PCT/EP2011/070868**

(71) Applicant: **Glaxo Group Limited**
**Brentford, Middlesex TW8 9GS (GB)**

(72) Inventors:
- **Griggs, Jeremy**
  **Stevenage, Hertfordshire SG1 2NY (GB)**
- **Parmar, Radha Shah**
  **Stevenage, Hertfordshire SG1 2NY (GB)**
- **Steward, Michael**
  **Stevenage, Hertfordshire SG1 2NY (GB)**

(74) Representative: **Fowler, Gavin James et al**
**GlaxoSmithKline**
**Global Patents (CN925.1)**
**980 Great West Road**
**Brentford, Middlesex TW8 9GS (GB)**

Remarks:
This application was filed on 14-11-2014 as a divisional application to the application mentioned under INID code 62.

(54) **Multispecific antigen binding proteins targeting HGF**

(57) The invention relates to combinations of HGF-antagonists with VEGF antagonists, and provides antigen-binding proteins which bind to HGF comprising a protein scaffold which are linked to one or more epitope-binding domains wherein the antigen-binding protein has at least two antigen binding sites at least one of which is from an epitope binding domain and at least one of which is from a paired VH/VL domain, methods of making such constructs and uses thereof.



Printed by Jouve, 75001 PARIS (FR)

SEQUENCE LISTING

<110> Griggs, Jeremy
Shah, Radha
Steward, Michael

<120> ANTIGEN BINDING PROTEINS

<130> PB64390

<140> US 61/416844
<141> 2010-11-24

<160> 196

<170> FastSEQ for Windows Version 4.0

<210> 1
<211> 57
<212> DNA
<213> Homo Sapiens

<400> 1
atgggctggt cctgcatcat cctgtttctg gtggccaccg ccaccggcgt gcacagc 57

<210> 2
<211> 19
<212> PRT
<213> Homo Sapiens

<400> 2
Met Gly Trp Ser Cys Ile Ile Leu Phe Leu Val Ala Thr Ala Thr Gly
1 5 10 15
Val His Ser

<210> 3
<211> 340
<212> DNA
<213> Mus-musculus

<400> 3
cagattcagc tgcggcagtc tggagctgga ctgatgaagc ctggggcctc agtgaagctt 60
tcctgcaagg ctactggcta cacattcact ggctactgga tagagtgggt aaagcagagg 120
cctggacatg accttgagtg gattggagag atttacctg gaagtggtac tactaactac 180
aatgagaagt tcaagggcaa ggccacattc actgcagata catcctccaa cacagcctac 240
atgcaactca gcagcctgac aactgaggac tctgccatct attactgtgc aaggggggt 300
tattactacg gtagtagcta cgactcctgg ggccaaggca 340

<210> 4
<211> 113
<212> PRT
<213> Mus-musculus

<400> 4
Gln Ile Gln Leu Arg Gln Ser Gly Ala Gly Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Asp Leu Glu Trp Ile
35 40 45
Gly Glu Ile Leu Pro Gly Ser Gly Thr Thr Asn Tyr Asn Glu Lys Phe

```
    50                  55                  60
Lys Gly Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Tyr Tyr Gly Ser Ser Tyr Asp Ser Trp Gly Gln
            100                 105                 110
Gly
```

<210> 5
<211> 336
<212> DNA
<213> Mus-musculus

<400> 5

```
gacattgtgc tgacccaatc tccagcttct ttggctgtgt ctctagggca gagggccacc 60
atctcctgca gagccagtga aagtgtcagt attcatggta ctcatttaat gcactggtac 120
caacagaaac caggacagcc acccaaactc ctcatctatg ctgcatccaa cctagaatct 180
ggagtccctg ccaggttcag tggcagtggg tctgagacag acttcaccct caacatccat 240
cctgtggagg aggaggatgc tgcaacctat ttctgtcagc aaagtattga ggatccgtac 300
acgttcggag gggggaccaa gctggaaata aaacgg                           336
```

<210> 6
<211> 112
<212> PRT
<213> Mus-musculus

<400> 6

```
Asp Ile Val Leu Thr Gln Ser Pro Ala Ser Leu Ala Val Ser Leu Gly
1               5                   10                  15
Gln Arg Ala Thr Ile Ser Cys Arg Ala Ser Glu Ser Val Ser Ile His
            20                  25                  30
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Glu Thr Asp Phe Thr Leu Asn Ile His
65                  70                  75                  80
Pro Val Glu Glu Glu Asp Ala Ala Thr Tyr Phe Cys Gln Gln Ser Ile
                85                  90                  95
Glu Asp Pro Tyr Thr Phe Gly Gly Gly Thr Lys Leu Glu Ile Lys Arg
            100                 105                 110
```

<210> 7
<211> 343
<212> DNA
<213> Mus-musculus

<400> 7

```
caggttcagc tgcagcagtc tggagctgag ctgatgaagc ctggggcctc agtgaagctt 60
tcctgcaagg ctactggcta cacattcact ggctactgga tagagtgggt aaagcagagg 120
cctggacatg gccttgagtg gattggagag attttacctg gaagtggtag tactaactac 180
aatgagaagt tcaagggcaa ggccacattc actgcagata catcctccaa cacagcctac 240
atgcaactca gcagcctgac aactgaggac tctgccatct attactgtgc aagagggggg 300
tatggttacc acgacgcctg gtttgcttac tggggccaag gac                   343
```

<210> 8
<211> 114
<212> PRT
<213> Mus-musculus

<400> 8

```
Gln Val Gln Leu Gln Gln Ser Gly Ala Glu Leu Met Lys Pro Gly Ala
1               5                   10                  15
Ser Val Lys Leu Ser Cys Lys Ala Thr Gly Tyr Thr Phe Thr Gly Tyr
            20                  25                  30
Trp Ile Glu Trp Val Lys Gln Arg Pro Gly His Gly Leu Glu Trp Ile
        35                  40                  45
Gly Glu Ile Leu Pro Gly Ser Gly Ser Thr Asn Tyr Asn Glu Lys Phe
    50                  55                  60
Lys Gly Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Gly Tyr His Asp Ala Trp Phe Ala Tyr Trp Gly
            100                 105                 110
Gln Gly
```

<210> 9
<211> 315
<212> DNA
<213> Mus-musculus

<400> 9

```
gacattgtga tgtcacagtc tccatcctcc ctagctgtgt cagttggaga gaaggttact 60
atgagctgca agtccagtca gagcctttta tatagtagca atcaaaagaa ctacttggcc 120
tggtaccagc agaaaccagg gcagtctcct aaactgctga tttactgggc atccactagg 180
gaatctgggg tccctgatcg cttcacaggc agtggatctg ggacagattt cactctcacc 240
atcagcagtg tgaaggctga agacctggca gtttattact gtcagcaata ttatagctat 300
ccgtacacgt tcgga                                                  315
```

<210> 10
<211> 105
<212> PRT
<213> Mus-musculus

<400> 10

```
Asp Ile Val Met Ser Gln Ser Pro Ser Ser Leu Ala Val Ser Val Gly
1               5                   10                  15
Glu Lys Val Thr Met Ser Cys Lys Ser Ser Gln Ser Leu Leu Tyr Ser
            20                  25                  30
Ser Asn Gln Lys Asn Tyr Leu Ala Trp Tyr Gln Gln Lys Pro Gly Gln
        35                  40                  45
Ser Pro Lys Leu Leu Ile Tyr Trp Ala Ser Thr Arg Glu Ser Gly Val
    50                  55                  60
Pro Asp Arg Phe Thr Gly Ser Gly Ser Gly Thr Asp Phe Thr Leu Thr
65                  70                  75                  80
Ile Ser Ser Val Lys Ala Glu Asp Leu Ala Val Tyr Tyr Cys Gln Gln
                85                  90                  95
Tyr Tyr Ser Tyr Pro Tyr Thr Phe Gly
            100                 105
```

<210> 11
<211> 339
<212> DNA
<213> Mus-musculus

<400> 11

```
caggttcagc tgcagcagtc tggagctgag ctgatgaagc ctggggcctc agtgaagctt 60
tcctgcaagg ctactggcta cacattcact ggctactgga tagagtgggt aaagcagagg 120
cctggacatg gccttgagtg gattggagag atttacctg gaagtggtag tactaactac 180
aatgagaagt tcaagggcaa ggccacattc actgcagata catcctccaa cacagcctac 240
atgcaactca gcagcctgac aactgaggac tctgccatct attactgtgc aagggggggt 300
tattactacg gtagtagctt tgactactgg ggccaaggc                        339
```

<210> 12
<211> 113
<212> PRT
<213> Mus-musculus

<400> 12
Gln Val Gln Leu Gln Gln Ser Gly Ala Glu Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Gly Leu Glu Trp Ile
35 40 45
Gly Glu Ile Leu Pro Gly Ser Gly Ser Thr Asn Tyr Asn Glu Lys Phe
50 55 60
Lys Gly Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Tyr Tyr Gly Ser Ser Phe Asp Tyr Trp Gly Gln
100 105 110
Gly

<210> 13
<211> 327
<212> DNA
<213> Mus-musculus

<400> 13
caaattgttc tcacccagtc tccagcaatc atgtctgcat ctctagggga acgggtcacc 60
atgacctgca ctgccagctc aagtgtaagt tccagttact tgcactggta ccagcagaag 120
ccaggatcct cccccaaact ctggatttat agcacatcca acctggcttc tggagtccca 180
gctcgcttca gtggcagtgg gtctgggacc tcttactctc tcacaatcag cagcatggag 240
gctgaagatg ctgccactta ttactgccac cagtatcatc gttccccgct cacgttcggt 300
gctgggacca agctggagct gaaacgg 327

<210> 14
<211> 109
<212> PRT
<213> Mus-musculus

<400> 14
Gln Ile Val Leu Thr Gln Ser Pro Ala Ile Met Ser Ala Ser Leu Gly
1 5 10 15
Glu Arg Val Thr Met Thr Cys Thr Ala Ser Ser Ser Val Ser Ser Ser
20 25 30
Tyr Leu His Trp Tyr Gln Gln Lys Pro Gly Ser Ser Pro Lys Leu Trp
35 40 45
Ile Tyr Ser Thr Ser Asn Leu Ala Ser Gly Val Pro Ala Arg Phe Ser
50 55 60
Gly Ser Gly Ser Gly Thr Ser Tyr Ser Leu Thr Ile Ser Ser Met Glu
65 70 75 80
Ala Glu Asp Ala Ala Thr Tyr Tyr Cys His Gln Tyr His Arg Ser Pro
85 90 95
Leu Thr Phe Gly Ala Gly Thr Lys Leu Glu Leu Lys Arg
100 105

<210> 15
<211> 360
<212> DNA
<213> Mus-musculus

<400> 15
caggttcagc tgcagcagtc tggagctgag ctgatgaagc ctggggcctc agtgaagctt 60
tcctgcaagg ctactggcta cacattcact ggctactgga tagagtgggt aaaacagagg 120
cctggacatg gccttgagtg gattggagag attttacctg gaagttctag tactaactac 180
aatgagaagt tcaaggacaa ggccacattc actgcagata catcctccaa cacagcctac 240
atgcaactca gcagcctgac aactgaggac tctgccatct attactgtgc aagaggggga 300
tattactacg gtagtcctat ggactactgg ggtcaaggaa cctcagtcac cgtctcctca 360

<210> 16
<211> 120
<212> PRT
<213> Mus-musculus

<400> 16
Gln Val Gln Leu Gln Gln Ser Gly Ala Glu Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Gly Leu Glu Trp Ile
35 40 45
Gly Glu Ile Leu Pro Gly Ser Ser Ser Thr Asn Tyr Asn Glu Lys Phe
50 55 60
Lys Asp Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Tyr Tyr Gly Ser Pro Met Asp Tyr Trp Gly Gln
100 105 110
Gly Thr Ser Val Thr Val Ser Ser
115 120

<210> 17
<211> 318
<212> DNA
<213> Mus-musculus

<400> 17
caaattgttc tcacccagtc tccagcaatc atgtctgcat ctccagggga gaaggtcacc 60
atgacctgca gtgccaggtc aagtgtaagt tacatgcact ggtaccagca gaagtcaggc 120
acctccccca aaagatggat ttatgacaca tccaaactgg cttctggagt ccctgctcgc 180
ttcagtggca gtgggtctgg gacctcttac tctctcacaa tcagcagcat ggaggctgaa 240
gatgctgcca cttattactg ccagcagtgg agtagtaacc cacccacgtt cggtggaggc 300
accaagctgg aaatcaaa 318

<210> 18
<211> 106
<212> PRT
<213> Mus-musculus

<400> 18
Gln Ile Val Leu Thr Gln Ser Pro Ala Ile Met Ser Ala Ser Pro Gly
1 5 10 15
Glu Lys Val Thr Met Thr Cys Ser Ala Arg Ser Ser Val Ser Tyr Met
20 25 30
His Trp Tyr Gln Gln Lys Ser Gly Thr Ser Pro Lys Arg Trp Ile Tyr
35 40 45
Asp Thr Ser Lys Leu Ala Ser Gly Val Pro Ala Arg Phe Ser Gly Ser
50 55 60
Gly Ser Gly Thr Ser Tyr Ser Leu Thr Ile Ser Ser Met Glu Ala Glu
65 70 75 80
Asp Ala Ala Thr Tyr Tyr Cys Gln Gln Trp Ser Ser Asn Pro Pro Thr
85 90 95
Phe Gly Gly Gly Thr Lys Leu Glu Ile Lys

100 105

<210> 19
<211> 5
<212> PRT
<213> Mus-musculus

<400> 19
Gly Tyr Trp Ile Glu
1 5

<210> 20
<211> 17
<212> PRT
<213> Mus-musculus

<400> 20
Glu Ile Leu Pro Gly Ser Gly Thr Thr Asn Tyr Asn Glu Lys Phe Lys
1 5 10 15
Gly

<210> 21
<211> 11
<212> PRT
<213> Mus-musculus

<400> 21
Gly Gly Tyr Tyr Tyr Gly Ser Ser Tyr Asp Ser
1 5 10

<210> 22
<211> 15
<212> PRT
<213> Mus-musculus

<400> 22
Arg Ala Ser Glu Ser Val Ser Ile His Gly Thr His Leu Met His
1 5 10 15

<210> 23
<211> 7
<212> PRT
<213> Mus-musculus

<400> 23
Ala Ala Ser Asn Leu Glu Ser
1 5

<210> 24
<211> 9
<212> PRT
<213> Mus-musculus

<400> 24
Gln Gln Ser Ile Glu Asp Pro Tyr Thr
1 5

<210> 25
<211> 5
<212> PRT
<213> Mus-musculus

<400> 25
Gly Tyr Trp Ile Glu
1 5

<210> 26
<211> 17
<212> PRT
<213> Mus-musculus

<400> 26
Glu Ile Leu Pro Gly Ser Gly Ser Thr Asn Tyr Asn Glu Lys Phe Lys
1 5 10 15
Gly

<210> 27
<211> 12
<212> PRT
<213> Mus-musculus

<400> 27
Gly Gly Tyr Gly Tyr His Asp Ala Trp Phe Ala Tyr
1 5 10

<210> 28
<211> 17
<212> PRT
<213> Mus-musculus

<400> 28
Lys Ser Ser Gln Ser Leu Leu Tyr Ser Ser Asn Gln Lys Asn Tyr Leu
1 5 10 15
Ala

<210> 29
<211> 7
<212> PRT
<213> Mus-musculus

<400> 29
Trp Ala Ser Thr Arg Glu Ser
1 5

<210> 30
<211> 9
<212> PRT
<213> Mus-musculus

<400> 30
Gln Gln Tyr Tyr Ser Tyr Pro Tyr Thr
1 5

<210> 31

<211> 5
<212> PRT
<213> Mus-musculus

<400> 31
Gly Tyr Trp Ile Glu
1 5

<210> 32
<211> 17
<212> PRT
<213> Mus-musculus

<400> 32
Glu Ile Leu Pro Gly Ser Gly Ser Thr Asn Tyr Asn Glu Lys Phe Lys
1 5 10 15
Gly

<210> 33
<211> 11
<212> PRT
<213> Mus-musculus

<400> 33
Gly Gly Tyr Tyr Tyr Gly Ser Ser Phe Asp Tyr
1 5 10

<210> 34
<211> 12
<212> PRT
<213> Mus-musculus

<400> 34
Thr Ala Ser Ser Ser Val Ser Ser Ser Tyr Leu His
1 5 10

<210> 35
<211> 7
<212> PRT
<213> Mus-musculus

<400> 35
Ser Thr Ser Asn Leu Ala Ser
1 5

<210> 36
<211> 9
<212> PRT
<213> Mus-musculus

<400> 36
His Gln Tyr His Arg Ser Pro Leu Thr
1 5

<210> 37
<211> 5
<212> PRT
<213> Mus-musculus

<400> 37
Gly Tyr Trp Ile Glu
1 5

<210> 38
<211> 17
<212> PRT
<213> Mus-musculus

<400> 38
Glu Ile Leu Pro Gly Ser Ser Ser Thr Asn Tyr Asn Glu Lys Phe Lys
1 5 10 15
Asp

<210> 39
<211> 11
<212> PRT
<213> Mus-musculus

<400> 39
Gly Gly Tyr Tyr Tyr Gly Ser Pro Met Asp Tyr
1 5 10

<210> 40
<211> 10
<212> PRT
<213> Mus-musculus

<400> 40
Ser Ala Arg Ser Ser Val Ser Tyr Met His
1 5 10

<210> 41
<211> 7
<212> PRT
<213> Mus-musculus

<400> 41
Asp Thr Ser Lys Leu Ala Ser
1 5

<210> 42
<211> 9
<212> PRT
<213> Mus-musculus

<400> 42
Gln Gln Trp Ser Ser Asn Pro Pro Thr
1 5

<210> 43
<211> 1350
<212> DNA
<213> Artificial Sequence

<220>
<223> murine-human chimeric heavy chain

<400> 43
cagatccagc tgcgccagtc cggcgccggc ctgatgaagc ccggcgcctc cgtgaagctg 60
tcctgcaagg ccaccggcta caccttcacc ggctactgga tcgagtgggt gaagcagcgc 120
cccggccacg acctggagtg gatcggcgag atcctgcccg gctccggcac caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tactactacg gctcctccta cgactcctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag 1350

<210> 44
<211> 450
<212> PRT
<213> Artificial Sequence

<220>
<223> murine-human chimeric heavy chain

<400> 44
Gln Ile Gln Leu Arg Gln Ser Gly Ala Gly Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Asp Leu Glu Trp Ile
35 40 45
Gly Glu Ile Leu Pro Gly Ser Gly Thr Thr Asn Tyr Asn Glu Lys Phe
50 55 60
Lys Gly Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys
450

<210> 45
<211> 654
<212> DNA
<213> Artificial Sequence

<220>
<223> murine-human chimeric light chain

<400> 45
gacatcgtgc tgacccagtc ccccgcctcc ctggccgtgt ccctgggcca gcgcgccacc 60
atctcctgcc gcgcctccga gtccgtgtcc atccacggca cccacctgat gcactggtac 120
cagcagaagc ccggccagcc ccccaagctg ctgatctacg ccgcctccaa cctggagtcc 180
ggcgtgcccg cccgcttctc cggctccggc tccgagaccg acttcaccct gaacatccac 240
cccgtggagg aggaggacgc cgccacctac ttctgccagc agtccatcga ggacccctac 300
accttcggcg gcggcaccaa gctggagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgc 654

<210> 46
<211> 218
<212> PRT
<213> Artificial Sequence

<220>
<223> murine-human chimeric light chain

<400> 46
Asp Ile Val Leu Thr Gln Ser Pro Ala Ser Leu Ala Val Ser Leu Gly
1 5 10 15
Gln Arg Ala Thr Ile Ser Cys Arg Ala Ser Glu Ser Val Ser Ile His
20 25 30
Gly Thr His Leu Met His Trp Tyr Gln Gln Lys Pro Gly Gln Pro Pro

35 40 45
Lys Leu Leu Ile Tyr Ala Ala Ser Asn Leu Glu Ser Gly Val Pro Ala
50 55 60
Arg Phe Ser Gly Ser Gly Ser Glu Thr Asp Phe Thr Leu Asn Ile His
65 70 75 80
Pro Val Glu Glu Glu Asp Ala Ala Thr Tyr Phe Cys Gln Gln Ser Ile
85 90 95
Glu Asp Pro Tyr Thr Phe Gly Gly Gly Thr Lys Leu Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys
210 215

<210> 47
<211> 1353
<212> DNA
<213> Artificial Sequence

<220>
<223> murine-human chimeric heavy chain

<400> 47
caggtgcagc tgcagcagtc cggcgccgag ctgatgaagc ccggcgcctc cgtgaagctg 60
tcctgcaagg ccaccggcta caccttcacc ggctactgga tcgagtgggt gaagcagcgc 120
cccggccacg gcctggagtg gatcggcgag atcctgcccg gctccggctc caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tacggctacc acgacgcctg gttcgcctac tggggccagg gcacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc ccccccctgcc ctgcccccga gctgctggga 720
ggccccagcg tgttcctgtt ccccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac caccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag 1353

<210> 48
<211> 451
<212> PRT
<213> Artificial Sequence

<220>
<223> murine-human chimeric heavy chain

<400> 48
Gln Val Gln Leu Gln Gln Ser Gly Ala Glu Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Gly Leu Glu Trp Ile
35 40 45
Gly Glu Ile Leu Pro Gly Ser Gly Ser Thr Asn Tyr Asn Glu Lys Phe
50 55 60
Lys Gly Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Gly Tyr His Asp Ala Trp Phe Ala Tyr Trp Gly
100 105 110
Gln Gly Thr Leu Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Leu Leu Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Pro Ala Pro Ile
325 330 335
Glu Lys Thr Ile Ser Lys Ala Lys Gly Gln Pro Arg Glu Pro Gln Val
340 345 350
Tyr Thr Leu Pro Pro Ser Arg Asp Glu Leu Thr Lys Asn Gln Val Ser
355 360 365
Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys
450

<210> 49
<211> 660
<212> DNA

<213> Artificial Sequence

<220>
<223> murine-human chimeric light chain

<400> 49
gacatcgtga tgtcccagtc cccctcctcc ctggccgtgt ccgtgggcga gaaggtgacc 60
atgtcctgca agtcctccca gtccctgctg tactcctcca accagaagaa ctacctggcc 120
tggtaccagc agaagcccgg ccagtccccc aagctgctga tctactgggc ctccacccgc 180
gagtccggcg tgcccgaccg cttcaccggc tccggctccg gcaccgactt caccctgacc 240
atctcctccg tgaaggccga ggacctggcc gtgtactact gccagcagta ctactcctac 300
ccctacacct tcggcggcgg caccaagctg gagatcaagc gtacggtggc cgcccccagc 360
gtgttcatct tcccccccag cgatgagcag ctgaagagcg gcaccgccag cgtggtgtgt 420
ctgctgaaca acttctaccc ccgggaggcc aaggtgcagt ggaaggtgga caatgccctg 480
cagagcggca acagccagga gagcgtgacc gagcaggaca gcaaggactc cacctacagc 540
ctgagcagca ccctgaccct gagcaaggcc gactacgaga agcacaaggt gtacgcctgt 600
gaggtgaccc accagggcct gtccagcccc gtgaccaaga gcttcaaccg gggcgagtgc 660

<210> 50
<211> 220
<212> PRT
<213> Artificial Sequence

<220>
<223> murine-human chimeric light chain

<400> 50
Asp Ile Val Met Ser Gln Ser Pro Ser Ser Leu Ala Val Ser Val Gly
1 5 10 15
Glu Lys Val Thr Met Ser Cys Lys Ser Ser Gln Ser Leu Leu Tyr Ser
20 25 30
Ser Asn Gln Lys Asn Tyr Leu Ala Trp Tyr Gln Gln Lys Pro Gly Gln
35 40 45
Ser Pro Lys Leu Leu Ile Tyr Trp Ala Ser Thr Arg Glu Ser Gly Val
50 55 60
Pro Asp Arg Phe Thr Gly Ser Gly Ser Gly Thr Asp Phe Thr Leu Thr
65 70 75 80
Ile Ser Ser Val Lys Ala Glu Asp Leu Ala Val Tyr Tyr Cys Gln Gln
85 90 95
Tyr Tyr Ser Tyr Pro Tyr Thr Phe Gly Gly Gly Thr Lys Leu Glu Ile
100 105 110
Lys Arg Thr Val Ala Ala Pro Ser Val Phe Ile Phe Pro Pro Ser Asp
115 120 125
Glu Gln Leu Lys Ser Gly Thr Ala Ser Val Val Cys Leu Leu Asn Asn
130 135 140
Phe Tyr Pro Arg Glu Ala Lys Val Gln Trp Lys Val Asp Asn Ala Leu
145 150 155 160
Gln Ser Gly Asn Ser Gln Glu Ser Val Thr Glu Gln Asp Ser Lys Asp
165 170 175
Ser Thr Tyr Ser Leu Ser Ser Thr Leu Thr Leu Ser Lys Ala Asp Tyr
180 185 190
Glu Lys His Lys Val Tyr Ala Cys Glu Val Thr His Gln Gly Leu Ser
195 200 205
Ser Pro Val Thr Lys Ser Phe Asn Arg Gly Glu Cys
210 215 220

<210> 51
<211> 1350
<212> DNA
<213> Artificial Sequence

<220>
<223> murine-human chimeric heavy chain

<400> 51
caggtgcagc tgcagcagtc cggcgccgag ctgatgaagc ccggcgcctc cgtgaagctg 60
tcctgcaagg ccaccggcta caccttcacc ggctactgga tcgagtgggt gaagcagcgc 120
cccggccacg gcctggagtg gatcggcgag atcctgcccg gctccggctc caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tactactacg gctcctcctt cgactactgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag 1350

<210> 52
<211> 450
<212> PRT
<213> Artificial Sequence

<220>
<223> murine-human chimeric heavy chain

<400> 52
Gln Val Gln Leu Gln Gln Ser Gly Ala Glu Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Gly Leu Glu Trp Ile
35 40 45
Gly Glu Ile Leu Pro Gly Ser Gly Ser Thr Asn Tyr Asn Glu Lys Phe
50 55 60
Lys Gly Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Tyr Tyr Gly Ser Ser Phe Asp Tyr Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys
450

<210> 53
<211> 645
<212> DNA
<213> Artificial Sequence

<220>
<223> murine-human chimeric light chain

<400> 53
cagatcgtgc tgacccagtc ccccgccatc atgtccgcct ccctgggcga gcgcgtgacc 60
atgacctgca ccgcctcctc ctccgtgtcc tcctcctacc tgcactggta ccagcagaag 120
cccggctcct cccccaagct gtggatctac tccacctcca acctggcctc cggcgtgccc 180
gcccgcttct ccggctccgg ctccggcacc tcctactccc tgaccatctc ctccatggag 240
gccgaggacg ccgccaccta ctactgccac cagtaccacc gctcccccct gaccttcggc 300
gccggcacca agctggagat caagcgtacg gtggccgccc ccagcgtgtt catcttcccc 360
cccagcgatg agcagctgaa gagcggcacc gccagcgtgg tgtgtctgct gaacaacttc 420
tacccccggg aggccaaggt gcagtggaag gtggacaatg ccctgcagag cggcaacagc 480
caggagagcg tgaccgagca ggacagcaag gactccacct acagcctgag cagcaccctg 540
accctgagca aggccgacta cgagaagcac aaggtgtacg cctgtgaggt gacccaccag 600
ggcctgtcca gccccgtgac caagagcttc aaccggggcg agtgc 645

<210> 54
<211> 215
<212> PRT
<213> Artificial Sequence

<220>
<223> murine-human chimeric light chain

<400> 54
Gln Ile Val Leu Thr Gln Ser Pro Ala Ile Met Ser Ala Ser Leu Gly
1 5 10 15
Glu Arg Val Thr Met Thr Cys Thr Ala Ser Ser Ser Val Ser Ser Ser
20 25 30
Tyr Leu His Trp Tyr Gln Gln Lys Pro Gly Ser Ser Pro Lys Leu Trp

```
            35                  40                  45
Ile Tyr Ser Thr Ser Asn Leu Ala Ser Gly Val Pro Ala Arg Phe Ser
    50                  55                  60
Gly Ser Gly Ser Gly Thr Ser Tyr Ser Leu Thr Ile Ser Ser Met Glu
65                  70                  75                  80
Ala Glu Asp Ala Ala Thr Tyr Tyr Cys His Gln Tyr His Arg Ser Pro
                85                  90                  95
Leu Thr Phe Gly Ala Gly Thr Lys Leu Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215
```

<210> 55
<211> 1350
<212> DNA
<213> Artificial Sequence

<220>
<223> murine-human chimeric heavy chain

<400> 55

```
caggtgcagc tccagcagag cggagccgag ctgatgaaac ccggggccag cgtgaagctg 60
agctgcaagg ccaccggcta caccttcacc ggctactgga tcgagtgggt gaagcagagg 120
cccggccacg gcctggagtg gatcggcgaa atcctgcccg gcagcagcag caccaactac 180
aacgagaagt tcaaggacaa ggccaccttc accgccgaca ctagcagcaa caccgcctac 240
atgcagctga gcagcctgac aaccgaggac tccgcaatct actactgcgc caggggcggc 300
tactactacg gcagccccat ggactattgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350
```

<210> 56
<211> 450
<212> PRT
<213> Artificial Sequence

<220>
<223> murine-human chimeric heavy chain

<400> 56

Gln Val Gln Leu Gln Gln Ser Gly Ala Glu Leu Met Lys Pro Gly Ala
1               5                   10                  15
Ser Val Lys Leu Ser Cys Lys Ala Thr Gly Tyr Thr Phe Thr Gly Tyr
            20                  25                  30
Trp Ile Glu Trp Val Lys Gln Arg Pro Gly His Gly Leu Glu Trp Ile
        35                  40                  45
Gly Glu Ile Leu Pro Gly Ser Ser Ser Thr Asn Tyr Asn Glu Lys Phe
    50                  55                  60
Lys Asp Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Tyr Tyr Gly Ser Pro Met Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210> 57
<211> 639
<212> DNA

<213> Artificial Sequence

<220>
<223> murine-human chimeric light chain

<400> 57
```
cagatcgtgc tgacccagag ccccgccatt atgagcgcta gccccgggga gaaggtgacc 60
atgacctgca gcgccaggag cagcgtgagc tacatgcact ggtaccagca gaagagcggc 120
accagcccca agaggtggat ctacgacacc agcaagctgg cctcaggcgt gcccgccagg 180
ttcagcggct ctggcagcgg caccagctac agcctgacca tctccagcat ggaggccgag 240
gacgccgcca cctactattg ccagcagtgg agcagcaacc ctcccacttt cggcggcggc 300
accaaactgg agatcaagcg tacggtggcc gcccccagcg tgttcatctt cccccccagc 360
gatgagcagc tgaagagcgg caccgccagc gtggtgtgtc tgctgaacaa cttctacccc 420
cgggaggcca aggtgcagtg gaaggtggac aatgccctgc agagcggcaa cagccaggag 480
agcgtgaccg agcaggacag caaggactcc acctacagcc tgagcagcac cctgaccctg 540
agcaaggccg actacgagaa gcacaaggtg tacgcctgtg aggtgaccca ccagggcctg 600
tccagccccg tgaccaagag cttcaaccgg ggcgagtgc                        639
```

<210> 58
<211> 213
<212> PRT
<213> Artificial Sequence

<220>
<223> murine-human chimeric light chain

<400> 58
```
Gln Ile Val Leu Thr Gln Ser Pro Ala Ile Met Ser Ala Ser Pro Gly
1               5                   10                  15
Glu Lys Val Thr Met Thr Cys Ser Ala Arg Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Pro Thr
                85                  90                  95
Phe Gly Gly Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210
```

<210> 59
<211> 360
<212> DNA
<213> Artificial Sequence

<220>
<223> murine-human chimeric heavy chain variable domain

<400> 59
cagatccagc tgcgccagtc cggcgccggc ctgatgaagc ccggcgcctc cgtgaagctg 60
tcctgcaagg ccaccggcta caccttcacc ggctactgga tcgagtgggt gaagcagcgc 120
cccggccacg acctggagtg gatcggcgag atcctgcccg gctccggcac caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tactactacg gctcctccta cgactcctgg ggccagggca cactagtgac cgtgtccagc 360

<210> 60
<211> 120
<212> PRT
<213> Artificial Sequence

<220>
<223> murine-human chimeric heavy chain variable domain

<400> 60
Gln Ile Gln Leu Arg Gln Ser Gly Ala Gly Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Asp Leu Glu Trp Ile
35 40 45
Gly Glu Ile Leu Pro Gly Ser Gly Thr Thr Asn Tyr Asn Glu Lys Phe
50 55 60
Lys Gly Lys Ala Thr Phe Thr Ala Asp Thr Ser Ser Asn Thr Ala Tyr
65 70 75 80
Met Gln Leu Ser Ser Leu Thr Thr Glu Asp Ser Ala Ile Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser
115 120

<210> 61
<211> 336
<212> DNA
<213> Artificial Sequence

<220>
<223> murine-human chimeric light chain variable domain

<400> 61
gacatcgtgc tgacccagtc ccccgcctcc ctggccgtgt ccctgggcca gcgcgccacc 60
atctcctgcc gcgcctccga gtccgtgtcc atccacggca cccacctgat gcactggtac 120
cagcagaagc ccggccagcc ccccaagctg ctgatctacg ccgcctccaa cctggagtcc 180
ggcgtgcccg cccgcttctc cggctccggc tccgagaccg acttcaccct gaacatccac 240
cccgtggagg aggaggacgc cgccacctac ttctgccagc agtccatcga ggacccctac 300
accttcggcg gcggcaccaa gctggagatc aagcgt 336

<210> 62
<211> 112
<212> PRT
<213> Artificial Sequence

<220>
<223> murine-human chimeric light chain variable domain

<400> 62
Asp Ile Val Leu Thr Gln Ser Pro Ala Ser Leu Ala Val Ser Leu Gly
1 5 10 15
Gln Arg Ala Thr Ile Ser Cys Arg Ala Ser Glu Ser Val Ser Ile His
20 25 30

```
Gly Thr His Leu Met His Trp Tyr Gln Gln Lys Pro Gly Gln Pro Pro
        35                  40                  45
Lys Leu Leu Ile Tyr Ala Ala Ser Asn Leu Glu Ser Gly Val Pro Ala
    50                  55                  60
Arg Phe Ser Gly Ser Gly Ser Glu Thr Asp Phe Thr Leu Asn Ile His
65                  70                  75                  80
Pro Val Glu Glu Glu Asp Ala Ala Thr Tyr Phe Cys Gln Gln Ser Ile
                85                  90                  95
Glu Asp Pro Tyr Thr Phe Gly Gly Gly Thr Lys Leu Glu Ile Lys Arg
            100                 105                 110
```

<210> 63
<211> 363
<212> DNA
<213> Artificial Sequence

<220>
<223> murine-human chimeric heavy chain variable domain

<400> 63

```
caggtgcagc tgcagcagtc cggcgccgag ctgatgaagc ccggcgcctc cgtgaagctg 60
tcctgcaagg ccaccggcta caccttcacc ggctactgga tcgagtgggt gaagcagcgc 120
cccggccacg gcctggagtg gatcggcgag atcctgcccg gctccggctc caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tacggctacc acgacgcctg gttcgcctac tggggccagg gcacactagt gaccgtgtcc 360
agc                                                               363
```

<210> 64
<211> 121
<212> PRT
<213> Artificial Sequence

<220>
<223> murine-human chimeric heavy chain variable domain

<400> 64

```
Gln Val Gln Leu Gln Gln Ser Gly Ala Glu Leu Met Lys Pro Gly Ala
1               5                   10                  15
Ser Val Lys Leu Ser Cys Lys Ala Thr Gly Tyr Thr Phe Thr Gly Tyr
            20                  25                  30
Trp Ile Glu Trp Val Lys Gln Arg Pro Gly His Gly Leu Glu Trp Ile
        35                  40                  45
Gly Glu Ile Leu Pro Gly Ser Gly Ser Thr Asn Tyr Asn Glu Lys Phe
    50                  55                  60
Lys Gly Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Gly Tyr His Asp Ala Trp Phe Ala Tyr Trp Gly
            100                 105                 110
Gln Gly Thr Leu Val Thr Val Ser Ser
        115                 120
```

<210> 65
<211> 342
<212> DNA
<213> Artificial Sequence

<220>
<223> murine-human chimeric light chain variable domain

<400> 65

gacatcgtga tgtcccagtc cccctcctcc ctggccgtgt ccgtgggcga gaaggtgacc 60
atgtcctgca agtcctccca gtccctgctg tactcctcca accagaagaa ctacctggcc 120
tggtaccagc agaagcccgg ccagtccccc aagctgctga tctactgggc ctccacccgc 180
gagtccggcg tgcccgaccg cttcaccggc tccggctccg gcaccgactt caccctgacc 240
atctcctccg tgaaggccga ggacctggcc gtgtactact gccagcagta ctactcctac 300
ccctacacct tcggcggcgg caccaagctg gagatcaagc gt 342

<210> 66
<211> 114
<212> PRT
<213> Artificial Sequence

<220>
<223> murine-human chimeric light chain variable domain

<400> 66
Asp Ile Val Met Ser Gln Ser Pro Ser Ser Leu Ala Val Ser Val Gly
1 5 10 15
Glu Lys Val Thr Met Ser Cys Lys Ser Ser Gln Ser Leu Leu Tyr Ser
20 25 30
Ser Asn Gln Lys Asn Tyr Leu Ala Trp Tyr Gln Gln Lys Pro Gly Gln
35 40 45
Ser Pro Lys Leu Leu Ile Tyr Trp Ala Ser Thr Arg Glu Ser Gly Val
50 55 60
Pro Asp Arg Phe Thr Gly Ser Gly Ser Gly Thr Asp Phe Thr Leu Thr
65 70 75 80
Ile Ser Ser Val Lys Ala Glu Asp Leu Ala Val Tyr Tyr Cys Gln Gln
85 90 95
Tyr Tyr Ser Tyr Pro Tyr Thr Phe Gly Gly Gly Thr Lys Leu Glu Ile
100 105 110
Lys Arg

<210> 67
<211> 360
<212> DNA
<213> Artificial Sequence

<220>
<223> murine-human chimeric heavy chain variable domain

<400> 67
caggtgcagc tgcagcagtc cggcgccgag ctgatgaagc ccggcgcctc cgtgaagctg 60
tcctgcaagg ccaccggcta caccttcacc ggctactgga tcgagtgggt gaagcagcgc 120
cccggccacg gcctggagtg gatcggcgag atcctgcccg gctccggctc caccaactac 180
aacgagaagt tcaagggcaa ggccaccttc accgccgaca cctcctccaa caccgcctac 240
atgcagctgt cctccctgac caccgaggac tccgccatct actactgcgc ccgcggcggc 300
tactactacg gctcctcctt cgactactgg ggccagggca cactagtgac cgtgtccagc 360

<210> 68
<211> 120
<212> PRT
<213> Artificial Sequence

<220>
<223> murine-human chimeric heavy chain variable domain

<400> 68
Gln Val Gln Leu Gln Gln Ser Gly Ala Glu Leu Met Lys Pro Gly Ala
1 5 10 15
Ser Val Lys Leu Ser Cys Lys Ala Thr Gly Tyr Thr Phe Thr Gly Tyr
20 25 30
Trp Ile Glu Trp Val Lys Gln Arg Pro Gly His Gly Leu Glu Trp Ile

```
        35                  40                  45
Gly Glu Ile Leu Pro Gly Ser Gly Ser Thr Asn Tyr Asn Glu Lys Phe
    50                  55                  60
Lys Gly Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Tyr Tyr Gly Ser Ser Phe Asp Tyr Trp Gly Gln
            100                 105                 110
Gly Thr Leu Val Thr Val Ser Ser
        115                 120


<210> 69
<211> 327
<212> DNA
<213> Artificial Sequence

<220>
<223> murine-human chimeric light chain variable domain

<400> 69
cagatcgtgc tgacccagtc ccccgccatc atgtccgcct ccctgggcga gcgcgtgacc 60
atgacctgca ccgcctcctc ctccgtgtcc tcctcctacc tgcactggta ccagcagaag 120
cccggctcct cccccaagct gtggatctac tccacctcca acctggcctc cggcgtgccc 180
gcccgcttct ccggctccgg ctccggcacc tcctactccc tgaccatctc ctccatggag 240
gccgaggacg ccgccaccta ctactgccac cagtaccacc gctccccccct gaccttcggc 300
gccggcacca agctggagat caagcgt                                     327

<210> 70
<211> 109
<212> PRT
<213> Artificial Sequence

<220>
<223> murine-human chimeric light chain variable domain

<400> 70
Gln Ile Val Leu Thr Gln Ser Pro Ala Ile Met Ser Ala Ser Leu Gly
1               5                   10                  15
Glu Arg Val Thr Met Thr Cys Thr Ala Ser Ser Ser Val Ser Ser Ser
            20                  25                  30
Tyr Leu His Trp Tyr Gln Gln Lys Pro Gly Ser Ser Pro Lys Leu Trp
        35                  40                  45
Ile Tyr Ser Thr Ser Asn Leu Ala Ser Gly Val Pro Ala Arg Phe Ser
    50                  55                  60
Gly Ser Gly Ser Gly Thr Ser Tyr Ser Leu Thr Ile Ser Ser Met Glu
65                  70                  75                  80
Ala Glu Asp Ala Ala Thr Tyr Tyr Cys His Gln Tyr His Arg Ser Pro
                85                  90                  95
Leu Thr Phe Gly Ala Gly Thr Lys Leu Glu Ile Lys Arg
            100                 105


<210> 71
<211> 360
<212> DNA
<213> Artificial Sequence

<220>
<223> murine-human chimeric heavy chain variable domain

<400> 71
caggtgcagc tccagcagag cggagccgag ctgatgaaac ccggggccag cgtgaagctg 60
agctgcaagg ccaccggcta caccttcacc ggctactgga tcgagtgggt gaagcagagg 120
```

cccggccacg gcctggagtg gatcggcgaa atcctgcccg gcagcagcag caccaactac 180
aacgagaagt tcaaggacaa ggccaccttc accgccgaca ctagcagcaa caccgcctac 240
atgcagctga gcagcctgac aaccgaggac tccgcaatct actactgcgc caggggcggc 300
tactactacg gcagccccat ggactattgg ggccagggca cactagtgac cgtgtccagc 360

<210> 72
<211> 120
<212> PRT
<213> Artificial Sequence

<220>
<223> murine-human chimeric heavy chain variable domain

<400> 72
Gln Val Gln Leu Gln Gln Ser Gly Ala Glu Leu Met Lys Pro Gly Ala
1               5                   10                  15
Ser Val Lys Leu Ser Cys Lys Ala Thr Gly Tyr Thr Phe Thr Gly Tyr
            20                  25                  30
Trp Ile Glu Trp Val Lys Gln Arg Pro Gly His Gly Leu Glu Trp Ile
        35                  40                  45
Gly Glu Ile Leu Pro Gly Ser Ser Ser Thr Asn Tyr Asn Glu Lys Phe
    50                  55                  60
Lys Asp Lys Ala Thr Phe Thr Ala Asp Thr Ser Ser Asn Thr Ala Tyr
65                  70                  75                  80
Met Gln Leu Ser Ser Leu Thr Thr Glu Asp Ser Ala Ile Tyr Tyr Cys
                85                  90                  95
Ala Arg Gly Gly Tyr Tyr Tyr Gly Ser Pro Met Asp Tyr Trp Gly Gln
            100                 105                 110
Gly Thr Leu Val Thr Val Ser Ser
        115                 120

<210> 73
<211> 321
<212> DNA
<213> Artificial Sequence

<220>
<223> murine-human chimeric light chain variable domain

<400> 73
cagatcgtgc tgacccagag ccccgccatt atgagcgcta gccccgggga gaaggtgacc 60
atgacctgca gcgccaggag cagcgtgagc tacatgcact ggtaccagca gaagagcggc 120
accagcccca agaggtggat ctacgacacc agcaagctgg cctcaggcgt gcccgccagg 180
ttcagcggct ctggcagcgg caccagctac agcctgacca tctccagcat ggaggccgag 240
gacgccgcca cctactattg ccagcagtgg agcagcaacc ctcccacttt cggcggcggc 300
accaaactgg agatcaagcg t 321

<210> 74
<211> 107
<212> PRT
<213> Artificial Sequence

<220>
<223> murine-human chimeric light chain variable domain

<400> 74
Gln Ile Val Leu Thr Gln Ser Pro Ala Ile Met Ser Ala Ser Pro Gly
1               5                   10                  15
Glu Lys Val Thr Met Thr Cys Ser Ala Arg Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser

```
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Gln Gln Trp Ser Ser Asn Pro Pro Thr
                85                  90                  95
Phe Gly Gly Gly Thr Lys Leu Glu Ile Lys Arg
            100                 105
```

<210> 75
<211> 1350
<212> DNA
<213> Artificial Sequence

<220>
<223> humanized heavy chain

<400> 75

```
cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatgggcgag atcctgcccg ggtctggcac caccaactac 180
aacgagaagt tcaagggccg cgtgaccatc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350
```

<210> 76
<211> 450
<212> PRT
<213> Artificial Sequence

<220>
<223> humanized heavy chain

<400> 76

```
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Gly Thr Thr Asn Tyr Asn Glu Lys Phe
    50                  55                  60
Lys Gly Arg Val Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
```

```
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

<210> 77
<211> 1350
<212> DNA
<213> Artificial Sequence

<220>
<223> humanized heavy chain

<400> 77

```
cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
```

```
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350
```

<210> 78
<211> 450
<212> PRT
<213> Artificial Sequence

<220>
<223> humanized heavy chain

<400> 78

```
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
```

```
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

```
<210> 79
<211> 1350
<212> DNA
<213> Artificial Sequence

<220>
<223> humanized heavy chain

<400> 79
cagatccagc tgaggcagag cggcgccgag gtgaaaaagc ccggcgccag cgtgaagctg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggcaa ggccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350

<210> 80
<211> 450
<212> PRT
<213> Artificial Sequence

<220>
<223> humanized heavy chain

<400> 80
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
```

```
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

<210> 81
<211> 1350
<212> DNA
<213> Artificial Sequence

<220>
<223> humanized heavy chain

<400> 81
cagatccagc tggtgcagag cggcgccgag gtgaaaaagc ccggcagcag cgtgaaggtg 60

```
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca agtccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350
```

<210> 82
<211> 450
<212> PRT
<213> Artificial Sequence

<220>
<223> humanized heavy chain

<400> 82

```
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
```

```
        260                 265                 270
Asp Pro Glu Val Lys Phe Asn Trp Tyr Val Asp Gly Val Glu Val His
    275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
        340                 345                 350
Thr Leu Pro Pro Ser Arg Asp Glu Leu Thr Lys Asn Gln Val Ser Leu
    355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
        420                 425                 430
Glu Ala Leu His Asn His Tyr Thr Gln Lys Ser Leu Ser Leu Ser Pro
    435                 440                 445
Gly Lys
    450
```

<210> 83
<211> 1350
<212> DNA
<213> Artificial Sequence

<220>
<223> humanized heavy chain

<400> 83

```
caggtgcagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag                                  1350
```

<210> 84
<211> 450
<212> PRT
<213> Artificial Sequence

<220>
<223> humanized heavy chain

<400> 84
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210> 85

<211> 654
<212> DNA
<213> Artificial Sequence

<220>
<223> humanized light chain

<400> 85
gacatcgtga tgacccagtc ccccgatagc ctggctgtgt cactggggga gagggccacc 60
atcaactgca gggccagcga gtccgtgagc atccacggca cccacctgat gcactggtac 120
cagcagaagc ccggccagcc ccctaagctg ctgatctacg ccgccagcaa cctcgaaagc 180
ggcgtccccg acaggttcag cggcagcggc agcggcaccg acttcaccct gactatcagc 240
agcctgcagg ccgaggacgt ggccgtctac tactgccagc agagcatcga ggacccctac 300
accttcggcc agggcaccaa gctggagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgc 654

<210> 86
<211> 218
<212> PRT
<213> Artificial Sequence

<220>
<223> humanized light chain

<400> 86
Asp Ile Val Met Thr Gln Ser Pro Asp Ser Leu Ala Val Ser Leu Gly
1 5 10 15
Glu Arg Ala Thr Ile Asn Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Asn Leu Glu Ser Gly Val Pro Asp
50 55 60
Arg Phe Ser Gly Ser Gly Ser Gly Thr Asp Phe Thr Leu Thr Ile Ser
65 70 75 80
Ser Leu Gln Ala Glu Asp Val Ala Val Tyr Tyr Cys Gln Gln Ser Ile
85 90 95
Glu Asp Pro Tyr Thr Phe Gly Gln Gly Thr Lys Leu Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys
210 215

<210> 87
<211> 654
<212> DNA
<213> Artificial Sequence

<220>

<223> humanized light chain

<400> 87
gacatcgtgc tgacccagtc ccccgatagc ctggctgtgt cactggggga gagggccacc 60
atcaactgca gggccagcga gtccgtgagc atccacggca cccacctgat gcactggtac 120
cagcagaagc ccggccagcc ccctaagctg ctgatctacg ccgccagcaa cctcgaaagc 180
ggcgtccccg acaggttcag cggcagcggc agcgagaccg acttcaccct gactatcagc 240
agcctgcagg ccgaggacgt ggccgtctac tactgccagc agagcatcga ggacccctac 300
accttcggcc agggcaccaa gctggagatc aagcgtacgg tggccgcccc cagcgtgttc 360
atcttccccc ccagcgatga gcagctgaag agcggcaccg ccagcgtggt gtgtctgctg 420
aacaacttct acccccggga ggccaaggtg cagtggaagg tggacaatgc cctgcagagc 480
ggcaacagcc aggagagcgt gaccgagcag gacagcaagg actccaccta cagcctgagc 540
agcaccctga ccctgagcaa ggccgactac gagaagcaca aggtgtacgc ctgtgaggtg 600
acccaccagg gcctgtccag ccccgtgacc aagagcttca accggggcga gtgc 654

<210> 88
<211> 218
<212> PRT
<213> Artificial Sequence

<220>
<223> humanized light chain

<400> 88
Asp Ile Val Leu Thr Gln Ser Pro Asp Ser Leu Ala Val Ser Leu Gly
1 5 10 15
Glu Arg Ala Thr Ile Asn Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Asn Leu Glu Ser Gly Val Pro Asp
50 55 60
Arg Phe Ser Gly Ser Gly Ser Glu Thr Asp Phe Thr Leu Thr Ile Ser
65 70 75 80
Ser Leu Gln Ala Glu Asp Val Ala Val Tyr Tyr Cys Gln Gln Ser Ile
85 90 95
Glu Asp Pro Tyr Thr Phe Gly Gln Gly Thr Lys Leu Glu Ile Lys Arg
100 105 110
Thr Val Ala Ala Pro Ser Val Phe Ile Phe Pro Pro Ser Asp Glu Gln
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys
210 215

<210> 89
<211> 360
<212> DNA
<213> Artificial Sequence

<220>
<223> humanized heavy chain variable domain

<400> 89
cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120

```
cccggacagg gcctggagtg gatgggcgag atcctgcccg ggtctggcac caccaactac 180
aacgagaagt tcaagggccg cgtgaccatc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360


<210> 90
<211> 120
<212> PRT
<213> Artificial Sequence

<220>
<223> humanized heavy chain variable domain

<400> 90
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Met
        35                  40                  45
Gly Glu Ile Leu Pro Gly Ser Gly Thr Thr Asn Tyr Asn Glu Lys Phe
    50                  55                  60
Lys Gly Arg Val Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser
        115                 120


<210> 91
<211> 360
<212> DNA
<213> Artificial Sequence

<220>
<223> humanized heavy chain variable domain

<400> 91
cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360


<210> 92
<211> 120
<212> PRT
<213> Artificial Sequence

<220>
<223> humanized heavy chain variable domain

<400> 92
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
```

```
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser
        115                 120
```

<210> 93
<211> 360
<212> DNA
<213> Artificial Sequence

<220>
<223> humanized heavy chain variable domain

<400> 93

```
cagatccagc tgaggcagag cggcgccgag gtgaaaaagc ccggcgccag cgtgaagctg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggcaa ggccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
```

<210> 94
<211> 120
<212> PRT
<213> Artificial Sequence

<220>
<223> humanized heavy chain variable domain

<400> 94

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser
        115                 120
```

<210> 95
<211> 360
<212> DNA
<213> Artificial Sequence

<220>
<223> humanized heavy chain variable domain

<400> 95

cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca agtccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360

<210> 96
<211> 120
<212> PRT
<213> Artificial Sequence

<220>
<223> humanized heavy chain variable domain

<400> 96
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser
115 120

<210> 97
<211> 360
<212> DNA
<213> Artificial Sequence

<220>
<223> humanized heavy chain variable domain

<400> 97
caggtgcagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360

<210> 98
<211> 120
<212> PRT
<213> Artificial Sequence

<220>
<223> humanized heavy chain variable domain

<400> 98
Gln Val Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30

Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser
115 120

<210> 99
<211> 336
<212> DNA
<213> Artificial Sequence

<220>
<223> humanized heavy chain variable domain

<400> 99
gacatcgtga tgacccagtc ccccgatagc ctggctgtgt cactggggga gagggccacc 60
atcaactgca gggccagcga gtccgtgagc atccacggca cccacctgat gcactggtac 120
cagcagaagc ccggccagcc ccctaagctg ctgatctacg ccgccagcaa cctcgaaagc 180
ggcgtccccg acaggttcag cggcagcggc agcggcaccg acttcaccct gactatcagc 240
agcctgcagg ccgaggacgt ggccgtctac tactgccagc agagcatcga ggacccctac 300
accttcggcc agggcaccaa gctggagatc aagcgt 336

<210> 100
<211> 112
<212> PRT
<213> Artificial Sequence

<220>
<223> humanized light chain variable domain

<400> 100
Asp Ile Val Met Thr Gln Ser Pro Asp Ser Leu Ala Val Ser Leu Gly
1 5 10 15
Glu Arg Ala Thr Ile Asn Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Asn Leu Glu Ser Gly Val Pro Asp
50 55 60
Arg Phe Ser Gly Ser Gly Ser Gly Thr Asp Phe Thr Leu Thr Ile Ser
65 70 75 80
Ser Leu Gln Ala Glu Asp Val Ala Val Tyr Tyr Cys Gln Gln Ser Ile
85 90 95
Glu Asp Pro Tyr Thr Phe Gly Gln Gly Thr Lys Leu Glu Ile Lys Arg
100 105 110

<210> 101
<211> 336
<212> DNA
<213> Artificial Sequence

<220>
<223> humanized light chain variable domain

<400> 101
gacatcgtgc tgacccagtc ccccgatagc ctggctgtgt cactggggga gagggccacc 60

atcaactgca gggccagcga gtccgtgagc atccacggca cccacctgat gcactggtac 120
cagcagaagc ccggccagcc ccctaagctg ctgatctacg ccgccagcaa cctcgaaagc 180
ggcgtccccg acaggttcag cggcagcggc agcgagaccg acttcaccct gactatcagc 240
agcctgcagg ccgaggacgt ggccgtctac tactgccagc agagcatcga ggacccctac 300
accttcggcc agggcaccaa gctggagatc aagcgt 336

<210> 102
<211> 112
<212> PRT
<213> Artificial Sequence

<220>
<223> humanized light chain variable domain

<400> 102
Asp Ile Val Leu Thr Gln Ser Pro Asp Ser Leu Ala Val Ser Leu Gly
1 5 10 15
Glu Arg Ala Thr Ile Asn Cys Arg Ala Ser Glu Ser Val Ser Ile His
20 25 30
Gly Thr His Leu Met His Trp Tyr Gln Gln Lys Pro Gly Gln Pro Pro
35 40 45
Lys Leu Leu Ile Tyr Ala Ala Ser Asn Leu Glu Ser Gly Val Pro Asp
50 55 60
Arg Phe Ser Gly Ser Gly Ser Glu Thr Asp Phe Thr Leu Thr Ile Ser
65 70 75 80
Ser Leu Gln Ala Glu Asp Val Ala Val Tyr Tyr Cys Gln Gln Ser Ile
85 90 95
Glu Asp Pro Tyr Thr Phe Gly Gln Gly Thr Lys Leu Glu Ile Lys Arg
100 105 110

<210> 103
<211> 372
<212> DNA
<213> Artificial Sequence

<220>
<223> domain antibody

<400> 103
gaggtgcagc tgttggagtc tgggggaggc ttggtacagc ctggggggtc cctgcgtctc 60
tcctgtgcag cctccggatt cacctttaag gattatgata tgtggtgggt ccgccaggct 120
ccagggaagg gtctagagtg ggtctcatct atttctgtgg agggtgttca gacatactac 180
gcagactccg tgaaaggccg gttcaccatc tcccgcgaca attccaagaa cacgctgtat 240
ctgcaaatga acagcctgcg tgccgaggac accgcggtat attactgtgc gaaaaatatt 300
cgttatgtgg ggaatcggtc gtggtggacg tttgactact ggggtcaggg aaccctggtc 360
accgtctcga gc 372

<210> 104
<211> 124
<212> PRT
<213> Artificial Sequence

<220>
<223> domain antibody

<400> 104
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Lys Asp Tyr
20 25 30
Asp Met Trp Trp Val Arg Gln Ala Pro Gly Lys Gly Leu Glu Trp Val
35 40 45
Ser Ser Ile Ser Val Glu Gly Val Gln Thr Tyr Tyr Ala Asp Ser Val
50 55 60

Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Lys Asn Ile Arg Tyr Val Gly Asn Arg Ser Trp Trp Thr Phe Asp
100 105 110
Tyr Trp Gly Gln Gly Thr Leu Val Thr Val Ser Ser
115 120

<210> 105
<211> 381
<212> DNA
<213> Artificial Sequence

<220>
<223> domain antibody

<400> 105
gaggtgcagc tgttggagtc tgggggaggc ttggtacagc ctggggggtc cctgcgtctc 60
tcctgtgcag cctccggatt cacctttaag gattatgata tgtggtgggt ccgccaggct 120
ccagggaagg gtctagagtg ggtctcatct atttctgtgg agggtgttca gacatactac 180
gcagactccg tgaaaggccg gttcaccatc tcccgcgaca attccaagaa cacgctgtat 240
ctgcaaatga acagcctgcg tgccgaggac accgcggtat attactgtgc gaaaaatatt 300
cgttatgtgg ggaatcggtc gtggtggacg tttgactact ggggtcaggg aaccctggtc 360
accgtctcga gcgcggccgc c 381

<210> 106
<211> 127
<212> PRT
<213> Artificial Sequence

<220>
<223> domain antibody

<400> 106
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Lys Asp Tyr
20 25 30
Asp Met Trp Trp Val Arg Gln Ala Pro Gly Lys Gly Leu Glu Trp Val
35 40 45
Ser Ser Ile Ser Val Glu Gly Val Gln Thr Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Lys Asn Ile Arg Tyr Val Gly Asn Arg Ser Trp Trp Thr Phe Asp
100 105 110
Tyr Trp Gly Gln Gly Thr Leu Val Thr Val Ser Ser Ala Ala Ala
115 120 125

<210> 107
<211> 324
<212> DNA
<213> Artificial Sequence

<220>
<223> domain antibody

<400> 107
gacatccaga tgacccagtc tccatcctcc ctgtctgcat ctgtaggaga ccgtgtcacc 60
atcacttgcc gggcaagtca gtggattggt cctgagttaa gttggtacca gcagaaacca 120

gggaaagccc ctaagctcct gatctatcat ggttccattt tgcaaagtgg ggtcccatca 180
cgtttcagtg gcagtggatc tgggacagac ttcactctca ccatcagcag tctgcaacct 240
gaagattttg ctacgtacta ctgtcaacag tatatgtatt atcctcatac gttcggccaa 300
gggaccaagg tggaaatcaa acgg 324

<210> 108
<211> 108
<212> PRT
<213> Artificial Sequence

<220>
<223> domain antibody

<400> 108
Asp Ile Gln Met Thr Gln Ser Pro Ser Ser Leu Ser Ala Ser Val Gly
1 5 10 15
Asp Arg Val Thr Ile Thr Cys Arg Ala Ser Gln Trp Ile Gly Pro Glu
20 25 30
Leu Ser Trp Tyr Gln Gln Lys Pro Gly Lys Ala Pro Lys Leu Leu Ile
35 40 45
Tyr His Gly Ser Ile Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Met Tyr Tyr Pro His
85 90 95
Thr Phe Gly Gln Gly Thr Lys Val Glu Ile Lys Arg
100 105

<210> 109
<211> 348
<212> DNA
<213> Artificial Sequence

<220>
<223> domain antibody

<400> 109
gaggtgcagc tcctggtcag cggcggcggc ctggtccagc ccggaggctc actgaggctg 60
agctgcgccg ctagcggctt caccttcaag gcctacccca tgatgtgggt caggcaggcc 120
cccggcaaag gcctggagtg ggtgtctgag atcagcccca gcggcagcta cacctactac 180
gccgacagcg tgaagggcag gttcaccatc agcagggaca acagcaagaa caccctgtac 240
ctgcagatga actctctgag ggccgaggac accgccgtgt actactgcgc caaggacccc 300
aggaagctgg actattgggg ccagggcact ctggtgaccg tgagcagc 348

<210> 110
<211> 116
<212> PRT
<213> Artificial Sequence

<220>
<223> domain antibody

<400> 110
Glu Val Gln Leu Leu Val Ser Gly Gly Gly Leu Val Gln Pro Gly Gly
1 5 10 15
Ser Leu Arg Leu Ser Cys Ala Ala Ser Gly Phe Thr Phe Lys Ala Tyr
20 25 30
Pro Met Met Trp Val Arg Gln Ala Pro Gly Lys Gly Leu Glu Trp Val
35 40 45
Ser Glu Ile Ser Pro Ser Gly Ser Tyr Thr Tyr Tyr Ala Asp Ser Val
50 55 60
Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95
Ala Lys Asp Pro Arg Lys Leu Asp Tyr Trp Gly Gln Gly Thr Leu Val
100 105 110
Thr Val Ser Ser
115

<210> 111
<211> 1722
<212> DNA
<213> Artificial Sequence

<220>
<223> humanized antibody-linker-domain antibody bispecific construct

<400> 111
cagatccagc tggtgcagag cggcgccgag gtgaaaaagc ccggcagcag cgtgaaggtg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggccg cgccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag accgtggccg ccccctcggg atccgaggtg 1380
cagctcctgg tcagcggcgg cggcctggtc cagcccggag gctcactgag gctgagctgc 1440
gccgctagcg gcttcacctt caaggcctac cccatgatgt gggtcaggca ggcccccggc 1500
aaaggcctgg agtgggtgtc tgagatcagc cccagcggca gctacaccta ctacgccgac 1560
agcgtgaagg gcaggttcac catcagcagg gacaacagca agaacaccct gtacctgcag 1620
atgaactctc tgagggccga ggacaccgcc gtgtactact gcgccaagga ccccaggaag 1680
ctggactatt ggggccaggg cactctggtg accgtgagca gc 1722

<210> 112
<211> 574
<212> PRT
<213> Artificial Sequence

<220>
<223> humanized antibody-linker-domain antibody bispecific construct

<400> 112
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe

```
                50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                    85                  90                  95
Ala Arg Gly Gly Tyr Tyr Tyr Gly Ser Ser Tyr Asp Ser Trp Gly Gln
                100                 105                 110
Gly Thr Leu Val Thr Val Ser Ser Ala Ser Thr Lys Gly Pro Ser Val
            115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Gly Ser Glu Val Gln Leu Leu Val
    450                 455                 460
Ser Gly Gly Gly Leu Val Gln Pro Gly Gly Ser Leu Arg Leu Ser Cys
465                 470                 475                 480
Ala Ala Ser Gly Phe Thr Phe Lys Ala Tyr Pro Met Met Trp Val Arg
                485                 490                 495
Gln Ala Pro Gly Lys Gly Leu Glu Trp Val Ser Glu Ile Ser Pro Ser
            500                 505                 510
Gly Ser Tyr Thr Tyr Tyr Ala Asp Ser Val Lys Gly Arg Phe Thr Ile
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asp Pro Arg Lys
545                 550                 555                 560
Leu Asp Tyr Trp Gly Gln Gly Thr Leu Val Thr Val Ser Ser
                565                 570
```

<210> 113
<211> 1722
<212> DNA
<213> Artificial Sequence

<220>
<223> humanized antibody-linker-domain antibody
bispecific construct

<400> 113
cagatccagc tgaggcagag cggcgccgag gtgaaaaagc ccggcgccag cgtgaagctg 60
agctgcaagg ccagcggcta caccttcacc ggctactgga tcgagtgggt gaggcaggct 120
cccggacagg gcctggagtg gatcggcgag atcctgcccg ggtctggcac caccaactac 180
aacgagaagt tcaagggcaa ggccaccttc actgccgaca cctccaccag caccgcctac 240
atggaactga gcagcctcag gagcgaagac accgccgtct actattgcgc caggggcggc 300
tactactacg gcagcagcta cgacagctgg ggccagggca cactagtgac cgtgtccagc 360
gccagcacca agggccccag cgtgttcccc ctggccccca gcagcaagag caccagcggc 420
ggcacagccg ccctgggctg cctggtgaag gactacttcc ccgaaccggt gaccgtgtcc 480
tggaacagcg gagccctgac cagcggcgtg cacaccttcc ccgccgtgct gcagagcagc 540
ggcctgtaca gcctgagcag cgtggtgacc gtgcccagca gcagcctggg cacccagacc 600
tacatctgta acgtgaacca caagcccagc aacaccaagg tggacaagaa ggtggagccc 660
aagagctgtg acaagaccca cacctgcccc ccctgccctg cccccgagct gctgggaggc 720
cccagcgtgt tcctgttccc ccccaagcct aaggacaccc tgatgatcag cagaaccccc 780
gaggtgacct gtgtggtggt ggatgtgagc cacgaggacc ctgaggtgaa gttcaactgg 840
tacgtggacg gcgtggaggt gcacaatgcc aagaccaagc ccagggagga gcagtacaac 900
agcacctacc gggtggtgtc cgtgctgacc gtgctgcacc aggattggct gaacggcaag 960
gagtacaagt gtaaggtgtc caacaaggcc ctgcctgccc ctatcgagaa aaccatcagc 1020
aaggccaagg gccagcccag agagccccag gtgtacaccc tgccccctag cagagatgag 1080
ctgaccaaga accaggtgtc cctgacctgc ctggtgaagg gcttctaccc cagcgacatc 1140
gccgtggagt gggagagcaa cggccagccc gagaacaact acaagaccac cccccctgtg 1200
ctggacagcg atggcagctt cttcctgtac agcaagctga ccgtggacaa gagcagatgg 1260
cagcagggca acgtgttcag ctgctccgtg atgcacgagg ccctgcacaa tcactacacc 1320
cagaagagcc tgagcctgtc ccctggcaag accgtggccg ccccctcggg atccgaggtg 1380
cagctcctgg tcagcggcgg cggcctggtc cagcccggag gctcactgag gctgagctgc 1440
gccgctagcg gcttcacctt caaggcctac cccatgatgt gggtcaggca ggcccccggc 1500
aaaggcctgg agtgggtgtc tgagatcagc cccagcggca gctacaccta ctacgccgac 1560
agcgtgaagg gcaggttcac catcagcagg gacaacagca agaacaccct gtacctgcag 1620
atgaactctc tgagggccga ggacaccgcc gtgtactact gcgccaagga ccccaggaag 1680
ctggactatt ggggccaggg cactctggtg accgtgagca gc 1722

<210> 114
<211> 574
<212> PRT
<213> Artificial Sequence

<220>
<223> humanized antibody-linker-domain antibody
bispecific construct

<400> 114
Gln Ile Gln Leu Arg Gln Ser Gly Ala Glu Val Lys Lys Pro Gly Ala
1 5 10 15
Ser Val Lys Leu Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Lys Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln

```
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Gly Ser Glu Val Gln Leu Leu Val
    450                 455                 460
Ser Gly Gly Gly Leu Val Gln Pro Gly Gly Ser Leu Arg Leu Ser Cys
465                 470                 475                 480
Ala Ala Ser Gly Phe Thr Phe Lys Ala Tyr Pro Met Met Trp Val Arg
                485                 490                 495
Gln Ala Pro Gly Lys Gly Leu Glu Trp Val Ser Glu Ile Ser Pro Ser
            500                 505                 510
Gly Ser Tyr Thr Tyr Tyr Ala Asp Ser Val Lys Gly Arg Phe Thr Ile
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asp Pro Arg Lys
545                 550                 555                 560
Leu Asp Tyr Trp Gly Gln Gly Thr Leu Val Thr Val Ser Ser
                565                 570
```

<210> 115
<211> 582
<212> PRT
<213> Artificial Sequence

<220>
<223> humanized antibody-linker-domain antibody
bispecific construct

<400> 115
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Gly Ser Glu Val Gln Leu Leu Glu
    450                 455                 460
Ser Gly Gly Gly Leu Val Gln Pro Gly Gly Ser Leu Arg Leu Ser Cys

```
465                 470                 475                 480
Ala Ala Ser Gly Phe Thr Phe Lys Asp Tyr Asp Met Trp Trp Val Arg
                485                 490                 495
Gln Ala Pro Gly Lys Gly Leu Glu Trp Val Ser Ser Ile Ser Val Glu
            500                 505                 510
Gly Val Gln Thr Tyr Tyr Ala Asp Ser Val Lys Gly Arg Phe Thr Ile
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asn Ile Arg Tyr
545                 550                 555                 560
Val Gly Asn Arg Ser Trp Trp Thr Phe Asp Tyr Trp Gly Gln Gly Thr
                565                 570                 575
Leu Val Thr Val Ser Ser
            580
```

<210> 116
<211> 582
<212> PRT
<213> Artificial Sequence

<220>
<223> humanized antibody-linker-domain antibody bispecific construct

<400> 116

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
```

Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Glu Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Lys Asp Tyr Asp Met Trp Trp Val Arg
485 490 495
Gln Ala Pro Gly Lys Gly Leu Glu Trp Val Ser Ser Ile Ser Val Glu
500 505 510
Gly Val Gln Thr Tyr Tyr Ala Asp Ser Val Lys Gly Arg Phe Thr Ile
515 520 525
Ser Arg Asp Asn Ser Lys Asn Thr Leu Tyr Leu Gln Met Asn Ser Leu
530 535 540
Arg Ala Glu Asp Thr Ala Val Tyr Tyr Cys Ala Lys Asn Ile Arg Tyr
545 550 555 560
Val Gly Asn Arg Ser Trp Trp Thr Phe Asp Tyr Trp Gly Gln Gly Thr
565 570 575
Leu Val Thr Val Ser Ser
580

<210> 117
<211> 582
<212> PRT
<213> Artificial Sequence

<220>
<223> humanized antibody-linker-domain antibody bispecific construct

<400> 117
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala

130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Glu Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Lys Asp Tyr Asp Met Trp Trp Val Arg
485 490 495
Gln Ala Pro Gly Lys Gly Leu Glu Trp Val Ser Ser Ile Ser Val Glu
500 505 510
Gly Val Gln Thr Tyr Tyr Ala Asp Ser Val Lys Gly Arg Phe Thr Ile
515 520 525
Ser Arg Asp Asn Ser Lys Asn Thr Leu Tyr Leu Gln Met Asn Ser Leu
530 535 540
Arg Ala Glu Asp Thr Ala Val Tyr Tyr Cys Ala Lys Asn Ile Arg Tyr
545 550 555 560
Val Gly Asn Arg Ser Trp Trp Thr Phe Asp Tyr Trp Gly Gln Gly Thr
565 570 575
Leu Val Thr Val Ser Ser
580

<210> 118
<211> 582
<212> PRT
<213> Artificial Sequence

<220>

<223> humanized antibody-linker-domain antibody bispecific construct

<400> 118

Gln Val Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Glu Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Lys Asp Tyr Asp Met Trp Trp Val Arg

```
                485                 490                 495
Gln Ala Pro Gly Lys Gly Leu Glu Trp Val Ser Ser Ile Ser Val Glu
            500                 505                 510
Gly Val Gln Thr Tyr Tyr Ala Asp Ser Val Lys Gly Arg Phe Thr Ile
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asn Ile Arg Tyr
545                 550                 555                 560
Val Gly Asn Arg Ser Trp Trp Thr Phe Asp Tyr Trp Gly Gln Gly Thr
                565                 570                 575
Leu Val Thr Val Ser Ser
            580
```

```
<210> 119
<211> 580
<212> PRT
<213> Artificial Sequence

<220>
<223> humanized antibody-linker-domain antibody
      bispecific construct

<400> 119
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
```

```
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Glu Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Lys Asp Tyr Asp Met Trp Trp Val Arg Gln Ala
                485                 490                 495
Pro Gly Lys Gly Leu Glu Trp Val Ser Ser Ile Ser Val Glu Gly Val
            500                 505                 510
Gln Thr Tyr Tyr Ala Asp Ser Val Lys Gly Arg Phe Thr Ile Ser Arg
        515                 520                 525
Asp Asn Ser Lys Asn Thr Leu Tyr Leu Gln Met Asn Ser Leu Arg Ala
    530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser
            580


<210> 120
<211> 580
<212> PRT
<213> Artificial Sequence

<220>
<223> humanized antibody-linker-domain antibody
      bispecific construct

<400> 120
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
```

```
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Glu Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Lys Asp Tyr Asp Met Trp Trp Val Arg Gln Ala
                485                 490                 495
Pro Gly Lys Gly Leu Glu Trp Val Ser Ser Ile Ser Val Glu Gly Val
            500                 505                 510
Gln Thr Tyr Tyr Ala Asp Ser Val Lys Gly Arg Phe Thr Ile Ser Arg
        515                 520                 525
Asp Asn Ser Lys Asn Thr Leu Tyr Leu Gln Met Asn Ser Leu Arg Ala
    530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser
            580
```

<210> 121
<211> 580
<212> PRT
<213> Artificial Sequence

<220>
<223> humanized antibody-linker-domain antibody bispecific construct

<400> 121

Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Glu Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Lys Asp Tyr Asp Met Trp Trp Val Arg Gln Ala
                485                 490                 495
Pro Gly Lys Gly Leu Glu Trp Val Ser Ser Ile Ser Val Glu Gly Val

```
                500                 505                 510
Gln Thr Tyr Tyr Ala Asp Ser Val Lys Gly Arg Phe Thr Ile Ser Arg
            515                 520                 525
Asp Asn Ser Lys Asn Thr Leu Tyr Leu Gln Met Asn Ser Leu Arg Ala
    530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser
            580
```

<210> 122
<211> 580
<212> PRT
<213> Artificial Sequence

<220>
<223> humanized antibody-linker-domain antibody bispecific construct

<400> 122

```
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
```

```
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Glu Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Lys Asp Tyr Asp Met Trp Trp Val Arg Gln Ala
                485                 490                 495
Pro Gly Lys Gly Leu Glu Trp Val Ser Ser Ile Ser Val Glu Gly Val
            500                 505                 510
Gln Thr Tyr Tyr Ala Asp Ser Val Lys Gly Arg Phe Thr Ile Ser Arg
        515                 520                 525
Asp Asn Ser Lys Asn Thr Leu Tyr Leu Gln Met Asn Ser Leu Arg Ala
    530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser
            580
```

<210> 123
<211> 585
<212> PRT
<213> Artificial Sequence

<220>
<223> humanized antibody-linker-domain antibody bispecific construct

<400> 123

```
Gln Ile Gln Leu Val Gln Ser Gly Ala Glu Val Lys Lys Pro Gly Ser
 1               5                  10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
```

```
                        165                 170                 175
Leu Gln Ser Ser Gly Leu Tyr Ser Leu Ser Ser Val Val Thr Val Pro
                    180                 185                 190
Ser Ser Ser Leu Gly Thr Gln Thr Tyr Ile Cys Asn Val Asn His Lys
                195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Gly Ser Glu Val Gln Leu Leu Glu
    450                 455                 460
Ser Gly Gly Gly Leu Val Gln Pro Gly Gly Ser Leu Arg Leu Ser Cys
465                 470                 475                 480
Ala Ala Ser Gly Phe Thr Phe Lys Asp Tyr Asp Met Trp Trp Val Arg
                485                 490                 495
Gln Ala Pro Gly Lys Gly Leu Glu Trp Val Ser Ser Ile Ser Val Glu
            500                 505                 510
Gly Val Gln Thr Tyr Tyr Ala Asp Ser Val Lys Gly Arg Phe Thr Ile
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asn Ile Arg Tyr
545                 550                 555                 560
Val Gly Asn Arg Ser Trp Trp Thr Phe Asp Tyr Trp Gly Gln Gly Thr
                565                 570                 575
Leu Val Thr Val Ser Ser Ala Ala Ala
            580                 585
```

<210> 124
<211> 585
<212> PRT
<213> Artificial Sequence

<220>
<223> humanized antibody-linker-domain antibody bispecific construct

<400> 124

Gln Ile Gln Leu Arg Gln Ser Gly Ala Glu Val Lys Lys Pro Gly Ala
1 5 10 15
Ser Val Lys Leu Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Lys Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Glu Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Lys Asp Tyr Asp Met Trp Trp Val Arg
485 490 495
Gln Ala Pro Gly Lys Gly Leu Glu Trp Val Ser Ser Ile Ser Val Glu
500 505 510
Gly Val Gln Thr Tyr Tyr Ala Asp Ser Val Lys Gly Arg Phe Thr Ile

```
        515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asn Ile Arg Tyr
545                 550                 555                 560
Val Gly Asn Arg Ser Trp Trp Thr Phe Asp Tyr Trp Gly Gln Gly Thr
                565                 570                 575
Leu Val Thr Val Ser Ser Ala Ala Ala
            580                 585


<210> 125
<211> 585
<212> PRT
<213> Artificial Sequence

<220>
<223> humanized antibody-linker-domain antibody
      bispecific construct

<400> 125
Gln Ile Gln Leu Val Gln Ser Gly Ala Glu Val Lys Lys Pro Gly Ser
 1               5                  10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
```

Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Glu Val Gln Leu Leu Glu
450 455 460
Ser Gly Gly Gly Leu Val Gln Pro Gly Gly Ser Leu Arg Leu Ser Cys
465 470 475 480
Ala Ala Ser Gly Phe Thr Phe Lys Asp Tyr Asp Met Trp Trp Val Arg
485 490 495
Gln Ala Pro Gly Lys Gly Leu Glu Trp Val Ser Ser Ile Ser Val Glu
500 505 510
Gly Val Gln Thr Tyr Tyr Ala Asp Ser Val Lys Gly Arg Phe Thr Ile
515 520 525
Ser Arg Asp Asn Ser Lys Asn Thr Leu Tyr Leu Gln Met Asn Ser Leu
530 535 540
Arg Ala Glu Asp Thr Ala Val Tyr Tyr Cys Ala Lys Asn Ile Arg Tyr
545 550 555 560
Val Gly Asn Arg Ser Trp Trp Thr Phe Asp Tyr Trp Gly Gln Gly Thr
565 570 575
Leu Val Thr Val Ser Ser Ala Ala Ala
580 585

<210> 126
<211> 585
<212> PRT
<213> Artificial Sequence

<220>
<223> humanized antibody-linker-domain antibody bispecific construct

<400> 126
Gln Val Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro

```
                180                 185                 190
Ser Ser Ser Leu Gly Thr Gln Thr Tyr Ile Cys Asn Val Asn His Lys
            195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
                260                 265                 270
Asp Pro Glu Val Lys Phe Asn Trp Tyr Val Asp Gly Val Glu Val His
            275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
                340                 345                 350
Thr Leu Pro Pro Ser Arg Asp Glu Leu Thr Lys Asn Gln Val Ser Leu
            355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
                420                 425                 430
Glu Ala Leu His Asn His Tyr Thr Gln Lys Ser Leu Ser Leu Ser Pro
            435                 440                 445
Gly Lys Thr Val Ala Ala Pro Ser Gly Ser Glu Val Gln Leu Leu Glu
    450                 455                 460
Ser Gly Gly Gly Leu Val Gln Pro Gly Gly Ser Leu Arg Leu Ser Cys
465                 470                 475                 480
Ala Ala Ser Gly Phe Thr Phe Lys Asp Tyr Asp Met Trp Trp Val Arg
                485                 490                 495
Gln Ala Pro Gly Lys Gly Leu Glu Trp Val Ser Ser Ile Ser Val Glu
                500                 505                 510
Gly Val Gln Thr Tyr Tyr Ala Asp Ser Val Lys Gly Arg Phe Thr Ile
            515                 520                 525
Ser Arg Asp Asn Ser Lys Asn Thr Leu Tyr Leu Gln Met Asn Ser Leu
    530                 535                 540
Arg Ala Glu Asp Thr Ala Val Tyr Tyr Cys Ala Lys Asn Ile Arg Tyr
545                 550                 555                 560
Val Gly Asn Arg Ser Trp Trp Thr Phe Asp Tyr Trp Gly Gln Gly Thr
                565                 570                 575
Leu Val Thr Val Ser Ser Ala Ala Ala
                580                 585
```

<210> 127
<211> 583
<212> PRT
<213> Artificial Sequence

<220>
<223> humanized antibody-linker-domain antibody bispecific construct

<400> 127

```
Gln Ile Gln Leu Val Gln Ser Gly Ala Glu Val Lys Lys Pro Gly Ser
1               5                   10                  15
```

Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Glu Val Gln Leu Leu Glu Ser Gly
450 455 460
Gly Gly Leu Val Gln Pro Gly Gly Ser Leu Arg Leu Ser Cys Ala Ala
465 470 475 480
Ser Gly Phe Thr Phe Lys Asp Tyr Asp Met Trp Trp Val Arg Gln Ala
485 490 495
Pro Gly Lys Gly Leu Glu Trp Val Ser Ser Ile Ser Val Glu Gly Val
500 505 510
Gln Thr Tyr Tyr Ala Asp Ser Val Lys Gly Arg Phe Thr Ile Ser Arg
515 520 525
Asp Asn Ser Lys Asn Thr Leu Tyr Leu Gln Met Asn Ser Leu Arg Ala

```
        530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser Ala Ala Ala
            580
```

<210> 128
<211> 583
<212> PRT
<213> Artificial Sequence

<220>
<223> humanized antibody-linker-domain antibody bispecific construct

<400> 128

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
```

Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Glu Val Gln Leu Leu Glu Ser Gly
450 455 460
Gly Gly Leu Val Gln Pro Gly Gly Ser Leu Arg Leu Ser Cys Ala Ala
465 470 475 480
Ser Gly Phe Thr Phe Lys Asp Tyr Asp Met Trp Trp Val Arg Gln Ala
485 490 495
Pro Gly Lys Gly Leu Glu Trp Val Ser Ser Ile Ser Val Glu Gly Val
500 505 510
Gln Thr Tyr Tyr Ala Asp Ser Val Lys Gly Arg Phe Thr Ile Ser Arg
515 520 525
Asp Asn Ser Lys Asn Thr Leu Tyr Leu Gln Met Asn Ser Leu Arg Ala
530 535 540
Glu Asp Thr Ala Val Tyr Tyr Cys Ala Lys Asn Ile Arg Tyr Val Gly
545 550 555 560
Asn Arg Ser Trp Trp Thr Phe Asp Tyr Trp Gly Gln Gly Thr Leu Val
565 570 575
Thr Val Ser Ser Ala Ala Ala
580

<210> 129
<211> 583
<212> PRT
<213> Artificial Sequence

<220>
<223> humanized antibody-linker-domain antibody bispecific construct

<400> 129
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys

```
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Glu Val Gln Leu Leu Glu Ser Gly
    450                 455                 460
Gly Gly Leu Val Gln Pro Gly Gly Ser Leu Arg Leu Ser Cys Ala Ala
465                 470                 475                 480
Ser Gly Phe Thr Phe Lys Asp Tyr Asp Met Trp Trp Val Arg Gln Ala
                485                 490                 495
Pro Gly Lys Gly Leu Glu Trp Val Ser Ser Ile Ser Val Glu Gly Val
            500                 505                 510
Gln Thr Tyr Tyr Ala Asp Ser Val Lys Gly Arg Phe Thr Ile Ser Arg
        515                 520                 525
Asp Asn Ser Lys Asn Thr Leu Tyr Leu Gln Met Asn Ser Leu Arg Ala
    530                 535                 540
Glu Asp Thr Ala Val Tyr Tyr Cys Ala Lys Asn Ile Arg Tyr Val Gly
545                 550                 555                 560
Asn Arg Ser Trp Trp Thr Phe Asp Tyr Trp Gly Gln Gly Thr Leu Val
                565                 570                 575
Thr Val Ser Ser Ala Ala Ala
            580
```

```
<210> 130
<211> 583
<212> PRT
<213> Artificial Sequence

<220>
<223> humanized antibody-linker-domain antibody
      bispecific construct

<400> 130
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
```

Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Glu Val Gln Leu Leu Glu Ser Gly
450 455 460
Gly Gly Leu Val Gln Pro Gly Gly Ser Leu Arg Leu Ser Cys Ala Ala
465 470 475 480
Ser Gly Phe Thr Phe Lys Asp Tyr Asp Met Trp Trp Val Arg Gln Ala
485 490 495
Pro Gly Lys Gly Leu Glu Trp Val Ser Ser Ile Ser Val Glu Gly Val
500 505 510
Gln Thr Tyr Tyr Ala Asp Ser Val Lys Gly Arg Phe Thr Ile Ser Arg
515 520 525
Asp Asn Ser Lys Asn Thr Leu Tyr Leu Gln Met Asn Ser Leu Arg Ala
530 535 540
Glu Asp Thr Ala Val Tyr Tyr Cys Ala Lys Asn Ile Arg Tyr Val Gly

545 550 555 560
Asn Arg Ser Trp Trp Thr Phe Asp Tyr Trp Gly Gln Gly Thr Leu Val
565 570 575
Thr Val Ser Ser Ala Ala Ala
580

<210> 131
<211> 566
<212> PRT
<213> Artificial Sequence

<220>
<223> humanized antibody-linker-domain antibody bispecific construct

<400> 131
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380

```
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Gly Ser Asp Ile Gln Met Thr Gln
    450                 455                 460
Ser Pro Ser Ser Leu Ser Ala Ser Val Gly Asp Arg Val Thr Ile Thr
465                 470                 475                 480
Cys Arg Ala Ser Gln Trp Ile Gly Pro Glu Leu Ser Trp Tyr Gln Gln
                485                 490                 495
Lys Pro Gly Lys Ala Pro Lys Leu Leu Ile Tyr His Gly Ser Ile Leu
            500                 505                 510
Gln Ser Gly Val Pro Ser Arg Phe Ser Gly Ser Gly Ser Gly Thr Asp
        515                 520                 525
Phe Thr Leu Thr Ile Ser Ser Leu Gln Pro Glu Asp Phe Ala Thr Tyr
    530                 535                 540
Tyr Cys Gln Gln Tyr Met Tyr Tyr Pro His Thr Phe Gly Gln Gly Thr
545                 550                 555                 560
Lys Val Glu Ile Lys Arg
                565


<210> 132
<211> 566
<212> PRT
<213> Artificial Sequence

<220>
<223> humanized antibody-linker-domain antibody
      bispecific construct

<400> 132
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
```

225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Asp Ile Gln Met Thr Gln
450 455 460
Ser Pro Ser Ser Leu Ser Ala Ser Val Gly Asp Arg Val Thr Ile Thr
465 470 475 480
Cys Arg Ala Ser Gln Trp Ile Gly Pro Glu Leu Ser Trp Tyr Gln Gln
485 490 495
Lys Pro Gly Lys Ala Pro Lys Leu Leu Ile Tyr His Gly Ser Ile Leu
500 505 510
Gln Ser Gly Val Pro Ser Arg Phe Ser Gly Ser Gly Ser Gly Thr Asp
515 520 525
Phe Thr Leu Thr Ile Ser Ser Leu Gln Pro Glu Asp Phe Ala Thr Tyr
530 535 540
Tyr Cys Gln Gln Tyr Met Tyr Tyr Pro His Thr Phe Gly Gln Gly Thr
545 550 555 560
Lys Val Glu Ile Lys Arg
565

<210> 133
<211> 566
<212> PRT
<213> Artificial Sequence

<220>
<223> humanized antibody-linker-domain antibody bispecific construct

<400> 133
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Gly Ser Asp Ile Gln Met Thr Gln
450 455 460
Ser Pro Ser Ser Leu Ser Ala Ser Val Gly Asp Arg Val Thr Ile Thr
465 470 475 480
Cys Arg Ala Ser Gln Trp Ile Gly Pro Glu Leu Ser Trp Tyr Gln Gln
485 490 495
Lys Pro Gly Lys Ala Pro Lys Leu Leu Ile Tyr His Gly Ser Ile Leu
500 505 510
Gln Ser Gly Val Pro Ser Arg Phe Ser Gly Ser Gly Ser Gly Thr Asp
515 520 525
Phe Thr Leu Thr Ile Ser Ser Leu Gln Pro Glu Asp Phe Ala Thr Tyr
530 535 540
Tyr Cys Gln Gln Tyr Met Tyr Tyr Pro His Thr Phe Gly Gln Gly Thr
545 550 555 560
Lys Val Glu Ile Lys Arg
565

<210> 134

<211> 566
<212> PRT
<213> Artificial Sequence

<220>
<223> humanized antibody-linker-domain antibody
bispecific construct

<400> 134
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445

Gly Lys Thr Val Ala Ala Pro Ser Gly Ser Asp Ile Gln Met Thr Gln
    450                 455                 460
Ser Pro Ser Ser Leu Ser Ala Ser Val Gly Asp Arg Val Thr Ile Thr
465                 470                 475                 480
Cys Arg Ala Ser Gln Trp Ile Gly Pro Glu Leu Ser Trp Tyr Gln Gln
                485                 490                 495
Lys Pro Gly Lys Ala Pro Lys Leu Leu Ile Tyr His Gly Ser Ile Leu
            500                 505                 510
Gln Ser Gly Val Pro Ser Arg Phe Ser Gly Ser Gly Ser Gly Thr Asp
        515                 520                 525
Phe Thr Leu Thr Ile Ser Ser Leu Gln Pro Glu Asp Phe Ala Thr Tyr
    530                 535                 540
Tyr Cys Gln Gln Tyr Met Tyr Tyr Pro His Thr Phe Gly Gln Gly Thr
545                 550                 555                 560
Lys Val Glu Ile Lys Arg
                565

<210> 135
<211> 564
<212> PRT
<213> Artificial Sequence

<220>
<223> humanized antibody-linker-domain antibody
      bispecific construct

<400> 135
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg

```
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Asp Ile Gln Met Thr Gln Ser Pro
    450                 455                 460
Ser Ser Leu Ser Ala Ser Val Gly Asp Arg Val Thr Ile Thr Cys Arg
465                 470                 475                 480
Ala Ser Gln Trp Ile Gly Pro Glu Leu Ser Trp Tyr Gln Gln Lys Pro
                485                 490                 495
Gly Lys Ala Pro Lys Leu Leu Ile Tyr His Gly Ser Ile Leu Gln Ser
            500                 505                 510
Gly Val Pro Ser Arg Phe Ser Gly Ser Gly Ser Gly Thr Asp Phe Thr
        515                 520                 525
Leu Thr Ile Ser Ser Leu Gln Pro Glu Asp Phe Ala Thr Tyr Tyr Cys
    530                 535                 540
Gln Gln Tyr Met Tyr Tyr Pro His Thr Phe Gly Gln Gly Thr Lys Val
545                 550                 555                 560
Glu Ile Lys Arg
```

```
<210> 136
<211> 564
<212> PRT
<213> Artificial Sequence

<220>
<223> humanized antibody-linker-domain antibody
      bispecific construct

<400> 136
Gln Ile Gln Leu Arg Gln Ser Gly Ala Glu Val Lys Lys Pro Gly Ala
 1               5                  10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
```

Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Asp Ile Gln Met Thr Gln Ser Pro
450 455 460
Ser Ser Leu Ser Ala Ser Val Gly Asp Arg Val Thr Ile Thr Cys Arg
465 470 475 480
Ala Ser Gln Trp Ile Gly Pro Glu Leu Ser Trp Tyr Gln Gln Lys Pro
485 490 495
Gly Lys Ala Pro Lys Leu Leu Ile Tyr His Gly Ser Ile Leu Gln Ser
500 505 510
Gly Val Pro Ser Arg Phe Ser Gly Ser Gly Ser Gly Thr Asp Phe Thr
515 520 525
Leu Thr Ile Ser Ser Leu Gln Pro Glu Asp Phe Ala Thr Tyr Tyr Cys
530 535 540
Gln Gln Tyr Met Tyr Tyr Pro His Thr Phe Gly Gln Gly Thr Lys Val
545 550 555 560
Glu Ile Lys Arg

<210> 137
<211> 564
<212> PRT
<213> Artificial Sequence

<220>
<223> humanized antibody-linker-domain antibody
bispecific construct

<400> 137
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Asp Ile Gln Met Thr Gln Ser Pro
450 455 460
Ser Ser Leu Ser Ala Ser Val Gly Asp Arg Val Thr Ile Thr Cys Arg
465 470 475 480
Ala Ser Gln Trp Ile Gly Pro Glu Leu Ser Trp Tyr Gln Gln Lys Pro
485 490 495
Gly Lys Ala Pro Lys Leu Leu Ile Tyr His Gly Ser Ile Leu Gln Ser
500 505 510

Gly Val Pro Ser Arg Phe Ser Gly Ser Gly Ser Gly Thr Asp Phe Thr
515 520 525
Leu Thr Ile Ser Ser Leu Gln Pro Glu Asp Phe Ala Thr Tyr Tyr Cys
530 535 540
Gln Gln Tyr Met Tyr Tyr Pro His Thr Phe Gly Gln Gly Thr Lys Val
545 550 555 560
Glu Ile Lys Arg

<210> 138
<211> 564
<212> PRT
<213> Artificial Sequence

<220>
<223> humanized antibody-linker-domain antibody bispecific construct

<400> 138
Gln Val Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu

```
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Asp Ile Gln Met Thr Gln Ser Pro
    450                 455                 460
Ser Ser Leu Ser Ala Ser Val Gly Asp Arg Val Thr Ile Thr Cys Arg
465                 470                 475                 480
Ala Ser Gln Trp Ile Gly Pro Glu Leu Ser Trp Tyr Gln Gln Lys Pro
                485                 490                 495
Gly Lys Ala Pro Lys Leu Leu Ile Tyr His Gly Ser Ile Leu Gln Ser
            500                 505                 510
Gly Val Pro Ser Arg Phe Ser Gly Ser Gly Ser Gly Thr Asp Phe Thr
        515                 520                 525
Leu Thr Ile Ser Ser Leu Gln Pro Glu Asp Phe Ala Thr Tyr Tyr Cys
    530                 535                 540
Gln Gln Tyr Met Tyr Tyr Pro His Thr Phe Gly Gln Gly Thr Lys Val
545                 550                 555                 560
Glu Ile Lys Arg
```

<210> 139
<211> 584
<212> PRT
<213> Artificial Sequence

<220>
<223> humanized antibody-linker-domain antibody bispecific construct

<400> 139

```
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
```

Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Gly Ser Thr Val Ala Ala Pro Ser Gly Ser Thr Val Ala Ala
450 455 460
Pro Ser Gly Ser Thr Val Ala Ala Pro Ser Gly Ser Asp Ile Gln Met
465 470 475 480
Thr Gln Ser Pro Ser Ser Leu Ser Ala Ser Val Gly Asp Arg Val Thr
485 490 495
Ile Thr Cys Arg Ala Ser Gln Trp Ile Gly Pro Glu Leu Ser Trp Tyr
500 505 510
Gln Gln Lys Pro Gly Lys Ala Pro Lys Leu Leu Ile Tyr His Gly Ser
515 520 525
Ile Leu Gln Ser Gly Val Pro Ser Arg Phe Ser Gly Ser Gly Ser Gly
530 535 540
Thr Asp Phe Thr Leu Thr Ile Ser Ser Leu Gln Pro Glu Asp Phe Ala
545 550 555 560
Thr Tyr Tyr Cys Gln Gln Tyr Met Tyr Tyr Pro His Thr Phe Gly Gln
565 570 575
Gly Thr Lys Val Glu Ile Lys Arg
580

<210> 140
<211> 584
<212> PRT
<213> Artificial Sequence

<220>
<223> humanized antibody-linker-domain antibody bispecific construct

<400> 140
Gln Ile Gln Leu Arg Gln Ser Gly Ala Glu Val Lys Lys Pro Gly Ala
1 5 10 15
Ser Val Lys Leu Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile

```
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Gly Ser Thr Val Ala Ala Pro Ser Gly Ser Thr Val Ala Ala
    450                 455                 460
Pro Ser Gly Ser Thr Val Ala Ala Pro Ser Gly Ser Asp Ile Gln Met
465                 470                 475                 480
Thr Gln Ser Pro Ser Ser Leu Ser Ala Ser Val Gly Asp Arg Val Thr
                485                 490                 495
Ile Thr Cys Arg Ala Ser Gln Trp Ile Gly Pro Glu Leu Ser Trp Tyr
            500                 505                 510
Gln Gln Lys Pro Gly Lys Ala Pro Lys Leu Leu Ile Tyr His Gly Ser
        515                 520                 525
Ile Leu Gln Ser Gly Val Pro Ser Arg Phe Ser Gly Ser Gly Ser Gly
    530                 535                 540
Thr Asp Phe Thr Leu Thr Ile Ser Ser Leu Gln Pro Glu Asp Phe Ala
545                 550                 555                 560
```

Thr Tyr Tyr Cys Gln Gln Tyr Met Tyr Tyr Pro His Thr Phe Gly Gln
565 570 575
Gly Thr Lys Val Glu Ile Lys Arg
580

<210> 141
<211> 584
<212> PRT
<213> Artificial Sequence

<220>
<223> humanized antibody-linker-domain antibody bispecific construct

<400> 141
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val

```
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Gly Ser Thr Val Ala Ala Pro Ser Gly Ser Thr Val Ala Ala
    450                 455                 460
Pro Ser Gly Ser Thr Val Ala Ala Pro Ser Gly Ser Asp Ile Gln Met
465                 470                 475                 480
Thr Gln Ser Pro Ser Ser Leu Ser Ala Ser Val Gly Asp Arg Val Thr
                485                 490                 495
Ile Thr Cys Arg Ala Ser Gln Trp Ile Gly Pro Glu Leu Ser Trp Tyr
            500                 505                 510
Gln Gln Lys Pro Gly Lys Ala Pro Lys Leu Leu Ile Tyr His Gly Ser
        515                 520                 525
Ile Leu Gln Ser Gly Val Pro Ser Arg Phe Ser Gly Ser Gly Ser Gly
    530                 535                 540
Thr Asp Phe Thr Leu Thr Ile Ser Ser Leu Gln Pro Glu Asp Phe Ala
545                 550                 555                 560
Thr Tyr Tyr Cys Gln Gln Tyr Met Tyr Tyr Pro His Thr Phe Gly Gln
                565                 570                 575
Gly Thr Lys Val Glu Ile Lys Arg
            580
```

<210> 142
<211> 584
<212> PRT
<213> Artificial Sequence

<220>
<223> humanized antibody-linker-domain antibody bispecific construct

<400> 142

```
Gln Val Gln Leu Val Gln Ser Gly Ala Glu Val Lys Lys Pro Gly Ser
 1               5                  10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
```

Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Gly Ser Thr Val Ala Ala Pro Ser Gly Ser Thr Val Ala Ala
450 455 460
Pro Ser Gly Ser Thr Val Ala Ala Pro Ser Gly Ser Asp Ile Gln Met
465 470 475 480
Thr Gln Ser Pro Ser Ser Leu Ser Ala Ser Val Gly Asp Arg Val Thr
485 490 495
Ile Thr Cys Arg Ala Ser Gln Trp Ile Gly Pro Glu Leu Ser Trp Tyr
500 505 510
Gln Gln Lys Pro Gly Lys Ala Pro Lys Leu Leu Ile Tyr His Gly Ser
515 520 525
Ile Leu Gln Ser Gly Val Pro Ser Arg Phe Ser Gly Ser Gly Ser Gly
530 535 540
Thr Asp Phe Thr Leu Thr Ile Ser Ser Leu Gln Pro Glu Asp Phe Ala
545 550 555 560
Thr Tyr Tyr Cys Gln Gln Tyr Met Tyr Tyr Pro His Thr Phe Gly Gln
565 570 575
Gly Thr Lys Val Glu Ile Lys Arg
580

<210> 143
<211> 576
<212> PRT
<213> Artificial Sequence

<220>
<223> humanized antibody-linker-domain antibody bispecific construct

<400> 143
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe

```
        50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Thr Val Ala Ala Pro Ser Thr Val
    450                 455                 460
Ala Ala Pro Ser Asp Ile Gln Met Thr Gln Ser Pro Ser Ser Leu Ser
465                 470                 475                 480
Ala Ser Val Gly Asp Arg Val Thr Ile Thr Cys Arg Ala Ser Gln Trp
                485                 490                 495
Ile Gly Pro Glu Leu Ser Trp Tyr Gln Gln Lys Pro Gly Lys Ala Pro
            500                 505                 510
Lys Leu Leu Ile Tyr His Gly Ser Ile Leu Gln Ser Gly Val Pro Ser
        515                 520                 525
Arg Phe Ser Gly Ser Gly Ser Gly Thr Asp Phe Thr Leu Thr Ile Ser
    530                 535                 540
Ser Leu Gln Pro Glu Asp Phe Ala Thr Tyr Tyr Cys Gln Gln Tyr Met
545                 550                 555                 560
Tyr Tyr Pro His Thr Phe Gly Gln Gly Thr Lys Val Glu Ile Lys Arg
                565                 570                 575
```

<210> 144
<211> 576
<212> PRT
<213> Artificial Sequence

<220>
<223> humanized antibody-linker-domain antibody bispecific construct

<400> 144

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
```

```
                420                 425                 430
Glu Ala Leu His Asn His Tyr Thr Gln Lys Ser Leu Ser Leu Ser Pro
            435                 440                 445
Gly Lys Thr Val Ala Ala Pro Ser Thr Val Ala Ala Pro Ser Thr Val
    450                 455                 460
Ala Ala Pro Ser Asp Ile Gln Met Thr Gln Ser Pro Ser Ser Leu Ser
465                 470                 475                 480
Ala Ser Val Gly Asp Arg Val Thr Ile Thr Cys Arg Ala Ser Gln Trp
                485                 490                 495
Ile Gly Pro Glu Leu Ser Trp Tyr Gln Gln Lys Pro Gly Lys Ala Pro
            500                 505                 510
Lys Leu Leu Ile Tyr His Gly Ser Ile Leu Gln Ser Gly Val Pro Ser
        515                 520                 525
Arg Phe Ser Gly Ser Gly Ser Gly Thr Asp Phe Thr Leu Thr Ile Ser
    530                 535                 540
Ser Leu Gln Pro Glu Asp Phe Ala Thr Tyr Tyr Cys Gln Gln Tyr Met
545                 550                 555                 560
Tyr Tyr Pro His Thr Phe Gly Gln Gly Thr Lys Val Glu Ile Lys Arg
                565                 570                 575


<210> 145
<211> 576
<212> PRT
<213> Artificial Sequence

<220>
<223> humanized antibody-linker-domain antibody
      bispecific construct

<400> 145
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
```

Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Thr Val Ala Ala Pro Ser Thr Val Ala Ala Pro Ser Thr Val
    450                 455                 460
Ala Ala Pro Ser Asp Ile Gln Met Thr Gln Ser Pro Ser Ser Leu Ser
465                 470                 475                 480
Ala Ser Val Gly Asp Arg Val Thr Ile Thr Cys Arg Ala Ser Gln Trp
                485                 490                 495
Ile Gly Pro Glu Leu Ser Trp Tyr Gln Gln Lys Pro Gly Lys Ala Pro
            500                 505                 510
Lys Leu Leu Ile Tyr His Gly Ser Ile Leu Gln Ser Gly Val Pro Ser
        515                 520                 525
Arg Phe Ser Gly Ser Gly Ser Gly Thr Asp Phe Thr Leu Thr Ile Ser
    530                 535                 540
Ser Leu Gln Pro Glu Asp Phe Ala Thr Tyr Tyr Cys Gln Gln Tyr Met
545                 550                 555                 560
Tyr Tyr Pro His Thr Phe Gly Gln Gly Thr Lys Val Glu Ile Lys Arg
                565                 570                 575

<210> 146
<211> 576
<212> PRT
<213> Artificial Sequence

<220>
<223> humanized antibody-linker-domain antibody bispecific construct

<400> 146
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val

115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Thr Val Ala Ala Pro Ser Thr Val Ala Ala Pro Ser Thr Val
450 455 460
Ala Ala Pro Ser Asp Ile Gln Met Thr Gln Ser Pro Ser Ser Leu Ser
465 470 475 480
Ala Ser Val Gly Asp Arg Val Thr Ile Thr Cys Arg Ala Ser Gln Trp
485 490 495
Ile Gly Pro Glu Leu Ser Trp Tyr Gln Gln Lys Pro Gly Lys Ala Pro
500 505 510
Lys Leu Leu Ile Tyr His Gly Ser Ile Leu Gln Ser Gly Val Pro Ser
515 520 525
Arg Phe Ser Gly Ser Gly Ser Gly Thr Asp Phe Thr Leu Thr Ile Ser
530 535 540
Ser Leu Gln Pro Glu Asp Phe Ala Thr Tyr Tyr Cys Gln Gln Tyr Met
545 550 555 560
Tyr Tyr Pro His Thr Phe Gly Gln Gly Thr Lys Val Glu Ile Lys Arg
565 570 575

<210> 147
<211> 574
<212> PRT
<213> Artificial Sequence

<220>

<223> humanized antibody-linker-domain antibody bispecific construct

<400> 147

```
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Asp Glu Thr Tyr Val Pro Lys Glu Phe Asn Ala Glu Thr Phe
    450                 455                 460
Gly Ser Asp Ile Gln Met Thr Gln Ser Pro Ser Ser Leu Ser Ala Ser
465                 470                 475                 480
Val Gly Asp Arg Val Thr Ile Thr Cys Arg Ala Ser Gln Trp Ile Gly
```

```
                    485                     490                     495
Pro Glu Leu Ser Trp Tyr Gln Gln Lys Pro Gly Lys Ala Pro Lys Leu
                500                     505                     510
Leu Ile Tyr His Gly Ser Ile Leu Gln Ser Gly Val Pro Ser Arg Phe
            515                     520                     525
Ser Gly Ser Gly Ser Gly Thr Asp Phe Thr Leu Thr Ile Ser Ser Leu
    530                     535                     540
Gln Pro Glu Asp Phe Ala Thr Tyr Tyr Cys Gln Gln Tyr Met Tyr Tyr
545                     550                     555                     560
Pro His Thr Phe Gly Gln Gly Thr Lys Val Glu Ile Lys Arg
                565                     570
```

<210> 148
<211> 574
<212> PRT
<213> Artificial Sequence

<220>
<223> humanized antibody-linker-domain antibody bispecific construct

<400> 148

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
```

Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Asp Glu Thr Tyr Val Pro Lys Glu Phe Asn Ala Glu Thr Phe
450 455 460
Gly Ser Asp Ile Gln Met Thr Gln Ser Pro Ser Ser Leu Ser Ala Ser
465 470 475 480
Val Gly Asp Arg Val Thr Ile Thr Cys Arg Ala Ser Gln Trp Ile Gly
485 490 495
Pro Glu Leu Ser Trp Tyr Gln Gln Lys Pro Gly Lys Ala Pro Lys Leu
500 505 510
Leu Ile Tyr His Gly Ser Ile Leu Gln Ser Gly Val Pro Ser Arg Phe
515 520 525
Ser Gly Ser Gly Ser Gly Thr Asp Phe Thr Leu Thr Ile Ser Ser Leu
530 535 540
Gln Pro Glu Asp Phe Ala Thr Tyr Tyr Cys Gln Gln Tyr Met Tyr Tyr
545 550 555 560
Pro His Thr Phe Gly Gln Gly Thr Lys Val Glu Ile Lys Arg
565 570

<210> 149
<211> 574
<212> PRT
<213> Artificial Sequence

<220>
<223> humanized antibody-linker-domain antibody bispecific construct

<400> 149
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro

```
                180                 185                 190
Ser Ser Ser Leu Gly Thr Gln Thr Tyr Ile Cys Asn Val Asn His Lys
            195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Asp Glu Thr Tyr Val Pro Lys Glu Phe Asn Ala Glu Thr Phe
    450                 455                 460
Gly Ser Asp Ile Gln Met Thr Gln Ser Pro Ser Ser Leu Ser Ala Ser
465                 470                 475                 480
Val Gly Asp Arg Val Thr Ile Thr Cys Arg Ala Ser Gln Trp Ile Gly
                485                 490                 495
Pro Glu Leu Ser Trp Tyr Gln Gln Lys Pro Gly Lys Ala Pro Lys Leu
            500                 505                 510
Leu Ile Tyr His Gly Ser Ile Leu Gln Ser Gly Val Pro Ser Arg Phe
        515                 520                 525
Ser Gly Ser Gly Ser Gly Thr Asp Phe Thr Leu Thr Ile Ser Ser Leu
    530                 535                 540
Gln Pro Glu Asp Phe Ala Thr Tyr Tyr Cys Gln Gln Tyr Met Tyr Tyr
545                 550                 555                 560
Pro His Thr Phe Gly Gln Gly Thr Lys Val Glu Ile Lys Arg
                565                 570


<210> 150
<211> 574
<212> PRT
<213> Artificial Sequence

<220>
<223> humanized antibody-linker-domain antibody
      bispecific construct

<400> 150
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
```

```
Trp Ile Glu Trp Val Arg Gln Ala Pro Gly Gln Gly Leu Glu Trp Ile
            35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
            115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
            195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
            275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
            355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
            435                 440                 445
Gly Lys Asp Glu Thr Tyr Val Pro Lys Glu Phe Asn Ala Glu Thr Phe
    450                 455                 460
Gly Ser Asp Ile Gln Met Thr Gln Ser Pro Ser Ser Leu Ser Ala Ser
465                 470                 475                 480
Val Gly Asp Arg Val Thr Ile Thr Cys Arg Ala Ser Gln Trp Ile Gly
                485                 490                 495
Pro Glu Leu Ser Trp Tyr Gln Gln Lys Pro Gly Lys Ala Pro Lys Leu
            500                 505                 510
Leu Ile Tyr His Gly Ser Ile Leu Gln Ser Gly Val Pro Ser Arg Phe
            515                 520                 525
Ser Gly Ser Gly Ser Gly Thr Asp Phe Thr Leu Thr Ile Ser Ser Leu
    530                 535                 540
Gln Pro Glu Asp Phe Ala Thr Tyr Tyr Cys Gln Gln Tyr Met Tyr Tyr
```

545 550 555 560
Pro His Thr Phe Gly Gln Gly Thr Lys Val Glu Ile Lys Arg
565 570

<210> 151
<211> 572
<212> PRT
<213> Artificial Sequence

<220>
<223> humanized antibody-linker-domain antibody bispecific construct

<400> 151
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400

```
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Asp Glu Thr Tyr Val Pro Lys Glu Phe Asn Ala Glu Thr Phe
    450                 455                 460
Asp Ile Gln Met Thr Gln Ser Pro Ser Ser Leu Ser Ala Ser Val Gly
465                 470                 475                 480
Asp Arg Val Thr Ile Thr Cys Arg Ala Ser Gln Trp Ile Gly Pro Glu
                485                 490                 495
Leu Ser Trp Tyr Gln Gln Lys Pro Gly Lys Ala Pro Lys Leu Leu Ile
            500                 505                 510
Tyr His Gly Ser Ile Leu Gln Ser Gly Val Pro Ser Arg Phe Ser Gly
        515                 520                 525
Ser Gly Ser Gly Thr Asp Phe Thr Leu Thr Ile Ser Ser Leu Gln Pro
    530                 535                 540
Glu Asp Phe Ala Thr Tyr Tyr Cys Gln Gln Tyr Met Tyr Tyr Pro His
545                 550                 555                 560
Thr Phe Gly Gln Gly Thr Lys Val Glu Ile Lys Arg
                565                 570


<210> 152
<211> 572
<212> PRT
<213> Artificial Sequence

<220>
<223> humanized antibody-linker-domain antibody
      bispecific construct

<400> 152
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
```

```
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Asp Glu Thr Tyr Val Pro Lys Glu Phe Asn Ala Glu Thr Phe
    450                 455                 460
Asp Ile Gln Met Thr Gln Ser Pro Ser Ser Leu Ser Ala Ser Val Gly
465                 470                 475                 480
Asp Arg Val Thr Ile Thr Cys Arg Ala Ser Gln Trp Ile Gly Pro Glu
                485                 490                 495
Leu Ser Trp Tyr Gln Gln Lys Pro Gly Lys Ala Pro Lys Leu Leu Ile
            500                 505                 510
Tyr His Gly Ser Ile Leu Gln Ser Gly Val Pro Ser Arg Phe Ser Gly
        515                 520                 525
Ser Gly Ser Gly Thr Asp Phe Thr Leu Thr Ile Ser Ser Leu Gln Pro
    530                 535                 540
Glu Asp Phe Ala Thr Tyr Tyr Cys Gln Gln Tyr Met Tyr Tyr Pro His
545                 550                 555                 560
Thr Phe Gly Gln Gly Thr Lys Val Glu Ile Lys Arg
                565                 570
```

<210> 153
<211> 572
<212> PRT
<213> Artificial Sequence

<220>
<223> humanized antibody-linker-domain antibody bispecific construct

<400> 153

```
Gln Ile Gln Leu Val Gln Ser Gly Ala Glu Val Lys Lys Pro Gly Ser
 1               5                  10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
```

Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Asp Glu Thr Tyr Val Pro Lys Glu Phe Asn Ala Glu Thr Phe
450 455 460
Asp Ile Gln Met Thr Gln Ser Pro Ser Ser Leu Ser Ala Ser Val Gly
465 470 475 480
Asp Arg Val Thr Ile Thr Cys Arg Ala Ser Gln Trp Ile Gly Pro Glu
485 490 495
Leu Ser Trp Tyr Gln Gln Lys Pro Gly Lys Ala Pro Lys Leu Leu Ile
500 505 510
Tyr His Gly Ser Ile Leu Gln Ser Gly Val Pro Ser Arg Phe Ser Gly
515 520 525
Ser Gly Ser Gly Thr Asp Phe Thr Leu Thr Ile Ser Ser Leu Gln Pro
530 535 540
Glu Asp Phe Ala Thr Tyr Tyr Cys Gln Gln Tyr Met Tyr Tyr Pro His
545 550 555 560
Thr Phe Gly Gln Gly Thr Lys Val Glu Ile Lys Arg
565 570

<210> 154
<211> 572
<212> PRT

<213> Artificial Sequence

<220>
<223> humanized antibody-linker-domain antibody bispecific construct

<400> 154

```
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Asp Glu Thr Tyr Val Pro Lys Glu Phe Asn Ala Glu Thr Phe
    450                 455                 460
```

Asp Ile Gln Met Thr Gln Ser Pro Ser Ser Leu Ser Ala Ser Val Gly
465 470 475 480
Asp Arg Val Thr Ile Thr Cys Arg Ala Ser Gln Trp Ile Gly Pro Glu
485 490 495
Leu Ser Trp Tyr Gln Gln Lys Pro Gly Lys Ala Pro Lys Leu Leu Ile
500 505 510
Tyr His Gly Ser Ile Leu Gln Ser Gly Val Pro Ser Arg Phe Ser Gly
515 520 525
Ser Gly Ser Gly Thr Asp Phe Thr Leu Thr Ile Ser Ser Leu Gln Pro
530 535 540
Glu Asp Phe Ala Thr Tyr Tyr Cys Gln Gln Tyr Met Tyr Tyr Pro His
545 550 555 560
Thr Phe Gly Gln Gly Thr Lys Val Glu Ile Lys Arg
565 570

<210> 155
<211> 581
<212> PRT
<213> Artificial Sequence

<220>
<223> humanized antibody-linker-domain antibody bispecific construct

<400> 155
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys

305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Glu Val Asp Glu Thr Tyr Val Pro Lys Glu Phe Asn Ala Glu
450 455 460
Thr Phe Thr Phe His Ala Asp Gly Ser Asp Ile Gln Met Thr Gln Ser
465 470 475 480
Pro Ser Ser Leu Ser Ala Ser Val Gly Asp Arg Val Thr Ile Thr Cys
485 490 495
Arg Ala Ser Gln Trp Ile Gly Pro Glu Leu Ser Trp Tyr Gln Gln Lys
500 505 510
Pro Gly Lys Ala Pro Lys Leu Leu Ile Tyr His Gly Ser Ile Leu Gln
515 520 525
Ser Gly Val Pro Ser Arg Phe Ser Gly Ser Gly Ser Gly Thr Asp Phe
530 535 540
Thr Leu Thr Ile Ser Ser Leu Gln Pro Glu Asp Phe Ala Thr Tyr Tyr
545 550 555 560
Cys Gln Gln Tyr Met Tyr Tyr Pro His Thr Phe Gly Gln Gly Thr Lys
565 570 575
Val Glu Ile Lys Arg
580

<210> 156
<211> 581
<212> PRT
<213> Artificial Sequence

<220>
<223> humanized antibody-linker-domain antibody bispecific construct

<400> 156
Gln Ile Gln Leu Arg Gln Ser Gly Ala Glu Val Lys Lys Pro Gly Ala
1 5 10 15
Ser Val Lys Leu Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Lys Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Glu Val Asp Glu Thr Tyr Val Pro Lys Glu Phe Asn Ala Glu
450 455 460
Thr Phe Thr Phe His Ala Asp Gly Ser Asp Ile Gln Met Thr Gln Ser
465 470 475 480
Pro Ser Ser Leu Ser Ala Ser Val Gly Asp Arg Val Thr Ile Thr Cys
485 490 495
Arg Ala Ser Gln Trp Ile Gly Pro Glu Leu Ser Trp Tyr Gln Gln Lys
500 505 510
Pro Gly Lys Ala Pro Lys Leu Leu Ile Tyr His Gly Ser Ile Leu Gln
515 520 525
Ser Gly Val Pro Ser Arg Phe Ser Gly Ser Gly Ser Gly Thr Asp Phe
530 535 540
Thr Leu Thr Ile Ser Ser Leu Gln Pro Glu Asp Phe Ala Thr Tyr Tyr
545 550 555 560
Cys Gln Gln Tyr Met Tyr Tyr Pro His Thr Phe Gly Gln Gly Thr Lys
565 570 575
Val Glu Ile Lys Arg
580

<210> 157
<211> 581
<212> PRT
<213> Artificial Sequence

<220>
<223> humanized antibody-linker-domain antibody

bispecific construct

<400> 157

Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Glu Val Asp Glu Thr Tyr Val Pro Lys Glu Phe Asn Ala Glu
    450                 455                 460
Thr Phe Thr Phe His Ala Asp Gly Ser Asp Ile Gln Met Thr Gln Ser
465                 470                 475                 480
Pro Ser Ser Leu Ser Ala Ser Val Gly Asp Arg Val Thr Ile Thr Cys
                485                 490                 495

Arg Ala Ser Gln Trp Ile Gly Pro Glu Leu Ser Trp Tyr Gln Gln Lys
500 505 510
Pro Gly Lys Ala Pro Lys Leu Leu Ile Tyr His Gly Ser Ile Leu Gln
515 520 525
Ser Gly Val Pro Ser Arg Phe Ser Gly Ser Gly Ser Gly Thr Asp Phe
530 535 540
Thr Leu Thr Ile Ser Ser Leu Gln Pro Glu Asp Phe Ala Thr Tyr Tyr
545 550 555 560
Cys Gln Gln Tyr Met Tyr Tyr Pro His Thr Phe Gly Gln Gly Thr Lys
565 570 575
Val Glu Ile Lys Arg
580

<210> 158
<211> 581
<212> PRT
<213> Artificial Sequence

<220>
<223> humanized antibody-linker-domain antibody bispecific construct

<400> 158
Gln Val Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Thr Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu

```
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Glu Val Asp Glu Thr Tyr Val Pro Lys Glu Phe Asn Ala Glu
    450                 455                 460
Thr Phe Thr Phe His Ala Asp Gly Ser Asp Ile Gln Met Thr Gln Ser
465                 470                 475                 480
Pro Ser Ser Leu Ser Ala Ser Val Gly Asp Arg Val Thr Ile Thr Cys
                485                 490                 495
Arg Ala Ser Gln Trp Ile Gly Pro Glu Leu Ser Trp Tyr Gln Gln Lys
            500                 505                 510
Pro Gly Lys Ala Pro Lys Leu Leu Ile Tyr His Gly Ser Ile Leu Gln
        515                 520                 525
Ser Gly Val Pro Ser Arg Phe Ser Gly Ser Gly Ser Gly Thr Asp Phe
    530                 535                 540
Thr Leu Thr Ile Ser Ser Leu Gln Pro Glu Asp Phe Ala Thr Tyr Tyr
545                 550                 555                 560
Cys Gln Gln Tyr Met Tyr Tyr Pro His Thr Phe Gly Gln Gly Thr Lys
                565                 570                 575
Val Glu Ile Lys Arg
            580


<210> 159
<211> 579
<212> PRT
<213> Artificial Sequence

<220>
<223> humanized antibody-linker-domain antibody
      bispecific construct

<400> 159
Gln Ile Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
```

Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr
340 345 350
Thr Leu Pro Pro Ser Arg Asp Glu Leu Thr Lys Asn Gln Val Ser Leu
355 360 365
Thr Cys Leu Val Lys Gly Phe Tyr Pro Ser Asp Ile Ala Val Glu Trp
370 375 380
Glu Ser Asn Gly Gln Pro Glu Asn Asn Tyr Lys Thr Thr Pro Pro Val
385 390 395 400
Leu Asp Ser Asp Gly Ser Phe Phe Leu Tyr Ser Lys Leu Thr Val Asp
405 410 415
Lys Ser Arg Trp Gln Gln Gly Asn Val Phe Ser Cys Ser Val Met His
420 425 430
Glu Ala Leu His Asn His Tyr Thr Gln Lys Ser Leu Ser Leu Ser Pro
435 440 445
Gly Lys Glu Val Asp Glu Thr Tyr Val Pro Lys Glu Phe Asn Ala Glu
450 455 460
Thr Phe Thr Phe His Ala Asp Asp Ile Gln Met Thr Gln Ser Pro Ser
465 470 475 480
Ser Leu Ser Ala Ser Val Gly Asp Arg Val Thr Ile Thr Cys Arg Ala
485 490 495
Ser Gln Trp Ile Gly Pro Glu Leu Ser Trp Tyr Gln Gln Lys Pro Gly
500 505 510
Lys Ala Pro Lys Leu Leu Ile Tyr His Gly Ser Ile Leu Gln Ser Gly
515 520 525
Val Pro Ser Arg Phe Ser Gly Ser Gly Ser Gly Thr Asp Phe Thr Leu
530 535 540
Thr Ile Ser Ser Leu Gln Pro Glu Asp Phe Ala Thr Tyr Tyr Cys Gln
545 550 555 560
Gln Tyr Met Tyr Tyr Pro His Thr Phe Gly Gln Gly Thr Lys Val Glu
565 570 575
Ile Lys Arg

<210> 160
<211> 579
<212> PRT
<213> Artificial Sequence

<220>
<223> humanized antibody-linker-domain antibody bispecific construct

<400> 160

```
Gln Ile Gln Leu Arg Gln Ser Gly Ala Glu Val Lys Lys Pro Gly Ala
1               5                   10                  15
Ser Val Lys Leu Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Lys Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Glu Val Asp Glu Thr Tyr Val Pro Lys Glu Phe Asn Ala Glu
    450                 455                 460
Thr Phe Thr Phe His Ala Asp Asp Ile Gln Met Thr Gln Ser Pro Ser
465                 470                 475                 480
Ser Leu Ser Ala Ser Val Gly Asp Arg Val Thr Ile Thr Cys Arg Ala
                485                 490                 495
Ser Gln Trp Ile Gly Pro Glu Leu Ser Trp Tyr Gln Gln Lys Pro Gly
            500                 505                 510
```

Lys Ala Pro Lys Leu Leu Ile Tyr His Gly Ser Ile Leu Gln Ser Gly
515 520 525
Val Pro Ser Arg Phe Ser Gly Ser Gly Ser Gly Thr Asp Phe Thr Leu
530 535 540
Thr Ile Ser Ser Leu Gln Pro Glu Asp Phe Ala Thr Tyr Tyr Cys Gln
545 550 555 560
Gln Tyr Met Tyr Tyr Pro His Thr Phe Gly Gln Gly Thr Lys Val Glu
565 570 575
Ile Lys Arg

<210> 161
<211> 579
<212> PRT
<213> Artificial Sequence

<220>
<223> humanized antibody-linker-domain antibody bispecific construct

<400> 161
Gln Ile Gln Leu Val Gln Ser Gly Ala Glu Val Lys Lys Pro Gly Ser
1 5 10 15
Ser Val Lys Val Ser Cys Lys Ala Ser Gly Tyr Thr Phe Thr Gly Tyr
20 25 30
Trp Ile Glu Trp Val Arg Gln Ala Pro Gly Gln Gly Leu Glu Trp Ile
35 40 45
Gly Glu Ile Leu Pro Gly Ser Gly Thr Thr Asn Tyr Asn Glu Lys Phe
50 55 60
Lys Gly Arg Ala Thr Phe Thr Ala Asp Lys Ser Thr Ser Thr Ala Tyr
65 70 75 80
Met Glu Leu Ser Ser Leu Arg Ser Glu Asp Thr Ala Val Tyr Tyr Cys
85 90 95
Ala Arg Gly Gly Tyr Tyr Tyr Gly Ser Ser Tyr Asp Ser Trp Gly Gln
100 105 110
Gly Thr Leu Val Thr Val Ser Ser Ala Ser Thr Lys Gly Pro Ser Val
115 120 125
Phe Pro Leu Ala Pro Ser Ser Lys Ser Thr Ser Gly Gly Thr Ala Ala
130 135 140
Leu Gly Cys Leu Val Lys Asp Tyr Phe Pro Glu Pro Val Thr Val Ser
145 150 155 160
Trp Asn Ser Gly Ala Leu Thr Ser Gly Val His Thr Phe Pro Ala Val
165 170 175
Leu Gln Ser Ser Gly Leu Tyr Ser Leu Ser Ser Val Val Thr Val Pro
180 185 190
Ser Ser Ser Leu Gly Thr Gln Thr Tyr Ile Cys Asn Val Asn His Lys
195 200 205
Pro Ser Asn Thr Lys Val Asp Lys Lys Val Glu Pro Lys Ser Cys Asp
210 215 220
Lys Thr His Thr Cys Pro Pro Cys Pro Ala Pro Glu Leu Leu Gly Gly
225 230 235 240
Pro Ser Val Phe Leu Phe Pro Pro Lys Pro Lys Asp Thr Leu Met Ile
245 250 255
Ser Arg Thr Pro Glu Val Thr Cys Val Val Val Asp Val Ser His Glu
260 265 270
Asp Pro Glu Val Lys Phe Asn Trp Tyr Val Asp Gly Val Glu Val His
275 280 285
Asn Ala Lys Thr Lys Pro Arg Glu Glu Gln Tyr Asn Ser Thr Tyr Arg
290 295 300
Val Val Ser Val Leu Thr Val Leu His Gln Asp Trp Leu Asn Gly Lys
305 310 315 320
Glu Tyr Lys Cys Lys Val Ser Asn Lys Ala Leu Pro Ala Pro Ile Glu
325 330 335
Lys Thr Ile Ser Lys Ala Lys Gly Gln Pro Arg Glu Pro Gln Val Tyr

```
                340                     345                     350
Thr Leu Pro Pro Ser Arg Asp Glu Leu Thr Lys Asn Gln Val Ser Leu
            355                     360                     365
Thr Cys Leu Val Lys Gly Phe Tyr Pro Ser Asp Ile Ala Val Glu Trp
        370                     375                     380
Glu Ser Asn Gly Gln Pro Glu Asn Asn Tyr Lys Thr Thr Pro Pro Val
385                     390                     395                     400
Leu Asp Ser Asp Gly Ser Phe Phe Leu Tyr Ser Lys Leu Thr Val Asp
                    405                     410                     415
Lys Ser Arg Trp Gln Gln Gly Asn Val Phe Ser Cys Ser Val Met His
                420                     425                     430
Glu Ala Leu His Asn His Tyr Thr Gln Lys Ser Leu Ser Leu Ser Pro
            435                     440                     445
Gly Lys Glu Val Asp Glu Thr Tyr Val Pro Lys Glu Phe Asn Ala Glu
        450                     455                     460
Thr Phe Thr Phe His Ala Asp Asp Ile Gln Met Thr Gln Ser Pro Ser
465                     470                     475                     480
Ser Leu Ser Ala Ser Val Gly Asp Arg Val Thr Ile Thr Cys Arg Ala
                    485                     490                     495
Ser Gln Trp Ile Gly Pro Glu Leu Ser Trp Tyr Gln Gln Lys Pro Gly
                500                     505                     510
Lys Ala Pro Lys Leu Leu Ile Tyr His Gly Ser Ile Leu Gln Ser Gly
            515                     520                     525
Val Pro Ser Arg Phe Ser Gly Ser Gly Ser Gly Thr Asp Phe Thr Leu
        530                     535                     540
Thr Ile Ser Ser Leu Gln Pro Glu Asp Phe Ala Thr Tyr Tyr Cys Gln
545                     550                     555                     560
Gln Tyr Met Tyr Tyr Pro His Thr Phe Gly Gln Gly Thr Lys Val Glu
                    565                     570                     575
Ile Lys Arg
```

<210> 162
<211> 579
<212> PRT
<213> Artificial Sequence

<220>
<223> humanized antibody-linker-domain antibody bispecific construct

<400> 162

```
Gln Val Gln Leu Val Gln Ser Gly Ala Glu Val Lys Lys Pro Gly Ser
1               5                   10                  15
Ser Val Lys Val Ser Cys Lys Ala Ser Gly Tyr Thr Phe Thr Gly Tyr
            20                  25                  30
Trp Ile Glu Trp Val Arg Gln Ala Pro Gly Gln Gly Leu Glu Trp Ile
        35                  40                  45
Gly Glu Ile Leu Pro Gly Ser Gly Thr Thr Asn Tyr Asn Glu Lys Phe
    50                  55                  60
Lys Gly Arg Ala Thr Phe Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Gly Tyr Tyr Tyr Gly Ser Ser Tyr Asp Ser Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
```

```
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys Glu Val Asp Glu Thr Tyr Val Pro Lys Glu Phe Asn Ala Glu
    450                 455                 460
Thr Phe Thr Phe His Ala Asp Asp Ile Gln Met Thr Gln Ser Pro Ser
465                 470                 475                 480
Ser Leu Ser Ala Ser Val Gly Asp Arg Val Thr Ile Thr Cys Arg Ala
                485                 490                 495
Ser Gln Trp Ile Gly Pro Glu Leu Ser Trp Tyr Gln Gln Lys Pro Gly
            500                 505                 510
Lys Ala Pro Lys Leu Leu Ile Tyr His Gly Ser Ile Leu Gln Ser Gly
        515                 520                 525
Val Pro Ser Arg Phe Ser Gly Ser Gly Ser Gly Thr Asp Phe Thr Leu
    530                 535                 540
Thr Ile Ser Ser Leu Gln Pro Glu Asp Phe Ala Thr Tyr Tyr Cys Gln
545                 550                 555                 560
Gln Tyr Met Tyr Tyr Pro His Thr Phe Gly Gln Gly Thr Lys Val Glu
                565                 570                 575
Ile Lys Arg
```

```
<210> 163
<211> 8
<212> PRT
<213> Artificial Sequence

<220>
<223> linker

<400> 163
Thr Val Ala Ala Pro Ser Gly Ser
 1               5
```

<210> 164
<211> 6
<212> PRT
<213> Artificial Sequence

<220>
<223> linker

<400> 164
Thr Val Ala Ala Pro Ser
1 5

<210> 165
<211> 26
<212> PRT
<213> Artificial Sequence

<220>
<223> linker

<400> 165
Gly Ser Thr Val Ala Ala Pro Ser Gly Ser Thr Val Ala Ala Pro Ser
1 5 10 15
Gly Ser Thr Val Ala Ala Pro Ser Gly Ser
20 25

<210> 166
<211> 18
<212> PRT
<213> Artificial Sequence

<220>
<223> linker

<400> 166
Thr Val Ala Ala Pro Ser Thr Val Ala Ala Pro Ser Thr Val Ala Ala
1 5 10 15
Pro Ser

<210> 167
<211> 16
<212> PRT
<213> Artificial Sequence

<220>
<223> linker

<400> 167
Asp Glu Thr Tyr Val Pro Lys Glu Phe Asn Ala Glu Thr Phe Gly Ser
1 5 10 15

<210> 168
<211> 14
<212> PRT
<213> Artificial Sequence

<220>
<223> linker

<400> 168
Asp Glu Thr Tyr Val Pro Lys Glu Phe Asn Ala Glu Thr Phe
1 5 10

<210> 169
<211> 23
<212> PRT
<213> Artificial Sequence

<220>
<223> linker

<400> 169
Glu Val Asp Glu Thr Tyr Val Pro Lys Glu Phe Asn Ala Glu Thr Phe
1 5 10 15
Thr Phe His Ala Asp Gly Ser
20

<210> 170
<211> 21
<212> PRT
<213> Artificial Sequence

<220>
<223> linker

<400> 170
Glu Val Asp Glu Thr Tyr Val Pro Lys Glu Phe Asn Ala Glu Thr Phe
1 5 10 15
Thr Phe His Ala Asp
20

<210> 171
<211> 1710
<212> DNA
<213> Artificial Sequence

<220>
<223> domain antibody

<400> 171
gaggtgcagc tggtggagtc tggcggcgga ctggtgcagc ccggcagaag cctgagactg 60
agctgtgccg ccagcggctt caccttcgac gactacgcca tgcactgggt gaggcaggcc 120
cctggcaagg gcctggagtg ggtgtccgcc atcacctgga atagcggcca catcgactac 180
gccgacagcg tggagggcag attcaccatc agccgggaca acgccaagaa cagcctgtac 240
ctgcagatga acagcctgag agccgaggac accgccgtgt actactgtgc caaggtgtcc 300
tacctgagca ccgccagcag cctggactac tggggccagg gcaccctggt gacagtctcg 360
agcgctagca ccaagggccc cagcgtgttc cccctggccc ccagcagcaa gagcaccagc 420
ggcggcacag ccgccctggg ctgcctggtg aaggactact tccccgagcc tgtgaccgtg 480
tcctggaata gcggagccct gacctccggc gtgcacacct tccccgccgt gctgcagagc 540
agcggcctgt actccctgag cagcgtggtg accgtgccca gcagcagcct gggcacccag 600
acctacatct gcaacgtgaa ccacaagccc agcaacacca aagtggacaa gaaagtggag 660
cccaagagct gcgataagac ccacacctgc ccccctgcc ctgcccccga gctggccggc 720
gcccctagcg tgttcctgtt cccccccaag cctaaggaca ccctgatgat cagcaggacc 780
cccgaagtga cctgcgtggt ggtggatgtg agccacgagg accctgaagt gaagttcaac 840
tggtacgtgg acggcgtgga agtgcacaac gccaagacca agcccagaga ggagcagtac 900
aacagcacct accgcgtggt gtctgtgctg accgtgctgc accaggattg gctgaacggc 960
aaggagtaca agtgcaaagt gagcaacaag gccctgcctg cccctatcga gaaaaccatc 1020
agcaaggcca agggccagcc tagagagccc caggtctaca ccctgcctcc ctccagagat 1080
gagctgacca agaaccaggt gtccctgacc tgtctggtga agggcttcta ccccagcgac 1140
atcgccgtgg agtgggagag caacggccag cccgagaaca actacaagac cacccccct 1200

```
gtgctggaca gcgatggcag cttcttcctg tactccaagc tgaccgtgga caagagcaga 1260
tggcagcagg gcaacgtgtt cagctgcagc gtgatgcacg aggccctgca caatcactac 1320
acccagaaga gtctgagcct gtcccctggc aagtcgaccg gtgaggtgca gctgctggtg 1380
tctggcggcg gactggtgca gcctggcggc agcctgagac tgagctgcgc cgccagcggc 1440
ttcaccttca aggcctaccc catgatgtgg gtgcggcagg cccctggcaa gggcctggaa 1500
tgggtgtccg agatcagccc cagcggcagc tacacctact acgccgacag cgtgaagggc 1560
cggttcacca tcagccggga caacagcaag aacaccctgt acctgcagat gaacagcctg 1620
cgggccgagg acaccgccgt gtactactgc gccaaggacc cccggaagct ggactactgg 1680
ggccagggca ccctggtgac cgtgagcagc                                  1710
```

<210> 172
<211> 570
<212> PRT
<213> Artificial Sequence

<220>
<223> domain antibody

<400> 172

```
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Ala Ile Thr Trp Asn Ser Gly His Ile Asp Tyr Ala Asp Ser Val
    50                  55                  60
Glu Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Ala Gly
225                 230                 235                 240
Ala Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
```

Leu Thr Cys Leu Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Tyr Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro Gly Lys Ser Thr Gly Glu Val Gln Leu Leu Val Ser Gly Gly Gly
450 455 460
Leu Val Gln Pro Gly Gly Ser Leu Arg Leu Ser Cys Ala Ala Ser Gly
465 470 475 480
Phe Thr Phe Lys Ala Tyr Pro Met Met Trp Val Arg Gln Ala Pro Gly
485 490 495
Lys Gly Leu Glu Trp Val Ser Glu Ile Ser Pro Ser Gly Ser Tyr Thr
500 505 510
Tyr Tyr Ala Asp Ser Val Lys Gly Arg Phe Thr Ile Ser Arg Asp Asn
515 520 525
Ser Lys Asn Thr Leu Tyr Leu Gln Met Asn Ser Leu Arg Ala Glu Asp
530 535 540
Thr Ala Val Tyr Tyr Cys Ala Lys Asp Pro Arg Lys Leu Asp Tyr Trp
545 550 555 560
Gly Gln Gly Thr Leu Val Thr Val Ser Ser
565 570

<210> 173
<211> 642
<212> DNA
<213> Artificial Sequence

<220>
<223> domain antibody

<400> 173
gatatccaga tgacccagag ccccagcagc ctgagcgcct ctgtgggcga tagagtgacc 60
atcacctgcc gggccagcca gggcatcaga aactacctgg cctggtatca gcagaagcct 120
ggcaaggccc ctaagctgct gatctacgcc gccagcaccc tgcagagcgg cgtgcccagc 180
agattcagcg gcagcggctc cggcaccgac ttcaccctga ccatcagcag cctgcagccc 240
gaggacgtgg ccacctacta ctgccagcgg tacaacagag ccccttacac cttcggccag 300
ggcaccaagg tggagatcaa gcgtacggtg gccgccccca gcgtgttcat cttccccccc 360
agcgatgagc agctcaagag cggcaccgcc agcgtggtgt gtctgctgaa caacttctac 420
ccccgggagg ccaaagtgca gtggaaagtg gacaacgccc tgcagagcgg caacagccag 480
gagagcgtga ccgagcagga cagcaaggac tccacctaca gcctgagcag caccctgacc 540
ctgagcaagg ccgactacga gaagcacaaa gtgtacgcct gcgaagtgac ccaccagggc 600
ctgtccagcc ccgtgaccaa gagcttcaac cggggcgagt gc 642

<210> 174
<211> 214
<212> PRT
<213> Artificial Sequence

<220>
<223> domain antibody

<400> 174
Asp Ile Gln Met Thr Gln Ser Pro Ser Ser Leu Ser Ala Ser Val Gly
1 5 10 15
Asp Arg Val Thr Ile Thr Cys Arg Ala Ser Gln Gly Ile Arg Asn Tyr
20 25 30
Leu Ala Trp Tyr Gln Gln Lys Pro Gly Lys Ala Pro Lys Leu Leu Ile
35 40 45
Tyr Ala Ala Ser Thr Leu Gln Ser Gly Val Pro Ser Arg Phe Ser Gly

```
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Arg Ala Pro Tyr
                85                  90                  95
Thr Phe Gly Gln Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 175
<211> 1353
<212> DNA
<213> Artificial Sequence

<220>
<223> antibody heavy chain

<400> 175

```
caggtccaat tagtgcaatc tgggtctgag ttgaagaagc ctggggcctc agtgaaggtt 60
tcctgcaagg cctctggata caccttcact aactatggaa tgaactgggt gcgacaggcc 120
cctggacaag ggctcgagtg gatgggatgg ataaacacca gaaatggaaa gtcaacatat 180
gttgatgact tcaaggggcg gtttgtcttc tccttggaca cctctgtcag cacggcatat 240
ctacagatca gcagcctaaa ggctgacgac actgcagtgt attactgtgc gagagaaggg 300
aatatggatg gttacttccc ttttacttac tggggccagg gtacactagt gaccgtgtcc 360
agcgccagca ccaagggccc cagcgtgttc cccctggccc ccagcagcaa gagcaccagc 420
ggcggcacag ccgccctggg ctgcctggtg aaggactact tccccgaacc ggtgaccgtg 480
tcctggaaca gcggagccct gaccagcggc gtgcacacct tccccgccgt gctgcagagc 540
agcggcctgt acagcctgag cagcgtggtg accgtgccca gcagcagcct gggcacccag 600
acctacatct gtaacgtgaa ccacaagccc agcaacacca aggtggacaa gaaggtggag 660
cccaagagct gtgacaagac ccacacctgc cccccctgcc ctgcccccga gctgctggga 720
ggccccagcg tgttcctgtt ccccccccaag cctaaggaca ccctgatgat cagcagaacc 780
cccgaggtga cctgtgtggt ggtggatgtg agccacgagg accctgaggt gaagttcaac 840
tggtacgtgg acggcgtgga ggtgcacaat gccaagacca agcccaggga ggagcagtac 900
aacagcacct accgggtggt gtccgtgctg accgtgctgc accaggattg gctgaacggc 960
aaggagtaca agtgtaaggt gtccaacaag gccctgcctg cccctatcga gaaaaccatc 1020
agcaaggcca agggccagcc cagagagccc caggtgtaca ccctgccccc tagcagagat 1080
gagctgacca agaaccaggt gtccctgacc tgcctggtga agggcttcta ccccagcgac 1140
atcgccgtgg agtgggagag caacggccag cccgagaaca actacaagac cacccccccct 1200
gtgctggaca gcgatggcag cttcttcctg tacagcaagc tgaccgtgga caagagcaga 1260
tggcagcagg gcaacgtgtt cagctgctcc gtgatgcacg aggccctgca caatcactac 1320
acccagaaga gcctgagcct gtcccctggc aag                              1353
```

<210> 176
<211> 451
<212> PRT
<213> Artificial Sequence

<220>
<223> antibody heavy chain

<400> 176

```
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Asn Tyr
            20                  25                  30
Gly Met Asn Trp Val Arg Gln Ala Pro Gly Gln Gly Leu Glu Trp Met
        35                  40                  45
Gly Trp Ile Asn Thr Arg Asn Gly Lys Ser Thr Tyr Val Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Asp Asp Thr Ala Val Tyr Tyr Cys
                85                  90                  95
Ala Arg Glu Gly Asn Met Asp Gly Tyr Phe Pro Phe Thr Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
Asp Lys Thr His Thr Cys Pro Pro Cys Pro Ala Pro Glu Leu Leu Gly
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Pro Ala Pro Ile
                325                 330                 335
Glu Lys Thr Ile Ser Lys Ala Lys Gly Gln Pro Arg Glu Pro Gln Val
            340                 345                 350
Tyr Thr Leu Pro Pro Ser Arg Asp Glu Leu Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450
```

<210> 177
<211> 642
<212> DNA
<213> Artificial Sequence

<220>
<223> antibody light chain

<400> 177
gatattgtca tgactcagtc tccatcatcc ctgtccgcat cagtaggaga cagggtcacc 60
atcacctgca aggcttctca gaatgtgggt actaatgtag cctggtatca acagaaacca 120
gggaaagctc ctaaagcact gatttactcg gcatcctatc ggtacagtgg agtccctgat 180
cgcttctcag gcagtggatc cgggacagat ttcactctca ccatcagcag tctgcagcct 240
gaagacttcg caacgtatta ctgtcagcaa tataacagct atcctctcac gttcggtggt 300
ggtaccaagg tggaaataaa acgtacggtg gccgccccca gcgtgttcat cttccccccc 360
agcgatgagc agctgaagag cggcaccgcc agcgtggtgt gtctgctgaa caacttctac 420
ccccgggagg ccaaggtgca gtggaaggtg gacaatgccc tgcagagcgg caacagccag 480
gagagcgtga ccgagcagga cagcaaggac tccacctaca gcctgagcag caccctgacc 540
ctgagcaagg ccgactacga gaagcacaag gtgtacgcct gtgaggtgac ccaccagggc 600
ctgtccagcc ccgtgaccaa gagcttcaac cggggcgagt gc 642

<210> 178
<211> 214
<212> PRT
<213> Artificial Sequence

<220>
<223> antibody light chain

<400> 178
Asp Ile Val Met Thr Gln Ser Pro Ser Ser Leu Ser Ala Ser Val Gly
1 5 10 15
Asp Arg Val Thr Ile Thr Cys Lys Ala Ser Gln Asn Val Gly Thr Asn
20 25 30
Val Ala Trp Tyr Gln Gln Lys Pro Gly Lys Ala Pro Lys Ala Leu Ile
35 40 45
Tyr Ser Ala Ser Tyr Arg Tyr Ser Gly Val Pro Asp Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Asn Ser Tyr Pro Leu
85 90 95
Thr Phe Gly Gly Gly Thr Lys Val Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 179
<211> 118
<212> PRT
<213> Artificial Sequence

<220>
<223> humanized antibody heavy chain variable domain

<400> 179
Glu Val Gln Leu Val Glu Ser Gly Gly Gly Leu Val Gln Pro Gly Gly

```
 1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu
        115
```

<210> 180
<211> 110
<212> PRT
<213> Artificial Sequence

<220>
<223> humanized antibody light chain variable domain

<400> 180

```
Asp Ile Gln Leu Thr Gln Ser Pro Ser Ser Leu Ser Ala Ser Val Gly
 1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly Thr Lys Val Glu Ile Lys Arg Thr Val
            100                 105                 110
```

<210> 181
<211> 152
<212> PRT
<213> Artificial Sequence

<220>
<223> anticalin

<400> 181

```
Asp Gly Gly Gly Ile Arg Arg Ser Met Ser Gly Thr Trp Tyr Leu Lys
 1               5                   10                  15
Ala Met Thr Val Asp Arg Glu Phe Pro Glu Met Asn Leu Glu Ser Val
            20                  25                  30
Thr Pro Met Thr Leu Thr Leu Leu Lys Gly His Asn Leu Glu Ala Lys
        35                  40                  45
Val Thr Met Leu Ile Ser Gly Arg Cys Gln Glu Val Lys Ala Val Leu
    50                  55                  60
Gly Arg Thr Lys Glu Arg Lys Lys Tyr Thr Ala Asp Gly Gly Lys His
65                  70                  75                  80
Val Ala Tyr Ile Ile Pro Ser Ala Val Arg Asp His Val Ile Phe Tyr
                85                  90                  95
Ser Glu Gly Gln Leu His Gly Lys Pro Val Arg Gly Val Lys Leu Val
            100                 105                 110
```

Gly Arg Asp Pro Lys Asn Asn Leu Glu Ala Leu Glu Asp Phe Glu Lys
        115                 120                 125
Ala Ala Gly Ala Arg Gly Leu Ser Thr Glu Ser Ile Leu Ile Pro Arg
    130                 135                 140
Gln Ser Glu Thr Cys Ser Pro Gly
145                 150

<210> 182
<211> 86
<212> PRT
<213> Artificial Sequence

<220>
<223> adnectin

<400> 182
Glu Val Val Ala Ala Thr Pro Thr Ser Leu Leu Ile Ser Trp Arg His
1               5                   10                  15
Pro His Phe Pro Thr Arg Tyr Tyr Arg Ile Thr Tyr Gly Glu Thr Gly
            20                  25                  30
Gly Asn Ser Pro Val Gln Glu Phe Thr Val Pro Leu Gln Pro Pro Thr
        35                  40                  45
Ala Thr Ile Ser Gly Leu Lys Pro Gly Val Asp Tyr Thr Ile Thr Val
    50                  55                  60
Tyr Ala Val Thr Asp Gly Arg Asn Gly Arg Leu Leu Ser Ile Pro Ile
65                  70                  75                  80
Ser Ile Asn Tyr Arg Thr
                85

<210> 183
<211> 126
<212> PRT
<213> Artificial Sequence

<220>
<223> nanobody

<400> 183
Glu Val Gln Leu Val Glu Ser Gly Gly Gly Leu Val Gln Ala Gly Gly
1               5                   10                  15
Ser Leu Arg Leu Ser Cys Ala Ala Ser Gly Arg Thr Phe Arg Ser Tyr
            20                  25                  30
Pro Met Gly Trp Phe Arg Gln Ala Pro Gly Lys Glu Arg Glu Phe Val
        35                  40                  45
Ala Ser Ile Thr Gly Ser Gly Gly Ser Thr Tyr Tyr Ala Asp Ser Val
    50                  55                  60
Lys Gly Arg Phe Thr Ile Ser Arg Asp Asn Ala Lys Asn Thr Val Tyr
65                  70                  75                  80
Leu Gln Met Asn Ser Leu Arg Pro Glu Asp Thr Ala Val Tyr Ser Cys
                85                  90                  95
Ala Ala Tyr Ile Arg Pro Asp Thr Tyr Leu Ser Arg Asp Tyr Arg Lys
            100                 105                 110
Tyr Asp Tyr Trp Gly Gln Gly Thr Gln Val Thr Val Ser Ser
        115                 120                 125

<210> 184
<211> 126
<212> PRT
<213> Artificial Sequence

<220>
<223> nanobody

<400> 184
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Pro Met Gly Trp Phe Arg Gln Ala Pro Gly Lys Gly Arg Glu Phe Val
        35                  40                  45
Ser Ser Ile Thr Gly Ser Gly Gly Ser Thr Tyr Tyr Ala Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Pro Glu Asp Thr Ala Val Tyr Tyr Cys
                85                  90                  95
Ala Ala Tyr Ile Arg Pro Asp Thr Tyr Leu Ser Arg Asp Tyr Arg Lys
            100                 105                 110
Tyr Asp Tyr Trp Gly Gln Gly Thr Leu Val Thr Val Ser Ser
        115                 120                 125

<210> 185
<211> 108
<212> PRT
<213> Artificial Sequence

<220>
<223> antibody light chain variable domain

<400> 185
Asp Ile Gln Met Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly Thr Lys Val Glu Ile Lys Arg
            100                 105

<210> 186
<211> 123
<212> PRT
<213> Artificial Sequence

<220>
<223> antibody heavy chain variable domain

<400> 186
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Thr Phe Thr Asn Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys

```
                        85                  90                  95
Ala Lys Tyr Pro His Tyr Tyr Gly Ser Ser His Trp Tyr Phe Asp Val
            100                 105                 110
Trp Gly Gln Gly Thr Leu Val Thr Val Ser Ser
        115                 120
```

<210> 187
<211> 13
<212> PRT
<213> Artificial Sequence

<220>
<223> linker

<400> 187

```
Asp Asp Asn Pro Asn Leu Pro Arg Leu Val Arg Pro Glu
 1               5                  10
```

<210> 188
<211> 14
<212> PRT
<213> Artificial Sequence

<220>
<223> linker

<400> 188

```
Asp Glu Met Pro Ala Asp Leu Pro Ser Leu Ala Ala Asp Phe
 1               5                  10
```

<210> 189
<211> 19
<212> PRT
<213> Artificial Sequence

<220>
<223> linker

<400> 189

```
His Lys Asp Asp Asn Pro Asn Leu Pro Arg Leu Val Arg Pro Glu Val
 1               5                  10                  15
Asp Val Met
```

<210> 190
<211> 21
<212> PRT
<213> Artificial Sequence

<220>
<223> linker

<400> 190

```
Glu Asn Asp Glu Met Pro Ala Asp Leu Pro Ser Leu Ala Ala Asp Phe
 1               5                  10                  15
Val Glu Ser Lys Asp
            20
```

<210> 191
<211> 108

<212> PRT
<213> Artificial Sequence

<220>
<223> domain antibody

<400> 191
Asp Ile Gln Met Thr Gln Ser Pro Ser Ser Leu Ser Ala Ser Val Gly
1 5 10 15
Asp Arg Val Thr Ile Thr Cys Arg Ala Ser Gln Trp Ile Gly Pro Glu
20 25 30
Leu Ser Trp Tyr Gln Gln Lys Pro Gly Lys Ala Pro Lys Leu Leu Ile
35 40 45
Tyr His Gly Ser Ile Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Met Tyr Tyr Pro His
85 90 95
Thr Phe Gly Gln Gly Thr Lys Val Glu Ile Lys Arg
100 105

<210> 192
<211> 108
<212> PRT
<213> Artificial Sequence

<220>
<223> domain antibody

<400> 192
Asp Ile Gln Met Thr Gln Ser Pro Ser Ser Leu Ser Ala Ser Val Gly
1 5 10 15
Asp Arg Val Thr Ile Thr Cys Arg Ala Ser Gln Trp Ile Gly Pro Glu
20 25 30
Leu Lys Trp Tyr Gln Gln Lys Pro Gly Lys Ala Pro Lys Leu Leu Ile
35 40 45
Tyr His Gly Ser Ile Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Met Tyr Tyr Pro Glu
85 90 95
Thr Phe Gly Gln Gly Thr Lys Val Glu Ile Lys Arg
100 105

<210> 193
<211> 108
<212> PRT
<213> Artificial Sequence

<220>
<223> domain antibody

<400> 193
Asp Ile Gln Met Thr Gln Ser Pro Ser Ser Leu Ser Ala Ser Val Gly
1 5 10 15
Asp Arg Val Thr Ile Thr Cys Arg Ala Ser Gln Trp Ile Gly Pro Glu
20 25 30
Leu Lys Trp Tyr Gln Gln Lys Pro Gly Lys Ala Pro Lys Leu Leu Ile
35 40 45
Tyr His Gly Ser Ile Leu Gln Ser Gly Val Pro Ser Arg Phe Ser Gly
50 55 60

Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Met Tyr Tyr Pro Lys
85 90 95
Thr Phe Gly Gln Gly Thr Lys Val Glu Ile Lys Arg
100 105

<210> 194
<211> 108
<212> PRT
<213> Artificial Sequence

<220>
<223> domain antibody

<400> 194
Asp Ile Gln Met Thr Gln Ser Pro Ser Ser Leu Ser Ala Ser Val Gly
1 5 10 15
Asp Arg Val Thr Ile Thr Cys Arg Ala Ser Gln Trp Ile Gly Pro Glu
20 25 30
Leu Lys Trp Tyr Gln Gln Lys Pro Gly Lys Ala Pro Lys Leu Leu Ile
35 40 45
Tyr His Gly Ser Ile Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Tyr Met Tyr Tyr Pro His
85 90 95
Thr Phe Gly Gln Gly Thr Lys Val Glu Ile Lys Arg
100 105

<210> 195
<211> 18
<212> PRT
<213> Artificial Sequence

<220>
<223> linker

<400> 195
Thr Gly Leu Asp Ser Pro Thr Gly Leu Asp Ser Pro Thr Gly Leu Asp
1 5 10 15
Ser Pro

<210> 196
<211> 24
<212> PRT
<213> Artificial Sequence

<220>
<223> linker

<400> 196
Thr Gly Leu Asp Ser Pro Thr Gly Leu Asp Ser Pro Thr Gly Leu Asp
1 5 10 15
Ser Pro Thr Gly Leu Asp Ser Pro
20

## Description

## BACKGROUND OF THE INVENTION

Antibodies are well known for use in therapeutic applications. Antibodies are heteromultimeric glycoproteins comprising at least two heavy and two light chains. Aside from IgM, intact antibodies are usually heterotetrameric glycoproteins of approximately 150Kda, composed of two identical light (L) chains and two identical heavy (H) chains. Typically, each light chain is linked to a heavy chain by one covalent disulfide bond while the number of disulfide linkages between the heavy chains of different immunoglobulin isotypes varies. Each heavy and light chain also has intrachain disulfide bridges. Each heavy chain has at one end a variable domain (VH) followed by a number of constant regions. Each light chain has a variable domain (VL) and a constant region at its other end; the constant region of the light chain is aligned with the first constant region of the heavy chain and the light chain variable domain is aligned with the variable domain of the heavy chain. The light chains of antibodies from most vertebrate species can be assigned to one of two types called Kappa and Lambda based on the amino acid sequence of the constant region. Depending on the amino acid sequence of the constant region of their heavy chains, human antibodies can be assigned to five different classes, IgA, IgD, IgE, IgG and IgM. IgG and IgA can be further subdivided into subclasses, IgG1, IgG2, IgG3 and IgG4; and IgA1 and IgA2. Species variants exist with mouse and rat having at least IgG2a, IgG2b. The variable domain of the antibody confers binding specificity upon the antibody with certain regions displaying particular variability called complementarity determining regions (CDRs). The more conserved portions of the variable region are called Framework regions (FR). The variable domains of intact heavy and light chains each comprise four FR connected by three CDRs. The CDRs in each chain are held together in close proximity by the FR regions and with the CDRs from the other chain contribute to the formation of the antigen-binding site of antibodies. The constant regions are not directly involved in the binding of the antibody to the antigen but exhibit various effector functions such as participation in antibody dependent cell-mediated cytotoxicity (ADCC), phagocytosis via binding to Fcγ receptor, half-life/clearance rate via neonatal Fc receptor (FcRn) and complement dependent cytotoxicity via the C1q component of the complement cascade.

The nature of the structure of an IgG antibody is such that there are two antigen-binding sites, both of which are specific for the same epitope. They are therefore, monospecific. A bispecific antibody is an antibody having binding specificities for at least two different epitopes. Methods of making such antibodies are known in the art. Traditionally, the recombinant production of bispecific antibodies is based on the co-expression of two immunoglobulin H chain-L chain pairs, where the two H chains have different binding specificities see Millstein et al, Nature 305 537-539 (1983), WO93/08829 and Traunecker et al EMBO, 10, 1991, 3655-3659. Because of the random assortment of H and L chains, a potential mixture often different antibody structures are produced of which only one has the desired binding specificity. An alternative approach involves fusing the variable domains with the desired binding specificities to heavy chain constant region comprising at least part of the hinge region, CH2 and CH3 regions. It is preferred to have the CH1 region containing the site necessary for light chain binding present in at least one of the fusions. DNA encoding these fusions, and if desired the L chain are inserted into separate expression vectors and are then co-transfected into a suitable host organism. It is possible though to insert the coding sequences for two or all three chains into one expression vector. In one approach, a bispecific antibody is composed of a H chain with a first binding specificity in one arm and a H-L chain pair, providing a second binding specificity in the other arm, see WO94/04690. Also see Suresh et al Methods in Enzymology 121, 210, 1986. Other approaches include antibody molecules which comprise single domain binding sites which is set out in WO2007/095338. A number of formats of bispecific antibodies have been described, some of which use linkers to attach one protein domain to another. Examples of such formats include antibody molecules attached to single chain Fv domains, as described in WO0190192, antibody molecules attached to single domain binding sites for example as set out in WO2007/095338, the mAbdAb format as described in WO2009/068649, the Dual Variable Domain format as described in WO2007/024715, and dual-specificity antibody fusions as described in WO2009/040562.

HGF (Hepatocyte Growth Factor or Scatter Factor, SF) is a pleiotropic cytokine that, together with its receptor MET (Mesenchymal Epithelial Transition factor, also known as c-MET or Hepatocyte Growth Factor receptor), is able to convey in cells a unique combination of pro-migratory, anti - apoptoic and pro-mitogenic signals. Native to most tissues, HGF is expressed by cells of mesenchymal origin and is localized within the extracellular matrix where it remains in its inactive (pro-HGF) form until cleaved by proteases. Under normal physiological conditions this occurs in response to tissue injury or during embryonic development. MET is expressed by cells of epithelial origin and, consistent with their tissue localization, the effects of HGF/ MET signal transduction are important in epithelial -mesenchymal interactions, cell mobilization, migration and rapid cell divisions that are essential for tissue repair in the adult and organogenesis in the embryo. Activation of HGF/ MET signalling coordinates a wide array of cellular processes including, proliferation, scattering/migration, induction of cell polarity and angiogenesis, where the effects are dependent on cell type and environment. In the adult animal, the pathway is relatively quiescent although it is integral to processes such as liver regeneration, repair to kidney damage, skin healing and intestinal injury where a coordinated process of invasive growth, mediated by HGF/MET signalling in cells at the wound edge, is essential for restoration of tissue integrity. Whilst regulated

HGF/MET, together coordinated genetic programmes that orchestrate embryonic development and tissue morphogenesis, are essential features of normal physiology, unregulated HGF/MET expression in cancer cells is a key feature of neoplastic dissemination of tumours. This unregulated expression can occur as a result of activating mutations, genomic amplification, transcriptional upregulation and paracrine or autocrine activation. Indeed, it has been shown that propagation of HGF/MET-dependent invasive growth signals is a general feature of highly aggressive tumours that can yield cells which migrate and infiltrate adjacent tissues and establish metastatic lesions at sites distal to the primary tumour. Coupled with the fact that HGF is a potent angiogenic factor and that MET is known to be expressed by endothelial cells, therapeutic targeting of HGF/MET has considerable potential to inhibit cancer onset, tumour progression and metastasis.

The Vascular Endothelial Growth Factor (VEGF) family of growth factors and their receptors are essential regulators of angiogenesis and vascular permeability. The VEGF family comprises VEGF-A, PIGF (placenta growth factor), VEGF-B, VEGF-C, VEGF-E and snake venom VEGF and each is thought to have a distinct role in vascular patterning and vessel development. Due to alternative splicing of mRNA transcribed from a single 8-exon gene, VEGF-A has at least 9 subtypes (isoforms) identified by the number of amino acids remaining after signal peptide cleavage. For example, in humans the most prominent isoform is $VEGF_{165}$, which exists in equilibrium between a soluble and cell associated form. Longer isoforms ($VEGF_{183}$, $VEGF_{189}$ & $VEGF_{206}$) possess C-terminal regions that are highly positively charged and mediate association with cell surface glycans and heparin that modulates their bioavailability. All VEGF-A isoforms form homodimers with the association occurring via a core of approximately 110 N-terminal residues that constitutes the receptor-binding VEGF fragment. Under normal circumstances, and in the centre of solid tumours, expression of VEGF is principally mediated by hypoxic conditions, signifying a shortage of vascular supply. The hypoxia causes dimerization of the hypoxia inducible factor HIF-1$\alpha$ with the constitutively expressed HIF-1$\alpha$, forming a transcription factor that binds to hypoxic response elements in the promoter region of the VEGF gene. Under normoxia, the HIF-1$\alpha$ protein undergoes ubiquitin-mediated degradation as a consequence of multiple proline hydroxylation events. Other tumour-associated VEGF up-regulation occurs due to activation via oncogene pathways (i.e. ras) via inflammatory cytokines & growth factors as well as by mechanical forces.

The active VEGF homodimer is bound at the cell surface by receptors of the VEGFR family. The principal vascular endothelium associated receptors for VEGF-A are VEGFR1 (Flt1) and VEGFR2 (Flk-2; KDR). Both receptors are members of the tyrosine kinase family and require ligand-mediated dimerization for activation. Upon dimerization the kinase domains undergo autophosphorylation, although the extent of the kinase activity in VEGFR2 is greater than that in VEGFR1. It has been demonstrated that the angiogenic signalling of VEGF is mediated largely through VEGFR2, although the affinity of VEGF is approximately 3-fold greater for VEGFR1 (KD ~30pM compared with 100pM for VEGFR2). This has led to the proposal that VEGFR1 principally acts as a decoy receptor to sequester VEGF and moderate the extent of VEGFR2 activation. Although VEGFR1 expression is associated with some tumours, its principal role appears to be during embryonic development & organogenesis. VEGF-$A_{165}$ is also bound by the neuropilin receptors NRP1 & NRP2. Although these receptors lack TK domains, they are believed to acts as co-receptors for VEGFR2 and augment signalling by transferring the VEGF to the VEGFR2.

Numerous studies have helped confirm VEGF-A as a key factor in tumour angiogenesis. For example VEGF-A is expressed in most tumours and in tumour associated stroma. In the absence of a well developed and expanding vasculature system to support growth, tumour cells become necrotic and apoptotic thereby imposing a limit to the increase in tumour volume (of the order 1 mm3) that can result from continuous cell proliferation. The expression of VEGF-A is highest in hypoxic tumour cells adjacent to necrotic areas indicating that the induction of VEGF-A by hypoxia in growing tumours can change the balance of activators and inhibitors of angiogenesis, leading to the growth of new blood vessels in the tumour. Consistent with this hypothesis, a number of approaches, including small-molecular weight tyrosine kinase inhibitors, monoclonal antibodies, antisense oligonucleotides etc., that inhibit or capture either VEGF-A or block its signalling receptor, VEGFR-2, have been developed as therapeutic agents.

Thus, there is a need for dual specific HGF and VEGF antagonists.

## SUMMARY OF INVENTION

The present invention relates to novel anti-human growth factor (HGF) antagonists, to a bispecific HGF antagonist and Vascular Endothelial Growth Factor (VEGF) antagonist, and to the use of such antagonists in therapy. More specifically, the invention relates to a panel of novel humanised anti-HGF antibodies, derived from the murine anti-HGF antibody S260116C12 (also identified herein as "16C12"). The invention also relates to an antigen-binding protein comprising at least one paired VH/VL domain which is linked to one or more epitope-binding domains wherein the antigen-binding protein has at least two antigen-binding sites, and wherein at least one of the antigen-binding sites binds to HGF or to VEGF, or wherein at least one of the antigen-binding sites binds to HGF and at least one of the antigen-binding sites binds to VEGF. In particular, the paired VH/VL domain may comprise VH and VL regions derived from 16C12.

Accordingly, in a first aspect, the invention provides an antigen-binding protein comprising at least one paired VH/VL domain linked to one or more epitope-binding domains, wherein the antigen-binding protein comprises at least

two antigen binding sites, at least one of the antigen binding sites being provided by the at least one epitope binding domain and at least one of the antigen binding sites being provided by the at least one paired VH/VL domain, wherein the or each paired VH/VL domain specifically binds to HGF and comprises a VH amino acid sequence of SEQ ID NO: 92, 94, 96 or 98.

In another aspect, the invention provides an anti-HGF antibody comprising a CDRH3 with a sequence set forth in SEQ ID NO:21. In an embodiment, the anti-HGF antibody comprises a CDRH1 (SEQ ID NO:19), CDRH2 (SEQ ID NO:20), CDRH3 (SEQ ID NO:21), CDRL1 (SEQ ID NO:22), CDRL2 (SEQ ID NO:23), and CDRL3 (SEQ ID NO:24). In an embodiment, the anti-HGF antibody comprises a heavy chain variable domain comprising an amino acid sequence set forth in any of SEQ ID NO:90, 92,94, 96 or 98, and a light chain variable domain comprising an amino acid sequence set forth in SEQ ID NO: 100 or 102. In another embodiment, the anti-HGF antibody comprises a heavy chain sequence set forth in SEQ ID NO:76, 78, 80, 82 or 84, and a light chain sequence set forth in SEQ ID NO: 86 or 88.

In another aspect, the invention provides an anti-HGF antibody according to the abovementioned aspect of the invention, linked to an epitope binding domain by a linker, wherein the epitope binding domain specifically binds to VEGF. In an embodiment, the epitope binding domain is an anti-VEGF immunoglobulin single variable domain, optionally having an amino acid sequence set forth in SEQ ID NO:104, 106, 108, 110, 191, 192, 193 or 194, or an amino acid sequence within 80, 85, 90, 95, 98, or 99% identity thereto.

In a particular embodiment, the immunoglobulin single variable domain is a domain antibody having the amino acid sequence of SEQ ID NO:194.

In an embodiment, the epitope binding domain is linked to the anti-HGF antibody by a linker having an amino acid sequence set forth in SEQ ID NO:163, 164, 165, 166, 167, 168, 169, 170, 195 or 196. In an embodiment, the epitope binding domain is linked to the heavy chain of the anti-HGF antibody, optionally at the C- or N-terminus thereof.

In another aspect, the invention provides a bispecific antigen-binding protein comprising an anti-HGF antibody linked to an anti-VEGF epitope binding domain, the anti-HGF antibody comprising at least a CDRH3 as set forth in SEQ ID NO:21, wherein the anti-VEGF epitope binding domain has a sequence set forth in SEQ ID NO: 104, 106, 108, 110, 191, 192, 193 or 194. In an embodiment, the anti-HGF antibody comprises a CDRH1 (SEQ ID NO:19), CDRH2 (SEQ ID NO:20), CDRH3 (SEQ ID NO:21), CDRL1 (SEQ ID NO:22), CDRL2 (SEQ ID NO:23), and CDRL3 (SEQ ID NO:24). In an embodiment, the anti-HGF antibody comprises a heavy chain variable domain comprising an amino acid sequence set forth in any of SEQ ID NO:76, 78, 80, 82 or 84, and a light chain variable domain comprising an amino acid sequence set forth in SEQ ID NO: 86 or 88. In a particular embodiment, the anti-HGF antibody comprises a heavy chain variable domain of SEQ ID NO:78 and a light chain variable domain of SEQ ID NO:86.

In another aspect, the invention provides an antigen-binding protein comprising an anti-HGF antibody comprsing a heavy chain having a VH amino acid sequence of SEQ ID NO:90, 92, 94, 96 or 98, and a light chain having a VL sequence of SEQ ID NO:100 or 102, and at least one epitope binding domain linked to said antibody by a linker comprising from 1 to 20 amino acids, said epitope binding domain specifically binding to VEGF.

The epitope binding domain may have an amino acid sequence of SEQ ID NO:191, 192, 193 or 194. In a particular embodiment, the anti-HGF antibody comprises a VH amino acid sequence of SEQ ID NO:92 and a VL domain of SEQ ID NO:100.

The invention also provides a polynucleotide sequence encoding a heavy chain of any of the antigen-binding proteins described herein, and a polynucleotide encoding a light chain of any of the antigen-binding proteins described herein. Such polynucleotides represent the coding sequence which corresponds to the equivalent polypeptide sequences, however it will be understood that such polynucleotide sequences could be cloned into an expression vector along with a start codon, an appropriate signal sequence and a stop codon.

The invention also provides a recombinant transformed or transfected host cell comprising one or more polynucleotides encoding a heavy chain and a light chain of any of the antigen-binding proteins described herein.

The invention further provides a method for the production of any of the antigen-binding proteins described herein which method comprises the step of culturing a host cell comprising a first and second vector, said first vector comprising a polynucleotide encoding a heavy chain of any of the antigen-binding proteins described herein and said second vector comprising a polynucleotide encoding a light chain of any of the antigen-binding proteins described herein, in a suitable culture media, for example serum- free culture media.

The invention further provides a pharmaceutical composition comprising an antigen-binding protein as described herein a pharmaceutically acceptable carrier.

BRIEF DESCRIPTION OF DRAWINGS

Figure 1 - Biacore binding data of bispecific antibodies to VEGF and HGF.
Figure 2 - MvlLu cell cell proliferation assay.

Figure 3 - PcMET in Human umbilical cord endothelial cells (HUVEC) for HGF.
Figure 4 - PcMET in Human umbilical cord endothelial cells (HUVEC) for HGF and VEGF.
Figure 5 - Bx-PC3 pMET detection assay.
Figure 6 - Bx-PC3 cell migration assay.

**Definitions**

The term 'Protein Scaffold' as used herein includes but is not limited to an immunoglobulin (Ig) scaffold, for example an IgG scaffold, which may be a four chain or two chain antibody, or which may comprise only the Fc region of an antibody, or which may comprise one or more constant regions from an antibody, which constant regions may be of human or primate origin, or which may be an artificial chimera of human and primate constant regions. Such protein scaffolds may comprise antigen-binding sites in addition to the one or more constant regions, for example where the protein scaffold comprises a full IgG. Such protein scaffolds will be capable of being linked to other protein domains, for example protein domains which have antigen-binding sites, for example epitope-binding domains or ScFv domains. The VH and VL domains or the heavy and light chains of the embodiments of the invention may exist as part of a protein scaffold.

Thus, in an aspect, the invention provides an antigen-binding protein comprising a protein scaffold which comprises at least one paired VH/VL domain linked to one or more epitope-binding domains, wherein the paired VH/VL domain specifically binds to HGF, and the epitope binding domain specifically binds to the VEGF, and wherein the VH of the at least one paired VH domain comprises the amino acid sequence of SEQ ID NO:92, 94, 96 or 98.

A "domain" is a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. An "antibody single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain ($V_H$, $V_{HH}$, $V_L$) that specifically binds an antigen or epitope independently of a different V region or domain. An immunoglobulin single variable domain can be present in a format (*e,g*., homo- or hetero-multimer) with other, different variable regions or variable domains where the other regions or domains are not required for antigen binding by the single immunoglobulin variable domain (*i.e.,* where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and *Camelid*$V_{HH}$ dAbs. Camelid $V_{HH}$ are immunoglobulin single variable domain polypeptides that are derived from species including camel, llama, alpaca, dromedary, and guanaco, which produce heavy chain antibodies naturally devoid of light chains. Such $V_{HH}$ domains may be humanised according to standard techniques available in the art, and such domains are still considered to be "domain antibodies" according to the invention. As used herein "$V_H$ includes camelid $V_{HH}$ domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a immunoglobulin single variable domain, for example a human, camelid or shark immunoglobulin single variable domain or it may be a domain which is a derivative of a non-Immunoglobulin scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than its natural ligand.

CTLA-4 (Cytotoxic T Lymphocyte-associated Antigen 4) is a CD28-family receptor expressed on mainly CD4+ T-cells. Its extracellular domain has a variable domain-like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001)

Lipocalins are a family of extracellular proteins which transport small hydrophobic molecules such as steroids,

bilins, retinoids and lipids. They have a rigid β-sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633

An affibody is a scaffold derived from Protein A of *Staohylococcus aureus* which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1

Avimers are multidomain proteins derived from the A-domain scaffold family. The native domains of approximately 35 amino acids adopt a defined disulphide bonded structure. Diversity is generated by shuffling of the natural variation exhibited by the family of A-domaihs. For further details see Nature Biotechnology 23(12), 1556 - 1561 (2005) and Expert Opinion on Investigational Drugs 16(6), 909-917 (June 2007)

A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Bibl. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two α-helices and a β-turn. They can be engineered to bind different target antigens by randomising residues in the first α-helix and a β-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the β-sandwich can be engineered to enable an Adnectin to specifically recognize a therapeutic target of interest. For further details see Protein Eng. Des. Sel. 18, 435-444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataB1 and conotoxin and knottins. The microproteins have a loop which can be engineered to include upto 25 amino acids without affecting the overall fold of the microprotein. For further details of engineered knottin domains, see WO2008098796.

Other epitope binding domains include proteins which have been used as a scaffold to engineer different target antigen binding properties include human γ-crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Epitope binding domains of the present invention could be derived from any of these alternative protein domains.

As used herein, the terms "paired VH domain", "paired VL domain", and "paired VH/VL domains" refer to antibody variable domains which specifically bind antigen only when paired with their partner variable domain. There is always one VH and one VL in any pairing, and the term "paired VH domain" refers to the VH partner, the term "paired VL domain" refers to the VL partner, and the term "paired VH/VL domains" refers to the two domains together.

The term "antigen binding protein" as used herein refers to antibodies, antibody fragments, for example a domain antibody (dAb), ScFv, FAb, $FAb_2$, and other protein constructs which are capable of binding to HGF and/or VEGF. Antigen binding molecules may comprise at least one Ig variable domain, for example antibodies, domain antibodies, Fab, Fab', F(ab')2, Fv, ScFv, diabodies, mAbdAbs, affibodies, heteroconjugate antibodies or bispecifics. In one embodiment the antigen binding molecule is an antibody. In another embodiment the antigen binding molecule is a dAb, i.e. an immunoglobulin single variable domain such as a VH, VHH or VL that specifically binds an antigen or epitope independently of a different V region or domain. Antigen binding molecules may be capable of binding to two targets, I.e. they may be dual targeting proteins. Antigen binding molecules may be a combination of antibodies and antigen binding fragments such as for example, one or more domain antibodies and/or one or more ScFvs linked to a monoclonal antibody. Antigen binding molecules may also comprise a non-Immunoglobulin domain for example a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEI and GroES; transferrin (trans-body); ankyrin repeat protein (DARPin); peptide aptamer; C-type lectin domain (Tetranectin); human γ-crystallin and' human ubiquitin (affilins); PDZ domains; scorpion toxinkunitz type domains of human protease inhibitors; and fibronectin (adnectin); which has been subjected to protein engineering in order to obtain binding to HGF or VEGF. As used herein "antigen binding protein" will be capable of antagonising and/or neutralising human HGF and/or

VEGF. In addition, an antigen binding protein may block HGF and/or VEGF activity by binding to HGF and/or VEGF and preventing a natural ligand from binding and/or activating the receptor.

As used herein "VEGF antagonist" includes any compound capable of reducing and or eliminating at least one activity of VEGF. By way of example, an VEGF antagonist may bind to VEGF and that binding may directly reduce or eliminate VEGF activity or it may work indirectly by blocking at least one ligand from binding the receptor.

As used herein "HGF antagonist" includes any compound capable of reducing and or eliminating at least one activity of HGF. By way of example, an HGF antagonist may bind to HGF and that binding may directly reduce or eliminate HGF activity or it may work indirectly by blocking at least one ligand from binding the receptor.

In one embodiment of the invention the antigen-binding site binds to antigen with a Kd of at least 1mM, for example a Kd of 10nM, 1nM, 500pM, 200pM, 100pM, to each antigen as measured by Biacore™.

As used herein, the term "antigen-binding site" refers to a site on a construct which is capable of specifically binding to antigen, this may be a single domain, for example an epitope-binding domain, or it may be paired VH/VL domains as can be found on a standard antibody. In some aspects of the invention single-chain Fv (ScFv) domains can provide antigen-binding sites.

The term "Constant Light Chain" is used herein to refer to the constant domain of an immunoglobulin light chain.

## DETAILED DESCRIPTION OF INVENTION

The present invention provides compositions comprising a Human Growth Factor (HGF) antagonist and/or a Vascular Endothelial Growth Factor (VEGF) antagonist. The present invention also provides the combination of an HGF antagonist and a VEGF antagonist, for example for use in therapy. The present invention also provides a method of treating disease by administering an HGF antagonist in combination with a VEGF antagonist. The HGF antagonist and the VEGF antagonist may be administered separately, sequentially or simultaneously.

Inhibition of angiogenesis is a therapeutic approach that has been established with the aim of starving the blood (and hence limiting the oxygen and nutrient) supply to the growing tumour. Multiple angiogenesis inhibitors have been therapeutically validated in preclinical cancer models and several clinical trials. Avastin (Bevacizumab), a monoclonal antibody targeting VEGF, has been approved as a first line therapy for the treatment of metastatic colorectal cancer (CRC) and non small lung carcinoma (NSCLC) in combination with chemotherapy and many small molecule compounds are in preclinical and clinical development. In certain cancers, such as breast and colon, agents such as these can slow the progression of the disease and lead to increased patient survival times of several months when given in combination with chemotherapy, but not when given alone. Indeed in several clinical trials the Bevacizumab-only arm was terminated early due to inferior performance relative to the plus chemotherapy (CT) arms. Initially this observation appeared paradoxical, since reducing the tumour blood supply has been shown to restrict the extent to which CT can be delivered to the tumour. Attempts to rationalize this observation are based on the proposition that an effect of Bevacizumab is to "normalize" the characteristically disordered vasculature of tumours. One postulated effect of the vascular normalization is the reduction of interstitial fluid pressure (IFP), resulting in increased blood flow and penetration of the CT agents to the core of the tumour. An alternative theory for the effectiveness of Bevacizumab in combination with CT suggests that the blockade of VEGF reduces nutrient and oxygen supply and triggers pro-apoptotic events that augment those induced by the CT.

Recent work in *in vivo* models has begun to cast more light on the lack of long term efficacy of anti-angiogenesis inhibitors when used in mono-therapy to target inhibition of the VEGF pathway in the clinic. Several reports demonstrate the anti-tumour effects of such an approach but also show concomitant tumour adaptation and progression to stages of greater malignancy, with heightened invasiveness and in some cases increased lymphatic and distant metastasis. Therefore, a consequence of 'starving' cancer cells of oxygen (hypoxia), additional to its beneficial effect on the primary tumour growth, appears to be to drive the tumour cells elsewhere in search of it. In other words, anti-angiogenic therapy that produces anti-tumour effects and survival benefit by effectively inhibiting neo-vascularization can additionally alter the phenotype of tumours by increasing invasion and metastasis. Other reports have shown that hypoxia induces cancer cells to produce MET and to have increased signalling via HGF/MET mediated pathways which in turn causes those cells to become highly motile and to move to distal sites (metastatic spread). Furthermore, extended use of VEGF inhibitors alone may promote the use of alternative neo-angiogenesis pathways, opening the possibility of drug resistance as survival rates increase.

Hence, a bispecific molecule will combine in a single agent the activity of an HGF antibody (suppression of tumour growth, angiogenesis and metastasis) with the anti-angiogenic effects of VEGF blockade, and has several advantages over the use of each component separately. There is a potential for synergistic effects since the simultaneous neutralization of HGF and VEGF could suppress the metastatic response of the cells to hypoxia whilst delivering improved angiogenic control. Furthermore, the combination of these two activities could limit the potential for drug resistance to single agent anti-angiogenesis therapies as patient survival rates increase.

Such antagonists may be antibodies or epitope binding domains for example immunoglobulin single variable

domains. The antagonists may be administered as a mixture of separate molecules which are administered at the same time i.e. co-administered, or are administered within 24 hours of each other, for example within 20 hours, or within 15 hours or within 12 hours, or within 10 hours, or within 8 hours, or within 6 hours, or within 4 hours, or within 2 hours, or within 1 hour, or within 30 minutes of each other.

Other HGF antagonists of use in the present invention comprise anti-c-MET antibodies, for example, the antibodies described in WO2009/007427.

In another aspect of the invention there is provided an antigen binding protein which comprises the heavy chain variable sequence selected from SEQ ID NO:92, SEQ ID NO:94, SEQ ID NO:96 or SEQ ID NO:98. In an embodiment, the antigen binding protein is a monoclonal antibody. The antigen binding protein may comprise a light chain variable sequence selected from SEQ ID NO:100 or SEQ ID NO:102, or a light chain of SEQ ID NO:86 or 88.

In one aspect of the invention as described herein there is provided an antigen binding protein which comprises an amino acid sequence selected from SEQ ID NO:104, SEQ ID NO:106, SEQ ID NO:108, SEQ ID NO:110, SEQ ID NO:191, SEQ ID NO:192, SEQ ID NO:193 or SEQ ID NO:194.

In one embodiment there is provided an antigen binding protein according to the invention described herein wherein the antigen binding protein binds to VEGF, for example the antigen binding protein comprises an epitope-binding domain which binds to VEGF and wherein the antigen binding protein comprises an amino acid sequence that has at least 70%, or at least 75%, or at least 80% or at least 85% or at least 90% or at least 95% or at least 99% sequence identity to an amino acid sequence selected from SEQ ID NO:104, SEQ ID NO:106, SEQ ID NO:108 or SEQ ID NO:110, SEQ ID NO:191, SEQ ID NO:192, SEQ ID NO:193 or SEQ ID NO:194. The antigen binding protein may further comprise an anti-HGF antibody according to the abovedescribed aspect of the invention. The anti-HGF antibody may be linked to the VEGF specific epitope-binding domain by a linker.

In one embodiment there is provided an antigen binding protein according to the invention described herein wherein the antigen binding protein binds to HGF, for example the antigen binding protein comprises an epitope-binding domain which binds to HGF, and wherein the antigen binding protein comprises an amino acid sequence that has at least 70%, or at least 75%, or at least 80% or at least 85% or at least 90% or at least 95% or at least 99% sequence identity to a heavy chain sequence selected from SEQ ID NO:76, SEQ ID NO:78, SEQ ID NO:80 or SEQ ID NO:82.

In a further embodiment of the invention as described herein the antigen binding protein may further comprise a linker sequence for example a linker selected from SEQ ID NO: 163-170, SEQ ID NO:187-190, or SEQ ID NO:195 or 196.

In a further embodiment the antagonists are present as one molecule capable of binding to two or more antigens, for example the invention provides a dual targeting molecule which is capable of binding to HGF and VEGF or which is capable of binding to HGF and VEGFR2, or which is capable of binding c-MET and VEGF.

The present invention provides an antigen-binding protein comprising a paired VH/VL domain which is linked to one or more epitope-binding domains wherein the antigen-binding protein has at least two antigen-binding sites at least one of which is from an epitope binding domain and wherein at least one of the antigen-binding sites binds to HGF.

The present invention provides an antigen-binding protein comprising a paired VH/VL domain which is linked to one or more epitope-binding domains wherein the antigen-binding protein has at least two antigen-binding sites at least one of which is from an epitope binding domain and at least one of which is from a paired VH/VL domain and wherein at least one of the antigen-binding sites binds to VEGF.

Such antigen-binding proteins comprise a paired VH/VL, for example a monoclonal antibody, which is linked to one or more epitope-binding domains, for example a domain antibody, wherein the binding protein has at least two antigen-binding sites, at least one of which is from an epitope binding domain, and wherein at least one of the antigen-binding sites binds to HGF, and to methods of producing and uses thereof, particularly uses in therapy.

The antigen-binding proteins of the present invention are also referred to as mAbdAbs.

The antigen-binding protein of the present invention has at least two antigen-binding sites, for examples it has two binding sites, for example where the first binding site has specificity for a first epitope on an antigen and the second binding site has specificity for a second epitope on the same antigen. In a further embodiment there are 4 antigen-binding sites, or 6 antigen-binding sites, or 8 antigen-binding sites, or 10 or more antigen-binding sites. In one embodiment the antigen-binding protein has specificity for more than one antigen, for example two antigens, or for three antigens, or for four antigens.

In one embodiment of the present invention the epitope binding domain is an immunoglobulin single variable domain.

It will be understood that any of the antigen-binding proteins described herein will be capable of neutralising one or more antigens, for example they will be capable of neutralising HGF and they will also be capable of neutralising VEGF.

The term "neutralises" and grammatical variations thereof as used throughout the present specification in relation to antigen-binding proteins of the invention means that a biological activity of the target is reduced, either totally or partially, in the presence of the antigen-binding proteins of the present invention in comparison to the activity of the target in the absence of such antigen-binding proteins. Neutralisation may be due to but not limited to one or more of

blocking ligand binding, preventing the ligand activating the receptor, down regulating the receptor or affecting effector functionality.

Levels of neutralisation can be measured in several ways, for example by use of any of the assays as set out in the examples below, for example in an assay which measures inhibition of ligand binding to receptor which may be carried out for example as described in Example 10. The neutralisation of HGF, in this assay is measured by assessing the decrease in phosphorylation of MET (Met phosphorylation is stimulated by HGF) in the presence of neutralising antigen-binding protein. HGF protein suitable for use in this assay includes the HGF protein comprising the sequence of NCBI Reference Sequence: NM_000601.4 (UniProt ID P14210). Levels of neutralisation of VEGF can be measured for example by the assay described in Example 11. VEGF protein suitable for use in this assay include $VEGF_{165}$ which comprises the sequence of NCBI Reference NP_001020539.2 (UniProt ID: P15692).

Other methods of assessing neutralisation, for example, by assessing the decreased binding between the ligand and its receptor in the presence of neutralising antigen-binding protein are known in the art, and include, for example, Biacore™ assays.

In one embodiment there is therefore provided antigen binding proteins according to the invention described herein which are neutralizing for HGF or neutralizing for VEGF. In a further embodiment there is provided antigen binding proteins according to the invention described herein wherein the antigen binding protein is neutralizing for both HGF and VEGF.

In an alternative aspect of the present invention there is provided antigen-binding proteins which have at least substantially equivalent neutralising activity to the antigen binding proteins exemplified herein.

The antigen-binding proteins of the invention have specificity for HGF, for example they comprise an epitope-binding domain which is capable of binding to HGF, and/or they comprise a paired VH/VL which binds to HGF. The antigen-binding protein may comprise an antibody which is capable of binding to HGF. The antigen-binding protein may comprise an immunoglobulin single variable domain which is capable of binding to HGF.

The antigen-binding proteins of the invention have specificity for VEGF, for example they comprise an epitope-binding domain which is capable of binding to VEGF, and/or they comprise a paired VH/VL which binds to VEGF. The antigen-binding protein may comprise an antibody which is capable of binding to VEGF. The antigen-binding protein may comprise an immunoglobulin single variable domain which is capable of binding to VEGF.

In one embodiment there is provided an antigen binding protein which compete with the antigen binding proteins herein described. For example an antigen binding protein which competes for binding to VEGF with an antigen binding protein which comprises an amino acid sequence selected from SEQ ID NO:104, SEQ ID NO:106, SEQ ID NO:108, SEQ ID NO:110, SEQ ID NO:191, SEQ ID NO:192, SEQ ID NO:193 or SEQ ID NO:194.

For example an antigen binding protein which competes for binding to HGF with an antigen binding protein which comprises the variable heavy chain sequence selected from SEQ ID NO:92, SEQ ID NO:94, SEQ ID NO:96 or SEQ ID NO:98.

In a further embodiment there is provided an antigen binding protein which competes for binding to both HGF and VEGF and comprises the variable heavy chain sequence selected from SEQ ID NO:92, SEQ ID NO:94, SEQ ID NO:96 or SEQ ID NO:98, SEQ ID NO:104, SEQ ID NO106, SEQ ID NO:108, SEQ ID NO:110, SEQ ID NO:191, SEQ ID NO:192, SEQ ID NO:193 or SEQ ID NO:194.

In one embodiment there is provided an antigen binding protein according to the invention described herein wherein the antigen binding protein binds to VEGF, for example the antigen binding protein comprises an epitope-binding domain which binds to VEGF and wherein the antigen binding protein comprises an amino acid sequence that has at least 70%, or at least 75%, or at least 80% or at least 85% or at least 90% or at least 95% or at least 99% sequence identity to a sequence selected from SEQ ID NO:104, SEQ ID NO106, SEQ ID NO:108, SEQ ID NO:110, SEQ ID NO:191, SEQ ID NO:192, SEQ ID NO:193 or SEQ ID NO:194

In one embodiment the antigen-binding protein of the present invention has specificity for more than one antigen, for example it may be capable of binding HGF and VEGF. In one embodiment the antigen-binding protein of the present invention is capable of binding HGF and VEGF simultaneously.

In one embodiment there is provided an antigen binding protein according to the invention described herein wherein the antigen binding protein binds to HGF, for example the antigen binding protein comprises an epitope-binding domain which binds to HGF and wherein the antigen binding protein comprises an amino acid sequence that has at least 70%, or at least 75%, or at least 80% or at least 85% or at least 90% or at least 95% or at least 99% sequence identity to a heavy chain sequence selected from SEQ ID NO:76, SEQ ID NO:78, SEQ ID NO:80 or SEQ ID NO:82.

It will be understood that any of the antigen-binding proteins described herein may be capable of binding two or more antigens simultaneously, for example, as determined by stochiometry analysis by using a suitable assay such as that described in Example 7.

Examples of such antigen-binding proteins include anti-VEGF antibodies which have an epitope binding domain which is a HGF antagonist, for example an anti-HGF immunoglobulin single variable domain, attached to the C-terminus or the N-terminus of the heavy chain or the C-terminus or N-terminus of the light chain.

Examples of such antigen-binding proteins include anti-HGF antibodies which have an epitope binding domain which is a VEGF antagonist attached to the C-terminus or the N-terminus of the heavy chain or the C-terminus or the N-terminus of the light chain. Examples include an antigen binding protein comprising the heavy chain sequence set out in SEQ ID NO: 76, 78, 80, 82 or 84 and/or the light chain sequence set out in SEQ ID NO: 86 or 88, wherein one or both of the Heavy and Light chain further comprise one or more epitope-binding domains which is capable of antagonising VEGF, for example by binding to VEGF or to a VEGF receptor for example VEGFR2. Such epitope-binding domains can be selected from those set out in SEQ ID NO: 181, 182 and SEQ ID NO: 104, 106, 108, 110, 191, 192, 193 or 194.

In a particular embodiment, the antigen binding protein comprises the heavy chain sequence of SEQ ID NO:78 and the light chain sequence of SEQ ID NO:86, wherein one or both of the heavy and light chains, optionally the heavy chains, further comprise an epitope-binding domain attached thereto which is capable of antagonizing VEGF or VEGF receptor, optionally VEGF. In a particular embodiment, the epitope binding domain has a sequence set forth in SEQ ID NO:194. The epitope binding domain may be attached to the antigen binding protein by means of a linker of 1 to 20 amino acids in length, as described herein.

Examples of such antigen-binding proteins include HGF antibodies which have an epitope binding domain comprising a VEGF immunoglobulin single variable domain attached to the c-terminus or the n-terminus of the heavy chain or the c-terminus or n-terminus of the light chain, for example an antigen binding protein having the heavy chain sequence set out in SEQ ID NO: 112-162, and the light chain sequence set out in SEQ ID NO: 86 or 88.

In one embodiment the antigen-binding protein will comprise an anti-HGF antibody linked to an epitope binding domain which is a VEGF antagonist, wherein the anti-HGF antibody has the same CDRs as the antibody which has the heavy chain sequence of SEQ ID NO:78, 82 or 84 and the light chain sequence of SEQ ID NO: 86, or the antibody which has the heavy chain sequence of SEQ ID NO:48, and the light chain sequence of SEQ ID NO: 50, or the antibody which has the heavy chain sequence of SEQ ID NO: 52, and the light chain sequence of SEQ ID NO: 54 , or the antibody which has the heavy chain sequence of SEQ ID NO: 56, and the light chain sequence of SEQ ID NO: 58.

In one embodiment the antigen-binding protein will comprise an anti-HGF antibody linked to an epitope binding domain which is a $V_EG_F$ antagonist, wherein the heavy chain sequence comprises SEQ ID NO:112, 114, 115, 117, 118, 123, 125, 126, 131, 133 or 134 and the light chain sequence comprises SEQ ID NO:86 or 88.

Further details of HGF antibodies which are of use in the present invention are given in WO2005/017107, WO2007/143098 and WO2007/115049. Other examples of such antigen-binding proteins include anti-HGF antibodies which have an anti-VEGF epitope binding domain, attached to the c-terminus or the n-terminus of the heavy chain or the c-terminus or n-terminus of the light chain wherein the VEGF epitope binding domain is a VEGF dAb which is selected from any of the VEGF dAb sequences which are set out in SEQ ID NO: 104, 106, 108, 110, 191, 192, 193 or 194.

In one embodiment the antigen-binding proteins include anti-HGF antibodies which have an anti-VEGF epitope binding domain, attached to the c-terminus or the n-terminus of the heavy chain or the c-terminus or n-terminus of the light chain wherein the VEGF epitope binding domain is a VEGF dAb which is selected from any of the VEGF dAb sequences which are set out in SEQ ID NO: 104, 106, 108, 110, 191, 192, 193 or 194.

The term "Effector Function" as used herein is meant to refer to one or more of Antibody dependant cell mediated cytotoxic activity (ADCC), Complement-dependant cytotoxic activity (CDC) mediated responses, Fc-mediated phagocytosis and antibody recycling via the FcRn receptor. For IgG antibodies, effector functionalities including ADCC and ADCP are mediated by the interaction of the heavy chain constant region with a family of Fcγ receptors present on the surface of immune cells. In humans these include FcyRI (CD64), FcyRII (CD32) and FcγRIII (CD16). Interaction between the antigen binding protein bound to antigen and the formation of the Fc/ Fcγ complex induces a range of effects including cytotoxicity, immune cell activation, phagocytosis and release of inflammatory cytokines.

The interaction between the constant region of an antigen binding protein and various Fc receptors (FcR) is believed to mediate the effector functions of the antigen binding protein. Significant biological effects can be a consequence of effector functionary, in particular, antibody-dependent cellular cytotoxicity (ADCC), fixation of complement (complement dependent cytotoxicity or CDC), and half-life/clearance of the antigen binding protein. Usually, the ability to mediate effector function requires binding of the antigen binding protein to an antigen and not all antigen binding proteins will mediate every effector function.

Effector function can be measured in a number of ways including for example via binding of the FcyRIII to Natural Killer cells or via FcyRI to monocytes/macrophages to measure for ADCC effector function. For example an antigen binding protein of the present invention can be assessed for ADCC effector function in a Natural Killer cell assay. Examples of such assays can be found in Shields et al, 2001 The Journal of Biological Chemistry, Vol. 276, p6591-6604; Chappel et al, 1993 The Journal of Biological Chemistry, Vol 268, p25124-25131; Lazar et al, 2006 PNAS, 103; 4005-4010.

Examples of assays to determine CDC function include that described in 1995 J Imm Meth 184:29-38.

Some isotypes of human constant regions, in particular IgG4 and IgG2 isotypes, essentially lack the functions of a) activation of complement by the classical pathway; and b) antibody-dependent cellular cytotoxicity. Various modifications to the heavy chain constant region of antigen binding proteins may be carried out depending on the desired effector property. IgG1 constant regions containing specific mutations have separately been described to reduce binding

to Fc receptors and therefore reduce ADCC and CDC (Duncan et al. Nature 1988, 332; 563-564; Lund et al. J. Immunol. 1991, 147; 2657-2662; Chappel et al. PNAS 1991, 88; 9036-9040; Burton and Woof, Adv. Immunol. 1992, 51;1-84; Morgan et al., Immunology 1995, 86; 319-324; Hezareh et al., J. Virol. 2001, 75 (24); 12161-12168).

In one embodiment of the present invention there is provided an antigen binding protein comprising a constant region such that the antigen binding protein has reduced ADCC and/or complement activation or effector functionality. In one such embodiment the heavy chain constant region may comprise a naturally disabled constant region of IgG2 or IgG4 isotype or a mutated IgG1 constant region. Examples of suitable modifications are described in EP0307434. One example comprises the substitutions of alanine residues at positions 235 and 237 (EU index numbering).

Human IgG1 constant regions containing specific mutations or altered glycosylation on residue Asn297 have also been described to enhance binding to Fc receptors. In some cases these mutations have also been shown to enhance ADCC and CDC(Lazar et al. PNAS 2006, 103; 4005-4010; Shields et al. J Biol Chem 2001, 276; 6591-6604; Nechansky et al. Mol Immunol, 2007, 44; 1815-1817).

In one embodiment of the present invention, such mutations are in one or more of positions selected from 239, 332 and 330 (IgG1), or the equivalent positions in other IgG isotypes. Examples of suitable mutations are S239D and I332E and A330L. In one embodiment the antigen binding protein of the invention herein described is mutated at positions 239 and 332, for example S239D and I332E or in a further embodiment it is mutated at three or more positions selected from 239 and 332 and 330, for example S239D and I332E and A330L. (EU index numbering).

In one embodiment of the present invention, the antigen binding protein comprises a heavy chain constant region with an altered glycosylation profile such that the antigen binding protein has enhanced effector function. For example, wherein the antigen binding protein has enhanced ADCC or enhanced CDC or wherein it has both enhanced ADCC and CDC effector function. Examples of suitable methodologies to produce antigen binding proteins with an altered glycosylation profile are described in WO2003/011878, WO2006/014679 and EP1229125, all of which can be applied to the antigen binding proteins of the present invention.

The present invention also provides a method for the production of an antigen binding protein according to the invention comprising the steps of:

a) culturing a recombinant host cell comprising an expression vector comprising the isolated nucleic acid as described herein, wherein the FUT8 gene encoding alpha-1,6-fucosyltransferase has been inactivated in the recombinant host cell; and
b) recovering the antigen binding protein.

Such methods for the production of antigen binding proteins can be performed, for example, using the POTELLIGENT™ technology system available from BioWa, Inc. (Princeton, NJ) in which CHOK1SV cells lacking a functional copy of the FUT8 gene produce monoclonal antibodies having enhanced antibody dependent cell mediated cytotoxicity (ADCC) activity that is increased relative to an identical monoclonal antibody produced in a cell with a functional FUT8 gene. Aspects of the POTELLIGENT™ technology system are described in US7214775, US6946292, WO0061739 and WO0231240 all of which are incorporated herein by reference. Those of ordinary skill in the art will also recognize other appropriate systems.

In one embodiment of the present invention there is provided an antigen binding protein comprising a chimaeric heavy chain constant region for example an antigen binding protein comprising a chimaeric heavy chain constant region with at least one CH2 domain from IgG3 such that the antigen binding protein has enhanced effector function, for example wherein it has enhanced ADCC or enhanced CDC, or enhanced ADCC and CDC functions,. In one such embodiment, the antigen binding protein may comprise one CH2 domain from IgG3 or both CH2 domains may be from IgG3.

Also provided is a method of producing an antigen binding protein according to the invention comprising the steps of:

a) culturing a recombinant host cell comprising an expression vector comprising an isolated nucleic acid as described herein wherein the expression vector comprises a nucleic acid sequence encoding an Fc domain having both IgG1 and IgG3 Fc domain amino acid residues; and
b) recovering the antigen binding protein.

Such methods for the production of antigen binding proteins can be performed, for example, using the COMPLEGENT™ technology system available from BioWa, Inc. (Princeton, NJ) and Kyowa Hakko Kogyo (now, Kyowa Hakko Kirin Co., Ltd.) Co., Ltd. in which a recombinant host cell comprising an expression vector in which a nucleic acid sequence encoding a chimeric Fc domain having both IgG1 and IgG3 Fc domain amino acid residues is expressed to produce an antigen binding protein having enhanced complement dependent cytotoxicity (CDC) activity that is increased relative to an otherwise identical antigen binding protein lacking such a chimeric Fc domain. Aspects of the COMPLEGENT™ technology system are described in WO2007011041 and

US20070148165 each of which are incorporated herein by reference. In an alternative embodiment CDC activity may be increased by introducing sequence specific mutations into the Fc region of an IgG chain. Those of ordinary skill in the art will also recognize other appropriate systems.

It will be apparent to those skilled in the art that such modifications may not only be used alone but may be used in combination with each other in order to further enhance effector function.

In one such embodiment of the present invention there is provided an antigen binding protein comprising a heavy chain constant region which comprises a mutated and chimaeric heavy chain constant region for example wherein an antigen binding protein comprising at least one CH2 domain from IgG3 and one CH2 domain from IgG1, wherein the IgG1 CH2 domain has one or more mutations at positions selected from 239 and 332 and 330 (for example the mutations may be selected from S239D and I332E and A330L) such that the antigen binding protein has enhanced effector function, for example wherein it has one or more of the following functions, enhanced ADCC or enhanced CDC, for example wherein it has enhanced ADCC and enhanced CDC. In one embodiment the IgG1 CH2 domain has the mutations S239D and I332E.

In an alternative embodiment of the present invention there is provided an antigen binding protein comprising a chimaeric heavy chain constant region and which has an altered glycosylation profile. In one such embodiment the heavy chain constant region comprises at least one CH2 domain from IgG3 and one CH2 domain from IgG1 and has an altered glycosylation profile such that the ratio of fucose to mannose is 0.8:3 or less, for example wherein the antigen binding protein is defucosylated so that said antigen binding protein has an enhanced effector function in comparison with an equivalent antigen binding protein with an immunoglobulin heavy chain constant region lacking said mutations and altered glycosylation profile, for example wherein it has one or more of the following functions, enhanced ADCC or enhanced CDC, for example wherein it has enhanced ADCC and enhanced CDC

In an alternative embodiment the antigen binding protein has at least one IgG3 CH2 domain and at least one heavy chain constant domain from IgG1 wherein both IgG CH2 domains are mutated in accordance with the limitations described herein.

In one aspect of the invention there is provided a method of producing an antigen binding protein according to the invention described herein comprising the steps of:

a) culturing a recombinant host cell containing an expression vector containing an isolated nucleic acid as described herein, said expression vector further comprising a Fc nucleic acid sequence encoding a chimeric Fc domain having both IgG1 and IgG3 Fc domain amino acid residues, and wherein the FUT8 gene encoding alpha-1,6-fucosyltransferase has been inactivated in the recombinant host cell; and
b) recovering the antigen binding protein.

Such methods for the production of antigen binding proteins can be performed, for example, using the ACCRETAMAB™ technology system available from BioWa, Inc. (Princeton, NJ) which combines the POTELLIGENT™ and COMPLEGENT™ technology systems to produce an antigen binding protein having both ADCC and CDC enhanced activity that is increased relative to an otherwise identical monoclonal antibody lacking a chimeric Fc domain and which has fucose on the oligosaccharide.

In yet another embodiment of the present invention there is an antigen binding protein comprising a mutated and chimeric heavy chain constant region wherein said antigen binding protein has an altered glycosylation profile such that the antigen binding protein has enhanced effector function, for example wherein it has one or more of the following functions, enhanced ADCC or enhanced CDC. In one embodiment the mutations are selected from positions 239 and 332 and 330, for example the mutations are selected from S239D and I332E and A330L. In a further embodiment the heavy chain constant region comprises at least one CH2 domain from IgG3 and one Ch2 domain from IgG1. In one embodiment the heavy chain constant region has an altered glycosylation profile such that the ratio of fucose to mannose is 0.8:3 or less for example the antigen binding protein is defucosylated, so that said antigen binding protein has an enhanced effector function in comparison with an equivalent non-chimaeric antigen binding protein or with an immunoglobulin heavy chain constant region lacking said mutations and altered glycosylation profile.

Another means of modifying antigen binding proteins of the present invention involves increasing the in-vivo half life of such proteins by modification of the immunoglobulin constant domain or FcRn (Fc receptor neonate) binding domain.

In adult mammals, FcRn, also known as the neonatal Fc receptor, plays a key role in maintaining serum antibody levels by acting as a protective receptor that binds and salvages antibodies of the IgG isotype from degradation. IgG molecules are endocytosed by endothelial cells, and if they bind to FcRn, are recycled out into circulation. In contrast, IgG molecules that do not bind to FcRn enter the cells and are targeted to the lysosomal pathway where they are degraded.

The neonatal FcRn receptor is believed to be involved in both antibody clearance and the transcytosis across tissues (see Junghans R.P (1997) Immunol.Res 16. 29-57 and Ghetie et al (2000) Annu.Rev.Immunol. 18, 739-766). Human IgG1 residues determined to interact directly with human FcRn includes Ile253, Ser254, Lys288, Thr307, Gln311,

Asn434 and His435. Switches at any of these positions described in this section may enable increased serum half-life and/or altered effector properties of antigen binding proteins of the invention.

Antigen binding proteins of the present invention may have amino acid modifications that increase the affinity of the constant domain or fragment thereof for FcRn. Increasing the half-life of therapeutic and diagnostic IgG's and other bioactive molecules has many benefits including reducing the amount and/or frequency of dosing of these molecules. In one embodiment there is therefore provided an antigen binding according to the invention provided herein or a fusion protein comprising all or a portion (an FcRn binding portion) of an IgG constant domain having one or more of these amino acid modifications and a non-IgG protein or non-protein molecule conjugated to such a modified IgG constant domain, wherein the presence of the modified IgG constant domain increases the in vivo half life of the antigen binding protein.

PCT Publication No. WO 00/42072 discloses a polypeptide comprising a variant Fc region with altered FcRn binding affinity, which polypeptide comprises an amino acid modification at any one or more of amino acid positions 238, 252, 253, 254, 255, 256, 265, 272, 286, 288, 303, 305, 307, 309, 311, 312, 317, 340, 356, 360, 362, 376, 378, 380, 386,388, 400, 413, 415, 424,433, 434,435, 436, 439, and 447 of the Fc region, wherein the numbering of the residues in the Fc region is that of the EU index (Kabat et al).

PCT Publication No. WO 02/060919 A2 discloses a modified IgG comprising an IgG constant domain comprising one or more amino acid modifications relative to a wild-type IgG constant domain, wherein the modified IgG has an increased half-life compared to the half-life of an IgG having the wild-type IgG constant domain, and wherein the one or more amino acid modifications are at one or more of positions 251, 253, 255, 285-290, 308-314, 385-389, and 428-435.

Shields et al. (2001, J Biol Chem ; 276:6591-604) used alanine scanning mutagenesis to alter residues in the Fc region of a human IgG1 antibody and then assessed the binding to human FcRn. Positions that effectively abrogated binding to FcRn when changed to alanine include I253, S254, H435, and Y436. Other positions showed a less pronounced reduction in binding as follows: E233-G236, R255, K288, L309, S415, and H433. Several amino acid positions exhibited an improvement in FcRn binding when changed to alanine; notable among these are P238, T256, E272, V305, T307, Q311, D312, K317, D376, E380, E382, S424, and N434. Many other amino acid positions exhibited a slight improvement (D265, N286, V303, K360, Q362, and A378) or no change (S239, K246, K248, D249, M252, E258, T260, S267, H268, S269, D270, K274, N276, Y278, D280, V282, E283, H285, T289, K290, R292, E293, E294, Q295, Y296, N297, S298, R301, N315, E318, K320, K322, S324, K326, A327, P329, P331, E333, K334, T335, S337, K338, K340, Q342, R344, E345, Q345, Q347, R356, M358, T359, K3601 N361, Y373, S375, S383, N384, Q386, E388, N389, N390, K392, L398, S400, D401, K414, R416, Q418, Q419, N421, V422, E430, T437, K439, S440, S442, S444, and K447) in FcRn binding.

The most pronounced effect was found for combination variants with improved binding to FcRn. At pH 6.0, the E380A/N434A variant showed over 8-fold better binding to FcRn, relative to native IgG1, compared with 2-fold for E380A and 3.5-fold for N434A. Adding T307A to this effected a 12-fold improvement in binding relative to native IgG1. In one embodiment the antigen binding protein of the invention comprises the E380A/N434A mutations and has increased binding to FcRn.

Dall'Acqua et al. (2002, J Immunol.;169:5171-80) described random mutagenesis and screening of human IgG1 hinge-Fc fragment phage display libraries against mouse FcRn. They disclosed random mutagenesis of positions 251, 252, 254-256, 308, 309, 311, 312, 314, 385-387, 389,428,433,434, and 436. The major improvements in IgG1-human FcRn complex stability occur in substituting residues located in a band across the Fc-FcRn interface (M252, S254, T256, H433, N434, and Y436) and to lesser extend substitutions of residues at the periphery like V308, L309, Q311, G385, Q386, P387, and N389. The variant with the highest affinity to human FcRn was obtained by combining the M252Y/S254T/T256E and H433K/N434F/Y436H mutations and exhibited a 57-fold increase in affinity relative to the wild-type IgG1. The in vivo behaviour of such a mutated human IgG1 exhibited a nearly 4-fold increase in serum half-life in cynomolgus monkey as compared to wild-type IgG1.

The present invention therefore provides a variant of an antigen binding protein with optimized binding to FcRn. In a preferred embodiment, the said variant of an antigen binding protein comprises at least one amino acid modification in the Fc region of said antigen binding protein, wherein said modification is selected from the group consisting of 226, 227, 228, 230, 231, 233, 234, 239, 241, 243, 246, 250, 252, 256, 259, 264, 265, 267, 269, 270, 276, 284, 285, 288, 289, 290, 291, 292, 294, 297, 298, 299, 301, 302, 303, 305, 307, 308, 309, 311, 315, 317, 320, 322, 325, 327, 330, 332, 334, 335, 338, 340, 342, 343, 345, 347, 350, 352, 354, 355, 356, 359, 360, 361, 362, 369, 370, 371, 375, 378, 380, 382, 384, 385, 386, 387, 389, 390, 392, 393, 394, 395, 396, 397, 398, 399, 400, 401 403, 404, 408, 411, 412, 414, 415, 416, 418, 419, 420, 421, 422, 424, 426, 428, 433, 434, 438, 439, 440, 443, 444, 445, 446 and 447 of the Fc region as compared to said parent polypeptide, wherein the numbering of the amino acids in the Fc region is that of the EU index in Kabat.,

In a further aspect of the invention the modifications are M252Y/S254T/T256E.

Additionally, various publications describe methods for obtaining physiologically active molecules whose half-lives are modified either by introducing an FcRn-binding polypeptide into the molecules (WO 97/43316; U.S. Patent N° 5,869,046; U.S. Patent N° 5,747,035; WO 96/32478; WO 91/14438) or by fusing the molecules with antibodies whose FcRn-binding affinities are preserved but affinities for other Fc receptors have been greatly reduced (WO 99/43713) or

fusing with FcRn binding domains of antibodies (WO 00/09560; U.S. Patent N°4,703,039).

Additionally, methods of producing an antigen binding protein with a decreased biological half-life are also provided. A variant IgG in which His435 is mutated to alanine results in the selective loss of FcRn binding and a significantly reduced serum half-life (Firan et al. 2001, International immunology 13:993). U.S. Pat. No. 6,165,745 discloses a method of producing an antigen binding protein with a decreased biological half-life by introducing a mutation into the DNA segment encoding the antigen binding protein. The mutation includes an amino acid substitution at position 253, 310, 311, 433, or 434 of the Fc-hinge domain.

In one embodiment, the antigen-binding proteins comprise an epitope-binding domain which is a domain antibody (dAb), for example the epitope binding domain may be a human VH or human VL, or a camelid $V_{HH}$ (nanobody) or a shark dAb (NARV).

In one embodiment the antigen-binding proteins comprise an epitope-binding domain which is a derivative of a scaffold selected from the group consisting of

CTLA-4 (Evibody); lipocalin; Protein A derived molecules such as Z-domain of Protein A (Affibody, SpA), A-domain (Avimer/Maxibody); Heat shock proteins such as GroEI and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than its natural ligand.

The antigen-binding proteins of the present invention may comprise a protein scaffold attached to an epitope binding domain which is an adnectin, for example an IgG scaffold with an adnectin attached to the c-terminus of the heavy chain, or it may comprise a protein scaffold attached to an adnectin, for example an IgG scaffold with an adnectin attached to the n-terminus of the heavy chain, or it may comprise a protein scaffold attached to an adnectin, for example an IgG scaffold with an adnectin attached to the c-terminus of the light chain, or it may comprise a protein scaffold attached to an adnectin, for example an IgG scaffold with an adnectin attached to the n-terminus of the light chain.

In other embodiments it may comprise a protein scaffold, for example an IgG scaffold, attached to an epitope binding domain which is a CTLA-4, for example an IgG scaffold with a CTLA-4. attached to the n-terminus of the heavy chain, or it may comprise for example an IgG scaffold with a CTLA-4 attached to the c-terminus of the heavy chain, or it may comprise for example an IgG scaffold with CTLA-4 attached to the n-terminus of the light chain, or it may comprise an IgG scaffold with CTLA-4 attached to the c-terminus of the light chain.

In other embodiments it may comprise a protein scaffold, for example an IgG scaffold, attached to an epitope binding domain which is a lipocalin, for example an IgG scaffold with a lipocalin attached to the n-terminus of the heavy chain, or it may comprise for example an IgG scaffold with a lipocalin attached to the c-terminus of the heavy chain, or it may comprise for example an IgG scaffold with a lipocalin attached to the n-terminus of the light chain, or it may comprise an IgG scaffold with a lipocalin attached to the c-terminus of the light chain.

In other embodiments it may comprise a protein scaffold, for example an IgG scaffold, attached to an epitope binding domain which is an SpA, for example an IgG scaffold with an SpA attached to the n-terminus of the heavy chain, or it may comprise for example an IgG scaffold with an SpA attached to the c-terminus of the heavy chain, or it may comprise for example an IgG scaffold with an SpA attached to the n-terminus of the light chain, or it may comprise an IgG scaffold with an SpA attached to the c-terminus of the light chain.

In other embodiments it may comprise a protein scaffold, for example an IgG scaffold, attached to an epitope binding domain which is an affibody, for example an IgG scaffold with an affibody attached to the n-terminus of the heavy chain, or it may comprise for example an IgG scaffold with an affibody attached to the c-terminus of the heavy chain, or it may comprise for example an IgG scaffold with an affibody attached to the n-terminus of the light chain, or it may comprise an IgG scaffold with an affibody attached to the c-terminus of the light chain.

In other embodiments it may comprise a protein scaffold, for example an IgG scaffold, attached to an epitope binding domain which is an affimer, for example an IgG scaffold with an affimer attached to the n-terminus of the heavy chain, or it may comprise for example an IgG scaffold with an affimer attached to the c-terminus of the heavy chain, or it may comprise for example an IgG scaffold with an affimer attached to the n-terminus of the light chain, or it may comprise an IgG scaffold with an affimer attached to the c-terminus of the light chain.

In other embodiments it may comprise a protein scaffold, for example an IgG scaffold, attached to an epitope binding domain which is a GroEI, for example an IgG scaffold with a GroEI attached to the n-terminus of the heavy chain, or it may comprise for example an IgG scaffold with a GroEI attached to the c-terminus of the heavy chain, or it may comprise for example an IgG scaffold with a GroEI attached to the n-terminus of the light chain, or it may comprise an IgG scaffold with a GroEI attached to the c-terminus of the light chain.

In other embodiments it may comprise a protein scaffold, for example an IgG scaffold, attached to an epitope binding domain which is a transferrin, for example an IgG scaffold with a transferrin attached to the n-terminus of the heavy chain, or it may comprise for example an IgG scaffold with a transferrin attached to the c-terminus of the heavy chain, or it may comprise for example an IgG scaffold with a transferrin attached to the n-terminus of the light chain, or it may comprise an IgG scaffold with a transferrin attached to the c-terminus of the light chain.

In other embodiments it may comprise a protein scaffold, for example an IgG scaffold, attached to an epitope binding domain which is a GroES, for example an IgG scaffold with a GroES attached to the n-terminus of the heavy chain, or it may comprise for example an IgG scaffold with a GroES attached to the c-terminus of the heavy chain, or it may comprise for example an IgG scaffold with a GroES attached to the n-terminus of the light chain, or it may comprise an IgG scaffold with a GroES attached to the c-terminus of the light chain.

In other embodiments it may comprise a protein scaffold, for example an IgG scaffold, attached to an epitope binding domain which is a DARPin, for example an IgG scaffold with a DARPin attached to the n-terminus of the heavy chain, or it may comprise for example an IgG scaffold with a DARPin attached to the c-terminus of the heavy chain, or it may comprise for example an IgG scaffold with a DARPin attached to the n-terminus of the light chain, or it may comprise an IgG scaffold with a DARPin attached to the c-terminus of the light chain.

In other embodiments it may comprise a protein scaffold, for example an IgG scaffold, attached to an epitope binding domain which is a peptide aptamer, for example an IgG scaffold with a peptide aptamer attached to the n-terminus of the heavy chain, or it may comprise for example an IgG scaffold with a peptide aptamer attached to the c-terminus of the heavy chain, or it may comprise for example an IgG scaffold with a peptide aptamer attached to the n-terminus of the light chain, or it may comprise an IgG scaffold with a peptide aptamer attached to the c-terminus of the light chain.

In one embodiment of the present invention there are four epitope binding domains, for example four domain antibodies, two of the epitope binding domains may have specificity for the same antigen, or all of the epitope binding domains present in the antigen-binding protein may have specificity for the same antigen.

Paired VH/VL domains, antibodies, and protein scaffolds of the present invention may be linked to epitope-binding domains by the use of linkers. Examples of suitable linkers include amino acid sequences which may be from 1 amino acid to 150 amino acids in length, or from 1 amino acid to 140 amino acids, for example, from 1 amino acid to 130 amino acids, or from 1 to 120 amino acids, or from 1 to 80 amino acids, or from 1 to 50 amino acids, or from 1 to 20 amino acids, or from 1 to 10 amino acids, or from 5 to 18 amino acids. Such sequences may have their own tertiary structure, for example, a linker of the present invention may comprise a single variable domain. The size of a linker in one embodiment is equivalent to a single variable domain. Suitable linkers may be of a size from 1 to 20 angstroms, for example less than 15 angstroms, or less than 10 angstroms, or less than 5 angstroms.

In one embodiment of the present invention at least one of the epitope binding domains is directly attached to the Ig scaffold with a linker comprising from 1 to 150 amino acids, for example 1 to 20 amino acids, for example 1 to 10 amino acids. Such linkers may be the linker "GS" or may be one selected from any one of those set out in SEQ ID NO: 163-170,195 or 196, or multiples of such linkers.

Linkers of use in the antigen-binding proteins of the present invention may comprise alone or in addition to other linkers, one or more sets of GS residues, for example 'GSTVAAPS' or 'TVAAPSGS' or 'GSTVAAPSGS'. In one embodiment the linker comprises SEQ ID NO:163.

In one embodiment the epitope binding domain is linked to the Ig scaffold by the linker '$(PAS)_n(GS)_m$'. In another embodiment the epitope binding domain is linked to the Ig scaffold by the linker '$(GGGGS)_n(GS)_m$'. In another embodiment the epitope binding domain is linked to the Ig scaffold by the linker '$(TVAAPS)_n(GS)_m$'. In another embodiment the epitope binding domain is linked to the Ig scaffold by the linker '$(GS)_m(TVAAPSGS)_n$'. In another embodiment the epitope binding domain is linked to the Ig scaffold by the linker '$(PAVPPP)_n(GS)_m$'. In another embodiment the epitope binding domain is linked to the Ig scaffold by the tinker '$(TVSDVP)_n(GS)_m$'. In another embodiment the epitope binding domain is linked to the Ig scaffold by the linker '$(TGLDSP)_n(GS)_m$'. In all such embodiments, n = 1-10, and m = 0-4.

Examples of such linkers include $(PAS)_n(GS)_m$ wherein n=1 and m=1, $(PAS)_n(GS)_m$ wherein n=2 and m=1, $(PAS)_n(GS)_m$ wherein n=3 and m=1, $(PAS)_n(GS)_m$ wherein n=4 and m=1, $(PAS)_n(GS)_m$ wherein n=2 and m=0, $(PAS)_n(GS)_m$ wherein n=3 and m=0, $(PAS)_n(GS)_m$ wherein n=4 and m=0.

Examples of such linkers include $(GGGGS)_n(GS)_m$ wherein n=1 and m=1, $(GGGGS)_n(GS)_m$ wherein n=2 and m=1, $(GGGGS)_n(GS)_m$ wherein n=3 and m=1, $(GGGGS)_n(GS)_m$ wherein n=4 and m=1, $(GGGGS)_n(GS)_m$ wherein n=2 and m=0, $(GGGGS)_n(GS)_m$ wherein n=3 and m=0, $(GGGGS)_n(GS)_m$ wherein n=4 and m=0.

Examples of such linkers include $(TVAAPS)_n(GS)_m$ wherein n=1 and m=1 (SEQ ID NO:163), $(TVAAPS)_n(GS)_m$ wherein n=2 and m=1, $(TVAAPS)_n(GS)_m$ wherein n=3 and m=1, $(TVAAPS)_n(GS)_m$ wherein n=4 and m=1, $(TVAAPS)_n(GS)_m$ wherein n=1 and m=0 (SEQ ID NO:164), $(TVAAPS)_n(GS)_m$ wherein n=2 and m=0, $(TVAAPS)_n(GS)_m$ wherein n=3 and m=0 (SEQ ID NO:166), $(TVAAPS)_n(GS)_m$ wherein n=4 and m=0.

Examples of such linkers include $(GS)_m(TVAAPSGS)_n$ wherein n=1 and m=1, $(GS)_m(TVAAPSGS)_n$ wherein n=2 and m=1, $(GS)_m(TVAAPSGS)_n$ wherein n=3 and m=1, or $(GS)_m(TVAAPSGS)_n$ wherein n=4 and m=1, $(GS)_m(TVAAPSGS)_n$ wherein n=5 and m=1, $(GS)_m(TVAAPSGS)_n$ wherein n=6 and m=1, $(GS)_m(TVAAPSGS)_n$ wherein n=1 and m=0, $(GS)_m(TVAAPSGS)_n$ wherein n=2 and m=10, $(GS)_m(TVAAPSGS)_n$ wherein n=3 and m=0, $(GS)_m(TVAAPSGS)_n$ wherein n=3 and m=1 (SEQ ID NO:165), or $(GS)_m(TVAAPSGS)_n$ wherein n=0.

Examples of such linkers include $(PAVPPP)_n(GS)_m$ wherein n=1 and m=1, $(PAVPPP)_n(GS)_m$ wherein n=2 and m=1, $(PAVPPP)_n(GS)_m$ wherein n=3 and m=1, $(PAVPPP)_n(GS)_m$ wherein n=4 and m=1, $(PAVPPP)_n(GS)_m$ wherein n=2

and m=0, $(PAVPPP)_n(GS)_m$ wherein n=3 and m=0, $(PAVPPP)_n(GS)_m$ wherein n=4 and m=0.

Examples of such linkers include $(TVSDVP)_n(GS)_m$ wherein n=1 and m=1, $(TVSDVP)_n(GS)_m$ wherein n=2 and m=1, $(TVSDVP)_n(GS)_m$ wherein n=3 and m=1, $(TVSDVP)_n(GS)_m$ wherein n=4 and m=1, $(TVSDVP)_n(GS)_m$ wherein n=2 and m=0, $(TVSDVP)_n(GS)_m$ wherein n=3 and m=0, $(TVSDVP)_n(GS)_m$ wherein n=4 and m=0.

Examples of such linkers include $(TGLDSP)_n(GS)_m$ wherein n=1 and m=1 (SEQ ID NO:57), $(TGLDSP)_n(GS)_m$ wherein n=2 and m=1, $(TGLDSP)_n(GS)_m$ wherein n=3 and m=1, $(TGLDSP)_n(GS)_m$ wherein n=4 and m=1, $(TGLDSP)_n(GS)_m$ wherein n=2 and m=0, $(TGLDSP)_n(GS)_m$ wherein n=3 and m=0 (SEQ ID NO:195), $(TGLDSP)_n(GS)_m$ wherein n=4 and m=0 (SEQ ID NO:196).

In another embodiment there is no linker between the epitope binding domain and the Ig scaffold. In another embodiment the epitope binding domain is linked to the Ig scaffold by the linker 'TVAAPS'. In another embodiment the epitope binding domain, is linked to the Ig scaffold by the linker 'TVAAPSGS'. In another embodiment the epitope binding domain is linked to the Ig scaffold by the linker 'GS'. In another embodiment the epitope binding domain is linked to the Ig scaffold by the linker 'ASTKGPT'.

The linkers may be derived from human serum albumin, for example the linkers set out in SEQ ID NO:167, SEQ ID NO:168, SEQ ID NO:169, SEQ ID NO:170, SEQ ID NO:187, SEQ ID NO:188, SEQ ID NO:189 or SEQ ID NO:190. The linkers may further comprise some additional residues, for example, they may comprise additional Glycine and Serine residues. These additional residues may be at the beginning or end of the albumin-derived sequence, or may be within the albumin-derived sequence. Examples of such linkers include those set out in SEQ ID NO:167 and SEQ ID NO:169.

In one embodiment, the antigen-binding protein of the present invention comprises at least one antigen-binding site, for example at least one epitope binding domain, which is capable of binding human serum albumin.

In one embodiment, there are at least 3 antigen-binding sites, for example there are 4, or 5 or 6 or 8 or 10 antigen-binding sites and the antigen-binding protein is capable of binding at least 3 or 4 or 5 or 6 or 8 or 10 antigens, for example it is capable of binding 3 or 4 or 5 or 6 or 8 or 10 antigens simultaneously.

The invention also provides the antigen-binding proteins for use in medicine, for example for use in the manufacture of a medicament for treating solid tumours believed to require angiogenesis or to be associated with elevated levels of HGF (HGF/ Met signaling) and/or VEGF. Such tumours include colon, breast, ovarian, lung (small cell or non small cell), prostate, pancreatic, renal, liver, gastric, head and neck, melanoma, sarcoma. Also included are primary and secondary (metastatic) brain tumours including, but not limited to gliomas (including epenymomas), meningiomas, oligodendromas, astrocytomas (low grade, anaplastic and glioblastoma multiforme), medulloblastomas, gangliomas, schwannnomas and chordomas. Other diseases associated with undesirable angiogenesis that are suitable for treatment with the antigen binding proteins of the present invention include age-related macular degeneration, diabetic retinopathy, RA and psoriasis.

The invention provides a method of treating a patient suffering from solid tumours (including colon, breast, ovarian, lung (small cell or non small cell), prostate, pancreatic, renal, liver, gastric, head and neck, melanoma, sarcoma), primary and secondary (metastatic) brain tumours including, but not limited to gliomas (including epenymomas), meningiomas, oligodendromas, astrocytomas (low grade, anaplastic and glioblastoma multiforme), medulloblastomas, gangliomas, schwannnomas and chordomas, age-related macular degeneration, diabetic retinopathy, RA or psoriasis comprising administering a therapeutic amount of an antigen-binding protein of the invention.

The antigen-binding proteins of the invention may be used for the treatment of solid tumours (including colon, breast, ovarian, lung (small cell or non small cell), prostate, pancreatic, renal, liver, gastric, head and neck, melanoma, sarcoma), primary and secondary (metastatic) brain tumours including, but not limited to gliomas (including epenymomas), meningiomas, oligodendromas, astrocytomas (low grade, anaplastic and glioblastoma multiforme), medulloblastomas, gangliomas, schwannnomas and chordomas, age-related macular degeneration, diabetic retinopathy, RA or psoriasis or any other disease associated with the over production of HGF and/or VEGF.

Protein scaffolds of use in the present invention include full monoclonal antibody scaffolds comprising all the domains of an antibody, or protein scaffolds of the present invention may comprise a non-conventional antibody structure, such as a monovalent antibody. Such monovalent antibodies may comprise a paired heavy and light chain wherein the hinge region of the heavy chain is modified so that the heavy chain does not homodimerise, such as the monovalent antibody described in WO2007/059782. Other monovalent antibodies may comprise a paired heavy and light chain which dimerises with a second heavy chain which is lacking a functional variable region and CH1 region, wherein the first and second heavy chains are modified so that they will form heterodimers rather than homodimers, resulting in a monovalent antibody with two heavy chains and one light chain such as the monovalent antibody described in WO2006/015371. Such monovalent antibodies can provide the protein scaffold of the present invention to which epitope binding domains can be linked.

Epitope-binding domains of use in the present invention are domains that specifically bind an antigen or epitope independently of a different V region or domain, this may be a domain antibody or may be a domain which is a derivative of a non-immunoglobulin scaffold selected from the group consisting of CTLA-4 (Evibody); lipocalin; Protein A derived

molecules such as Z-domain of Protein A (Affibody, SpA), A-domain (Avimer/Maxibody); Heat shock proteins such as GroEI and GroES; transferrin (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than its natural ligand. In one embodiment this may be an domain antibody or other suitable domains such as a domain selected from the group consisting of CTLA-4, lipocallin, SpA, an Affibody, an avimer, GroEI, transferrin, GroES and fibronectin. In one embodiment this may be selected from a immunoglobulin single variable domain, an Affibody, an ankyrin repeat protein (DARPin) and an adnectin. In another embodiment this may be selected from an Affibody, an ankyrin repeat protein (DARPin) and an adnectin. In another embodiment this may be a domain antibody, for example a domain antibody selected from a human, camelid or shark (NARV) domain antibody.

Epitope-binding domains can be linked to the protein scaffold at one or more positions. These positions include the C-terminus and the N-terminus of the protein scaffold, for example at the C-terminus of the heavy chain and/or the C-terminus of the light chain of an IgG, or for example the N-terminus of the heavy chain and/or the N-terminus of the light chain of an IgG.

In one embodiment, a first epitope binding domain is linked to the protein scaffold comprising the paired VH/VL, and a second epitope binding domain is linked to the first epitope binding domain, for example where the protein scaffold is an IgG scaffold, a first epitope binding domain may be linked to the c-terminus of the heavy chain of the IgG scaffold, and that epitope binding domain can be linked at its C-terminus to a second epitope binding domain, or for example a first epitope binding domain may be linked to the C-terminus of the light chain of the IgG scaffold, and that first epitope binding domain may be further linked at its C-terminus to a second epitope binding domain, or for example a first epitope binding domain may be linked to the N-terminus of the light chain of the IgG scaffold, and that first epitope binding domain may be further linked at its N-terminus to a second epitope binding domain, or for example a first epitope binding domain may be linked to the N-terminus of the heavy chain of the IgG scaffold, and that first epitope binding domain may be further linked at its N-terminus to a second epitope binding domain.

When the epitope-binding domain is a domain antibody, some domain antibodies may be suited to particular positions within the scaffold.

Domain antibodies of use in the present invention can be linked at the C-terminal end of the heavy chain and/or the light chain of conventional IgGs. In addition some immunoglobulin single variable domains can be linked to the C-terminal ends of both the heavy chain and the light chain of conventional antibodies.

In constructs where the N-terminus of immunoglobulin single variable domains are fused to an antibody constant domain (either $C_H3$ or CL), a peptide linker may help the immunoglobulin single variable domain to bind to antigen. Indeed, the N-terminal end of a dAb is located closely to the complementarity-determining regions (CDRS) involved in antigen-binding activity. Thus a short peptide linker acts as a spacer between the epitope-binding, and the constant domain fo the protein scaffold, which may allow the dAb CDRs to more easily reach the antigen, which may therefore bind with high affinity.

The surroundings in which immunoglobulin single variable domains are linked to the IgG will differ depending on which antibody chain they are fused to:

When fused at the C-terminal end of the antibody light chain of an IgG scaffold, each immunoglobulin single variable domain is expected to be located in the vicinity of the antibody hinge and the Fc portion. It is likely that such immunoglobulin single variable domains will be located far apart from each other. In conventional antibodies, the angle between Fab fragments and the angle between each Fab fragment and the Fc portion can vary quite significantly. It is likely that - with mAbdAbs - the angle between the Fab fragments will not be widely different, whilst some angular restrictions may be observed with the angle between each Fab fragment and the Fc portion.

When fused at the C-terminal end of the antibody heavy chain of an IgG scaffold, each immunoglobulin single variable domain is expected to be located in the vicinity of the $C_H3$ domains of the Fc portion. This is not expected to impact on the Fc binding properties to Fc receptors (e.g. FcyRI, II, III an FcRn) as these receptors engage with the $C_H2$ domains (for the FcyRI, II and III class of receptors) or with the hinge between the $C_H2$ and $C_H3$ domains (e.g. FcRn receptor). Another feature of such antigen-binding proteins is that both immunoglobulin single variable domains are expected to be spatially close to each other and provided that flexibility is provided by provision of appropriate linkers, these immunoglobulin single variable domains may even form homodimeric species, hence propagating the 'zipped' quaternary structure of the Fc portion, which may enhance stability of the construct.

Such structural considerations can aid in the choice of the most suitable position to link an epitope-binding domain, for example a dAb, on to a protein scaffold, for example an antibody.

The size of the antigen, its localization (in blood or on cell surface), its quaternary structure (monomeric or multimeric) can vary. Conventional antibodies are naturally designed to function as adaptor constructs due to the presence of the hinge region, wherein the orientation of the two antigen-binding sites at the tip of the Fab fragments can vary

widely and hence adapt to the molecular feature of the antigen and its surroundings. In contrast immunoglobulin single variable domains linked to an antibody or other protein scaffold, for example a protein scaffold which comprises an antibody with no hinge region, may have less structural flexibility either directly or indirectly.

Understanding the solution state and mode of binding at the immunoglobulin single variable domain is also helpful. Evidence has accumulated that *in vitro* dAbs can predominantly exist in monomeric, homo-dimeric or multimeric forms in solution (Reiter et al. (1999) J Mol Biol 290 p685-698; Ewert et al (2003) J Mol Biol 325, p531-553, Jespers et al (2004) J Mol Biol 337 p893-903; Jespers et al (2004) Nat Biotechnol 22 p1161-1165; Martin et al (1997) Protein Eng. 10 p607-614; Sepulvada et al (2003) J Mol Biol 333 p355-365). This is fairly reminiscent to multimerisation events observed *in vivo* with Ig domains such as Bence-Jones proteins (which are dimers of immunoglobulin light chains (Epp et al (1975) Biochemistry 14 p4943-4952; Huan et al (1994) Biochemistry 33 p14848-14857; Huang et al (1997) Mol immunol 34 p1291-1301) and amyloid fibers (James et al. (2007) J Mol Biol. 367:603-8).

For example, it may be desirable to link dabs that tend to dimerise in solution to the C-terminal end of the Fc portion in preference to the C-terminal end of the light chain as linking to the C-terminal end of the Fc will allow those dAbs to dimerise in the context of the antigen-binding protein of the invention.

The antigen-binding proteins of the present invention may comprise antigen-binding sites specific for a single antigen, or may have antigen-binding sites specific for two or more antigens, or for two or more epitopes on a single antigen, or there may be antigen-binding sites each of which is specific for a different epitope on the same or different antigens.

In particular, the antigen-binding proteins of the present invention may be useful in treating diseases associated with HGF and VEGF for example solid tumours believed to require angiogenesis or to be associated with elevated levels of HGF (HGF/ Met signaling) and/or VEGF. Such tumours include colon, breast, ovarian, lung (small cell or non small cell), prostate, pancreatic, renal, liver, gastric, head and neck, melanoma, sarcoma. Also included are primary and secondary (metastatic) brain tumours including, but not limited to gliomas (including epenymomas), meningiomas, oligodendromas, astrocytomas (low grade, anaplastic and glioblastoma multiforme), medulloblastomas, gangliomas, schwannnomas and chordomas. Other diseases associated with undesirable angiogenesis that are suitable for treatment with the antigen binding proteins of the present invention include age-related macular degeneration, diabetic retinopathy, RA and psoriasis.

The antigen-binding proteins of the present invention may be produced by transfection of a host cell with an expression vector comprising the coding sequence for the antigen-binding protein of the invention. An expression vector or recombinant plasmid is produced by placing these coding sequences for the antigen-binding protein in operative association with conventional regulatory control sequences capable of controlling the replication and expression in, and/or secretion from, a host cell. Regulatory sequences include promoter sequences, e.g., CMV promoter, and signal sequences which can be derived from other known antibodies. Similarly, a second expression vector can be produced having a DNA sequence which encodes a complementary antigen-binding protein light or heavy chain. In certain embodiments this second expression vector is identical to the first except insofar as the coding sequences and selectable markers are concerned, so to ensure as far as possible that each polypeptide chain is functionally expressed. Alternatively, the heavy and light chain coding sequences for the antigen-binding protein may reside on a single vector, for example in two expression cassettes in the same vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antigen-binding protein of the invention. The antigen-binding protein which includes the association of both the recombinant heavy chain and/or light chain is screened from culture by appropriate assay, such as ELISA or RIA. Similar conventional techniques may be employed to construct other antigen-binding proteins.

Suitable vectors for the cloning and subcloning steps employed in the methods and construction of the compositions of this invention may be selected by one of skill in the art. For example, the conventional pUC series of cloning vectors may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning. Thus, the selection of the cloning vector is not a limiting factor in this invention.

The expression vectors may also be characterized by genes suitable for amplifying expression of the heterologous DNA sequences, e.g., the mammalian dihydrofolate reductase gene (DHFR). Other vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression

may also be selected for this purpose.

The present invention also encompasses a cell line transfected with a recombinant plasmid containing the coding sequences of the antigen-binding proteins of the present invention. Host cells useful for the cloning and other manipulations of these cloning vectors are also conventional. However, cells from various strains of E. coli may be used for replication of the cloning vectors and other steps in the construction of antigen-binding proteins of this invention.

Suitable host cells or cell lines for the expression of the antigen-binding proteins of the invention include mammalian cells such as NS0, Sp2/0, CHO (e.g. DG44), COS, HEK, a fibroblast cell (e.g., 3T3), and myeloma cells, for example it may be expressed in a CHO or a myeloma cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., Sambrook et al., cited above.

Bacterial cells may prove useful as host cells suitable for the expression of the recombinant Fabs or other embodiments of the present invention (see, e.g., Plückthun, A., Immunol. Rev., 130:151-188 (1992)). However, due to the tendency of proteins expressed in bacterial cells to be in an unfolded or improperly folded form or in a non-glycosylated form, any recombinant Fab produced in a bacterial cell would have to be screened for retention of antigen binding ability. If the molecule expressed by the bacterial cell was produced in a properly folded form, that bacterial cell would be a desirable host, or in alternative embodiments the molecule may express in the bacterial host and then be subsequently re-folded. For example, various strains of E. coli used for expression are well-known as host cells in the field of biotechnology. Various strains of B. subtilis, Streptomyces, other bacilli and the like may also be employed in this method.

Where desired, strains of yeast cells known to those skilled in the art are also available as host cells, as well as insect cells, e.g. Drosophila and Lepidoptera and viral expression systems. See, e.g. Miller et al., Genetic Engineering, 8:277-298, Plenum Press (1986) and references cited therein.

The general methods by which the vectors may be constructed, the transfection methods required to produce the host cells of the invention, and culture methods necessary to produce the antigen-binding protein of the invention from such host cell may all be conventional techniques. Typically, the culture method of the present invention is a serum-free culture method, usually by culturing cells serum-free in suspension. Likewise, once produced, the antigen-binding proteins of the invention may be purified from the cell culture contents according to standard procedures of the art, including ammonium sulfate precipitation, affinity columns, column chromatography, gel electrophoresis and the like. Such techniques are within the skill of the art and do not limit this invention. For example, preparation of altered antibodies are described in WO 99/58679 and WO 96/16990.

Yet another method of expression of the antigen-binding proteins may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

In a further aspect of the invention there is provided a method of producing an antibody of the invention which method comprises the step of culturing a host cell transformed or transfected with a vector encoding the light and/or heavy chain of the antibody of the invention and recovering the antibody thereby produced.

In accordance with the present invention there is provided a method of producing an antigen-binding protein of the present invention which method comprises the steps of;

(a) providing a first vector encoding a heavy chain of the antigen-binding protein;
(b) providing a second vector encoding a light chain of the antigen-binding protein;
(c) transforming a mammalian host cell (e.g. CHO) with said first and second vectors;
(d) culturing the host cell of step (c) under conditions conducive to the secretion of the antigen-binding protein from said host cell into said culture media;
(e) recovering the secreted antigen-binding protein of step (d).

Once expressed by the desired method, the antigen-binding protein is then examined for in vitro activity by use of an appropriate assay. Presently conventional ELISA assay formats are employed to assess qualitative and quantitative binding of the antigen-binding protein to its target. Additionally, other in vitro assays may also be used to verify neutralizing efficacy prior to subsequent human clinical studies performed to evaluate the persistence of the antigen-binding protein in the body despite the usual clearance mechanisms.

The dose and duration of treatment relates to the relative duration of the molecules of the present invention in the human circulation, and can be adjusted by one of skill in the art depending upon the condition being treated and the general health of the patient. It is envisaged that repeated dosing (e.g. once a week or once every two weeks) over an extended time period (e.g. four to six months) maybe required to achieve maximal therapeutic efficacy.

The mode of administration of the therapeutic agent of the invention may be any suitable route which delivers the agent to the host. The antigen-binding proteins, and pharmaceutical compositions of the invention are particularly

useful for parenteral administration, i.e., subcutaneously (s.c.), intrathecally, intraperitoneally, intramuscularly (i.m.), intravenously (i.v.), or intranasally.

Therapeutic agents of the invention may be prepared as pharmaceutical compositions containing an effective amount of the antigen-binding protein of the invention as an active ingredient in a pharmaceutically acceptable carrier. In the prophylactic agent of the invention, an aqueous suspension or solution containing the antigen-binding protein, may be buffered at physiological pH, in a form ready for injection. The compositions for parenteral administration will commonly comprise a solution of the antigen-binding protein of the invention or a cocktail thereof dissolved in a pharmaceutically acceptable carrier, for example an aqueous carrier. A variety of aqueous carriers may be employed, e.g., 0.9% saline, 0.3% glycine, and the like. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen-binding protein of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 mL sterile buffered water, and between about 1 ng to about 200 mg, e.g. about 50 ng to about 30 mg, or about 5 mg to about 25 mg, of an antigen-binding protein of the invention. Similarly, a pharmaceutical composition of the invention for intravenous infusion could be made up to contain about 250 ml of sterile Ringer's solution, and about 1 to about 30 or about 5 mg to about 25 mg of an antigen-binding protein of the invention per ml of Ringer's solution. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antigen-binding protein formulations of the invention see Lasmar U and Parkins D "The formulation of Biopharmaceutical products", Pharma. Sci.Tech.today, page 129-137, Vol.3 (3rd April 2000), Wang, W "Instability, stabilisation and formulation of liquid protein pharmaceuticals", Int. J. Pharm 185 (1999) 129-188, Stability of Protein Pharmaceuticals Part A and B ed Ahern T.J., Manning M.C., New York, NY: Plenum Press (1992), Akers,M.J. "Excipient-Drug interactions in Parenteral Formulations", J.Pharm Sci 91 (2002) 2283-2300, Imamura, K et al "Effects of types of sugar on stabilization of Protein in the dried state", J Pharm Sci 92 (2003) 266-274,Izutsu, Kkojima, S. "Excipient crystalinity and its protein-structure-stabilizing effect during freeze-drying", J Pharm. Pharmacol, 54 (2002) 1033-1039, Johnson, R, "Mannitol-sucrose mixtures-versatile formulations for protein lyophilization", J. Pharm. Sci, 91 (2002) 914-922.

Ha,E Wang W, Wang Y.j. "Peroxide formation in polysorbate 80 and protein stability", J. Pharm Sci, 91, 2252-2264,(2002) the entire contents of which are incorporated herein by reference and to which the reader is specifically referred.

In one embodiment the therapeutic agent of the invention, when in a pharmaceutical preparation, is present in unit dose forms. The appropriate therapeutically effective dose will be determined readily by those of skill in the art. Suitable doses may be calculated for patients according to their weight, for example suitable doses may be in the range of 0.01 to 20mg/kg, for example 0.1 to 20mg/kg, for example 1 to 20mg/kg, for example 10 to 20mg/kg or for example 1 to 15mg/kg, for example 10 to 15mg/kg. To effectively treat conditions of use in the present invention in a human, suitable doses may be within the range of 0.01 to 1000 mg, for example 0.1 to 1000mg, for example 0.1 to 500mg, for example 500mg, for example 0.1 to 100mg, or 0.1 to 80mg, or 0.1 to 60mg, or 0.1 to 40mg, or for example 1 to 100mg, or 1 to 50mg, of an antigen-binding protein of this invention, which may be administered parenterally, for example subcutaneously, intravenously or intramuscularly. Such dose may, if necessary, be repeated at appropriate time intervals selected as appropriate by a physician.

The antigen-binding proteins described herein can be lyophilized for storage and reconstituted in a suitable carrier prior to use. This technique has been shown to be effective with conventional immunoglobulins and art-known lyophilization and reconstitution techniques can be employed.

There are several methods known in the art which can be used to find epitope-binding domains of use in the present invention.

The term "library" refers to a mixture of heterogeneous polypeptides or nucleic acids. The library is composed of members, each of which has a single polypeptide or nucleic acid sequence. To this extent, "library" is synonymous with "repertoire." Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. In one example, each individual organism or cell contains only one or a limited number of library members. Advantageously, the nucleic acids are incorporated into expression vectors, in order to allow expression of the polypeptides encoded by the nucleic acids. In a one aspect, therefore, a library may take the form of a population of host organisms, each organism containing one or more copies of an expression vector containing a single member of the library in nucleic acid form

which can be expressed to produce its corresponding polypeptide member. Thus, the population of host organisms has the potential to encode a large repertoire of diverse polypeptides.

A "universal framework" is a single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. There may be a single framework, or a set of such frameworks, which has been found to permit the derivation of virtually any binding specificity though variation in the hypervariable regions alone.

Amino acid and nucleotide sequence alignments and homology, similarity or identity, as defined herein are in one embodiment prepared and determined using the algorithm BLAST 2 Sequences, using default parameters (Tatusova, T. A. et al., FEMS Microbiol Lett, 174:187-188 (1999)).

When a display system (*e.g.*, a display system that links coding function of a nucleic acid and functional characteristics of the peptide or polypeptide encoded by the nucleic acid) is used in the methods described herein, eg in the selection of a dAb or other epitope binding domain, it is frequently advantageous to amplify or increase the copy number of the nucleic acids that encode the selected peptides or polypeptides. This provides an efficient way of obtaining sufficient quantities of nucleic acids and/or peptides or polypeptides for additional rounds of selection, using the methods described herein or other suitable methods, or for preparing additional repertoires (*e.g.*, affinity maturation repertoires). Thus, in some embodiments, the methods of selecting epitope binding domains comprises using a display system (*e.g.*, that links coding function of a nucleic acid and functional characteristics of the peptide or polypeptide encoded by the nucleic acid, such as phage display) and further comprises amplifying or increasing the copy number of a nucleic acid that encodes a selected peptide or polypeptide. Nucleic acids can be amplified using any suitable methods, such as by phage amplification, cell growth or polymerase chain reaction.

In one example, the methods employ a display system that links the coding function of a nucleic acid and physical, chemical and/or functional characteristics of the polypeptide encoded by the nucleic acid. Such a display system can comprise a plurality of replicable genetic packages, such as bacteriophage or cells (bacteria). The display system may comprise a library, such as a bacteriophage display library. Bacteriophage display is an example of a display system.

A number of suitable bacteriophage display systems (*e.g.*, monovalent display and multivalent display systems) have been described. (See, *e.g.*, Griffiths etal., U.S. Patent No. 6,555,313 B1 (incorporated herein by reference); Johnson et al., U.S. Patent No. 5,733,743 (incorporated herein by reference); McCafferty et al., U.S. Patent No. 5,969,108 (incorporated herein by reference); Mulligan-Kehoe, U.S. Patent No. 5,702,892 (Incorporated herein by reference); Winter, G. et al., Annu. Rev. Immunol. 12:433-455 (1994); Soumillion, P. et al., Appl. Biochem. Biotechnol. 47(2-3):175-189 (1994); Castagnoli, L. etal., Comb. Chem. High Throughput Screen, 4(2):121-133 (2001).) The peptides or polypeptides displayed in a bacteriophage display system can be displayed on any suitable bacteriophage, such as a filamentous phage (*e.g.*, fd, M13, F1), a lytic phage (*e.g.*, T4, T7, lambda), or an RNA phage (*e.g.*, MS2), for example.

Generally, a library of phage that displays a repertoire of peptides or phagepolypeptides, as fusion proteins with a suitable phage coat protein (*e.g.*, fd pIII protein), is produced or provided. The fusion protein can display the peptides or polypeptides at the tip of the phage coat protein, or if desired at an internal position. For example, the displayed peptide or polypeptide can be present at a position that is amino-terminal to domain 1 of pIII. (Domain 1 of pIII is also referred to as N1.) The displayed polypeptide can be directly fused to pIII (*e.g.*, the N-terminus of domain 1 of pIII) or fused to pIII using a linker. If desired, the fusion can further comprise a tag (*e.g.*, myc epitope, His tag). Libraries that comprise a repertoire of peptides or polypeptides that are displayed as fusion proteins with a phage coat protein,can be produced using any suitable methods, such as by introducing a library of phage vectors or phagemid vectors encoding the displayed peptides or polypeptides into suitable host bacteria, and culturing the resulting bacteria to produce phage (*e.g.*, using a suitable helper phage or complementing plasmid if desired). The library of phage can be recovered from the culture using any suitable method, such as precipitation and centrifugation.

The display system can comprise a repertoire of peptides or polypeptides that contains any desired amount of diversity. For example, the repertoire can contain peptides or polypeptides that have amino acid sequences that correspond to naturally occurring polypeptides expressed by an organism, group of organisms, desired tissue or desired cell type, or can contain peptides or polypeptides that have random or randomized amino acid sequences. If desired, the polypeptides can share a common core or scaffold. For example, all polypeptides in the repertoire or library can be based on a scaffold selected from protein A, protein L, protein G, a fibronectin domain, an anticalin, CTLA4, a desired enzyme (e.g., a polymerase, a cellulase), or a polypeptide from the immunoglobulin superfamily, such as an antibody or antibody fragment (*e.g.*, an antibody variable domain). The polypeptides in such a repertoire or library can comprise defined regions of random or randomized amino acid sequence and regions of common amino acid sequence. In certain embodiments, all or substantially all polypeptides in a repertoire are of a desired type, such as a desired enzyme (*e.g.*, a polymerase) or a desired antigen-binding fragment of an antibody (*e.g.*, human $V_H$ or human $V_L$). In some embodiments, the polypeptide display system comprises a repertoire of polypeptides wherein each polypeptide comprises an antibody variable domain. For example, each polypeptide in the repertoire can contain a $V_H$, a $V_L$ or an Fv (*e.g.*, a single chain Fv).

Amino acid sequence diversity can be introduced into any desired region of a peptide or polypeptide or scaffold using any suitable method. For example, amino acid sequence diversity can be introduced into a target region, such as a complementarity determining region of an antibody variable domain or a hydrophobic domain, by preparing a library of nucleic acids that encode the diversified polypeptides using any suitable mutagenesis methods (*e.g.*, low fidelity PCR, oligonucleotide-mediated or site directed mutagenesis, diversification using NNK codons) or any other suitable method. If desired, a region of a polypeptide to be diversified can be randomized.

The size of the polypeptides that make up the repertoire is largely a matter of choice and uniform polypeptide size is not required. The polypeptides in the repertoire may have at least tertiary structure (form at least one domain).

Selection/Isolation/Recovery

An epitope binding domain or population of domains can be selected, isolated and/or recovered from a repertoire or library (*e.g.*, in a display system) using any suitable method. For example, a domain is selected or isolated based on a selectable characteristic (*e.g.*, physical characteristic, chemical characteristic, functional characteristic). Suitable selectable functional characteristics include biological activities of the peptides or polypeptides in the repertoire, for example, binding to a generic ligand (*e.g.*, a superantigen), binding to a target ligand (*e.g.*, an antigen, an epitope, a substrate), binding to an antibody (*e.g.*, through an epitope expressed on a peptide or polypeptide), and catalytic activity. (See, *e.g.*, Tomlinson et al., WO 99/20749; WO 01/57065; WO 99/58655.)

In some embodiments, the protease resistant peptide or polypeptide is selected and/or isolated from a library or repertoire of peptides or polypeptides in which substantially all domains share a common selectable feature. For example, the domain can be selected from a library or repertoire in which substantially all domains bind a common generic ligand, bind a common target ligand, bind (or are bound by) a common antibody, or possess a common catalytic activity. This type of selection is particularly useful for preparing a repertoire of domains that are based on a parental peptide or polypeptide that has a desired biological activity, for example, when performing affinity maturation of an immunoglobulin single variable domain.

Selection based on binding to a common generic ligand can yield a collection or population of domains that contain all or substantially all of the domains that were components of the original library or repertoire. For example, domains that bind a target ligand or a generic ligand, such as protein A, protein L or an antibody, can be selected, isolated and/or recovered by panning or using a suitable affinity matrix. Panning can be accomplished by adding a solution of ligand (*e.g.*, generic ligand, target ligand) to a suitable vessel (*e.g.*, tube, petri dish) and allowing the ligand to become deposited or coated onto the walls of the vessel. Excess ligand can be washed away and domains can be added to the vessel and the vessel maintained under conditions suitable for peptides or polypeptides to bind the immobilized ligand. Unbound domains can be washed away and bound domains can be recovered using any suitable method, such as scraping or lowering the pH, for example.

Suitable ligand affinity matrices generally contain a solid support or bead (*e.g.*, agarose) to which a ligand is covalently or noncovalently attached. The affinity matrix can be combined with peptides or polypeptides (*e.g.*, a repertoire that has been incubated with protease) using a batch process, a column process or any other suitable process under conditions suitable for binding of domains to the ligand on the matrix. domains that do not bind the affinity matrix can be washed away and bound domains can be eluted and recovered using any suitable method, such as elution with a lower pH buffer, with a mild denaturing agent (*e.g.*, urea), or with a peptide or domain that competes for binding to the ligand. In one example, a biotinylated target ligand is combined with a repertoire under conditions suitable for domains in the repertoire to bind the target ligand. Bound domains are recovered using immobilized avidin or streptavidin (*e.g.*, on a bead).

In some embodiments, the generic or target ligand is an antibody or antigen binding fragment thereof. Antibodies or antigen binding fragments that bind structural features of peptides or polypeptides that are substantially conserved in the peptides or polypeptides of a library or repertoire are particularly useful as generic ligands. Antibodies and antigen binding fragments suitable for use as ligands for isolating, selecting and/or recovering protease resistant peptides or polypeptides can be monoclonal or polyclonal and can be prepared using any suitable method.

Libraries/Repertoires

Libraries that encode and/or contain protease epitope binding domains can be prepared or obtained using any suitable method. A library can be designed to encode domains based on a domain or scaffold of interest (e.g., a domain selected from a library) or can be selected from another library using the methods described herein. For example, a library enriched in domains can be prepared using a suitable polypeptide display system.

Libraries that encode a repertoire of a desired type of domain can readily be produced using any suitable method. For example, a nucleic acid sequence that encodes a desired type of polypeptide (*e.g.*, an immunoglobulin variable domain) can be obtained and a collection of nucleic acids that each contain one or more mutations can be

prepared, for example by amplifying the nucleic acid using an error-prone polymerase chain reaction (PCR) system, by chemical mutagenesis (Deng et al., J. Biol. Chem., 269:9533 (1994)) or using bacterial mutator strains (Low et al., J. Mol. Biol., 260:359 (1996)).

In other embodiments, particular regions of the nucleic acid can be targeted for diversification. Methods for mutating selected positions are also well known in the art and include, for example, the use of mismatched oligonucleotides or degenerate oligonucleotides, with or without the use of PCR. For example, synthetic antibody libraries have been created by targeting mutations to the antigen binding loops. Random or semi-random antibody H3 and L3 regions have been appended to germline immunoblulin V gene segments to produce large libraries with unmutated framework regions (Hoogenboom and Winter (1992) supra; Nissim *et al.* (1994) supra; Griffiths *et al.* (1994) supra; DeKruif *et al.* (1995) supra). Such diversification has been extended to include some or all of the other antigen binding loops (Crameri et al. (1996) Nature Med., 2:100; Riechmann et al. (1995) Bio/Technology, 13:475; Morphosys, WO 97/08320, supra). In other embodiments, particular regions of the nucleic acid can be targeted for diversification by, for example, a two-step PCR strategy employing the product of the first PCR as a "mega-primer." (See, *e.g.,* Landt, O. et al., Gene 96:125-128 (1990).) Targeted diversification can also be accomplished, for example, by SOE PCR. (See, *e.g.*, Horton, R.M. et al., Gene 77:61-68 (1989).)

Sequence diversity at selected positions can be achieved by altering the coding sequence which specifies the sequence of the polypeptide such that a number of possible amino acids (*e.g.*, all 20 or a subset thereof) can be incorporated at that position. Using the IUPAC nomenclature, the most versatile codon is NNK, which encodes all amino acids as well as the TAG stop codon. The NNK codon may be used in order to introduce the required diversity. Other codons which achieve the same ends are also of use, including the NNN codon, which leads to the production of the additional stop codons TGA and TAA. Such a targeted approach can allow the full sequence space in a target area to be explored.

Some libraries comprise domains that are members of the immunoglobulin superfamily (*e.g.*, antibodies or portions thereof). For example the libraries can comprise domains that have a known main-chain conformation. (See, *e.g.*, Tomlinson et al., WO 99/20749.) Libraries can be prepared in a suitable plasmid or vector. As used herein, vector refers to a discrete element that is used to introduce heterologous DNA into cells for the expression and/or replication thereof. Any suitable vector can be used, including plasmids (*e.g.*, bacterial plasmids), viral or bacteriophage vectors, artificial chromosomes and episomal vectors. Such vectors may be used for simple cloning and mutagenesis, or an expression vector can be used to drive expression of the library. Vectors and plasmids usually contain one or more cloning sites (*e.g.*, a polylinker), an origin of replication and at least one selectable marker gene. Expression vectors can further contain elements to drive transcription and translation of a polypeptide, such as an enhancer element, promoter, transcription termination signal, signal sequences, and the like. These elements can be arranged in such a way as to be operably linked to a cloned insert encoding a polypeptide, such that the polypeptide is expressed and produced when such an expression vector is maintained under conditions suitable for expression (*e.g.*, in a suitable host cell).

Cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (*e.g.* SV40, adenovirus) are useful for cloning vectors in mammalian cells. Generally, the origin of replication is not needed for mammalian expression vectors, unless these are used in mammalian cells able to replicate high levels of DNA, such as COS cells.

Cloning or expression vectors can contain a selection gene also referred to as selectable marker. Such marker genes encode a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, *e.g.* ampicillin, neomycin, methotrexate or tetracycline, complement auxotrophic deficiencies, or supply critical nutrients not available in the growth media. Suitable expression vectors can contain a number of components, for example, an origin of replication, a selectable marker gene, one or more expression control elements, such as a transcription control element (*e.g.*, promoter, enhancer, terminator) and/or one or more translation signals, a signal sequence or leader sequence, and the like. Expression control elements and a signal or leader sequence, if present, can be provided by the vector or other source. For example, the transcriptional and/or translational control sequences of a cloned nucleic acid encoding an antibody chain can be used to direct expression.

A promoter can be provided for expression in a desired host cell. Promoters can be constitutive or inducible. For example, a promoter can be operably linked to a nucleic acid encoding an antibody, antibody chain or portion thereof, such that it directs transcription of the nucleic acid. A variety of suitable promoters for procaryotic (*e.g.*, the β-lactamase and lactose promoter systems, alkaline phosphatase, the tryptophan (trp) promoter system, lac, tac, T3, T7 promoters

for *E. coli*) and eucaryotic (*e.g.*, simian virus 40 early or late promoter, Rous sarcoma virus long terminal repeat promoter, cytomegalovirus promoter, adenovirus late promoter, EG-1a promoter) hosts are available.

In addition, expression vectors typically comprise a selectable marker for selection of host cells carrying the vector, and, in the case of a replicable expression vector, an origin of replication. Genes encoding products which confer antibiotic or drug resistance are common selectable markers and may be used in procaryotic (*e.g.*, β-lactamase gene (ampicillin resistance), *Tet* gene for tetracycline resistance) and eucaryotic cells (*e.g.*, neomycin (G418 or geneticin), gpt (mycophenolic acid), ampicillin, or hygromycin resistance genes). Dihydrofolate reductase marker genes permit selection with methotrexate in a variety of hosts. Genes encoding the gene product of auxotrophic markers of the host (*e.g.*, *LEU2, URA3, HIS3*) are often used as selectable markers in yeast. Use of viral (*e.g.*, baculovirus) or phage vectors, and vectors which are capable of integrating into the genome of the host cell, such as retroviral vectors, are also contemplated.

Suitable expression vectors for expression in prokaryotic (*e.g.*, bacterial cells such as *E. coli*) or mammalian cells include, for example, a pET vector (*e.g.*, pET-12a, pET-36, pET-37, pET-39, pET-40, Novagen and others), a phage vector (*e.g.*, pCANTAB 5 E, Pharmacia), pRIT2T (Protein A fusion vector, Pharmacia), pCDM8, pCDNA1.1/amp, pcDNA3.1, pRc/RSV, pEF-1 (Invitrogen, Carlsbad, CA), pCMV-SCRIPT, pFB, pSG5, pXT1 (Stratagene, La Jolla, CA), pCDEF3 (Goldman, L.A., et al., Biotechniques, 21:1013-1015 (1996)), pSVSPORT (GibcoBRL, Rockville, MD), pEF-Bos (Mizushima, S., et al., Nucleic Acids Res., 18:5322 (1990)) and the like. Expression vectors which are suitable for use in various expression hosts, such as prokaryotic cells (*E. coli*), insect cells *(Drosophila* Schnieder S2 cells, Sf9), yeast (*P. methanolica, P. pastoris, S. cerevisiae*) and mammalian cells (eg, COS cells) are available.

Some examples of vectors are expression vectors that enable the expression of a nucleotide sequence corresponding to a polypeptide library member. Thus, selection with generic and/or target ligands can be performed by separate propagation and expression of a single clone expressing the polypeptide library member. As described above, a particular selection display system is bacteriophage display. Thus, phage or phagemid vectors may be used, for example vectors may be phagemid vectors which have an *E. coli.* origin of replication (for double stranded replication) and also a phage origin of replication (for production of single-stranded DNA). The manipulation and expression of such vectors is well known in the art (Hoogenboom and Winter (1992) supra; Nissim *et al*. (1994) supra). Briefly, the vector can contain a β-lactamase gene to confer selectivity on the phagemid and a lac promoter upstream of an expression cassette that can contain a suitable leader sequence, a multiple cloning site, one or more peptide tags, one or more TAG stop codons and the phage protein pIII. Thus, using various suppressor and non-suppressor strains of *E. coli* and with the addition of glucose, iso-propyl thio-β-D-galactoside (IPTG) or a helper phage, such as VCS M13, the vector is able to replicate as a plasmid with no expression, produce large quantities of the polypeptide library member only or product phage, some of which contain at least one copy of the polypeptide-pIII fusion on their surface.

Antibody variable domains may comprise a target ligand binding site and/or a generic ligand binding site. In certain embodiments, the generic ligand binding site is a binding site for a superantigen, such as protein A, protein L or protein G. The variable domains can be based on any desired variable domain, for example a human VH (*e.g.*, $V_H$ 1a, $V_H$ 1b, $V_H$ 2, $V_H$ 3, $V_H$ 4, $V_H$ 5, $V_H$ 6), a human VL (*e.g.*, VLI, VLII, VLIII, VLIV, VLV, VLVI or VK1) or a human VK (*e.g.*, VK2, VK3, VK4, VK5, VK6, VK7, VK8, VK9 or VK10).

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

Characterisation of the epitope binding domains.

The binding of a domain to its specific antigen or epitope can be tested by methods which will be familiar to those skilled in the art and include ELISA. In one example, binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein.

The diversity of the selected phage monoclonal antibodies may also be assessed by gel electrophoresis of

PCR products (Marks *etal.* 1991, *supra*; Nissim *etal.* 1994 *supra),* probing (Tomlinson etal., 1992) J. Mol. Biol. 227, 776) or by sequencing of the vector DNA.

Structure of dAbs

In the case that the dAbs are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence may be located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

Scaffolds for use in Constructing dAbs

i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBOJ., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin etal. (1996) J. Mol. Biol., 263: 800; Shirai etal. (1996) FEBS Letters, 399: 1).

The dAbs are advantageously assembled from libraries of domains, such as libraries of $V_H$ domains and/or libraries of $V_L$ domains. In one aspect, libraries of domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human $V_K$ domain, the L1 loop can adopt one of four canonical structures, the L2 loop has a single canonical structure and that 90% of human $V_K$ domains adopt one of four or five canonical structures for the L3 loop (Tomlinson *etal.* (1995) supra); thus, in the $V_K$ domain alone, different canonical structures can combine to create a range of different main-chain conformations. Given that the V□ domain encodes a different range of canonical structures for the L1, L2 and L3 loops and that $V_K$ and VD domains can pair with any $V_H$ domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a one particular aspect, the dAbs possess a single known main-chain conformation.

The single main-chain conformation that is chosen may be commonplace among molecules of the immunoglobulin superfamily type in question. A conformation is commonplace when a significant number of naturally occurring molecules are observed to adopt it. Accordingly, in one aspect, the natural occurrence of the different main-chain conformations for each binding loop of an immunoglobulin domain are considered separately and then a naturally occurring variable domain is chosen which possesses the desired <u>combination</u> of main-chain conformations for the

different loops. If none is available, the nearest equivalent may be chosen. The desired combination of main-chain conformations for the different loops may be created by selecting germline gene segments which encode the desired main-chain conformations. In one example, the selected germline gene segments are frequently expressed in nature, and in particular they may be the most frequently expressed of all natural germline gene segments.

In designing libraries the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of $V_κ$(39%), L2 - CS 1 (100%), L3 - CS 1 of $V_κ$(36%) (calculation assumes a □:□ ratio of 70:30, Hood et al. (1967) Cold Spring Harbor Symp. Quant. Biol., 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the $V_H$ segment 3-23 (DP-47), the $J_H$ segment JH4b, the $V_□$ segment O2/O12 (DPK9) and the $J_□$ segment $J_□$1. $V_H$ segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five, or for all six of the antigen binding loops can be determined. Here, the chosen conformation may be commonplace in naturally occurring antibodies and may be observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

Diversification of the canonical sequence

Having selected several known main-chain conformations or a single known main-chain conformation, dAbs can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or they may be selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) . Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas etal. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas etal. (1992) Proc. Natl. Acad. Sci. USA, 89: 4457; Nissim etal. (1994) EMBO J., 13: 692; Griffiths et al. (1994) EMBO J., 13: 3245; De Kruif etal. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than $10^{15}$ structures for H3 alone and a similarly large number of variants for the other five loops, it is not feasible using current transformation technology or

even by using cell free systems to produce a library representing all possible combinations. For example, in one of the largest libraries constructed to date, 6 x $10^{10}$ different antibodies, which is only a fraction of the potential diversity for a library of this design, were generated (Griffiths *etal.* (1994) supra).

In a one embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

In one aspect, libraries of dAbs are used in which only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library.. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" or "dummy" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

It will be understood that the sequences described herein include sequences which are substantially identical, for example sequences which are at least 90% identical, for example which are at least 91%, or at least 92%, or at least 93%, or at least 94% or at least 95%, or at least 96%, or at least 97% or at least 98%, or at least 99% identical to the sequences described herein.

For nucleic acids, the term "substantial identity" indicates that two nucleic acids, or designated sequences thereof, when optimally aligned and compared, are identical, with appropriate nucleotide insertions or deletions, in at least about 80% of the nucleotides, usually at least about 90% to 95%, or at least about 98% to 99.5% of the nucleotides. Alternatively, substantial identity exists when the segments will hybridize under selective hybridization conditions, to the complement of the strand. For nucleotide and amino acid sequences, the term "identical" indicates the degree of identity between two nucleic acid or amino acid sequences when optimally aligned and compared with appropriate insertions or deletions. Alternatively, substantial identity exists when the DNA segments will hybridize under selective hybridization conditions, to the complement of the strand.

The percent identity between two sequences is a function of the number of identical positions shared by the sequences (i.e., % identity = # of identical positions/total # of positions times 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two nucleotide sequences can be determined using the GAP program in the GCG software package, using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

By way of example, a polypeptide sequence of the present invention may be identical to the reference sequence

encoded by SEQ ID NO: 38, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the polypeptide sequence encoded by SEQ ID NO: 38 by the numerical percent of the respective percent identity (divided by 100) and then subtracting that product from said total number of amino acids in the polypeptide sequence encoded by SEQ ID NO: 38, or:

$$na \leq xa - (xa \bullet y),$$

wherein na is the number of amino acid alterations, xa is the total number of amino acids in the polypeptide sequence encoded by SEQ ID NO: 38, and y is, for instance 0.70 for 70%, 0.80 for 80%, 0.85 for 85% etc., and wherein any non-integer product of xa and y is rounded down to the nearest integer prior to subtracting it from xa.

By the term "treating" and grammatical variations thereof as used herein, is meant therapeutic therapy. In reference to a particular condition, treating means: (1) to ameliorate or prevent the condition of one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy is also contemplated thereby. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As is understood in the art, the terms "complete remission," "complete response" and "complete regression" mean the disappearance of all detectable signs and/or symptoms of cancer in response to treatment. As is also understood in the art detectable signs or symptoms of cancer can be defined based on the type and stage of cancer being treated. By way of example, "complete response" to treatment in a subject suffering from hepatocellular carcinoma could be defined as no visible liver tumors observed with X-ray or CT scan. In some instances, clinical response can be defined by RECIST 1.0 criteria (Therasse P, Arbuck SG, Eisenhauer EA, Wanders J, Kaplan RS, Rubinstein L, et al. New guidelines to evaluate the response to treatment in solid tumors. European Organization for Research and Treatment of Cancer, National Cancer Institute of the United States, National Cancer Institute of Canada. J Natl Cancer Inst. 2000;92:205-16.) as described below:

Suitably, the present invention relates to a method for treating or lessening the severity of a cancer selected from: brain (gliomas), glioblastomas, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, including hepatocellucler carcinoma, melanoma, ovarian, pancreatic, prostate, sarcoma, osteosarcoma, giant cell tumor of bone, thyroid, Lymphoblastic T cell leukemia, Chronic myelogenous leukemia, Chronic lymphocytic leukemia, Hairy-cell leukemia, acute lymphoblastic leukemia, acute myelogenous leukemia, Chronic neutrophilic leukemia, Acute lymphoblastic T cell leukemia, Plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma, neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Suitably, the present invention relates to a method for treating or lessening the severity of a cancer selected from: brain (gliomas), glioblastomas, Bannayan-Zonana syndrome, Cowden disease, Lhermitte-Duclos disease, breast, colon, head and neck, kidney, lung, liver, melanoma, ovarian, pancreatic, prostate, sarcoma and thyroid.

"Cancer" refers to cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma;

Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinorna, glucagonoma, gastrinoma, carcinoid tumors, vipoma), small bowel (adenocarcinorna, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defomians), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, SertoliLeydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal glands: neuroblastoma.

As used herein, the terms "cancer," "neoplasm," and "tumor," are used interchangeably and in either the singular or plural form, refer to cells that have undergone a malignant transformation that makes them pathological to the host organism. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells. When referring to a type of cancer that normally manifests as a solid tumor, a "clinically detectable" tumor is one that is detectable on the basis of tumor mass; e.g., by procedures such as CAT scan, MR imaging, X-ray, ultrasound or palpation, and/or which is detectable because of the expression of one or more cancer-specific antigens in a sample obtainable from a patient. Tumors may be solid tumors such as heptocellular carcinoma (HCC) lesions. Tumors may be hematopoietic tumor, for example, tumors of blood cells or the like, meaning liquid tumors.

As used herein "overexpressed" and "overexpression" of a protein or polypeptide and grammatical variations thereof means that a given cell produces an increased number of a certain protein relative to a normal cell. By way of example, a c-Met protein may be overexpressed by a tumor cell relative to a non-tumor cell. Additionally, a mutant c-Met protein may be overexpressed compared to wild type c-Met protein in a cell. As is understood in the art, expression levels of a polypeptide in a cell can be normalized to a housekeeping gene such as actin. In some instances, a certain polypeptide may be underexpressed in a tumor cell compared with a non-tumor cell.

As used herein the term "amplification" and grammatical variations thereof refers to the presence of one or more extra gene copies in a chromosome complement. In certain embodiments a gene encoding a c-Met protein may be amplified in a cell. Amplification of the HER2 gene has been correlated with certain types of cancer. Amplification of the HER2 gene has been found in human salivary gland and gastric tumor-derived cell lines, gastric and colon adenocarcinomas, and mammary gland adenocarcinomas. Semba et al., Proc. Natl. Acad. Sci. USA, 82:6497-6501 (1985); Yokota et al., Oncogene, 2:283-287 (1988); Zhou et al., Cancer Res., 47:6123-6125 (1987); King et al., Science, 229:974-976 (1985); Kraus et al., EMBO J., 6:605-610 (1987); van de Vijver et al., Mol. Cell. Biol., 7:2019-2023 (1987); Yamamoto et al., Nature, 319:230-234 (1986).

Typically, any anti-neoplastic agent that has activity versus a susceptible tumor being treated may be co-administered in the treatment of cancer in the present invention. Examples of such agents can be found in Cancer Principles and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins;

topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; receptor tyrosine kinase inhibitors; serine-threonine kinase inhibitors; non-receptor tyrosine kinase inhibitors; angiogenesis inhibitors, immunotherapeutic agents; proapoptotic agents; and cell cycle signalling inhibitors.

The present invention also provides methods for treating cancer comprising administering at least one antigen binding protein of the present invention (herein referred to as Compound A) or pharmaceutically acceptable salt thereof with or without another anti-neoplastic agent (Compound B).

By the term "specified period" and grammatical variations thereof, as used herein is meant the interval of time between the administration of one of Compound A2 and Compound B2 and the other of Compound A2 and Compound B2. Unless otherwise defined, the specified period can include simultaneous administration. Unless otherwise defined the specified period refers to administration of Compound A2 and Compound B2 during a single day.

By the term "duration of time" and grammatical variations thereof, as used herein is meant a compound of the invention is administered for an indicated number of consecutive days. Unless otherwise defined, the number of consecutive days does not have to commence with the start of treatment or terminate with the end of treatment, it is only required that the number of consecutive days occur at some point during the course of treatment.

Examples of a further active ingredient or ingredients (anti-neoplastic agent) for use in combination or co-administered with Compound A or pharmaceutically acceptable salt thereof are chemotherapeutic agents.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti-cancer agents that operate at the G2/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β,13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree Taxus brevifolia and is commercially available as an injectable solution TAXOL®. It is a member of the taxane family of terpenes. It was first isolated in 1971 by Wani et al. J. Am. Chem, Soc., 93:2325. 1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77:1561-1565 (1980); Schiff et al., Nature, 277:665-667 (1979); Kumar, J. Biol, Chem, 256: 10435-10441 (1981). For a review of synthesis and anticancer activity of some paclitaxel derivatives see: D. G. I. Kingston et al., Studies in Organic Chemistry vol. 26, entitled "New trends in Natural Products Chemistry 1986", Attaur-Rahman, P.W. Le Quesne, Eds. (Elsevier, Amsterdam, 1986) pp 219-235.

Paclitaxel has been approved for clinical use in the treatment of refractory ovarian cancer in the United States (Markman et al., Yale Journal of Biology and Medicine, 64:583, 1991; McGuire et al., Ann. Intem, Med., 111:273,1989) and for the treatment of breast cancer (Holmes et al., J. Nat. Cancer Inst., 83:1797,1991.) It is a potential candidate for treatment of neoplasms in the skin (Einzig et. al., Proc. Am. Soc. Clin. Oncol., 20:46) and head and neck carcinomas (Forastire et. al., Sem. Oncol., 20:56, 1990). The compound also shows potential for the treatment of polycystic kidney disease (Woo et. al., Nature, 368:750. 1994), lung cancer and malaria. Treatment of patients with paclitaxel results in bone marrow suppression (multiple cell lineages, Ignoff, R.J. et. al, Cancer Chemotherapy Pocket Guide, 1998) related to the duration of dosing above a threshold concentration (50nM) (Kearns, C.M. et. al., Seminars in Oncology, 3(6) p.16-23, 1995).

Docetaxel, (2R,3S)- N-carboxy-3-phenylisoserine,N-tert-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel q.v., prepared using a natural precursor, 10-deacetyl-baccatin III, extracted from the needle of the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine, Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and

neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo, aquation and form intra-and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as PLATINOL® as an injectable solution. Cisplatin is primarily indicated in the treatment of metastatic testicular and ovarian cancer and advanced bladder cancer. The primary dose limiting side effects of cisplatin are nephrotoxicity, which may be controlled by hydration and diuresis, and ototoxicity.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced ovarian carcinoma. Bone marrow suppression is the dose limiting toxicity of carboplatin.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias. Alopecia, nausea, vomiting and leukopenia are the most common dose limiting side effects of cyclophosphamide.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease. Bone marrow suppression is the most common dose limiting side effect of chlorambucil.

Busulfan, 1,4-butanediol dimethanesulfonate, is commercially available as MYLERAN® TABLETS. Busulfan is indicated for the palliative treatment of chronic myelogenous leukemia. Bone marrow suppression is the most common dose limiting side effects of busulfan.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease. Nausea, vomiting, and anorexia are the most common dose limiting side effects of dacarbazine.

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthracyclins such as daunorubicin and doxorubicin; and bleomycins.

Dactinomycin, also know as Actinomycin D, is commercially available in injectable form as COSMEGEN®. Dactinomycin is indicated for the treatment of Wilm's tumor and rhabdomyosarcoma. Nausea, vomiting, and anorexia are the most common dose limiting side effects of dactinomycin.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma. Myelosuppression

is the most common dose limiting side effect of daunorubicin.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas. Myelosuppression is the most common dose limiting side effect of doxorubicin.

Bleomycin, a mixture of cytotoxic glycopeptide antibiotics isolated from a strain of Streptomyces verticillus, is commercially available as BLENOXANE®. Bleomycin is indicated as a palliative treatment, as a single agent or in combination with other agents, of squamous cell carcinoma, lymphomas, and testicular carcinomas. Pulmonary and cutaneous toxicities are the most common dose limiting side effects of bleomycin.

Topoisomerase II inhibitors include, but are not limited to, epipodophyllotoxins. Epipodophyllotoxins are phase specific anti-neoplastic agents derived from the mandrake plant. Epipodophyllotoxins typically affect cells in the S and G2 phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as a single agent or in combination with other chemotherapy agents in the treatment of testicular and non-small cell lung cancers. Myelosuppression is the most common side effect of etoposide. The incidence of leucopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON® and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leucopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine. 5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2(1H)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leucopenia, thrombocytopenia, and mucositis.

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppression and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID®. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leucopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Gemcitabine, 2'-deoxy-2', 2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer. Myelosuppression, including leucopenia, thrombocytopenia, and anemia, is the most common dose limiting side effect of gemcitabine administration.

Methotrexate, N-[4[[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA

synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder. Myelosuppression (leucopenia, thrombocytopenia, and anemia) and mucositis are expected side effect of methotrexate administration.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCl, (4S)-4,11-diethyl-4-hydroxy-9-[(4-piperidinopiperidino)carbonyloxy]-1H-pyrano[3',4',6,7]indolizino[1,2,-b]quinoline-3,14(4H,12H)-dione hydrochloride, is commercially available as the injectable solution CAMPTOSAR®.

Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA : irintecan or SN-38 ternary complex with replication enzymes. Irinotecan is indicated for treatment of metastatic cancer of the colon or rectum. The dose limiting side effects of irinotecan HCl are myelosuppression, including neutropenia, and GI effects, including diarrhea.

Topotecan HCl, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer. The dose limiting side effect of topotecan HCl is myelosuppression, primarily neutropenia.

Pazopanib which commercially available as VOTRIENT® is a tyrosine kinase inhibitor (TKI). Pazopanib is presented as the hydrochloride salt, with the chemical name 5-[[4-[(2;3-dimethyl-2H-indazol-6-yl)methylamino]-2-pyrimidinyl]amino]-2-methylbenzenesulfonamide monohydrochloride. Pazoponib is approved for treatment of patients with advanced renal cell carcinoma.

Rituximab is a chimeric monoclonal antibody which is sold as RITUXAN® and MABTHERA®. Rituximab binds to CD20 on B cells and causes cell apoptosis. Rituximab is administered intravenously and is approved for treatment of rheumatoid arthritis and B-cell non-Hodgkin's lymphoma.

Ofatumumab is a fully human monoclonal antibody which is sold as ARZERRA®. Ofatumumab binds to CD20 on B cells and is used to treat chronic lymphocytic leukemia (CLL; a type of cancer of the white blood cells) in adults who are refractory to treatment with fludarabine (Fludara) and alemtuzumab (Campath).

mTOR inhibitors include but are not limited to rapamycin (FK506) and rapalogs, RAD001 or everolimus (Afinitor), CCI-779 or temsirolimus, AP23573, AZD8055, WYE-354, WYE-600, WYE-687 and Pp121.

Bexarotene is sold as Targretin® and is a member of a subclass of retinoids that selectively activate retinoid X receptors (RXRs). These retinoid receptors have biologic activity distinct from that of retinoic acid receptors (RARs). The chemical name is 4-[1-(5,6,7,8-tetrahydro-3,5,5,8,8-pentamethyl-2-naphthalenyl) ethenyl] benzoic acid. Bexarotene is used to treat cutaneous T-cell lymphoma (CTCL, a type of skin cancer) in people whose disease could not be treated successfully with at least one other medication.

Sorafenib marketed as Nexavar® is in a class of medications called multikinase inhibitors. Its chemical name is 4-[4-[[4-chloro-3-(trifluoromethyl)phenyl]carbamoylamino]phenoxy]-N-methyl-pyridine-2-carboxamide. Sorafenib is used to treat advanced renal cell carcinoma (a type of cancer that begins in the kidneys). Sorafenib is also used to treat unresectable hepatocellular carcinoma (a type of liver cancer that cannot be treated with surgery).

Examples of erbB inhibitors include lapatinib, erlotinib, and gefitinib. Lapatinib, N-(3-chloro-4-{[(3-fluorophenyl)methyl]oxy}phenyl)-6-[5-({[2-(methylsulfonyl)ethyl]amino}methyl)-2-furanyl]-4-quinazolinamine (represented by formula II, as illustrated), is a potent, oral, small-molecule, dual inhibitor of erbB-1 and erbB-2 (EGFR and HER2) tyrosine kinases that is approved in combination with capecitabine for the treatment of HER2-positive metastatic breast cancer.

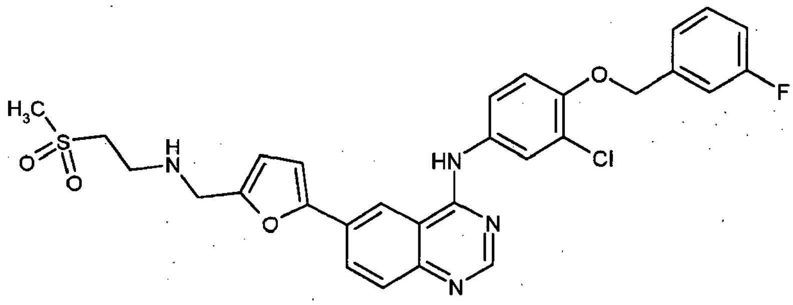

II

The free base, HCl salts, and ditosylate salts of the compound of formula (II) may be prepared according to the procedures disclosed in WO 99/35146, published July 15, 1999; and WO 02/02552 published January 10, 2002.

Erlotinib, N-(3-ethynylphenyl)-6,7-bis{[2-(methyloxy)ethyl]oxy}-4-quinazolinamine (commercially available under the tradename Tarceva) is represented by formula III, as illustrated:

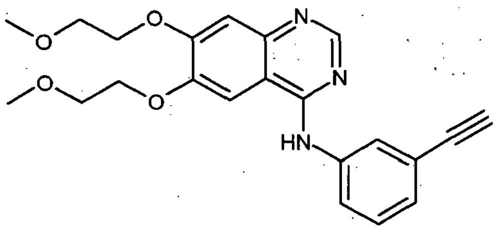

III

The free base and HCl salt of erlotinib may be prepared, for example, according to U.S. 5,747,498, Example 20.

Gefitinib, 4-quinazolinamine,N-(3-chloro-4-fluorophenyl)-7-methoxy-6-[3-4-morpholin)propoxy] is represented by formula IV, as illustrated:

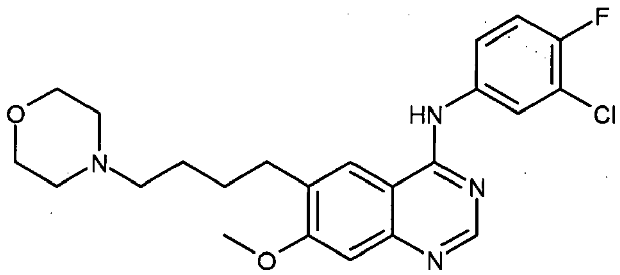

IV

Gefitinib, which is commercially available under the trade name IRESSA® (Astra-Zenenca) is an erbB-1 inhibitor that is indicated as monotherapy for the treatment of patients with locally advanced or metastatic non-small-cell lung cancer after failure of both platinum-based and docetaxel chemotherapies. The free base, HCl salts, and diHCl salts of gefitinib may be prepared according to the procedures of International Patent Application No. PCT/GB96/00961, filed April 23, 1996, and published as WO 96/33980 on October 31, 1996.

## EXAMPLES

### Example 1 - Production of neutralizing mAbs to human HGF

Recombinant human HGF protein produced in mouse myeloma NS0-derived cells was obtained from R&D Systems, catalogue number 294-HGN/CF. Two SJL/OlaHsd mice were immunized on days 0, 6 and 11 with R&D recombinant human HGF. Splenocytes and lymph nodes were isolated on day 14 and fused to mouse myeloma cells using a P3X63/Ag8.653-derived fusion partner. Immortalized antibody-producing cells were generated. HAT selection was used to deselect unfused myeloma cells.

Resulting hybridoma supernatants from active cultures were screened for specific binding and neutralization of HGF-cMet. Hits were identified, confirmed and cloned to monoclonality either by limiting dilution or growth in semi-solid media. Monoclonal antibodies with desired characteristics were scaled up in liquid culture and the antibody was purified by standard chromatography methods. The resulting purified antibodies were then further characterized for binding affinity and functional potency.

### Example 2 - Antibody Humanization - Cloning of Hybridoma Variable Regions

Total RNA was extracted from S260116C12, S260105B02, S260115C11 and S265109B10 hybridoma cells. Heavy and light variable domain cDNA sequence was generated by reverse transcription and polymerase chain reaction (RT-PCR). The forward primer for RT-PCR was a mixture of degenerate primers specific for murine immunoglobulin gene leader-sequences and the reverse primer was specific for the antibody constant regions, in this case isotype IgG1 for S260116C12, S260105B02, S265109B10 and isotype IgG2b for S260115C11. Primers were designed based on a strategy described by Jones and Bendig (Bio/Technology 9:88, 1991). RT-PCR was carried out for both V-region sequences to enable subsequent verification of the correct V-region sequences. DNA sequence data were obtained for the V-region products generated by the RT-PCR.

### Example 3 - Antibody Humanization - Cloning of chimeric antibodies

The DNA expression constructs encoding the chimeric antibodies were prepared *de novo* by build-up of overlapping oligonucleotides including restriction sites for cloning into mammalian expression vectors as well as a human signal sequence. *HindIII* and *SpeI* restriction sites were introduced to frame the $V_H$ domain containing the signal sequence (SEQ ID NO: 1) for cloning into mammalian expression vectors containing the human $\gamma 1$ constant region. *HindIII* and *BsiWI* restriction sites were introduced to frame the $V_L$ domain containing the signal sequence (SEQ ID NO: 1) for cloning into mammalian expression vector containing the human kappa constant region.

### Example 4 - Antibody **Humanization** - **Cloning of humanized variants**

The DNA expression constructs encoding the humanized antibody variants were prepared *de novo* by build-up of overlapping oligonucleotides including restriction sites for cloning into mammalian expression vectors as well as a human signal sequence. *HindIII* and *SpeI* restriction sites were introduced to frame the $V_H$ domain containing the signal sequence (SEQ ID NO: 1) for cloning into mammalian expression vectors containing the human $\gamma 1$ constant region. *HindIII* and *BsiWI* restriction sites were introduced to frame the $V_L$ domain containing the signal sequence (SEQ ID NO: 1) for cloning into mammalian expression vector containing the human kappa constant region.

### Example 5 - Expression of recombinant antibodies

Expression plasmids encoding the relevant heavy and light chains (listed in Table 1 below) were transiently co-transfected into HEK 293 6E cells and expressed at small scale to produce antibody. The antibodies were Protein A purified from the supernatants and quantified using the Nanodrop spectrophotometer.

**Table 1: Chimeric and humanized antibody variants**

| Antibody ID | Alternative Names | Description | SEQ ID NO: of DNA sequence | SE Q ID NO: of amino acid sequence |
|---|---|---|---|---|
| BPC 1840 | antiHGF S2651**09B10** Chimera | S2651**09B10** Chimeric heavy chain | 55 | 56 |
| | | S2651**09B10** Chimeric light chain | 57 | 58 |
| BPC 1854 | anti-HGF S2601**05B02** Chimera | S2601**05B02** Chimeric heavy chain | 47 | 48 |
| | | S2601**05B02** Chimeric light chain | 49 | 50 |
| BPC 1855 | anti-HGF S2601**15C11** Chimera | S2601**15C11** Chimeric heavy chain | 51 | 52 |
| | | S2601**15C11** Chimeric light chain | 53 | 54 |
| BPC 1856 | anti-HGF S2601**16C12**VL1 Chimera | S2601**16C12**VL1 Chimeric heavy chain | 59 | 60 |
| | | S2601**16C12**VL1 Chimeric light chain | 61 | 62 |
| BPC1873 | antiHGF humanised Ha2La1 | S2601**16C12** Ha2 heavy chain | 75 | 76 |
| | | S2601**16C12** La1 light chain | 87 | 88 |
| BPC 1880 | antiHGF humanised Ha4La0 | S2601**16C12** Ha4 heavy chain | 77 | 78 |
| | | S2601**16C12** La0 light chain | 85 | 86 |
| BPC 1881 | antiHGF humanised Ha4La1 | S2601**16C12** Ha4 heavy chain | 77 | 78 |
| | | S2601**16C12** La1 light chain | 87 | 88 |
| BPC 1884 | antiHGF humanised Ha5La0 | S2601**16C12** Ha5 heavy chain | 79 | 80 |
| | | S2601**16C12** La0 light chain | 85 | 86 |
| BPC 1885 | antiHGF humanised Ha5La1 | S2601**16C12** Ha5 heavy chain | 79 | 80 |
| | | S2601**16C12** La1 light chain | 87 | 88 |
| BPC 1930 | anti-HGF/VEGF Ha6La0 | S2601**16C12** Ha6 heavy chain | 81 | 82 |
| | | S2601**16C12** La0 light chain | 85 | 86 |
| BPC 1931 | anti-HGF/VEGF Ha7La0 | S2601**16C12** Ha7 heavy chain | 83 | 84 |
| | | S2601**16C12** La0 light chain | 85 | 86 |
| BPC 1923 | anti-HGF/VEGF Ha4-TVAAPSGS-593, La0 | S2601**16C12** Ha4-TVAAPSGS-593 heavy chain | 111 | 112 |
| | | S2601**16C12** La0 light chain | 85 | 86 |
| BPC 1924 | anti-HGF/VEGF Ha4-TVAAPSGS-593, La1 | S2601**16C12** Ha4-TVAARSGS-593 heavy chain | 111 | 112 |
| | | S2601**16C12** La1 light chain | 87 | 88 |
| BPC 1925 | anti-HGF/VEGF Ha5-TVAAPSGS-593, La0 | S2601**16C12** Ha5-TVAAPSGS-593 heavy chain | 113 | 114 |
| | | S2601**16C12** La0 light chain | 85 | 86 |

(continued)

| Antibody ID | Alternative Names | Description | SEQ ID NO: of DNA sequence | SE Q ID NO: of amino acid sequence |
|---|---|---|---|---|
| BPC 1926 | anti-HGF/VEGF Ha5-TVAAPSGS-593, La1 | S26011**6C12** Ha5-TVAAPSGS-593 heavy chain | 113 | 114 |
| | | S26011**6C12** La1 light chain | 87 | 88 |

**Example 6 - binding of antibodies to HGF on Biacore**

Anti-human IgG (Biacore BR-1008-39) was immobilised on a CM5 chip by primary amine coupling and this surface was used to capture the antibody molecules. Human HGFv1 and human HGFδ5 (GRITS38813) were used as analytes at 256nM, 64nM, 16nM, 4nM, and 1nM. Regeneration was carried out using 3M magnesium chloride. All binding curves were double referenced with a buffer injection (i.e. 0nM) and the data were fitted to the A100 evaluation software using the 1:1 model. The run was carried out at 37°C, using HBS-EP as the running buffer for the VEGF run and using HBS-N to which the following additions were made: 0.1M Lithium chloride, 0.1M Guanidine, 0.1% (v/v) triton X705, 0.1%(w/v) 3-(N,N-Dimethylmyristyl-ammonio)propanesulfonate (Sigma T7763). The data showed that all the molecules were capable of binding HGF (full length and shortened version). For most of the constructs tested affinity measurements were achieved within in the range measurable by Biacore for HGF (full length and shortened version), however, for BPC1880 binding failed to give affinity values measurable by Biacore, this was due to the off-rate being beyond the sensitivity of the machine in this assay, it does however indicate that the binding of BPC1880 to HGF is very tight.

Table 2 shows HGF binding of the humanized mAbs BPC1873, 1880, 1881, 1884, 1885 and chimeric mAb BPC1856.

**Table 2: Binding of antibodies to HGF**

| BPC Number | Analyte | ka (1/Ms) | kd (1/s) | KD(nM) | Comment |
|---|---|---|---|---|---|
| 1873 | HGF (delta 5) | 3.95E+05 | 4.63E-05 | 0.117 | |
| 1880 | HGF (delta 5) | 4.06E+05 | 7.55E-06 | 0.019 | off-rate beyond sensitivity of Biacore: very tight binder |
| 1881 | HGF (delta 5) | 4.22E+05 | 2.81E-05 | 0.067 | |
| 1884 | HGF (delta5) | 3.90E+05 | 1.50E-05 | 0.038 | |
| 1885 | HGF (delta5) | 4.19E+05 | 3.26E-05 | 0.078 | |
| 1856 | HGF (delta5) | 4.23E+05 | 1.78E-05 | 0.042 | |
| 1873 | HGF (fl) | 4.11E+05 | 3.74E-05 | 0.091 | |
| 1880 | HGF (fl) | 4.19E+05 | 5.17E-06 | 0.012 | off-rate beyond sensitivity of Biacore: very tight binder |
| 1881 | HGF (fl) | 4.13E+05 | 2.13E-05 | 0.052 | |
| 1884 | HGF (fl) | 3.88E+05 | 1.34E-05 | 0.035 | |
| 1885 | HGF (fl) | 4.18E+05 | 3.56E-05 | 0.085 | |
| 1856 | HGF (fl) | 4.34E+05 | 1.49E-05 | 0.034 | |

**Example 7 - Binding of HGF-VEGF bispecific antibodies to HGF and VEGF on Biacore**

Protein A was immobilised on a CM5 chip by primary amine coupling and this surface was then used to capture the antibody molecules. Human HGFvl (GRITS35238) and human HGFδ5 (GRITS38813) were used at 64nM, 16nM, 4nM, 1nM and 0.25nM and human VEGF was used at 256nM, 64nM, 16nM, 4nM and 1nM. Regeneration was carried out using 50mM NaOH. All binding curves were double referenced with a buffer injection (i.e. 0nM) and the data were fitted to the T100 evaluation software using the 1:1 model for HGF analysis and the 1:1 and bivalent analyte model for VEGF analysis. The run was carried out at 25°C, using HBS-EP as the running buffer for the VEGF run and using HBS-N to which the following additions were made: 0.1M Lithium chloride, 0.1M Guanidine, 0.1% (v/v) triton X705, 0.1%(w/v) 3-(N,N-Dimethylmyristyl-ammonio)propanesulfonate (Sigma T7763) for the HGF run. The data showed that the bispecific molecules were capable of binding both HGF (full length and shortened version) and VEGF. The data for the HGF (full length and shortened version) binding failed to give affinity values measurable by Biacore, this was due to the off-rate being beyond the sensitivity of the machine in this assay, it does however indicate that the molecules tested (BPC1923, BPC1924, BPC1925 and BPC1926) bind tightly to HGF.

Table 3 shows the results of the Biacore run and confirms that BPC1923, BPC1924, BPC1925 and BPC1926 are capable of binding to VEGF.

**Table 3: Binding of antibodies to VEGF**

| Sample | Fit | ka | kd | KD (nM) |
|---|---|---|---|---|
| BPC 1923 | 1:1 Binding | 4.90E+05 | 1.11E-05 | 0.023 |
| BPC 1924 | 1:1 Binding | 4.67E+05 | 2.39E-05 | 0.051 |
| BPC 1925 | 1:1 Binding | 4.69E+05 | 3.37E-05 | 0.072 |
| BPC 1926 | 1:1 Binding | 4.68E+05 | 4.05E-05 | 0.087 |
| DM S4000 | 1:1 Binding | 3.07E+05 | 1.08E-04 | 0.352 |
| BPC 1923 | Bivalent Analyte | 1.35E+05 | 1.66E-04 | 1.225 |
| BPC 1924 | Bivalent Analyte | 1.51E+05 | 1.33E-04 | 0.880 |
| BPC 1925 | Bivalent Analyte | 1.48E+05 | 1.90E-04 | 1.285 |
| BPC 1926 | Bivalent Analyte | 1.54E+05 | 1.72E-04 | 1.115 |
| DM S4000 | Bivalent Analyte | 1.64E+05 | 2.11E-04 | 1.289 |

Taken together the data suggestthat the humanised derivatives of 16C12, in particular 16C12 Ha4La0 (BPC1880), are very affinity antibodies against HGF, and are potential therapeutic antibodies. Importantly, this high affinity to HGF is retained, and high affinity to VEGF is added, in the bispecific (mAb-dAb) formats of 16C12Ha4La0 (as exemplified in BPC1923, BPC1924, BPC1925 and BPC1926).

### Example 8 - HGF and VEGF Bridging ELISA

A 96-well high binding plate was coated with 5μg/ml of recombinant human hVEGF-165 (TB090219, Domantis, 1.9mg/mL in PBS) in PBS and stored overnight at 4°C. The plate was washed twice with PBS. 200μL of blocking solution (5% BSA in PBS buffer) was added to each well and the plate was incubated for at least 1hour at room temperature. Another wash step was then performed. BPC1923, BPC1924, BPC1925, BPC1926, BPC1880 were successively diluted across the plate in blocking solution from 51.4nM or 60nM. Details of the HGF-VEGF mAbdAbs have been listed in the table below. A mAbdAb control containing anti-VEGF dAb moieties in-format with a mAb specific for an assay-irrelevant protein (designated DMS4000) was also included. After 1 hour incubation, the plate was washed. Biotinylated hHGF-v1 (GRITS37567, 1.62mg/mL, N13185-12) was diluted in blocking solution to 5μg/mL and 50μL was added to each well. The plate was incubated for one hour then washed. Streptavidin-HRP (GE Healthcare, RPN4401V) was diluted 1 in 4000 in blocking solution and 50μL was added to each well. After another wash step, 50μl of OPD SigmaFast substrate solution was added to each well and the reaction was stopped 15 minutes later by addition of 50μL of 2M sulphuric acid. Absorbance was read at 490nm using the VersaMax Tunable Microplate Reader (Molecular Devices) using a basic endpoint protocol.

Figure 1 shows the results of the HGF and VEGF bridging ELISA and confirms that BPC1923, BPC1924, BPC1925 and BPC1926 are capable of binding to both HGF and VEGF at the same time. BPC1880 and DMS4000 do not show binding to both targets.

### Example 9 - Mv1Lu Proliferation Assay

The Mv1Lu cell proliferation assay can be used to assess potency of putative HGF antagonists. TGF-beta inhibits MvlLu cell proliferation and this is overcome by the addition of HGF (J. Immunol Methods 1996, Jan 16, Vol 189 (1); 59-64). Hence, the differential in cell proliferation between HGF-treated and -untreated cells reflects HGF-mediated cell proliferation. The capacity of putative HGF antagonists to inhibit this effect can be quantitated.

To test the HGF-neutralisation capacity of S260116C12 and humanised variants thereof, MvlLu cells (ATCC) were incubated in serum-free medium supplemented with 40ng/ml human HGF, 1ng/ml TGF-beta (R&D Systems) and the test antibody. HGF was omitted from control wells as appropriate. All assays were performed in the presence of TGF-beta. All assays were performed in the presence of HGF, except for the negative control condition designated "-HGF". Antibodies were added at a final concentration of $1.3x10^{-08}$, $6.7x10^{-09}$, $3.3x10^{-09}$, $1.7x10^{-09}$, $8.3x10^{-10}$, $4.2x10^{-10}$, $2.1x10^{-10}$, $1.0x10^{-10}$ M. Where an irrelevant isotype control (designated hybrid control antibody) was used, it was applied at a final concentration of $1.3x10^{-08}$ M. Total cell number was determined after 48h using a luminescent ATP-dependent assay in which bioluminescence signal is proportional to viable cell number (CellTiterGlo™, Promega). All conditions were tested in triplicate. Data shown are presented as means +/- SD and are representative of at least two independent experiments.

The humanised anti-HGF monoclonal antibodies BPC1880, BPC1881, BPC1884 and BPC1885 each abrogated HGF-mediated MvlLu cell proliferation in a dose-dependent manner with profiles indistinguishable from the murine antibody S260116C12. The hybrid control antibody had no effect on HGF-mediated cell proliferation (Figure 2a-d).

To confirm that this HGF-neutralising capacity was retained in a mAbdAb format, a direct comparison was made between the murine anti-HGF mAb S260116C12 and representative humanised anti-HGF mAbdAb constructs BPC1923 and BPC1924 (corresponding to humanised mAb variants BPC1880 and BPC1881, respectively). These mAbdAbs contain the anti-HGF monoclonal antibody component in-format with anti-VEGF dAb moieties. Antibodies were added at a final concentration of $3.3x10^{-09}$, $1.7x10^{-08}$, $8.3x10^{-09}$, $4.2x10^{-09}$, $2.1x10^{-09}$, $1.0x10^{-09}$, $5.2x10^{-10}$ M. An hybrid control antibody and an irrelevant mAbdAb control (designated DMS4000) were used and each applied at a final concentration of $3.3x10^{-08}$ M. Data shown are presented as means +/- SD and are representative of at least two independent experiments.

Treatment with the mAbdAb constructs resulted in dose-dependent abrogation of HGF-mediated MvlLu cell proliferation that was indistinguishable from the corresponding mAb response profile (Figure 2e-f). Treatment with an irrelevant hybrid control antibody or an isotype control mAb comprising a monoclonal antibody moiety targeting an assay-irrelevant protein and anti-VEGF dAb moieties (DMS4000) had no effect on HGF-mediated cell proliferation, confirming that the observed effects of the test antibodies were due to the specific neutralisation of HGF.

Taken together, these data show that the murine anti-HGF mAb S260116C12 and humanised variants thereof, designated BPC1880, BPC1881, BPC1884 and BPC1885 abrogate HGF-dependent cell proliferation in a dose-dependent manner and that this activity is retained in a mAbdAb format as exemplified by BPC1923 and BPC1924.

### Example 10 - Human Umbilical Cord Endothelial Cell (HUVEC) c-MET phosphorylation assay

Treatment of serum-starved HUVEC monolayers with recombinant HGF results in phosphorylation of the HGF receptor cMET, which is detectable in cell lysates. Hence, neutralisation of HGF can be assessed by quantitation of phosphorylated-cMET from cells treated with HGF which has been pre-incubated with putative HGF antagonists.

Pooled donor HUVECs (Lonza) were seeded on gelatin-coated 96 well plates (Becton Dickenson) at 5,000cells/well in Bulletkit medium (Lonza) and incubated overnight at 37°C/5% $CO_2$. Medium was replaced with additive-free basal medium (e.g., EGM-2, Lonza) and cells were incubated for approximately four hours. Concentration titrations of test antibodies were preincubated with recombinant HGF for 15 minutes prior to addition to cells to achieve a final concentration of 25ng/ml HGF and a final concentration of 2.0, 1.0, 0.5, 0.25, 0.125, 0.06 μg/ml test antibody. Cells were incubated at 37°C for 20-25 minutes prior to washing and total cell lysates were prepared for p-cMET analysis using the MSD ELISA method according to the manufacturer's instructions (Mesoscale Discovery). All conditions were performed in at least triplicate. Data shown are means +/- SD and are representative of at least two independent experiments.

Preincubation of HGF with the murine anti-HGF antibody S260116C12 resulted in a dose-dependent neutralisation of HGF-mediated c-MET phosphorylation. Similar data were obtained by preincubation with each of the humanised variants of S260116C12, designated BPC1873, BPC1880, BPC1881, BPC1884 and BPC1885, confirming the HGF-neutralisation capacity of S260116C12 and its retention following humanisation. Preincubation with a hybrid control antibody had no effect on c-MET phosphorylation. Data are presented as raw MSD values (Figure 3a-c).

To confirm that this HGF-neutralising capacity was retained in a mAbdAb format, a direct comparison was made between the humanised anti-HGF mAbs BPC1880 and BPC1881 and the corresponding anti-HGF/anti-VEGF mAbdAb constructs BPC1923 and BPC1924. These mAbdAbs contain the anti-HGF monoclonal antibody component in-format with anti-VEGF dAb moieties. Antibodies were added over a concentration range (e.g., $1.3x10^{-08}$, $6.7x10^{-09}$, $3.3x10^{-09}$, $1.7x10^{-09}$, $8.3x10^{-10}$, $4.2x10^{-10}$ M. An irrelevant hybrid control antibody was used and applied at a final concentration of $1.3x10^{-08}$ M. Data shown are presented as the means of triplicate samples +/- SD and are representative of at least two independent experiments.

Treatment with the mAbdAb constructs resulted in dose-dependent abrogation of HGF-mediated cMET phosphorylation that was very similar to the corresponding mAb response profile (Figure 3d-e). Treatment with an irrelevant hybrid control antibody had no effect on HGF-mediated c-Met phosphorylation.

The data show that the capacity of the murine anti-HGF mAb S260116C12 and humanised variants thereof, designated BPC1880, BPC1881 to neutralise HGF-mediated c-Met phosphorylation in HUVECs is retained in a mAbdAb format as exemplified by BPC1923 and BPC1924.

## Example 11- Simultaneous inhibition of HGF-mediated cMET phosphorylation and VEGF-mediated VEGFR2 phosphorylation by mAbdAbs in HUVECs

Treatment of serum-starved HUVEC monolayers with recombinant vascular endothelial growth factor (VEGF) results in phosphorylation of the VEGF receptor VEGFR2, which is detectable in cell lysates. Hence, neutralisation of VEGF can be assessed by quantitation of phosphorylated-VEGFR2 from cells treated with VEGF which has been pre-incubated with putative VEGF antagonists. Furthermore, the capacity of anti-HGF, anti-VEGF mAbdAbs simultaneously to inhibit VEGF-mediated VEGFR2 phosphorylation and HGF-mediated cMET phosphorylation can similarly be assessed by treatment of cells with a preincubated combination of VEGF, HGF and putative bispecific VEGF/HGF antagonists, e.g,. mAbdAbs.

Pooled donor HUVECs (Lonza) were seeded on gelatin-coated 96 well plates (Becton Dickenson) at 5,000cells/well in Bulletkit medium (Lonza) and incubated overnight at 37°C/5% $CO_2$. Medium was replaced with additive-free basal medium (e.g., EGM-2, Lonza) and cells were incubated for approximately four hours. Concentration titrations of test antibodies were preincubated with recombinant HGF for 15 minutes prior to addition to cells to achieve a final concentration of 25ng/ml HGF 10ng/ml $VEGF_{165}$ and a final concentration of $1.3x10^{-08}$, $6.7x10^{-09}$, $3.3x10^{-09}$, $1.7x10^{-09}$, $8.3x10^{-10}$, $4.2x10^{-10}$ M test antibody. Cells were incubated at 37°C for 20-25 minutes prior to washing total cell lysates were prepared for p-cMET and p-VEGFR2 analysis using the MSD ELISA method according to the manufacturer's instructions (Mesoscale Discovery). All conditions were performed in at least triplicate. Data shown are means +/- SD and are representative of at least two independent experiments.

Treatment of cells with HGF or a combination of HGF and VEGF resulted in a detectable increase in p-cMET compared with untreated cells or those treated with VEGF alone. Preincubation of a combination of HGF and VEGF with the humanised anti-HGF antibody BPC1884 or the corresponding mAbdAb BPC1925 resulted in a dose-dependent neutralisation of HGF-mediated c-MET phosphorylation, consistent with the findings of example 10 showing that HGF-neutralisation capacity is retained in the mAbdAb format (Figure 4a).

Levels of p-VEGFR2 were quantitated in the same cell lysate samples. Treatment of cells with VEGF or a combination of VEGF and HGF resulted in a detectable increase in p-VEGFR2 compared with untreated cells or those treated with HGF alone. Preincubation of a combination of HGF and VEGF with the anti-HGF, anti-VEGF mAbdAb BPC1925 resulted in a reduction of detectable VEGFR2, reflecting neutralisation of VEGF simultaneous with the HGF neutralisation described in Figure 4a. In contrast, treatment with the corresponding humanised anti-HGF mAb BPC1884 did not affect p-VEGFR2 levels, confirming that VEGF neutralisation was specifically achieved by the VEGF-targeting moiety of the mAbdAb (Figure 4b).

The data show that VEGF and HGF can simultaneously be neutralised by an anti-HGF/anti-VEGF mAbdAb as exemplified by BPC1925 and determined by the measurement of HGF-mediated p-cMET and VEGF-mediated p-VEGFR2 in HUVECs.

## Example 12 - Angiogenesis Assay

This example is prophetic. It provides guidance for carrying out an additional assay in which the antigen binding proteins of the invention can be tested. This assay assesses the capacity of anti-HGF/anti-VEGF mAbdAbs or other antigen binding proteins to inhibit angiogenesis in an *in vitro* cellular assay.

The Angiokit™ is a commercially-available co-culture assay of endothelial cells and fibroblasts and can be used to test the capacity of putative anti-angiogenic agents to inhibit one or more parameters related to endothelial network formation in vitro. These parameters are quantitated using image analysis and include e.g. total endothelial cell area (field area), number of vessel branch points, mean tubule length, etc.

In a typical assay, angiogenesis co-culture assays (Angiokit™) are performed as directed by the manufacturer (TCS Cellworks). Briefly, medium is aspirated from 24 well format Angiokit™ co-culture plates and replaced with full growth medium with or without supplementation with human HGF and/or VEGF. Test compounds may be added at appropriate concentrations. Medium and test compounds are typically replaced on days 4, 7 and 9. Cells are fixed, typically on day 11, and endothelial cell networks visualised by anti-CD31 immunocytochemistry as directed by the manufacturer. Images are recorded by light microscopy and image analysis performed using appropriate software (e.g., AngioSys, TCS Cellworks) to achieve quantitation of a variety of angiogenic parameters. The effects of HGF-antagonism, VEGF-antagonism or simultaneous HGF- and VEGF-antagonism by test antigen binding proteins on various angiogenic processes can thereby be assessed and compared with appropriate positive and negative controls.

### Example 13 - Inhibition of tumour growth in animal models

This example is prophetic. It provides guidance for carrying out an additional assay in which the antigen binding proteins of the invention can be tested. This assay assesses the capacity of anti-HGF/anti-VEGF mAbdAbs or other antigen binding proteins to inhibit experimental tumour growth in an animal model. In a typical experiment, animals (e.g., mice) are inoculated with a suspension of tumour cells or a dissected tumour fragment, to initiate tumour growth. Such inoculation or implantation may be performed, for example: sub-cutaneously; intra-muscularly; into a specific tissue to generate an orthotopic tumour (dependent on the tumour cell type, e.g., intracranially or into the mammary fat pad) intravenously. Immunocompromised animals may be used to permit growth of a tumour xenograft, e.g., from a human tumour cell line.

Administration of mAbdAbs or other antigen binding proteins by an appropriate route (e.g., intravenous, intraperitoneal) can be commenced prior to, concomitant with or following tumour cell inoculation. Further dosing of test compounds is typically undertaken periodically for the duration of the experiment. The therapeutic effects of mAbdAbs or other antigen binding proteins in inhibiting tumour growth can be assessed by a variety of means including physical measurement of palpable tumour dimensions, bioluminescent imaging of tumour viability (in cases where an appropriate luciferase-expressing tumour cell line is employed) and by *post mortem* examination of primary and secondary tumours, including immunohistochemical and histological examination. The latter may provide a means for assessing the effects of putative therapeutic agents a variety of tumour characteristics specific for the targets of the antigen binding proteins, e.g., angiogenesis/microvessel density, necrosis, detection of phosphorylated VEGF receptors or HGF receptors. Quantitation of additional biomarkers may also be undertaken, e.g., detection of circulating levels of HGF, VEGF or other tumour-derived factors which may reflect tumour burden or tumour growth characteristics.

Such parameters of tumour growth in groups of animals treated with mAbdAbs can be compared with groups of animals treated with control substances or combinations of antigen binding proteins.

### Example 14 - Bx-PC3 cell cMET phosphorylation assay

Treatment of serum-starved Bx-PC3 cells with recombinant HGF results in phosphorylation of the HGF receptor cMET, which is detectable in cell lysates. Hence, neutralisation of HGF can be assessed by quantitation of phosphorylated-cMET from cells treated HGF subsequent to preincubation with putative HGF antagonists.

Bx-PC3 cells were seeded in Costar 96 well plates at 10,000cells per well in RPMI supplemented with glutamine and 10% FCS and incubated for 16 hours at 37°C/5% $CO_2$. The cells were washed with 100μl PBS and 100μl RPMI serum free medium added, with further incubation for 16 hours at 37°C/5%$CO_2$. Test antibodies were added to cells in duplicate over a concentration range ($6.67x10^{-07}$, $1.67x10^{-07}$, $4.2x10^{-08}$, $1.0x10^{-08}$, $2.6x10^{-09}$, $6.5x10^{-10}$, $1.6x10^{-10}$, $4.1x10^{-11}$, $1.0x10^{-11}$ M). After 15 minutes, recombinant HGF was added at a final concentration of 200ng/ml. Following incubation at 37°C/5%$CO_2$, medium was removed, cells washed with 100μl ice cold PBS and cell lysates prepared for cMET/phospho-cMET detection by the MSD ELISA method according to the manufacturer's instructions (Mesoscale Discovery). Data are presented as p-cMET as a percentage of total detectable cMET in cell lysates, +/- SE of duplicate values and are representative of at least two independent experiments.

Addition of the murine anti-HGF mAb S260116C12 resulted in a dose-dependent neutralisation of HGF-mediated cMET phosphorylation. Similar data were obtained by treatment with the humanised variants of S260116C12, designated BPC1880, BPC1881, BPC1884 and BPC1885, confirming that HGF neutralisation capacity had been retained following humanisation. Treatment with a hybrid antibody control had no substantive effect on cMET phosphorylation. To confirm that this HGF-neutralising capacity was retained in a mAbdAb format, cells were treated with anti-HGF/anti-VEGF mAbdAb constructs BPC1923, BPC1924, BPC1925, BPC1926 (corresponding to mAbs BPC1880, BPC1881, BPC1884, BPC1885, respectively). These mAbdAbs contain the anti-HGF monoclonal antibody component in-format with anti-VEGF dAb moieties. Treatment with the mAbdAb constructs resulted in dose-dependent abrogation of HGF-mediated cMET phosphorylation that was indistinguishable from the corresponding mAb response profile. Treatment with an irrelevant mAbdAb isotype control (DMS4000) had no effect on HGF-mediated c-Met phosphorylation (Figure 5).

These data describe the capacity of the murine anti-HGF mAb S260116C12 to inhibit HGF-mediated cMET phosphorylation in Bx-PC3 cells and confirm retention of this activity following humanisation and configuration as an anti-HGF/anti-VEGF mAbdAb.

### Example 15 - Bx-PC3 cell migration assay

The Oris cell migration assay (Amsbio™) consists of a sterile 96 well tissue culture plate with pre-inserted silicone seeding stoppers in each well. Cells are added and allowed to grow to confluence. The stopper is removed leaving a circular, cell-free area. Cell migration into this area is monitored over time following the addition of migration inhibitors or promoters. Hence, this assay provides a means for assessing putative inhibitors of factors, including HGF,

that are capable of modulating cell migration.

Bx-PC3 pancreatic cells were plated in an Oris cell migration 96 well plates at 100,000 cells per well in RPMI complete medium and incubated for 72 hours until confluent. Cell stoppers were removed to give a cell-free area. Cells were incubated for 24 hours in RPMI serum-free medium with test antibodies at various concentrations ($6.67x10^{-07}$, $1.67x10^{-07}$, $4.2x10^{-08}$, $1.0x10^{-08}$, $2.6x10^{-09}$, $6.5x10^{-10}$, $1.6x10^{-10}$, $4.1x10^{-11}$, $1.0x10^{-11}$ M) in combination with recombinant HGF at a final concentration of 25ng/ml HGF. Cell migration into the cell free area was quantified by fluorescently labelling cells with CellTracker (CellTracker™ Green CMFDA, Invitrogen #C2925) and measuring fluorescence using a plate reader (Envision). Exclusion of fluorescence derived from cells outside of the original cell-free area was achieved by application of a plate mask, according to the manufacturer's instructions (Amsbio). Data are presented as percentage of maximum migration and are representative of at least two independent experiments.

Treatment of cells with HGF resulted in a substantial increase of cell migration into the cell-free area compared with HGF-untreated controls. This HGF-mediated cell migration was inhibited in a dose-dependent manner by the murine anti-HGF mAb S260116C12. Treatment with the humanised S260116C12 variants BPC1873. BPC1880, BPC1881, BPC1884 and BPC1885 resulted in very similar inhibition profiles, confirming the retention of HGF-neutralisation capacity following humanisation **(Figure 6).**

### Example 16 - Generation of $V_k$ anti-VEGF dAbs

Vk dAbs were generated as described in USSN 61/512,143. Briefly, to generate domain antibodies (dAbs) with the ability to bind to human VEGF, selections were done using phage display libraries displaying Vk dAbs. Selections were performed on both biotinylated rhVEGF15 ("soluble selections") and on rhVEGF165 immobilized on plastic surfaces ("passive selections"). Using conventional phage panning techniques and three rounds of,selection against decreasing concentration of antigen, a panel of VEGF binding dAbs were identified. Sequence analysis identified 76 unique Vk sequences.

In order to identify dAbs in an appropriate architecture the dAb sequences were cloned onto the C-terminus of the heavy chain of a generic mAb of the human IgG1 isotype (Pascolizumab; anti-IL4) in a mammalian expression vector. The dAbs were linked to the mAb with one of two linkers; a short linker comprised of the sequence GSTVAAPST and a long linker comprised of the sequence GSTVAAPSGSTVAAPSGSTVAAPSGS.

The dAbs were analysed to determine their ability to bind to rhVEGF, and to block VEGF binding to recombinant VEGFR1 (flt-1) or VEGFR2 (KDR). The receptor binding assay (RBA) data demonstrated that the DT02-K-044 dAb (SEQ ID NO:191) was not only able to bind tightly to VEGF but that it was able to prevent VEGF binding to its natural receptors in a plate-based assay.

In an attempt to improve the off-rate (Kd) of the DT02-K-044, affinity maturation was carried out and candidate leads evaluated in the context of the Pascolizumab-(GSTVAAPS)3-T mAb heavy chain expression vector, co-expressed with the pascolizumab light chain in HEK cells. The expressed mAb-dAb in the transfection supernatant analysed for binding to VEGF using an SPR technique (Proteon). Samples were not quantitated but compared with the parent mAb-dAb that had been expressed alongside. In the Proteon analysis, the mutated clones were compared with the parent clone expressed in the same manner on each plate. All of the improvements were clustered at 3 of the 13 diversified CDR residues (S34, G51, H96 (numbering is based on the first residue of the Vk dAb (Asp) as residue 1)). Of these three positions, substitutions at positions S34 and H96 were the most significant (in terms of improving KD, by Proteon analysis and in a HUVEc proliferation assay). Table 4 below highlights Proteon estimation of KD (in nM) for certain positions where the affinity is significantly enhanced over the DT02-K-044 parent dAb (Table 4).

**Table 4: affinity matured variants of DT02-K-044 (SEQ ID NO:191)**

| Mutant ID | Amino Acid Substitution | KD nM |
|---|---|---|
| **DT02-K-044 wt** | N/A | 0.1 |
| **DT02-K-044-077** | S34A | 0.04 |
| **DT02-K-044-082** | S34G | 0.07 |
| **DT02-K-044-083** | S34H | 0.049 |
| **DT02-K-044-084** | S34I | 7.27E-27* |
| **DT02-K-044-085** | S34K | 1.12E-18* |
| **DT02-K-044-086** | S34L | 0.032 |
| **DT02-K-044-087** | S34M | 0.005* |

(continued)

| Mutant ID | Amino Acid Substitution | KD nM |
|---|---|---|
| **DT02-K-044-088** | S34N | 0.052 |
| **DT02-K-044-090** | S34Q | 0.02 |
| **DT02-K-044-092** | S34T | 0.003* |
| **DT02-K-044-093** | S34V | 0.034 |
| **DT02-K-044-095** | S34Y | 0.006* |
| **DT02-K-044-229** | H96A | 0.002 |
| **DT02-K-044-230** | H96C | 0.023 |
| **DT02-K-044-232** | H96E | 0.004* |
| **DT02-K-044-234** | H96G | 0.022 |
| **DT02-K-044-235** | H96I | 0.004 |
| **DT02-K-044-236** | H96K | 9.82E-18* |
| **DT02-K-044-237** | H96L | 0.031 |
| **DT02-K-044-238** | H96M | 0.044 |
| **DT02-K-044-239** | H96N | 0.013 |
| **DT02-K-044-240** | H96P | 0.043 |
| **DT02-K-044-247** | H96Y | 0.018 |

To determine whether or not these mutations, which were beneficial singly, could be addictive, combination mutants were constructed that combined these changes, which led to the identification, *interalia,* of the double mutants identified as DT02-K-044-251 (S34K plus H96E, SEQ ID NO:192) and DT02-K-044-255 (S34K plus H96K, SEQ ID NO:193).

These two dAbs, together with the preferred single mutant DT02-K-044-085 (S34K, SEQ ID NO:194) were expressed as fusions to pascolizumab with various linkers, including a 21 amino acid linker sequence derived from human serum albumin. These three constructs were tested for potency in the receptor binding assay, HUVEc proliferation assay and for kinetic affinity by Biacore SPR. All three molecules showed significantly enhanced affinity compared to the parental molecule, in the low pM range.

**SEQUENCE IDENTIFIERS**

**Table 5: Sequence identifier numbers of amino acid and DNA sequences**

| **Description** | **Sequence identifier (SEQ ID NO)** | |
|---|---|---|
| | **DNA** | **Amino acid** |
| Signal peptide sequence | 1 | 2 |
| S260116C12 mouse variable heavy | 3 | 4 |
| S260116C12 mouse variable light | 5 | 6 |
| S260105B02 mouse variable heavy | 7 | 8 |
| S260105B02 mouse variable light | 9 | 10 |
| S260115C11 mouse variable heavy | 11 | 12 |

(continued)

| Description | Sequence identifier (SEQ ID NO) | |
|---|---|---|
| | DNA | Amino acid |
| S260115C11 mouse variable light | 13 | 14 |
| S265109B10 mouse variable heavy | 15 | 16 |
| S265109B10 mouse variable light | 17 | 18 |
| S260116C12 CDR H1 | | 19 |
| S260116C12 CDR H2 | | 20 |
| S260116C12 CDR H3 | | 21 |
| S260116C12 CDR L1 | | 22 |
| S260116C12 CDR L2 | | 23 |
| S260116C12 CDR L3 | | 24 |
| S260105B02 CDR H1 | | 25 |
| S260105B02 CDR H2 | | 26 |
| S260105B02 CDR H3 | | 27 |
| S260105B02 CDR L1 | | 28 |
| S260105B02 CDR L2 | | 29 |
| S260105B02 CDR L3 | | 30 |
| S260115C11 CDR H1 | | 31 |
| S260115C11 CDR H2 | | 32 |
| S260115C11 CDR H3 | | 33 |
| S260115C11 CDR L1 | | 34 |
| S260115C11 CDR L2 | | 35 |
| S260115C11 CDR L3 | | 36 |
| S265109B10 CDR H1 | | 37 |
| S265109B10 CDR H2 | | 38 |
| S265109B10 CDR H3 | | 39 |
| S265109B10 CDR L1 | | 40 |
| S265109B10 CDR L2 | | 41 |
| S265109B10 CDR L3 | | 42 |
| S260116C12 chimera heavy chain | 43 | 44 |
| S260116C12 chimera light chain | 45 | 46 |
| S260105B02 chimera heavy chain | 47 | 48 |
| S260105B02 chimera light chain | 49 | 50 |
| S260115C11 chimera heavy chain | 51 | 52 |
| S260115C11 chimera light chain | 53 | 54 |
| S265109B10 chimera heavy chain | 55 | 56 |
| S265109B10 chimera light chain | 57 | 58 |

(continued)

| Description | Sequence identifier (SEQ ID NO) | |
|---|---|---|
| | DNA | Amino acid |
| S260116C12 chimera heavy chain variable region | 59 | 60 |
| S260116C12 chimera light chain variable region | 61 | 62 |
| S260105B02 chimera heavy chain variable region | 63 | 64 |
| S260105B02 chimera light chain variable region | 65 | 66 |
| S260115C11 chimera heavy chain variable region | 67 | 68 |
| S260115C11 chimera light chain variable region | 69 | 70 |
| S265109B10 chimera heavy chain variable region | 71 | 72 |
| S265109B10 chimera light chain variable region | 73 | 74 |
| S260116C12 humanized Ha2 heavy chain | 75 | 76 |
| S260116C12 humanized Ha4 heavy chain | 77 | 78 |
| S260116C12 humanized Ha5 heavy chain | 79 | 80 |
| S260116C12 humanized Ha6 heavy chain | 81 | 82 |
| S260116C12 humanized Ha7 heavy chain | 83 | 84 |
| S260116C12 humanized La0 light chain | 85 | 86 |
| S260116C12 humanized La1 light chain | 87 | 88 |
| S260116C12 humanized Ha2 heavy chain variable region | 89 | 90 |
| S260116C12 humanized Ha4 heavy chain variable region | 91 | 92 |
| S260116C12 humanized Ha5 heavy chain variable region | 93 | 94 |
| S260116C12 humanized Ha6 heavy chain variable region | 95 | 96 |
| S260116C12 humanized Ha7 heavy chain variable region | 97 | 98 |
| S260116C12 humanized La0 light chain variable region | 99 | 100 |
| S260116C12 humanized La1 light chain variable region | 101 | 102 |
| Anti-VEGF 098 dAb | 103 | 104 |
| Anti-VEGF 098AAA dAb | 105 | 106 |
| Anti-VEGF 044 dAb | 107 | 108 |
| Anti-VEGF 593 dAb | 109 | 110 |
| Anti-HGF-VEGF S260116C12 humanized Ha4-TVAAPSGS-593 heavy chain | 111 | 112 |
| Anti-HGF-VEGFS260116C12 humanized Ha5-TVAAPSGS-593 heavy chain | 113 | 114 |
| Anti-HGF-VEGF S260116C12 humanized Ha4-TVAAPSGS-098 heavy chain | | 115 |
| Anti-HGF-VEGF S260116C12 humanized Ha5-TVAAPSGS-098 heavy chain | | 116 |
| Anti-HGF-VEGF S260116C12 humanized Ha6-TVAAPSGS-098 heavy chain | | 117 |
| Anti-HGF-VEGF S260116C12 humanized Ha7-TVAAPSGS-098 heavy chain | | 118 |
| Anti-HGF-VEGF S260116C12 humanized Ha4-TVAAPS-098 heavy chain | | 119 |
| Anti-HGF-VEGF S260116C12 humanized Ha5-TVAAPS-098 heavy chain | | 120 |
| Anti-HGF-VEGF S260116C12 humanized Ha6-TVAAPS-098 heavy chain | | 121 |

(continued)

| Description | Sequence identifier (SEQ ID NO) | |
|---|---|---|
| | DNA | Amino acid |
| Anti-HGF-VEGF S260116C12 humanized Ha7-TVAAPS-098 heavy chain | | 122 |
| Anti-HGF-VEGF S260116C12 humanized Ha4-TVAAPSGS-098AAA heavy chain | | 123 |
| Anti-HGF-VEGF S260116C12 humanized Ha5-TVAAPSGS-098AAA heavy chain | | 124 |
| Anti-HGF-VEGF S260116C12 humanized Ha6-TVAAPSGS-098AAA heavy chain | | 125 |
| Anti-HGF-VEGF S260116C12 humanized Ha7-TVAAPSGS-098AAA heavy chain | | 126 |
| Anti-HGF-VEGF S260116C12 humanized Ha4-TVAAPS-098AAA heavy chain | | 127 |
| Anti-HGF-VEGF S260116C12 humanized Ha5-TVAAPS-098AAA heavy chain | | 128 |
| Anti-HGF-VEGF S260116C12 humanized Ha6-TVAAPS-098AAA heavy chain | | 129 |
| Anti-HGF-VEGF S260116C12 humanized Ha7-TVAAPS-098AAA heavy chain | | 130 |
| Anti-HGF-VEGF S260116C12 humanized Ha4-TVAAPSGS-044 heavy chain | | 131 |
| Anti-HGF-VEGF S260116C12 humanized Ha5-TVAAPSGS-044 heavy chain | | 132 |
| Anti-HGF-VEGF S260116C12 humanized Ha6-TVAAPSGS-044 heavy chain | | 133 |
| Anti-HGF-VEGF S260116C12 humanized Ha7-TVAAPSGS-044 heavy chain | | 134 |
| Anti-HGF-VEGF S260116C12 humanized Ha4-TVAAPS-044 heavy chain | | 135 |
| Anti-HGF-VEGF S260116C12 humanized Ha5-TVAAPS-044 heavy chain | | 136 |
| Anti-HGF-VEGF S260116C12 humanized Ha6-TVAAPS-044 heavy chain | | 137 |
| Anti-HGF-VEGF S260116C12 humanized Ha7-TVAAPS-044 heavy chain | | 138 |
| Anti-HGF-VEGF S260116C12 humanized Ha4-GS(TVAAPSGS)$_3$-044 heavy chain | | 139 |
| Anti-HGF-VEGF S260116C12 humanized Ha5-GS(TVAAPSGS)$_3$-044 heavy chain | | 140 |
| Anti-HGF-VEGF S260116C12 humanized Ha6-GS(TVAAPSGS)$_3$-044 heavy chain | | 141 |
| Anti-HGF-VEGF S260116C12 humanized Ha7-GS(TVAAPSGS)$_3$-044 heavy chain | | 142 |
| Anti-HGF-VEGF S260116C12 humanized Ha4-(TVAAPS)$_3$-044 heavy chain | | 143 |
| Anti-HGF-VEGF S260116C12 humanized Ha5-(TVAAPS)$_3$-044 heavy chain | | 144 |
| Anti-HGF-VEGF S260116C12 humanized Ha6-(TVAAPS)$_3$-044 heavy chain | | 145 |
| Anti-HGF-VEGF S260116C12 humanized Ha7-(TVAAPS)$_3$-044 heavy chain | | 146 |
| Anti-HGF-VEGF S260116C12 humanized Ha4-DETYVPKEFNAETFGS-044 heavy chain | | 147 |
| Anti-HGF-VEGF S260116C12 humanized Ha5-DETYVPKEFNAETFGS-044 heavy chain | | 148 |
| Anti-HGF-VEGF S260116C12 humanized Ha6-DETYVPKEFNAETFGS-044 heavy chain | | 149 |
| Anti-HGF-VEGF S260116C12 humanized Ha7-DETYVPKEFNAETFGS -044 heavy chain | | 150 |
| Anti-HGF-VEGF S260116C12 humanized Ha4-DETYVPKEFNAETF-044 heavy chain | | 151 |
| Anti-HGF-VEGF S260116C12 humanized Ha5-DETYVFKEFNAETF-044 heavy chain | | 152 |
| Anti-HGF-VEGF S260116C12 humanized Ha6-DETYVPKEFNAETF-044 heavy chain | | 153 |
| Anti-HGF-VEGF S260116C12 humanized Ha7-DETYVPKEFNAETF -044 heavy chain | | 154 |
| Anti-HGF-VEGF S260116C12 humanized Ha4-EVDETYVPKEFNAETFTFHADGS-044 heavy chain | | 155 |

(continued)

| Description | Sequence identifier (SEQ ID NO) | |
|---|---|---|
| | DNA | Amino acid |
| Anti-HGF-VEGF S260116C12 humanized Ha5-EVDETYVPKEFNAETFTFHADGS-044 heavy chain | | 156 |
| Anti-HGF-VEGF S260116C12 humanized Ha6-EVDETYVPKEFNAETFTFHADGS-044 heavy chain | | 157 |
| Anti-HGF-VEGF S260116C12 humanized Ha7-EVDETYVPKEFNAETFTFHADGS-044 heavy chain | | 158 |
| Anti-HGF-VEGF S260116C12 humanized Ha4-EVDETYVPKEFNAETFTFHAD-044 heavy chain | | 159 |
| Anti-HGF-VEGF S260116C12 humanized Ha5-EVDETYVPKEFNAETFTFHAD-044 heavy chain | | 160 |
| Anti-HGF-VEGF S260116C12 humanized Ha6-EVDETYVPKEFNAETFTFHAD-044 heavy chain | | 161 |
| Anti-HGF-VEGF S260116C12 humanized Ha7-EVDETYVPKEFNAETFTFHAD -044 heavy chain | | 162 |
| TVAAPSGS linker | | 163 |
| TVAAPS linker | | 164 |
| GS(TVAAPSGS)$_3$ | | 165 |
| (TVAAPS)$_3$ | | 166 |
| DETYVPKEFNAETFGS linker | | 167 |
| DETYVPKEFNAETF linker | | 168 |
| EVDETYVPKEFNAETFTFHADGS linker | | 169 |
| EVDETYVPKEFNAETFTFHAD linker | | 170 |
| DMS4000 heavy chain | 171 | 172 |
| DMS4000 light chain | 173 | 174 |
| Hybrid antibody control heavy chain | 175 | 176 |
| Hybrid antibody control light chain | 177 | 178 |
| Anti-VEGF Y0317 humanized antibody fragment VH region | | 179 |
| Anti-VEGF Y0317 humanized antibody fragment VL region | | 180 |
| Anti-VEGF anticalin | | 181 |
| Anti-VEGFR2 adnectin | | 182 |
| Humanised anti-HGF nanobody HGF13 | | 183 |
| Humanised anti-HGF nanobody HGF13hum5 | | 184 |
| Avastin Variable Light Chain | | 185 |
| Avastin Variable Heavy chain | | 186 |
| DDNPNLPRLVRPE Linker | | 187 |
| DEMPADLPSLAADF Linker | | 188 |
| HKDDNPNLPRLVRPEVDVM Linker | | 189 |

(continued)

| Description | Sequence identifier (SEQ ID NO) | |
|---|---|---|
| | DNA | Amino acid |
| ENDEMPADLPSLAADFVESKD Linker | | 190 |
| Anti-VEGF Vk DT02-K-044 dAb | | 191 |
| Anti-VEGF Vk dAb DT02-K-044-251 | | 192 |
| Anti-VEGF Vk dAb DT02-K-044-255 | | 193 |
| Anti-VEGF Vk dAb DT02-K-044-085 | | 194 |
| (TGLDSP)3 linker | | 195 |
| (TGLDSP)4 linker | | 196 |

**SEQUENCES**

**SEQ. ID NO.1 Signal peptide sequence (DNA sequence)**
ATGGGCTGGTCCTGCATCATCCTGTTTCTGGTGGCCACCGCCACCGGCGTGCACAGC
**SEQ. ID NO.2 Signal peptide sequence (amino acid sequence)**
MGWSCIILFLVATATGVHS
**SEQ. ID NO.3 S260116C12 mouse variable heavy (DNA sequence)**

CAGATTCAGCTGCGGCAGTCTGGAGCTGGACTGATGAAGCCTGGGGCCTCAGTGAAGCTTTCCTGCAAGGCTACT
GGCTACACATTCACTGGCTACTGGATAGAGTGGGTAAAGCAGAGGCCTGGACATGACCTTGAGTGGATTGGAGAG
ATTTTACCTGGAAGTGGTACTACTAACTACAATGAGAAGTTCAAGGGCAAGGCCACATTCACTGCAGATACATCCTC
CAACACAGCCTACATGCAACTCAGCAGCCTGACAACTGAGGACTCTGCCATCTATTACTGTGCAAGGGGGGGtTATT
ACTACGGTAGTAGCTACGACTCCTGGGGCCAAGGCA

**SEQ. ID NO.4 S260116C12 mouse variable heavy (amino acid sequence)**

QIQLRQSGAGLMKPGASVKLSCKATGYTFTGYWIEWVKQRPGHDLEWIGEILPGSGTTNYNEKFKGKATFTADTSSNT
AYMQLSSLTTEDSAIYYCARGGYYYGSSYDSWGQG

**SEQ. ID NO.5 S260116C12 mouse variable light (DNA sequence)**

GACATTGTGCTGACCCAATCTCCAGCTTCTTTGGCTGTGTCTCTAGGGCAGAGGGCCACCATCTCCTGCAGAGCCA
GTGAAAGTGTCAGTATTCATGGTACTCATTTAATGCACTGGTACCAACAGAAACCAGGACAGCCACCCAAACTCCTC
ATCTATGCTGCATCCAACCTAGAATCTGGAGTCCCTGCCAGGTTCAGTGGCAGTGGGTCTGAGACAGACTTCACCC
TCAACATCCATCCTGTGGAGGAGGAGGATGCTGCAACCTATTTCTGTCAGCAAAGTATTGAGGATCCGTACACGTT
CGGAGGGGGGACCAAGCTGGAAATAAAACGG

**SEQ. ID NO.6 S260116C12 mouse variable light (amino acid sequence)**

DIVLTQSPASLAVSLGQRATISCRASESVSIHGTHLMHWYQQKPGQPPKLLIYAASNLESGVPARFSGSGSETDFTLNIH
PVEEEDAATYFCQQSIEDPYTFGGGTKLEIKR

**SEQ. ID NO.7 S260105B02 mouse variable heavy (DNA sequence)**

CAGGTTCAGCTGCAGCAGTCTGGAGCTGAGCTGATGAAGCCTGGGGCCTCAGTGAAGCTTTCCTGCAAGGCTACT
GGCTACACATTCACTGGCTACTGGATAGAGTGGGTAAAGCAGAGGCCTGGACATGGCCTTGAGTGGATTGGAGAG
ATTTTACCTGGAAGTGGTAGTACTAACTACAATGAGAAGTTCAAGGGCAAGGCCACATTCACTGCAGATACATCCT
CCAACACAGCCTACATGCAACTCAGCAGCCTGACAACTGAGGACTCTGCCATCTATTACTGTGCAAGAGGGGGTA
TGGTTACCACGACGCCTGGTTTGCTTACTGGGGCCAAGGAC

**SEQ. ID NO.8 S260105B02 mouse variable heavy (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYGYHDAWFAYWGQG

**SEQ. ID NO.9 S260105B02 mouse variable light (DNA sequence)**

GACATTGTGATGTCACAGTCTCCATCCTCCCTAGCTGTGTCAGTTGGAGAGAAGGTTACTATGAGCTGCAAGTCCA
GTCAGAGCCTTTTATATAGTAGCAATCAAAAGAACTACTTGGCCTGGTACCAGCAGAAACCAGGGCAGTCTCCTAA
ACTGCTGATTTACTGGGCATCCACTAGGGAATCTGGGGTCCCTGATCGCTTCACAGGCAGTGGATCTGGGACAGAT
TTCACTCTCACCATCAGCAGTGTGAAGGCTGAAGACCTGGCAGTTTATTACTGTCAGCAATATTATAGCTATCCGTA
CACGTTCGGA

**SEQ. ID NO.10 S260105B02 mouse variable light (amino acid sequence)**

DIVMSQSPSSLAVSVGEKVTMSCKSSQSLLYSSNQKNYLAWYQQKPGQSPKLLIYWASTRESGVPDRFTGSGSGTDFT
LTISSVKAEDLAVYYCQQYYSYPYTFG

**SEQ. ID NO.11 S260115C11 mouse variable heavy (DNA sequence)**

CAGGTTCAGCTGCAGCAGTCTGGAGCTGAGCTGATGAAGCCTGGGGCCTCAGTGAAGCTTTCCTGCAAGGCTACT
GGCTACACATTCACTGGCTACTGGATAGAGTGGGTAAAGCAGAGGCCTGGACATGGCCTTGAGTGGATTGGAGAG
ATTTTACCTGGAAGTGGTAGTACTAACTACAATGAGAAGTTCAAGGGCAAGGCCACATTCACTGCAGATACATCCT
CCAACACAGCCTACATGCAACTCAGCAGCCTGACAACTGAGGACTCTGCCATCTATTACTGTGCAAGGGGGGGTTA
TTACTACGGTAGTAGCTTTGACTACTGGGGCCAAGGC

**SEQ. ID NO.12 S260115C11 mouse variable heavy (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYYYGSSFDYWGQG

**SEQ. ID NO.13 S260115C11 mouse variable light (DNA sequence)**

CAAATTGTTCTCACCCAGTCTCCAGCAATCATGTCTGCATCTCTAGGGGAACGGGTCACCATGACCTGCACTGCCA
GCTCAAGTGTAAGTTCCAGTTACTTGCACTGGTACCAGCAGAAGCCAGGATCCTCCCCCAAACTCTGGATTTATAG
CACATCCAACCTGGCTTCTGGAGTCCCAGCTCGCTTCAGTGGCAGTGGGTCTGGGACCTCTTACTCTCTCACAATC
AGCAGCATGGAGGCTGAAGATGCTGCCACTTATTACTGCCACCAGTATCATCGTTCCCCGCTCACGTTCGGTGCTG
GGACCAAGCTGGAGCTGAAACGG

**SEQ. ID NO.14 S260115C11 mouse variable light (amino acid sequence)**

QIVLTQSPAIMSASLGERVTMTCTASSSVSSSYLHWYQQKPGSSPKLWIYSTSNLASGVPARFSGSGSGTSYSLTISSM
EAEDAATYYCHQYHRSPLTFGAGTKLELKR

**SEQ. ID NO.15 S265109B10 mouse variable heavy (DNA sequence)**

CAGGTTCAGCTGCAGCAGTCTGGAGCTGAGCTGATGAAGCCTGGGGCCTCAGTGAAGCTTTCCTGCAAGGCTACT
GGCTACACATTCACTGGCTACTGGATAGAGTGGGTAAAACAGAGGCCTGGACATGGCCTTGAGTGGATTGGAGAG
ATTTTACCTGGAAGTTCTAGTACTAACTACAATGAGAAGTTCAAGGACAAGGCCACATTCACTGCAGATACATCCTC
CAACACAGCCTACATGCAACTCAGCAGCCTGACAACTGAGGACTCTGCCATCTATTACTGTGCAAGAGGGGGATAT
TACTACGGTAGTCCTATGGACTACTGGGGTCAAGGAACCTCAGTCACCGTCTCCTCA

**SEQ. ID NO.16 S265109B10 mouse variable heavy (amino acid sequence)**

QVQLQQSGAELMKPGASVKLSCKATGYTFTGYWIEWVKQRPGHGLEWIGEILPGSSSTNYNEKFKDKATFTADTSSN
TAYMQLSSLTTEDSAIYYCARGGYYYGSPMDYWGQGTSVTVSS

**SEQ. ID NO.17 S265109B10 mouse variable light (DNA sequence)**

CAAATTGTTCTCACCCAGTCTCCAGCAATCATGTCTGCATCTCCAGGGGAGAAGGTCACCATGACCTGCAGTGCCA
GGTCAAGTGTAAGTTACATGCACTGGTACCAGCAGAAGTCAGGCACCTCCCCCAAAAGATGGATTTATGACACATC
CAAACTGGCTTCTGGAGTCCCTGCTCGCTTCAGTGGCAGTGGGTCTGGGACCTCTTACTCTCTCACAATCAGCAGC
ATGGAGGCTGAAGATGCTGCCACTTATTACTGCCAGCAGTGGAGTAGTAACCCACCCACGTTCGGTGGAGGCACCA
AGCTGGAAATCAAA

**SEQ. ID NO.18 S265109B10 mouse variable light (amino acid sequence)**

QIVLTQSPAIMSASPGEKVTMTCSARSSVSYMHWYQQKSGTSPKRWIYDTSKLASGVPARFSGSGSGTSYSLTISSME
AEDAATYYCQQWSSNPPTFGGGTKLEIK

**SEQ. ID. No.19 S260116C12 CDR H1 (amino acid sequence)**
GYWIE
**SEQ. ID. No.20 S260116C12 CDR H2 (amino acid sequence)**
EILPGSGTTNYNEKFKG
**SEQ. ID. No.21 S260116C12 CDR H3 (amino acid sequence)**
GGYYYGSSYDS
**SEQ. ID. No.22 S260116C12 CDR L1 (amino acid sequence)**
RASESVSIHGTHLMH
**SEQ. ID. No.23 S260116C12 CDR L2 (amino acid sequence)**
AASNLES
**SEQ. ID. No.24 S260116C12 CDR L3 (amino acid sequence)**
QQSIEDPYT
**SEQ. ID. No.25 S260105B02 CDR H1 (amino acid sequence)**
GYWIE
**SEQ. ID. No.26 S260105B02 CDR H2 (amino acid sequence)**
EILPGSGSTNYNEKFKG
**SEQ. ID. No.27 S260105B02 CDR H3 (amino acid sequence)**
GGYGYHDAWFAY
**SEQ. ID. No.28 S260105B02 CDR L1 (amino acid sequence)**
KSSQSLLYSSNQKNYLA
**SEQ. ID. No.29 S260105B02 CDR L2 (amino acid sequence)**
WASTRES
**SEQ. ID. No.30 S260105B02 CDR L3 (amino acid sequence)**
QQYYSYPYT
**SEQ. ID. No.31 S260115C11 CDR H1 (amino acid sequence)**
GYWIE
**SEQ. ID. No.32 S260115C11 CDR H2 (amino acid sequence)**
EILPGSGSTNYNEKFKG

**SEQ. ID. No.33 S260115C11 CDR H3 (amino acid sequence)**
GGYYYGSSFDY
**SEQ. ID. No.34 S260115C11 CDR L1 (amino acid sequence)**
TASSSVSSSYLH
**SEQ. ID. No.35 S260115C11 CDR L2 (amino acid sequence)**
STSNLAS
**SEQ. ID. No.36 S260115C11 CDR L3 (amino acid sequence)**
HQYHRSPLT
**SEQ. ID. No.37 S265109B10 CDR H1 (amino acid sequence)**
GYWIE
**SEQ. ID. No.38 S265109B10 CDR H2 (amino acid sequence)**
EILPGSSSTNYNEKFKD
**SEQ. ID. No.39 S265109B10 CDR H3 (amino acid sequence)**
GGYYYGSPMDY
**SEQ. ID. No.40 S265109B10 CDR L1 (amino acid sequence)**
SARSSVSYMH
**SEQ. ID. No.41 S265109B10 CDR L2 (amino acid sequence)**
DTSKLAS
**SEQ. ID. No.42 S265109B10 CDR L3 (amino acid sequence)**
QQWSSNPPT
**SEQ. ID NO.43 S260116C12 chimera heavy chain (DNA sequence)**

CAGATCCAGCTGCGCCAGTCCGGCGCCGGCCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGACCTGGAGTGGATCGGCGAG
ATCCTGCCCGGCTCCGGCACCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CTACTACGGCTCCTCCTACGACTCCTGGGGCCAGGGCACACTAGTGACCGTGTCCAGCGCCAGCACCAAGGGCCCC
AGCGTGTTCCCCCTGGCCCCCAGCAGCAAGAGCACCAGCGGCGGCACAGCCGCCCTGGGCTGCCTGGTGAAGGAC
TACTTCCCCGAACCGGTGACCGTGTCCTGGAACAGCGGAGCCCTGACCAGCGGCGTGCACACCTTCCCCGCCGTGC
TGCAGAGCAGCGGCCTGTACAGCCTGAGCAGCGTGGTGACCGTGCCCAGCAGCAGCCTGGGCACCCAGACCTACA
TCTGTAACGTGAACCACAAGCCCAGCAACACCAAGGTGGACAAGAAGGTGGAGCCCAAGAGCTGTGACAAGACCCA
CACCTGCCCCCCCTGCCCTGCCCCCGAGCTGCTGGGAGGCCCCAGCGTGTTCCTGTTCCCCCCCAAGCCTAAGGAC
ACCCTGATGATCAGCAGAACCCCCGAGGTGACCTGTGTGGTGGTGGATGTGAGCCACGAGGACCCTGAGGTGAAG
TTCAACTGGTACGTGGACGGCGTGGAGGTGCACAATGCCAAGACCAAGCCCAGGGAGGAGCAGTACAACAGCACC
TACCGGGTGGTGTCCGTGCTGACCGTGCTGCACCAGGATTGGCTGAACGGCAAGGAGTACAAGTGTAAGGTGTCC
AACAAGGCCCTGCCTGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAGGGCTTCTACCCCAG

CGACATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAG

**SEQ. ID NO.44 S260116C12 chimera heavy chain (amino acid sequence)**

QIQLRQSGAGLMKPGASVKLSCKATGYTFTGYWIEWVKQRPGHDLEWIGEILPGSGTTNYNEKFKGKATFTADTSSNT
AYMQLSSLTTEDSAIYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.45 S260116C12 chimera light chain (DNA sequence)**

GACATCGTGCTGACCCAGTCCCCCGCCTCCCTGGCCGTGTCCCTGGGCCAGCGCGCCACCATCTCCTGCCGCGCCT
CCGAGTCCGTGTCCATCCACGGCACCCACCTGATGCACTGGTACCAGCAGAAGCCCGGCCAGCCCCCCAAGCTGCT
GATCTACGCCGCCTCCAACCTGGAGTCCGGCGTGCCCGCCCGCTTCTCCGGCTCCGGCTCCGAGACCGACTTCACC
CTGAACATCCACCCCGTGGAGGAGGAGGACGCCGCCACCTACTTCTGCCAGCAGTCCATCGAGGACCCCTACACCT
TCGGCGGCGGCACCAAGCTGGAGATCAAGCGTACGGTGGCCGCCCCCAGCGTGTTCATCTTCCCCCCCAGCGATG
AGCAGCTGAAGAGCGGCACCGCCAGCGTGGTGTGTCTGCTGAACAACTTCTACCCCCGGGAGGCCAAGGTGCAGT
GGAAGGTGGACAATGCCCTGCAGAGCGGCAACAGCCAGGAGAGCGTGACCGAGCAGGACAGCAAGGACTCCACCT
ACAGCCTGAGCAGCACCCTGACCCTGAGCAAGGCCGACTACGAGAAGCACAAGGTGTACGCCTGTGAGGTGACCC
ACCAGGGCCTGTCCAGCCCCGTGACCAAGAGCTTCAACCGGGGCGAGTGC

**SEQ. ID NO.46 S260116C12 chimera light chain (amino acid sequence)**

DIVLTQSPASLAVSLGQRATISCRASESVSIHGTHLMHWYQQKPGQPPKLLIYAASNLESGVPARFSGSGSETDFTLNIH
PVEEEDAATYFCQQSIEDPYTFGGGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ. ID NO.47 S260105B02 chimera heavy chain (DNA sequence)**

CAGGTGCAGCTGCAGCAGTCCGGCGCCGAGCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGGCCTGGAGTGGATCGGCGAG
ATCCTGCCCGGCTCCGGCTCCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CGGCTACCACGACGCCTGGTTCGCCTACTGGGGCCAGGGCACACTAGTGACCGTGTCCAGCGCCAGCACCAAGGG
CCCCAGCGTGTTCCCCCTGGCCCCCAGCAGCAAGAGCACCAGCGGCGGCACAGCCGCCCTGGGCTGCCTGGTGAA
GGACTACTTCCCCGAACCGGTGACCGTGTCCTGGAACAGCGGAGCCCTGACCAGCGGCGTGCACACCTTCCCCGCC
GTGCTGCAGAGCAGCGGCCTGTACAGCCTGAGCAGCGTGGTGACCGTGCCCAGCAGCAGCCTGGGCACCCAGACC
TACATCTGTAACGTGAACCACAAGCCCAGCAACACCAAGGTGGACAAGAAGGTGGAGCCCAAGAGCTGTGACAAGA
CCCACACCTGCCCCCCCTGCCCTGCCCCCGAGCTGCTGGGAGGCCCCAGCGTGTTCCTGTTCCCCCCCAAGCCTAA
GGACACCCTGATGATCAGCAGAACCCCCGAGGTGACCTGTGTGGTGGTGGATGTGAGCCACGAGGACCCTGAGGT
GAAGTTCAACTGGTACGTGGACGGCGTGGAGGTGCACAATGCCAAGACCAAGCCCAGGGAGGAGCAGTACAACAG
CACCTACCGGGTGGTGTCCGTGCTGACCGTGCTGCACCAGGATTGGCTGAACGGCAAGGAGTACAAGTGTAAGGT
GTCCAACAAGGCCCTGCCTGCCCCTATCGAGAAAACCATCAGCAAGGCCAAGGGCCAGCCCAGAGAGCCCCAGGTG
TACACCCTGCCCCCTAGCAGAGATGAGCTGACCAAGAACCAGGTGTCCCTGACCTGCCTGGTGAAGGGCTTCTACC
CCAGCGACATCGCCGTGGAGTGGGAGAGCAACGGCCAGCCCGAGAACAACTACAAGACCACCCCCCCTGTGCTGG
ACAGCGATGGCAGCTTCTTCCTGTACAGCAAGCTGACCGTGGACAAGAGCAGATGGCAGCAGGGCAACGTGTTCA
GCTGCTCCGTGATGCACGAGGCCCTGCACAATCACTACACCCAGAAGAGCCTGAGCCTGTCCCCTGGCAAG

**SEQ. ID NO.48 S260105B02 chimera heavy chain (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYGYHDAWFAYWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSPGK

**SEQ.** ID **NO.49 S260105B02** chimera light chain **(DNA sequence)**

GACATCGTGATGTCCCAGTCCCCCTCCTCCCTGGCCGTGTCCGTGGGCGAGAAGGTGACCATGTCCTGCAAGTCCT
CCCAGTCCCTGCTGTACTCCTCCAACCAGAAGAACTACCTGGCCTGGTACCAGCAGAAGCCCGGCCAGTCCCCCAA
GCTGCTGATCTACTGGGCCTCCACCCGCGAGTCCGGCGTGCCCGACCGCTTCACCGGCTCCGGCTCCGGCACCGAC
TTCACCCTGACCATCTCCTCCGTGAAGGCCGAGGACCTGGCCGTGTACTACTGCCAGCAGTACTACTCCTACCCCT
ACACCTTCGGCGGCGGCACCAAGCTGGAGATCAAGCGTACGGTGGCCGCCCCCAGCGTGTTCATCTTCCCCCCCAG
CGATGAGCAGCTGAAGAGCGGCACCGCCAGCGTGGTGTGTCTGCTGAACAACTTCTACCCCCGGGAGGCCAAGGT
GCAGTGGAAGGTGGACAATGCCCTGCAGAGCGGCAACAGCCAGGAGAGCGTGACCGAGCAGGACAGCAAGGACTC
CACCTACAGCCTGAGCAGCACCCTGACCCTGAGCAAGGCCGACTACGAGAAGCACAAGGTGTACGCCTGTGAGGTG
ACCCACCAGGGCCTGTCCAGCCCCGTGACCAAGAGCTTCAACCGGGGCGAGTGC

**SEQ. ID NO.50 S260105B02 chimera light chain (amino acid sequence)**

DIVMSQSPSSLAVSVGEKVTMSCKSSQSLLYSSNQKNYLAWYQQKPGQSPKLLIYWASTRESGVPDRFTGSGSGTDFT
LTISSVKAEDLAVYYCQQYYSYPYTFGGGTKLEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSFNRGEC

**SEQ. ID NO.51 S260115C11 chimera heavy chain (DNA sequence)**

CAGGTGCAGCTGCAGCAGTCCGGCGCCGAGCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGGCCTGGAGTGGATCGGCGAG
ATCCTGCCCGGCTCCGGCTCCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CTACTACGGCTCCTCCTTCGACTACTGGGGCCAGGGCACACTAGTGACCGTGTCCAGCGCCAGCACCAAGGGCCCC
AGCGTGTTCCCCCTGGCCCCCAGCAGCAAGAGCACCAGCGGCGGCACAGCCGCCCTGGGCTGCCTGGTGAAGGAC
TACTTCCCCGAACCGGTGACCGTGTCCTGGAACAGCGGAGCCCTGACCAGCGGCGTGCACACCTTCCCCGCCGTGC
TGCAGAGCAGCGGCCTGTACAGCCTGAGCAGCGTGGTGACCGTGCCCAGCAGCAGCCTGGGCACCCAGACCTACA
TCTGTAACGTGAACCACAAGCCCAGCAACACCAAGGTGGACAAGAAGGTGGAGCCCAAGAGCTGTGACAAGACCCA
CACCTGCCCCCCCTGCCCTGCCCCCGAGCTGCTGGGAGGCCCCAGCGTGTTCCTGTTCCCCCCCAAGCCTAAGGAC
ACCCTGATGATCAGCAGAACCCCCGAGGTGACCTGTGTGGTGGTGGATGTGAGCCACGAGGACCCTGAGGTGAAG
TTCAACTGGTACGTGGACGGCGTGGAGGTGCACAATGCCAAGACCAAGCCCAGGGAGGAGCAGTACAACAGCACC
TACCGGGTGGTGTCCGTGCTGACCGTGCTGCACCAGGATTGGCTGAACGGCAAGGAGTACAAGTGTAAGGTGTCC
AACAAGGCCCTGCCTGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAGGGCTTCTACCCCAG
CGACATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAG

**SEQ. ID NO.52 S260115C11 chimera heavy chain (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYYYGSSFDYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.53 S260115C11 chimera light chain (DNA sequence)**

CAGATCGTGCTGACCCAGTCCCCCGCCATCATGTCCGCCTCCCTGGGCGAGCGCGTGACCATGACCTGCACCGCCT
CCTCCTCCGTGTCCTCCTCCTACCTGCACTGGTACCAGCAGAAGCCCGGCTCCTCCCCCAAGCTGTGGATCTACTCC
ACCTCCAACCTGGCCTCCGGCGTGCCCGCCCGCTTCTCCGGCTCCGGCTCCGGCACCTCCTACTCCCTGACCATCT
CCTCCATGGAGGCCGAGGACGCCGCCACCTACTACTGCCACCAGTACCACCGCTCCCCCCTGACCTTCGGCGCCGG
CACCAAGCTGGAGATCAAGCGTACGGTGGCCGCCCCCAGCGTGTTCATCTTCCCCCCCAGCGATGAGCAGCTGAAG
AGCGGCACCGCCAGCGTGGTGTGTCTGCTGAACAACTTCTACCCCCGGGAGGCCAAGGTGCAGTGGAAGGTGGAC
AATGCCCTGCAGAGCGGCAACAGCCAGGAGAGCGTGACCGAGCAGGACAGCAAGGACTCCACCTACAGCCTGAGC
AGCACCCTGACCCTGAGCAAGGCCGACTACGAGAAGCACAAGGTGTACGCCTGTGAGGTGACCCACCAGGGCCTG
TCCAGCCCCGTGACCAAGAGCTTCAACCGGGGCGAGTGC

**SEQ.** ID **NO.54 S260115C11** chimera light chain (amino acid sequence)

QIVLTQSPAIMSASLGERVTMTCTASSSVSSSYLHWYQQKPGSSPKLWIYSTSNLASGVPARFSGSGSGTSYSLTISSM
EAEDAATYYCHQYHRSPLTFGAGTKLEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

**SEQ. ID NO.55 S265109B10 chimera heavy chain (DNA sequence)**

CAGGTGCAGCTCCAGCAGAGCGGAGCCGAGCTGATGAAACCCGGGGCCAGCGTGAAGCTGAGCTGCAAGGCCACC
GGCTACACCTTCACCGGCTACTGGATCGAGTGGGTGAAGCAGAGGCCCGGCCACGGCCTGGAGTGGATCGGCGAA
ATCCTGCCCGGCAGCAGCAGCACCAACTACAACGAGAAGTTCAAGGACAAGGCCACCTTCACCGCCGACACTAGCA
GCAACACCGCCTACATGCAGCTGAGCAGCCTGACAACCGAGGACTCCGCAATCTACTACTGCGCCAGGGGCGGCTA
CTACTACGGCAGCCCCATGGACTATTGGGGCCAGGGCACACTAGTGACCGTGTCCAGCGCCAGCACCAAGGGCCCC
AGCGTGTTCCCCCTGGCCCCCAGCAGCAAGAGCACCAGCGGCGGCACAGCCGCCCTGGGCTGCCTGGTGAAGGAC
TACTTCCCCGAACCGGTGACCGTGTCCTGGAACAGCGGAGCCCTGACCAGCGGCGTGCACACCTTCCCCGCCGTGC
TGCAGAGCAGCGGCCTGTACAGCCTGAGCAGCGTGGTGACCGTGCCCAGCAGCAGCCTGGGCACCCAGACCTACA
TCTGTAACGTGAACCACAAGCCCAGCAACACCAAGGTGGACAAGAAGGTGGAGCCCAAGAGCTGTGACAAGACCCA
CACCTGCCCCCCCTGCCCTGCCCCCGAGCTGCTGGGAGGCCCCAGCGTGTTCCTGTTCCCCCCCAAGCCTAAGGAC
ACCCTGATGATCAGCAGAACCCCCGAGGTGACCTGTGTGGTGGTGGATGTGAGCCACGAGGACCCTGAGGTGAAG
TTCAACTGGTACGTGGACGGCGTGGAGGTGCACAATGCCAAGACCAAGCCCAGGGAGGAGCAGTACAACAGCACC
TACCGGGTGGTGTCCGTGCTGACCGTGCTGCACCAGGATTGGCTGAACGGCAAGGAGTACAAGTGTAAGGTGTCC
AACAAGGCCCTGCCTGCCCCTATCGAGAAAACCATCAGCAAGGCCAAGGGCCAGCCCAGAGAGCCCCAGGTGTACA
CCCTGCCCCCTAGCAGAGATGAGCTGACCAAGAACCAGGTGTCCCTGACCTGCCTGGTGAAGGGCTTCTACCCCAG
CGACATCGCCGTGGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAACGTGTTCAGCTG
CTCCGTGATGCACGAGGCCCTGCACAATCACTACACCCAGAAGAGCCTGAGCCTGTCCCCTGGCAAG

**SEQ. ID NO.56 S265109B10 chimera heavy chain (amino acid sequence)**

QVQLQQSGAELMKPGASVKLSCKATGYTFTGYWIEWVKQRPGHGLEWIGEILPGSSSTNYNEKFKDKATFTADTSSN
TAYMQLSSLTTEDSAIYYCARGGYYYGSPMDYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.57 S265109B10 chimera light chain (DNA sequence)**

CAGATCGTGCTGACCCAGAGCCCCGCCATTATGAGCGCTAGCCCCGGGGAGAAGGTGACCATGACCTGCAGCGCC
AGGAGCAGCGTGAGCTACATGCACTGGTACCAGCAGAAGAGCGGCACCAGCCCCAAGAGGTGGATCTACGACACC
AGCAAGCTGGCCTCAGGCGTGCCCGCCAGGTTCAGCGGCTCTGGCAGCGGCACCAGCTACAGCCTGACCATCTCCA
GCATGGAGGCCGAGGACGCCGCCACCTACTATTGCCAGCAGTGGAGCAGCAACCCTCCCACTTTCGGCGGCGGCA
CCAAACTGGAGATCAAGCGTACGGTGGCCGCCCCCAGCGTGTTCATCTTCCCCCCCAGCGATGAGCAGCTGAAGAG
CGGCACCGCCAGCGTGGTGTGTCTGCTGAACAACTTCTACCCCCGGGAGGCCAAGGTGCAGTGGAAGGTGGACAA
TGCCCTGCAGAGCGGCAACAGCCAGGAGAGCGTGACCGAGCAGGACAGCAAGGACTCCACCTACAGCCTGAGCAG
CACCCTGACCCTGAGCAAGGCCGACTACGAGAAGCACAAGGTGTACGCCTGTGAGGTGACCCACCAGGGCCTGTCC
AGCCCCGTGACCAAGAGCTTCAACCGGGGCGAGTGC

**SEQ. ID NO.58 S265109B10 chimera light chain (amino acid sequence)**

QIVLTQSPAIMSASPGEKVTMTCSARSSVSYMHWYQQKSGTSPKRWIYDTSKLASGVPARFSGSGSGTSYSLTISSME
AEDAATYYCQQWSSNPPTFGGGTKLEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

**SEQ. ID NO.59 S260116C12 chimera heavy chain variable region (DNA sequence)**

CAGATCCAGCTGCGCCAGTCCGGCGCCGGCCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGACCTGGAGTGGATCGGCGAG
ATCCTGCCCGGCTCCGGCACCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CTACTACGGCTCCTCCTACGACTCCTGGGGCCAGGGCACACTAGTGACCGTGTCCAGC

**SEQ.** ID **NO.60 S260116C12 chimera heavy chain variable region (amino** acid sequence)

QIQLRQSGAGLMKPGASVKLSCKATGYTFTGYWIEWVKQRPGHDLEWIGEILPGSGTTNYNEKFKGKATFTADTSSNT
AYMQLSSLTTEDSAIYYCARGGYYYGSSYDSWGQGTLVTVSS

**SEQ. ID NO.61 S260116C12 chimera light chain variable region (DNA sequence)**

GACATCGTGCTGACCCAGTCCCCCGCCTCCCTGGCCGTGTCCCTGGGCCAGCGCGCCACCATCTCCTGCCGCGCCT
CCGAGTCCGTGTCCATCCACGGCACCCACCTGATGCACTGGTACCAGCAGAAGCCCGGCCAGCCCCCCAAGCTGCT
GATCTACGCCGCCTCCAACCTGGAGTCCGGCGTGCCCGCCCGCTTCTCCGGCTCCGGCTCCGAGACCGACTTCACC
CTGAACATCCACCCCGTGGAGGAGGAGGACGCCGCCACCTACTTCTGCCAGCAGTCCATCGAGGACCCCTACACCT
TCGGCGGCGGCACCAAGCTGGAGATCAAGCGT

**SEQ. ID NO.62 S260116C12 chimera light chain variable region (amino acid sequence)**

DIVLTQSPASLAVSLGQRATISCRASESVSIHGTHLMHWYQQKPGQPPKLLIYAASNLESGVPARFSGSGSETDFTLNIH
PVEEEDAATYFCQQSIEDPYTFGGGTKLEIKR

**SEQ. ID NO.63 S260105B02 chimera heavy chain variable region (DNA sequence)**

CAGGTGCAGCTGCAGCAGTCCGGCGCCGAGCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGGCCTGGAGTGGATCGGCGAG
ATCCTGCCCGGCTCCGGCTCCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CGGCTACCACGACGCCTGGTTCGCCTACTGGGGCCAGGGCACACTAGTGACCGTGTCCAGC

**SEQ. ID NO.64 S260105B02 chimera heavy chain variable region (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYGYHDAWFAYWGQGTLVTVSS

**SEQ. ID NO.65 S260105B02 chimera light chain variable region (DNA sequence)**

GACATCGTGATGTCCCAGTCCCCCTCCTCCCTGGCCGTGTCCGTGGGCGAGAAGGTGACCATGTCCTGCAAGTCCT
CCCAGTCCCTGCTGTACTCCTCCAACCAGAAGAACTACCTGGCCTGGTACCAGCAGAAGCCCGGCCAGTCCCCCAA
GCTGCTGATCTACTGGGCCTCCACCCGCGAGTCCGGCGTGCCCGACCGCTTCACCGGCTCCGGCTCCGGCACCGAC
TTCACCCTGACCATCTCCTCCGTGAAGGCCGAGGACCTGGCCGTGTACTACTGCCAGCAGTACTACTCCTACCCCT
ACACCTTCGGCGGCGGCACCAAGCTGGAGATCAAGCGT

**SEQ. ID NO.66 S260105B02 chimera light chain variable region (amino acid sequence)**

DIVMSQSPSSLAVSVGEKVTMSCKSSQSLLYSSNQKNYLAWYQQKPGQSPKLLIYWASTRESGVPDRFTGSGSGTDFT
LTISSVKAEDLAVYYCQQYYSYPYTFGGGTKLEIKR

**SEQ. ID NO.67 S260115C11 chimera heavy chain variable region (DNA sequence)**

CAGGTGCAGCTGCAGCAGTCCGGCGCCGAGCTGATGAAGCCCGGCGCCTCCGTGAAGCTGTCCTGCAAGGCCACC
GGCTACACCTTCACCGGCTACTGGATCGAGTGGGTGAAGCAGCGCCCCGGCCACGGCCTGGAGTGGATCGGCGAG
ATCCTGCCCGGCTCCGGCTCCACCAACTACAACGAGAAGTTCAAGGGCAAGGCCACCTTCACCGCCGACACCTCCT
CCAACACCGCCTACATGCAGCTGTCCTCCCTGACCACCGAGGACTCCGCCATCTACTACTGCGCCCGCGGCGGCTA
CTACTACGGCTCCTCCTTCGACTACTGGGGCCAGGGCACACTAGTGACCGTGTCCAGC

**SEQ. ID NO.68 S260115C11 chimera heavy chain variable region (amino acid sequence)**

QVQLQQSGAELMKPGASVKLSCKATGYTFTGYWIEWVKQRPGHGLEWIGEILPGSGSTNYNEKFKGKATFTADTSSN
TAYMQLSSLTTEDSAIYYCARGGYYYGSSFDYWGQGTLVTVSS

**SEQ. ID NO.69 S260115C11 chimera light chain variable region (DNA sequence)**

CAGATCGTGCTGACCCAGTCCCCCGCCATCATGTCCGCCTCCCTGGGCGAGCGCGTGACCATGACCTGCACCGCCT
CCTCCTCCGTGTCCTCCTCCTACCTGCACTGGTACCAGCAGAAGCCCGGCTCCTCCCCCAAGCTGTGGATCTACTCC
ACCTCCAACCTGGCCTCCGGCGTGCCCGCCCGCTTCTCCGGCTCCGGCTCCGGCACCTCCTACTCCCTGACCATCT
CCTCCATGGAGGCCGAGGACGCCGCCACCTACTACTGCCACCAGTACCACCGCTCCCCCCTGACCTTCGGCGCCGG
CACCAAGCTGGAGATCAAGCGT

**SEQ. ID NO.70 S260115C11 chimera light chain variable region (amino acid sequence)**

QIVLTQSPAIMSASLGERVTMTCTASSSVSSSYLHWYQQKPGSSPKLWIYSTSNLASGVPARFSGSGSGTSYSLTISSM
EAEDAATYYCHQYHRSPLTFGAGTKLEIKR

**SEQ. ID NO.71 S265109B10 chimera heavy chain variable region (DNA sequence)**

CAGGTGCAGCTCCAGCAGAGCGGAGCCGAGCTGATGAAACCCGGGGCCAGCGTGAAGCTGAGCTGCAAGGCCACC
GGCTACACCTTCACCGGCTACTGGATCGAGTGGGTGAAGCAGAGGCCCGGCCACGGCCTGGAGTGGATCGGCGAA
ATCCTGCCCGGCAGCAGCAGCACCAACTACAACGAGAAGTTCAAGGACAAGGCCACCTTCACCGCCGACACTAGCA

GCAACACCGCCTACATGCAGCTGAGCAGCCTGACAACCGAGGACTCCGCAATCTACTACTGCGCCAGGGGCGGCTA
CTACTACGGCAGCCCCATGGACTATTGGGGCCAGGGCACACTAGTGACCGTGTCCAGC

**SEQ. ID NO.72 S265109B10 chimera heavy chain variable region (amino acid sequence)**

QVQLQQSGAELMKPGASVKLSCKATGYTFTGYWIEWVKQRPGHGLEWIGEILPGSSSTNYNEKFKDKATFTADTSSN
TAYMQLSSLTTEDSAIYYCARGGYYYGSPMDYWGQGTLVTVSS

**SEQ. ID NO.73 S265109B10 chimera light chain variable region (DNA sequence)**

CAGATCGTGCTGACCCAGAGCCCCGCCATTATGAGCGCTAGCCCCGGGGAGAAGGTGACCATGACCTGCAGCGCC
AGGAGCAGCGTGAGCTACATGCACTGGTACCAGCAGAAGAGCGGCACCAGCCCCAAGAGGTGGATCTACGACACC
AGCAAGCTGGCCTCAGGCGTGCCCGCCAGGTTCAGCGGCTCTGGCAGCGGCACCAGCTACAGCCTGACCATCTCCA
GCATGGAGGCCGAGGACGCCGCCACCTACTATTGCCAGCAGTGGAGCAGCAACCCTCCCACTTTCGGCGGCGGCA
CCAAACTGGAGATCAAGCGT

**SEQ. ID NO.74 S265109B10 chimera light chain variable region (amino acid sequence)**

QIVLTQSPAIMSASPGEKVTMTCSARSSVSYMHWYQQKSGTSPKRWIYDTSKLASGVPARFSGSGSGTSYSLTISSME
AEDAATYYCQQWSSNPPTFGGGTKLEIKR

**SEQ. ID NO.75 S260116C12 humanized Ha2 heavy chain (DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATGGGCGAG
ATCCTGCCCGGGTCTGGCACCACCAACTACAACGAGAAGTTCAAGGGCCGCGTGACCATCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGCGCCAGCACCAAGGGCCC
CAGCGTGTTCCCCCTGGCCCCCAGCAGCAAGAGCACCAGCGGCGGCACAGCCGCCCTGGGCTGCCTGGTGAAGGA
CTACTTCCCCGAACCGGTGACCGTGTCCTGGAACAGCGGAGCCCTGACCAGCGGCGTGCACACCTTCCCCGCCGTG
CTGCAGAGCAGCGGCCTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCCCCCAAGCCTAAGGA
CACCCTGATGATCAGCAGAACCCCCGAGGTGACCTGTGTGGTGGTGGATGTGAGCCACGAGGACCCTGAGGTGAA
GTTCAACTGGTACGTGGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGTGTAC
ACCCTGCCCCCTAGCAGAGATGAGCTGACCAAGAACCAGGTGTCCCTGACCTGCCTGGTGAAGGGCTTCTACCCCA
GCGACATCGCCGTGGAGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGGGCAACGTGTTCAGCT
GCTCCGTGATGCACGAGGCCCTGCACAATCACTACACCCAGAAGAGCCTGAGCCTGTCCCCTGGCAAG

**SEQ. ID NO.76 S260116C12 humanized Ha2 heavy chain (amino acid sequence)**

QIQLVQSGAEVKKPGSSVKVSCKASGYTFTGYWIEWVRQAPGQGLEWMGEILPGSGTTNYNEKFKGRVTITADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.77 S260116C12 humanized Ha4 heavy chain (DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCCGCGCCACCTTCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGCGCCAGCACCAAGGGCCC
CAGCGTGTTCCCCCTGGCCCCCAGCAGCAAGAGCACCAGCGGCGGCACAGCCGCCCTGGGCTGCCTGGTGAAGGA
CTACTTCCCCGAACCGGTGACCGTGTCCTGGAACAGCGGAGCCCTGACCAGCGGCGTGCACACCTTCCCCGCCGTG
CTGCAGAGCAGCGGCCTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCCCCCAAGCCTAAGGA

CACCCTGATGATCAGCAGAACCCCCGAGGTGACCTGTGTGGTGGTGGATGTGAGCCACGAGGACCCTGAGGTGAA
GTTCAACTGGTACGTGGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGTGTAC
ACCCTGCCCCCTAGCAGAGATGAGCTGACCAAGAACCAGGTGTCCCTGACCTGCCTGGTGAAGGGCTTCTACCCCA
GCGACATCGCCGTGGAGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGGGCAACGTGTTCAGCT
GCTCCGTGATGCACGAGGCCCTGCACAATCACTACACCCAGAAGAGCCTGAGCCTGTCCCCTGGCAAG

**SEQ. ID NO.78 S260116C12 humanized Ha4 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.79 S260116C12 humanized Ha5 heavy chain (DNA sequence)**

CAGATCCAGCTGAGGCAGAGCGGCGCCGAGGTGAAAAAGCCCGGCGCCAGCGTGAAGCTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCAAGGCCACCTTCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGCGCCAGCACCAAGGGCCC
CAGCGTGTTCCCCCTGGCCCCCAGCAGCAAGAGCACCAGCGGCGGCACAGCCGCCCTGGGCTGCCTGGTGAAGGA
CTACTTCCCCGAACCGGTGACCGTGTCCTGGAACAGCGGAGCCCTGACCAGCGGCGTGCACACCTTCCCCGCCGTG
CTGCAGAGCAGCGGCCTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCCCCCAAGCCTAAGGA
CACCCTGATGATCAGCAGAACCCCCGAGGTGACCTGTGTGGTGGTGGATGTGAGCCACGAGGACCCTGAGGTGAA
GTTCAACTGGTACGTGGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGTGTAC
ACCCTGCCCCCTAGCAGAGATGAGCTGACCAAGAACCAGGTGTCCCTGACCTGCCTGGTGAAGGGCTTCTACCCCA
GCGACATCGCCGTGGAGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGGGCAACGTGTTCAGCT
GCTCCGTGATGCACGAGGCCCTGCACAATCACTACACCCAGAAGAGCCTGAGCCTGTCCCCTGGCAAG

**SEQ. ID NO.80 S260116C12 humanized Ha5 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ.** ID **NO.81** S260116C12 humanized Ha6 **heavy** chain **(DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCCGCGCCACCTTCACTGCCGACAAGTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGCGCCAGCACCAAGGGCCC
CAGCGTGTTCCCCCTGGCCCCCAGCAGCAAGAGCACCAGCGGCGGCACAGCCGCCCTGGGCTGCCTGGTGAAGGA
CTACTTCCCCGAACCGGTGACCGTGTCCTGGAACAGCGGAGCCCTGACCAGCGGCGTGCACACCTTCCCCGCCGTG
CTGCAGAGCAGCGGCCTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAGAGCTGTGACAAGACCC

ACACCTGCCCCCCCTGCCCTGCCCCCGAGCTGCTGGGAGGCCCCAGCGTGTTCCTGTTCCCCCCCAAGCCTAAGGA
CACCCTGATGATCAGCAGAACCCCCGAGGTGACCTGTGTGGTGGTGGATGTGAGCCACGAGGACCCTGAGGTGAA
GTTCAACTGGTACGTGGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGTGTAC
ACCCTGCCCCCTAGCAGAGATGAGCTGACCAAGAACCAGGTGTCCCTGACCTGCCTGGTGAAGGGCTTCTACCCCA
GCGACATCGCCGTGGAGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGGGCAACGTGTTCAGCT
GCTCCGTGATGCACGAGGCCCTGCACAATCACTACACCCAGAAGAGCCTGAGCCTGTCCCCTGGCAAG

**SEQ. ID NO.82 S260116C12 humanized Ha6 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.83 S260116C12 humanized Ha7 heavy chain (DNA sequence)**

CAGGTGCAGCTGGTGCAGAGCGGCGCCGAGGTGAAAAAGCCCGGCAGCAGCGTGAAGGTGAGCTGCAAGGCCAG
CGGCTACACCTTCACCGGCTACTGGATCGAGTGGGTGAGGCAGGCTCCCGGACAGGGCCTGGAGTGGATCGGCGA
GATCCTGCCCGGGTCTGGCACCACCAACTACAACGAGAAGTTCAAGGGCCGCGCCACCTTCACTGCCGACACCTCC
ACCAGCACCGCCTACATGGAACTGAGCAGCCTCAGGAGCGAAGACACCGCCGTCTACTATTGCGCCAGGGGCGGCT
ACTACTACGGCAGCAGCTACGACAGCTGGGGCCAGGGCACACTAGTGACCGTGTCCAGCGCCAGCACCAAGGGCC
CCAGCGTGTTCCCCCTGGCCCCCAGCAGCAAGAGCACCAGCGGCGGCACAGCCGCCCTGGGCTGCCTGGTGAAGG
ACTACTTCCCCGAACCGGTGACCGTGTCCTGGAACAGCGGAGCCCTGACCAGCGGCGTGCACACCTTCCCCGCCGT
GCTGCAGAGCAGCGGCCTGTACAGCCTGAGCAGCGTGGTGACCGTGCCCAGCAGCAGCCTGGGCACCCAGACCTA
CATCTGTAACGTGAACCACAAGCCCAGCAACACCAAGGTGGACAAGAAGGTGGAGCCCAAGAGCTGTGACAAGACC
CACACCTGCCCCCCCTGCCCTGCCCCCGAGCTGCTGGGAGGCCCCAGCGTGTTCCTGTTCCCCCCCAAGCCTAAGG
ACACCCTGATGATCAGCAGAACCCCCGAGGTGACCTGTGTGGTGGTGGATGTGAGCCACGAGGACCCTGAGGTGA
AGTTCAACTGGTACGTGGACGGCGTGGAGGTGCACAATGCCAAGACCAAGCCCAGGGAGGAGCAGTACAACAGCA
CCTACCGGGTGGTGTCCGTGCTGACCGTGCTGCACCAGGATTGGCTGAACGGCAAGGAGTACAAGTGTAAGGTGT
CCAACAAGGCCCTGCCTGCCCCTATCGAGAAAACCATCAGCAAGGCCAAGGGCCAGCCCAGAGAGCCCCAGGTGTA
CACCCTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTGGAC
AGCGATGGCAGCTTCTTCCTGTACAGCAAGCTGACCGTGGACAAGAGCAGATGGCAGCAGGGCAACGTGTTCAGC
TGCTCCGTGATGCACGAGGCCCTGCACAATCACTACACCCAGAAGAGCCTGAGCCTGTCCCCTGGCAAG

**SEQ. ID NO.84 S260116C12 humanized Ha7 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

**SEQ. ID NO.85 S260116C12 humanized La0 light chain (DNA sequence)**

GACATCGTGATGACCCAGTCCCCCGATAGCCTGGCTGTGTCACTGGGGGAGAGGGCCACCATCAACTGCAGGGCC
AGCGAGTCCGTGAGCATCCACGGCACCCACCTGATGCACTGGTACCAGCAGAAGCCCGGCCAGCCCCCTAAGCTGC
TGATCTACGCCGCCAGCAACCTCGAAAGCGGCGTCCCCGACAGGTTCAGCGGCAGCGGCAGCGGCACCGACTTCA
CCCTGACTATCAGCAGCCTGCAGGCCGAGGACGTGGCCGTCTACTACTGCCAGCAGAGCATCGAGGACCCCTACAC
CTTCGGCCAGGGCACCAAGCTGGAGATCAAGCGTACGGTGGCCGCCCCCAGCGTGTTCATCTTCCCCCCCAGCGAT
GAGCAGCTGAAGAGCGGCACCGCCAGCGTGGTGTGTCTGCTGAACAACTTCTACCCCCGGGAGGCCAAGGTGCAG
TGGAAGGTGGACAATGCCCTGCAGAGCGGCAACAGCCAGGAGAGCGTGACCGAGCAGGACAGCAAGGACTCCACC

TACAGCCTGAGCAGCACCCTGACCCTGAGCAAGGCCGACTACGAGAAGCACAAGGTGTACGCCTGTGAGGTGACCC
ACCAGGGCCTGTCCAGCCCCGTGACCAAGAGCTTCAACCGGGGCGAGTGC

**SEQ. ID NO.86 S260116C12 humanized La0 light chain (amino acid sequence)**

DIVMTQSPDSLAVSLGERATINCRASESVSIHGTHLMHWYQQKPGQPPKLLIYAASNLESGVPDRFSGSGSGTDFTLTI
SSLQAEDVAVYYCQQSIEDPYTFGQGTKLEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGEC

**SEQ. ID NO.87 S260116C12 humanized La1 light chain (DNA sequence)**

GACATCGTGCTGACCCAGTCCCCCGATAGCCTGGCTGTGTCACTGGGGGAGAGGGCCACCATCAACTGCAGGGCC
AGCGAGTCCGTGAGCATCCACGGCACCCACCTGATGCACTGGTACCAGCAGAAGCCCGGCCAGCCCCCTAAGCTGC
TGATCTACGCCGCCAGCAACCTCGAAAGCGGCGTCCCCGACAGGTTCAGCGGCAGCGGCAGCGAGACCGACTTCA
CCCTGACTATCAGCAGCCTGCAGGCCGAGGACGTGGCCGTCTACTACTGCCAGCAGAGCATCGAGGACCCCTACAC
CTTCGGCCAGGGCACCAAGCTGGAGATCAAGCGTACGGTGGCCGCCCCCAGCGTGTTCATCTTCCCCCCCAGCGAT
GAGCAGCTGAAGAGCGGCACCGCCAGCGTGGTGTGTCTGCTGAACAACTTCTACCCCCGGGAGGCCAAGGTGCAG
TGGAAGGTGGACAATGCCCTGCAGAGCGGCAACAGCCAGGAGAGCGTGACCGAGCAGGACAGCAAGGACTCCACC
TACAGCCTGAGCAGCACCCTGACCCTGAGCAAGGCCGACTACGAGAAGCACAAGGTGTACGCCTGTGAGGTGACCC
ACCAGGGCCTGTCCAGCCCCGTGACCAAGAGCTTCAACCGGGGCGAGTGC

**SEQ. ID NO.88 S260116C12 humanized La1 light chain (amino acid sequence)**

DIVLTQSPDSLAVSLGERATINCRASESVSIHGTHLMHWYQQKPGQPPKLLIYAASNLESGVPDRFSGSGSETDFTLTIS
SLQAEDVAVYYCQQSIEDPYTFGQGTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKSFNRGEC

**SEQ. ID NO.89.S260116C12 humanized Ha2 heavy chain variable region (DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATGGGCGAG
ATCCTGCCCGGGTCTGGCACCACCAACTACAACGAGAAGTTCAAGGGCCGCGTGACCATCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGC

**SEQ. ID NO.90 S260116C12 humanized Ha2 heavy chain variable region (amino acid sequence)**

QIQLVQSGAEVKKPGSSVKVSCKASGYTFTGYWIEWVRQAPGQGLEWMGEILPGSGTTNYNEKFKGRVTITADTSTS
TAYMELSSLRSEDTAVYYCARGGYYYGSSYDSWGQGTLVTVSS

**SEQ. ID NO.91 S260116C12 humanized Ha4 heavy chain variable region (DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCCGCGCCACCTTCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGC

**SEQ. ID NO.92 S260116C12 humanized Ha4 heavy chain variable region (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSS

**SEQ.** ID **NO.93 S260116C12 humanized Ha5 heavy chain variable region (DNA sequence)**

CAGATCCAGCTGAGGCAGAGCGGCGCCGAGGTGAAAAAGCCCGGCGCCAGCGTGAAGCTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCAAGGCCACCTTCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGC

**SEQ. ID NO.94 S260116C12 humanized Ha5 heavy chain variable region (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSS

**SEQ. ID NO.95 S260116C12 humanized Ha6 heavy chain variable region (DNA sequence)**

CAGATCCAGCTGGTGCAGAGCGGCGCCGAGGTGAAAAAGCCCGGCAGCAGCGTGAAGGTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCCGCGCCACCTTCACTGCCGACAAGTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGC

**SEQ. ID NO.96 S260116C12 humanized Ha6 heavy chain variable region (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSS

**SEQ. ID NO.97 S260116C12 humanized Ha7 heavy chain variable region (DNA sequence)**

CAGGTGCAGCTGGTGCAGAGCGGCGCCGAGGTGAAAAAGCCCGGCAGCAGCGTGAAGGTGAGCTGCAAGGCCAG
CGGCTACACCTTCACCGGCTACTGGATCGAGTGGGTGAGGCAGGCTCCCGGACAGGGCCTGGAGTGGATCGGCGA
GATCCTGCCCGGGTCTGGCACCACCAACTACAACGAGAAGTTCAAGGGCCGCGCCACCTTCACTGCCGACACCTCC
ACCAGCACCGCCTACATGGAACTGAGCAGCCTCAGGAGCGAAGACACCGCCGTCTACTATTGCGCCAGGGGCGGCT
ACTACTACGGCAGCAGCTACGACAGCTGGGGCCAGGGCACACTAGTGACCGTGTCCAGC

**SEQ. ID NO.98 S260116C12 humanized Ha7 heavy chain variable region (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSS

**SEQ. ID NO.99 S260116C12 humanized La0 light chain variable region (DNA sequence)**

GACATCGTGATGACCCAGTCCCCCGATAGCCTGGCTGTGTCACTGGGGGAGAGGGCCACCATCAACTGCAGGGCC
AGCGAGTCCGTGAGCATCCACGGCACCCACCTGATGCACTGGTACCAGCAGAAGCCCGGCCAGCCCCCTAAGCTGC
TGATCTACGCCGCCAGCAACCTCGAAAGCGGCGTCCCCGACAGGTTCAGCGGCAGCGGCAGCGGCACCGACTTCA
CCCTGACTATCAGCAGCCTGCAGGCCGAGGACGTGGCCGTCTACTACTGCCAGCAGAGCATCGAGGACCCCTACAC
CTTCGGCCAGGGCACCAAGCTGGAGATCAAGCGT

**SEQ. ID NO.100 S260116C12 humanized La0 light chain variable region (amino acid sequence)**

DIVMTQSPDSLAVSLGERATINCRASESVSIHGTHLMHWYQQKPGQPPKLLIYAASNLESGVPDRFSGSGSGTDFTLTI
SSLQAEDVAVYYCQQSIEDPYTFGQGTKLEIKR

**SEQ. ID NO.101 S260116C12 humanized La1** light chain **variable** region (DNA **sequence)**

GACATCGTGCTGACCCAGTCCCCCGATAGCCTGGCTGTGTCACTGGGGGAGAGGGCCACCATCAACTGCAGGGCC
AGCGAGTCCGTGAGCATCCACGGCACCCACCTGATGCACTGGTACCAGCAGAAGCCCGGCCAGCCCCCTAAGCTGC
TGATCTACGCCGCCAGCAACCTCGAAAGCGGCGTCCCCGACAGGTTCAGCGGCAGCGGCAGCGAGACCGACTTCA
CCCTGACTATCAGCAGCCTGCAGGCCGAGGACGTGGCCGTCTACTACTGCCAGCAGAGCATCGAGGACCCCTACAC
CTTCGGCCAGGGCACCAAGCTGGAGATCAAGCGT

**SEQ. ID NO.102 S260116C12 humanized La1 light chain variable region (amino acid sequence)**

DIVLTQSPDSLAVSLGERATINCRASESVSIHGTHLMHWYQQKPGQPPKLLIYAASNLESGVPDRFSGSGSETDFTLTIS SLQAEDVAVYYCQQSIEDPYTFGQGTKLEIKR

**SEQ. ID NO.103 Anti-VEGF 098 dAb (DNA sequence)**

GAGGTGCAGCTGTTGGAGTCTGGGGGAGGCTTGGTACAGCCTGGGGGGTCCCTGCGTCTCTCCTGTGCAGCCTCC GGATTCACCTTTAAGGATTATGATATGTGGTGGGTCCGCCAGGCTCCAGGGAAGGGTCTAGAGTGGGTCTCATCT ATTTCTGTGGAGGGTGTTCAGACATACTACGCAGACTCCGTGAAAGGCCGGTTCACCATCTCCCGCGACAATTCCA AGAACACGCTGTATCTGCAAATGAACAGCCTGCGTGCCGAGGACACCGCGGTATATTACTGTGCGAAAAATATTCG TTATGTGGGGAATCGGTCGTGGTGGACGTTTGACTACTGGGGTCAGGGAACCCTGGTCACCGTCTCGAGC

**SEQ. ID NO.104 Anti-VEGF 098 dAb (amino acid sequence)**

EVQLLESGGGLVQPGGSLRLSCAASGFTFKDYDMWWVRQAPGKGLEWVSSISVEGVQTYYADSVKGRFTISRDNSKN TLYLQMNSLRAEDTAVYYCAKNIRYVGNRSWWTFDYWGQGTLVTVSS

**SEQ. ID NO.105 Anti-VEGF 098AAA dAb (DNA sequence)**

GAGGTGCAGCTGTTGGAGTCTGGGGGAGGCTTGGTACAGCCTGGGGGGTCCCTGCGTCTCTCCTGTGCAGCCTCC GGATTCACCTTTAAGGATTATGATATGTGGTGGGTCCGCCAGGCTCCAGGGAAGGGTCTAGAGTGGGTCTCATCT

ATTTCTGTGGAGGGTGTTCAGACATACTACGCAGACTCCGTGAAAGGCCGGTTCACCATCTCCCGCGACAATTCCA AGAACACGCTGTATCTGCAAATGAACAGCCTGCGTGCCGAGGACACCGCGGTATATTACTGTGCGAAAAATATTCG TTATGTGGGGAATCGGTCGTGGTGGACGTTTGACTACTGGGGTCAGGGAACCCTGGTCACCGTCTCGAGCGCGGC CGCC

**SEQ. ID NO.106 Anti-VEGF 098AAA dAb (amino acid sequence)**

EVQLLESGGGLVQPGGSLRLSCAASGFTFKDYDMWWVRQAPGKGLEWVSSISVEGVQTYYADSVKGRFTISRDNSKN TLYLQMNSLRAEDTAVYYCAKNIRYVGNRSWWTFDYWGQGTLVTVSSAAA

**SEQ. ID NO.107 Anti-VEGF 044 dAb (DNA sequence)**

GACATCCAGATGACCCAGTCTCCATCCTCCCTGTCTGCATCTGTAGGAGACCGTGTCACCATCACTTGCCGGGCAA GTCAGTGGATTGGTCCTGAGTTAAGTTGGTACCAGCAGAAACCAGGGAAAGCCCCTAAGCTCCTGATCTATCATGG TTCCATTTTGCAAAGTGGGGTCCCATCACGTTTCAGTGGCAGTGGATCTGGGACAGACTTCACTCTCACCATCAGC AGTCTGCAACCTGAAGATTTTGCTACGTACTACTGTCAACAGTATATGTATTATCCTCATACGTTCGGCCAAGGGAC CAAGGTGGAAATCAAACGG

**SEQ. ID NO.108 Anti-VEGF 044 dAb (amino acid sequence)**

DIQMTQSPSSLSASVGDRVTITCRASQWIGPELSWYQQKPGKAPKLLIYHGSILQSGVPSRFSGSGSGTDFTLTISSLQ PEDFATYYCQQYMYYPHTFGQGTKVEIKR

**SEQ. ID NO.109 Anti-VEGF 593 dAb (DNA sequence)**

GAGGTGCAGCTCCTGGTCAGCGGCGGCGGCCTGGTCCAGCCCGGAGGCTCACTGAGGCTGAGCTGCGCCGCTAGC GGCTTCACCTTCAAGGCCTACCCCATGATGTGGGTCAGGCAGGCCCCCGGCAAAGGCCTGGAGTGGGTGTCTGAG ATCAGCCCCAGCGGCAGCTACACCTACTACGCCGACAGCGTGAAGGGCAGGTTCACCATCAGCAGGGACAACAGCA AGAACACCCTGTACCTGCAGATGAACTCTCTGAGGGCCGAGGACACCGCCGTGTACTACTGCGCCAAGGACCCCAG GAAGCTGGACTATTGGGGCCAGGGCACTCTGGTGACCGTGAGCAGC

**SEQ. ID NO.110 Anti-VEGF 593 dAb (amino acid sequence)**

EVQLLVSGGGLVQPGGSLRLSCAASGFTFKAYPMMWVRQAPGKGLEWVSEISPSGSYTYYADSVKGRFTISRDNSKNT LYLQMNSLRAEDTAVYYCAKDPRKLDYWGQGTLVTVSS

**SEQ. ID NO.111 Anti-HGF-VEGF S260116C12 humanized Ha4-TVAAPSGS-593 heavy chain (DNA sequence)**

CAGATCCAGCTGGTGCAGAGCGGCGCCGAGGTGAAAAAGCCCGGCAGCAGCGTGAAGGTGAGCTGCAAGGCCAGC GGCTACACCTTCACCGGCTACTGGATCGAGTGGGTGAGGCAGGCTCCCGGACAGGGCCTGGAGTGGATCGGCGAG ATCCTGCCCGGGTCTGGCACCACCAACTACAACGAGAAGTTCAAGGGCCGCGCCACCTTCACTGCCGACACCTCCA CCAGCACCGCCTACATGGAACTGAGCAGCCTCAGGAGCGAAGACACCGCCGTCTACTATTGCGCCAGGGGCGGCTA CTACTACGGCAGCAGCTACGACAGCTGGGGCCAGGGCACACTAGTGACCGTGTCCAGCGCCAGCACCAAGGGCCC CAGCGTGTTCCCCCTGGCCCCCAGCAGCAAGAGCACCAGCGGCGGCACAGCCGCCCTGGGCTGCCTGGTGAAGGA CTACTTCCCCGAACCGGTGACCGTGTCCTGGAACAGCGGAGCCCTGACCAGCGGCGTGCACACCTTCCCCGCCGTG CTGCAGAGCAGCGGCCTGTACAGCCTGAGCAGCGTGGTGACCGTGCCCAGCAGCAGCCTGGGCACCCAGACCTAC ATCTGTAACGTGAACCACAAGCCCAGCAACACCAAGGTGGACAAGAAGGTGGAGCCCAAGAGCTGTGACAAGACCC ACACCTGCCCCCCCTGCCCTGCCCCCGAGCTGCTGGGAGGCCCCAGCGTGTTCCTGTTCCCCCCCAAGCCTAAGGA CACCCTGATGATCAGCAGAACCCCCGAGGTGACCTGTGTGGTGGTGGATGTGAGCCACGAGGACCCTGAGGTGAA GTTCAACTGGTACGTGGACGGCGTGGAGGTGCACAATGCCAAGACCAAGCCCAGGGAGGAGCAGTACAACAGCAC CTACCGGGTGGTGTCCGTGCTGACCGTGCTGCACCAGGATTGGCTGAACGGCAAGGAGTACAAGTGTAAGGTGTC CAACAAGGCCCTGCCTGCCCCTATCGAGAAAACCATCAGCAAGGCCAAGGGCCAGCCCAGAGAGCCCCAGGTGTAC ACCCTGCCCCCTAGCAGAGATGAGCTGACCAAGAACCAGGTGTCCCTGACCTGCCTGGTGAAGGGCTTCTACCCCA GCGACATCGCCGTGGAGTGGGAGAGCAACGGCCAGCCCGAGAACAACTACAAGACCACCCCCCCTGTGCTGGACA GCGATGGCAGCTTCTTCCTGTACAGCAAGCTGACCGTGGACAAGAGCAGATGGCAGCAGGGCAACGTGTTCAGCT GCTCCGTGATGCACGAGGCCCTGCACAATCACTACACCCAGAAGAGCCTGAGCCTGTCCCCTGGCAAGACCGTGGC CGCCCCCTCGGGATCCGAGGTGCAGCTCCTGGTCAGCGGCGGCGGCCTGGTCCAGCCCGGAGGCTCACTGAGGCT GAGCTGCGCCGCTAGCGGCTTCACCTTCAAGGCCTACCCCATGATGTGGGTCAGGCAGGCCCCCGGCAAAGGCCT GGAGTGGGTGTCTGAGATCAGCCCCAGCGGCAGCTACACCTACTACGCCGACAGCGTGAAGGGCAGGTTCACCAT CAGCAGGGACAACAGCAAGAACACCCTGTACCTGCAGATGAACTCTCTGAGGGCCGAGGACACCGCCGTGTACTAC TGCGCCAAGGACCCCAGGAAGCTGGACTATTGGGGCCAGGGCACTCTGGTGACCGTGAGCAGC

SEQ. ID **NO.112 Anti-HGF-VEGF** S260116C12 humanized Ha4-TVAAPSGS-593 heavy chain **(amino** acid **sequence)**

QIQLVQSGAEVKKPGSSVKVSCKASGYTFTGYWIEWVRQAPGQGLEWIGEILPGSGTTNYNEKFKGRATFTADTSTST AYMELSSLRSEDTAVYYCARGGYYYGSSYDSWGQGTLVTVSSASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQVSLTCLVKGFYPSDIAVEWESNGQPENN YKTTPPVLDSDGSFFLYSKLTVDKSRWQQGNVFSCSVMHEALHNHYTQKSLSLSPGKTVAAPSGSEVQLLVSGGGLVQ PGGSLRLSCAASGFTFKAYPMMWVRQAPGKGLEWVSEISPSGSYTYYADSVKGRFTISRDNSKNTLYLQMNSLRAEDT AVYYCAKDPRKLDYWGQGTLVTVSS

**SEQ.** ID **NO.113** Anti-HGF-VEGF **S260116C12** humanized **Ha5-TVAAPSGS-593** heavy chain **(DNA sequence)**

CAGATCCAGCTGAGGCAGAGCGGCGCCGAGGTGAAAAAGCCCGGCGCCAGCGTGAAGCTGAGCTGCAAGGCCAGC
GGCTACACCTTCACCGGCTACTGGATCGAGTGGGTGAGGCAGGCTCCCGGACAGGGCCTGGAGTGGATCGGCGAG
ATCCTGCCCGGGTCTGGCACCACCAACTACAACGAGAAGTTCAAGGGCAAGGCCACCTTCACTGCCGACACCTCCA
CCAGCACCGCCTACATGGAACTGAGCAGCCTCAGGAGCGAAGACACCGCCGTCTACTATTGCGCCAGGGGCGGCTA
CTACTACGGCAGCAGCTACGACAGCTGGGGCCAGGGCACACTAGTGACCGTGTCCAGCGCCAGCACCAAGGGCCC
CAGCGTGTTCCCCCTGGCCCCCAGCAGCAAGAGCACCAGCGGCGGCACAGCCGCCCTGGGCTGCCTGGTGAAGGA
CTACTTCCCCGAACCGGTGACCGTGTCCTGGAACAGCGGAGCCCTGACCAGCGGCGTGCACACCTTCCCCGCCGTG
CTGCAGAGCAGCGGCCTGTACAGCCTGAGCAGCGTGGTGACCGTGCCCAGCAGCAGCCTGGGCACCCAGACCTAC
ATCTGTAACGTGAACCACAAGCCCAGCAACACCAAGGTGGACAAGAAGGTGGAGCCCAAGAGCTGTGACAAGACCC
ACACCTGCCCCCCCTGCCCTGCCCCCGAGCTGCTGGGAGGCCCCAGCGTGTTCCTGTTCCCCCCCAAGCCTAAGGA
CACCCTGATGATCAGCAGAACCCCCGAGGTGACCTGTGTGGTGGTGGATGTGAGCCACGAGGACCCTGAGGTGAA
GTTCAACTGGTACGTGGACGGCGTGGAGGTGCACAATGCCAAGACCAAGCCCAGGGAGGAGCAGTACAACAGCAC
CTACCGGGTGGTGTCCGTGCTGACCGTGCTGCACCAGGATTGGCTGAACGGCAAGGAGTACAAGTGTAAGGTGTC
CAACAAGGCCCTGCCTGCCCCTATCGAGAAAACCATCAGCAAGGCCAAGGGCCAGCCCAGAGAGCCCCAGGTGTAC
ACCCTGCCCCCTAGCAGAGATGAGCTGACCAAGAACCAGGTGTCCCTGACCTGCCTGGTGAAGGGCTTCTACCCCA
GCGACATCGCCGTGGAGTGGGAGAGCAACGGCCAGCCCGAGAACAACTACAAGACCACCCCCCCTGTGCTGGACA
GCGATGGCAGCTTCTTCCTGTACAGCAAGCTGACCGTGGACAAGAGCAGATGGCAGCAGGGCAACGTGTTCAGCT
GCTCCGTGATGCACGAGGCCCTGCACAATCACTACACCCAGAAGAGCCTGAGCCTGTCCCCTGGCAAGACCGTGGC
CGCCCCCTCGGGATCCGAGGTGCAGCTCCTGGTCAGCGGCGGCGGCCTGGTCCAGCCCGGAGGCTCACTGAGGCT
GAGCTGCGCCGCTAGCGGCTTCACCTTCAAGGCCTACCCCATGATGTGGGTCAGGCAGGCCCCCGGCAAAGGCCT
GGAGTGGGTGTCTGAGATCAGCCCCAGCGGCAGCTACACCTACTACGCCGACAGCGTGAAGGGCAGGTTCACCAT
CAGCAGGGACAACAGCAAGAACACCCTGTACCTGCAGATGAACTCTCTGAGGGCCGAGGACACCGCCGTGTACTAC
TGCGCCAAGGACCCCAGGAAGCTGGACTATTGGGGCCAGGGCACTCTGGTGACCGTGAGCAGC

**SEQ. ID NO.114 Anti-HGF-VEGF S260116C12** humanized **Ha5-TVAAPSGS-593** heavy chain **(amino** acid **sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLVSGGGLVQ
PGGSLRLSCAASGFTFKAYPMMWVRQAPGKGLEWVSEISPSGSYTYYADSVKGRFTISRDNSKNTLYLQMNSLRAEDT
AVYYCAKDPRKLDYWGQGTLVTVSS

**SEQ.** ID **NO.115 Anti-HGP-VEGF** S260116C12 humanized Ha4-TVAAPSGS-098 heavy chain **(amino** acid **sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ

DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSS

**SEQ. ID NO.116 Anti-HGP-VEGF S260116C12 humanized Ha5-TVAAPSGS-098 heavy chain (amino** acid **sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSS

**SEQ. ID NO.117 Anti-HGF-VEGF S260116C12 humanized Ha6-TVAAPSGS-098 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSS

**SEQ. ID NO.118 Anti-HGP-VEGF S260116C12 humanized Ha7-TVAAPSGS-098 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSS

**SEQ. ID NO.119 Anti-HGF-VEGF S260116C12 humanized Ha4-TVAAPS-098 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSS

**SEQ.** ID **NO.120 Anti-HGP-VEGF S260116C12** humanized **Ha5-TVAAPS-098** heavy chain **(amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN

YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSS

**SEQ. ID NO.121 Anti-HGP-VEGF S260116C12 humanized Ha6-TVAAPS-098 heavy chain (amino** acid **sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSS

**SEQ. ID NO.122 Anti-HGP-VEGF S260116C12 humanized Ha7-TVAAPS-098 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSS

**SEQ. ID NO.123 Anti-HGP-VEGF S260116C12 humanized Ha4-TVAAPSGS-098AAA heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSSAAA

**SEQ. ID NO.124 Anti-HGF-VEGF S260116C12 humanized Ha5-TVAAPSGS-098AAA heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSSAAA

**SEQ. ID NO.125 Anti-HGF-VEGF S260116C12 humanized Ha6-TVAAPSGS-098AAA heavy chain (amino acid**

**sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ

PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSSAAA

**SEQ. ID NO.126 Anti-HGF-VEGF S260116C12 humanized Ha7-TVAAPSGS-098AAA heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSEVQLLESGGGLVQ
PGGSLRLSCAASGFTFKDYDMWWVRQAPGKGLEWVSSISVEGVQTYYADSVKGRFTISRDNSKNTLYLQMNSLRAED
TAVYYCAKNIRYVGNRSWWTFDYWGQGTLVTVSSAAA

**SEQ. ID NO.127 Anti-HGP-VEGF S260116C12 humanized Ha4-TVAAPS-098AAA heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSSAAA

**SEQ. ID NO.128 Anti-HGP-VEGF S260116C12 humanized Ha5-TVAAPS-098AAA heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSSAAA

**SEQ. ID NO.129 Anti-HGP-VEGF S260116C12 humanized Ha6-TVAAPS-098AAA heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG
GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSSAAA

**SEQ. ID NO.130 Anti-HGF-VEGF S260116C12 humanized Ha7-TVAAPS-098AAA heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSEVQLLESGGGLVQPG

GSLRLSCAASGFTFKDYDMWWVRQAPGKGLEWVSSISVEGVQTYYADSVKGRFTISRDNSKNTLYLQMNSLRAEDTA
VYYCAKNIRYVGNRSWWTFDYWGQGTLVTVSSAAA

**SEQ. ID NO.131 Anti-HGP-VEGF S260116C12 humanized Ha4-TVAAPSGS-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSDIQMTQSPSSLSA
SVGDRVTITCRASQWIGPELSWYQQKPGKAPKLLIYHGSILQSGVPSRFSGSGSGTDFTLTISSLQPEDFATYYCQQYM
YYPHTFGQGTKVEIKR

**SEQ. ID NO.132 Anti-HGF-VEGF S260116C12 humanized Ha5-TVAAPSGS-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSDIQMTQSPSSLSA
SVGDRVTITCRASQWIGPELSWYQQKPGKAPKLLIYHGSILQSGVPSRFSGSGSGTDFTLTISSLQPEDFATYYCQQYM
YYPHTFGQGTKVEIKR

**SEQ. ID NO.133 Anti-HGF-VEGF S260116C12 humanized Ha6-TVAAPSGS-044 heavy -chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSDIQMTQSPSSLSA
SVGDRVTITCRASQWIGPELSWYQQKPGKAPKLLIYHGSILQSGVPSRFSGSGSGTDFTLTISSLQPEDFATYYCQQYM
YYPHTFGQGTKVEIKR

**SEQ. ID NO.134 Anti-HGF-VEGF S260116C12 humanized Ha7-TVAAPSGS-044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSGSDIQMTQSPSSLSA
SVGDRVTITCRASQWIGPELSWYQQKPGKAPKLLIYHGSILQSGVPSRFSGSGSGTDFTLTISSLQPEDFATYYCQQYM
YYPHTFGQGTKVEIKR

**SEQ. ID NO.135 Anti-HGF-VEGF S260116C12 humanized Ha4-TVAAPS-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSDIQMTQSPSSLSASV

GDRVTITCRASQWIGPELSWYQQKPGKAPKLLIYHGSILQSGVPSRFSGSGSGTDFTLTISSLQPEDFATYYCQQYMYY
PHTFGQGTKVEIKR

**SEQ. ID NO.136 Anti-HGF-VEGF S260116C12 humanized Ha5-TVAAPS-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSDIQMTQSPSSLSASV
GDRVTITCRASQWIGPELSWYQQKPGKAPKLLIYHGSILQSGVPSRFSGSGSGTDFTLTISSLQPEDFATYYCQQYMYY
PHTFGQGTKVEIKR

**SEQ. ID NO.137 Anti-HGF-VEGF S260116C12 humanized Ha6-TVAAPS-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSDIQMTQSPSSLSASV
GDRVTITCRASQWIGPELSWYQQKPGKAPKLLIYHGSILQSGVPSRFSGSGSGTDFTLTISSLQPEDFATYYCQQYMYY
PHTFGQGTKVEIKR

**SEQ. ID NO.138 Anti-HGF-VEGF S260116C12 humanized Ha7-TVAAPS-044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSDIQMTQSPSSLSASV
GDRVTITCRASQWIGPELSWYQQKPGKAPKLLIYHGSILQSGVPSRFSGSGSGTDFTLTISSLQPEDFATYYCQQYMYY
PHTFGQGTKVEIKR

**SEQ. ID NO.139 Anti-HGF-VEGF S260116C12 humanized Ha4-GS(TVAAPSGS)$_3$-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKGSTVAAPSGSTVAAPSGSTVA
APSGSDIQMTQSPSSLSASVGDRVTITCRASQWIGPELSWYQQKPGKAPKLLIYHGSILQSGVPSRFSGSGSGTDFTLT
ISSLQPEDFATYYCQQYMYYPHTFGQGTKVEIKR

**SEQ. ID NO.140 Anti-HGP-VEGF S260116C12** humanized **Ha5-GS(TVAAPSGS)$_3$-044** heavy chain **(amino** acid **sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKGSTVAAPSGSTVAAPSGSTVA

APSGSDIQMTQSPSSLSASVGDRVTITCRASQWIGPELSWYQQKPGKAPKLLIYHGSILQSGVPSRFSGSGSGTDFTLT
ISSLQPEDFATYYCQQYMYYPHTFGQGTKVEIKR

**SEQ. ID NO.141 Anti-HGP-VEGF S260116C12 humanized Ha6-GS(TVAAPSGS)$_3$-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKGSTVAAPSGSTVAAPSGSTVA
APSGSDIQMTQSPSSLSASVGDRVTITCRASQWIGPELSWYQQKPGKAPKLLIYHGSILQSGVPSRFSGSGSGTDFTLT
ISSLQPEDFATYYCQQYMYYPHTFGQGTKVEIKR

**SEQ. ID NO.142 Anti-HGP-VEGF S260116C12 humanized Ha7-GS(TVAAPSGS)$_3$-044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKGSTVAAPSGSTVAAPSGSTVA
APSGSDIQMTQSPSSLSASVGDRVTITCRASQWIGPELSWYQQKPGKAPKLLIYHGSILQSGVPSRFSGSGSGTDFTLT
ISSLQPEDFATYYCQQYMYYPHTFGQGTKVEIKR

**SEQ. ID NO.143 Anti-HGP-VEGF S260116C12 humanized Ha4-(TVAAPS)$_3$-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSTVAAPSTVAAPSDIQ
MTQSPSSLSASVGDRVTITCRASQWIGPELSWYQQKPGKAPKLLIYHGSILQSGVPSRFSGSGSGTDFTLTISSLQPED
FATYYCQQYMYYPHTFGQGTKVEIKR

**SEQ. ID NO.144 Anti-HGP-VEGF S260116C12 humanized Ha5-(TVAAPS)$_3$-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSTVAAPSTVAAPSDIQ
MTQSPSSLSASVGDRVTITCRASQWIGPELSWYQQKPGKAPKLLIYHGSILQSGVPSRFSGSGSGTDFTLTISSLQPED
FATYYCQQYMYYPHTFGQGTKVEIKR

**SEQ. ID NO.145 Anti-HGP-VEGF** S260116C12 humanized **Ha6-(TVAAPS)$_3$-044** heavy chain **(amino** acid **sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSTVAAPSTVAAPSDIQ

MTQSPSSLSASVGDRVTITCRASQWIGPELSWYQQKPGKAPKLLIYHGSILQSGVPSRFSGSGSGTDFTLTISSLQPED
FATYYCQQYMYYPHTFGQGTKVEIKR

**SEQ. ID NO.146 Anti-HGP-VEGF S260116C12 humanized Ha7-(TVAAPS)$_3$-044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKTVAAPSTVAAPSTVAAPSDIQ
MTQSPSSLSASVGDRVTITCRASQWIGPELSWYQQKPGKAPKLLIYHGSILQSGVPSRFSGSGSGTDFTLTISSLQPED
FATYYCQQYMYYPHTFGQGTKVEIKR

**SEQ. ID NO.147 Anti-HGP-VEGF S260116C12 humanized Ha4-DETYVPKEFNAETFGS-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFGSDIQMT
QSPSSLSASVGDRVTITCRASQWIGPELSWYQQKPGKAPKLLIYHGSILQSGVPSRFSGSGSGTDFTLTISSLQPEDFAT
YYCQQYMYYPHTFGQGTKVEIKR

**SEQ. ID NO.148 Anti-HGP-VEGF S260116C12 humanized Ha5-DETYVPKEFNAETFGS-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFGSDIQMT
QSPSSLSASVGDRVTITCRASQWIGPELSWYQQKPGKAPKLLIYHGSILQSGVPSRFSGSGSGTDFTLTISSLQPEDFAT
YYCQQYMYYPHTFGQGTKVEIKR

**SEQ. ID NO.149 Anti-HGF-VEGF S260116C12 humanized Ha6-DETYVPKEFNAETFGS-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFGSDIQMT
QSPSSLSASVGDRVTITCRASQWIGPELSWYQQKPGKAPKLLIYHGSILQSGVPSRFSGSGSGTDFTLTISSLQPEDFAT
YYCQQYMYYPHTFGQGTKVEIKR

**SEQ. ID NO.150 Anti-HGP-VEGF S260116C12 humanized Ha7-DETYVPKEFNAETFGS -044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFGSDIQMT

QSPSSLSASVGDRVTITCRASQWIGPELSWYQQKPGKAPKLLIYHGSILQSGVPSRFSGSGSGTDFTLTISSLQPEDFAT
YYCQQYMYYPHTFGQGTKVEIKR

**SEQ. ID NO.151 Anti-HGF-VEGF S260116C12 humanized Ha4-DETYVPKEFNAETF-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFDIQMTQS
PSSLSASVGDRVTITCRASQWIGPELSWYQQKPGKAPKLLIYHGSILQSGVPSRFSGSGSGTDFTLTISSLQPEDFATYY
CQQYMYYPHTFGQGTKVEIKR

**SEQ. ID NO.152 Anti-HGF-VEGF S260116C12 humanized Ha5-DETYVPKEFNAETF-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFDIQMTQS
PSSLSASVGDRVTITCRASQWIGPELSWYQQKPGKAPKLLIYHGSILQSGVPSRFSGSGSGTDFTLTISSLQPEDFATYY
CQQYMYYPHTFGQGTKVEIKR

**SEQ. ID NO.153 Anti-HGP-VEGF S260116C12 humanized Ha6-DETYVPKEFNAETF-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFDIQMTQS
PSSLSASVGDRVTITCRASQWIGPELSWYQQKPGKAPKLLIYHGSILQSGVPSRFSGSGSGTDFTLTISSLQPEDFATYY
CQQYMYYPHTFGQGTKVEIKR

**SEQ. ID NO.154 Anti-HGP-VEGF S260116C12 humanized Ha7-DETYVPKEFNAETF-044 heavy** chain **(amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKDETYVPKEFNAETFDIQMTQS
PSSLSASVGDRVTITCRASQWIGPELSWYQQKPGKAPKLLIYHGSILQSGVPSRFSGSGSGTDFTLTISSLQPEDFATYY
CQQYMYYPHTFGQGTKVEIKR

**SEQ. ID NO.155 Anti-HGP-VEGF S260116C12 humanized Ha4-EVDETYVPKEFNAETFTFHADGS-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD

GSDIQMTQSPSSLSASVGDRVTITCRASQWIGPELSWYQQKPGKAPKLLIYHGSILQSGVPSRFSGSGSGTDFTLTISS
LQPEDFATYYCQQYMYYPHTFGQGTKVEIKR

**SEQ. ID NO.156 Anti-HGP-VEGF S260116C12 humanized Ha5-EVDETYVPKEFNAETFTFHADGS-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
GSDIQMTQSPSSLSASVGDRVTITCRASQWIGPELSWYQQKPGKAPKLLIYHGSILQSGVPSRFSGSGSGTDFTLTISS
LQPEDFATYYCQQYMYYPHTFGQGTKVEIKR

**SEQ. ID NO.157 Anti-HGP-VEGF S260116C12 humanized Ha6-EVDETYVPKEFNAETFTFHADGS-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
GSDIQMTQSPSSLSASVGDRVTITCRASQWIGPELSWYQQKPGKAPKLLIYHGSILQSGVPSRFSGSGSGTDFTLTISS
LQPEDFATYYCQQYMYYPHTFGQGTKVEIKR

**SEQ. ID NO.158 Anti-HGF-VEGF S260116C12 humanized Ha7-EVDETYVPKEFNAETFTFHADGS -044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
GSDIQMTQSPSSLSASVGDRVTITCRASQWIGPELSWYQQKPGKAPKLLIYHGSILQSGVPSRFSGSGSGTDFTLTISS
LQPEDFATYYCQQYMYYPHTFGQGTKVEIKR

**SEQ. ID NO.159 Anti-HGF-VEGF S260116C12 humanized Ha4-EVDETYVPKEFNAETFTFHAD-044 heavy chain (amino acid sequence)**

QIQLVQSGAEVKKPGSSVKVSCKASGYTFTGYWIEWVRQAPGQGLEWIGEILPGSGTTNYNEKFKGRATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
DIQMTQSPSSLSASVGDRVTITCRASQWIGPELSWYQQKPGKAPKLLIYHGSILQSGVPSRFSGSGSGTDFTLTISSLQ
PEDFATYYCQQYMYYPHTFGQGTKVEIKR

**SEQ.** ID **NO.160 Anti-HGF-VEGF S260116C12** humanized **Ha5-EVDETYVPKEFNAETFTFHAD-044 heavy chain (amino acid sequence)**

QIQLRQSGAEVKKPGASVKLSCKASGYTFTGYWIEWVRQAPGQGLEWIGEILPGSGTTNYNEKFKGKATFTADTSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD

DIQMTQSPSSLSASVGDRVTITCRASQWIGPELSWYQQKPGKAPKLLIYHGSILQSGVPSRFSGSGSGTDFTLTISSLQ
PEDFATYYCQQYMYYPHTFGQGTKVEIKR

**SEQ. ID NO.161 Anti-HGF-VEGF S260116C12 humanized Ha6-EVDETYVPKEFNAETFTFHAD-044 heavy chain (amino acid sequence)**

QIQLVQSGAEVKKPGSSVKVSCKASGYTFTGYWIEWVRQAPGQGLEWIGEILPGSGTTNYNEKFKGRATFTADKSTST
AYMELSSLRSEDTAVYYCARGGYYYGSSYDSWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
DIQMTQSPSSLSASVGDRVTITCRASQWIGPELSWYQQKPGKAPKLLIYHGSILQSGVPSRFSGSGSGTDFTLTISSLQ
PEDFATYYCQQYMYYPHTFGQGTKVEIKR

**SEQ. ID NO.162 Anti-HGF-VEGF S260116C12 humanized Ha7-EVDETYVPKEENAETFTFHAD-044 heavy chain (amino acid sequence)**

QVQLVQSGAEVKKPGSSVKVSCKASGYTFTGYWIEWVRQAPGQGLEWIGEILPGSGTTNYNEKFKGRATFTADTSTS
TAYMELSSLRSEDTAVYYCARGGYYYGSSYDSWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGKEVDETYVPKEFNAETFTFHAD
DIQMTQSPSSLSASVGDRVTITCRASQWIGPELSWYQQKPGKAPKLLIYHGSILQSGVPSRFSGSGSGTDFTLTISSLQ
PEDFATYYCQQYMYYPHTFGQGTKVEIKR

**SEQ. ID NO.163 TVAAPSGS linker (amino acid sequence)**
**TVAAPSGS**
**SEQ. ID NO.164 TVAAPS linker (amino acid sequence)**
TVAAPS
**SEQ. ID NO.165 GS(TVAAPSGS)$_3$ (amino acid sequence)**
GSTVAAPSGSTVAAPSGSTVAAPSGS
**SEQ. ID NO.166 (TVAAPS)$_3$ (amino acid sequence)**
TVAAPSTVAAPSTVAAPS
**SEQ. ID NO.167 DETYVPKEFNAETFGS linker (amino acid sequence)**
DETYVPKEFNAETFGS
**SEQ. ID NO.168 DETYVPKEFNAETF linker (amino acid sequence)**
DETYVPKEFNAETF
**SEQ. ID NO.169 EVDETYVPKEFNAETFTFHADGS linker (amino acid sequence)**
EVDETYVPKEFNAETFTFHADGS
**SEQ. ID NO.170 EVDETYVPKEFNAETFTFHAD linker (amino acid sequence)**
EVDETYVPKEFNAETFTFHAD
**SEQ. ID NO.171 DMS4000 heavy chain (DNA sequence)**

GAGGTGCAGCTGGTGGAGTCTGGCGGCGGACTGGTGCAGCCCGGCAGAAGCCTGAGACTGAGCTGTGCCGCCAG
CGGCTTCACCTTCGACGACTACGCCATGCACTGGGTGAGGCAGGCCCCTGGCAAGGGCCTGGAGTGGGTGTCCGC
CATCACCTGGAATAGCGGCCACATCGACTACGCCGACAGCGTGGAGGGCAGATTCACCATCAGCCGGGACAACGCC
AAGAACAGCCTGTACCTGCAGATGAACAGCCTGAGAGCCGAGGACACCGCCGTGTACTACTGTGCCAAGGTGTCCT
ACCTGAGCACCGCCAGCAGCCTGGACTACTGGGGCCAGGGCACCCTGGTGACAGTCTCGAGCGCTAGCACCAAGG
GCCCCAGCGTGTTCCCCCTGGCCCCCAGCAGCAAGAGCACCAGCGGCGGCACAGCCGCCCTGGGCTGCCTGGTGA
AGGACTACTTCCCCGAGCCTGTGACCGTGTCCTGGAATAGCGGAGCCCTGACCTCCGGCGTGCACACCTTCCCCGC
CGTGCTGCAGAGCAGCGGCCTGTACTCCCTGAGCAGCGTGGTGACCGTGCCCAGCAGCAGCCTGGGCACCCAGAC
CTACATCTGCAACGTGAACCACAAGCCCAGCAACACCAAAGTGGACAAGAAAGTGGAGCCCAAGAGCTGCGATAAG
ACCCACACCTGCCCCCCCTGCCCTGCCCCCGAGCTGGCCGGCGCCCCTAGCGTGTTCCTGTTCCCCCCCAAGCCTA
AGGACACCCTGATGATCAGCAGGACCCCCGAAGTGACCTGCGTGGTGGTGGATGTGAGCCACGAGGACCCTGAAG
TGAAGTTCAACTGGTACGTGGACGGCGTGGAAGTGCACAACGCCAAGACCAAGCCCAGAGAGGAGCAGTACAACA

GCACCTACCGCGTGGTGTCTGTGCTGACCGTGCTGCACCAGGATTGGCTGAACGGCAAGGAGTACAAGTGCAAAG
TGAGCAACAAGGCCCTGCCTGCCCCTATCGAGAAAACCATCAGCAAGGCCAAGGGCCAGCCTAGAGAGCCCCAGGT
CTACACCCTGCCTCCCTCCAGAGATGAGCTGACCAAGAACCAGGTGTCCCTGACCTGTCTGGTGAAGGGCTTCTAC
CCCAGCGACATCGCCGTGGAGTGGGAGAGCAACGGCCAGCCCGAGAACAACTACAAGACCACCCCCCCTGTGCTG
GACAGCGATGGCAGCTTCTTCCTGTACTCCAAGCTGACCGTGGACAAGAGCAGATGGCAGCAGGGCAACGTGTTCA
GCTGCAGCGTGATGCACGAGGCCCTGCACAATCACTACACCCAGAAGAGTCTGAGCCTGTCCCCTGGCAAGTCGAC
CGGTGAGGTGCAGCTGCTGGTGTCTGGCGGCGGACTGGTGCAGCCTGGCGGCAGCCTGAGACTGAGCTGCGCCG
CCAGCGGCTTCACCTTCAAGGCCTACCCCATGATGTGGGTGCGGCAGGCCCCTGGCAAGGGCCTGGAATGGGTGT
CCGAGATCAGCCCCAGCGGCAGCTACACCTACTACGCCGACAGCGTGAAGGGCCGGTTCACCATCAGCCGGGACAA
CAGCAAGAACACCCTGTACCTGCAGATGAACAGCCTGCGGGCCGAGGACACCGCCGTGTACTACTGCGCCAAGGAC
CCCCGGAAGCTGGACTACTGGGGCCAGGGCACCCTGGTGACCGTGAGCAGC

**SEQ. ID NO.172 DMS4000 heavy chain (amino acid sequence)**

EVQLVESGGGLVQPGRSLRLSCAASGFTFDDYAMHWVRQAPGKGLEWVSAITWNSGHIDYADSVEGRFTISRDNAKN
SLYLQMNSLRAEDTAVYYCAKVSYLSTASSLDYWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNTKVDKKVEPKSCDKTHTCPPCPA
PELAGAPSVFLFPPKPKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPGKSTGEVQLLVSGGGLVQPGGS
LRLSCAASGFTFKAYPMMWVRQAPGKGLEWVSEISPSGSYTYYADSVKGRFTISRDNSKNTLYLQMNSLRAEDTAVYY
CAKDPRKLDYWGQGTLVTVSS

**SEQ. ID NO.173 DMS4000 light chain (DNA sequence)**

GATATCCAGATGACCCAGAGCCCCAGCAGCCTGAGCGCCTCTGTGGGCGATAGAGTGACCATCACCTGCCGGGCCA
GCCAGGGCATCAGAAACTACCTGGCCTGGTATCAGCAGAAGCCTGGCAAGGCCCCTAAGCTGCTGATCTACGCCGC
CAGCACCCTGCAGAGCGGCGTGCCCAGCAGATTCAGCGGCAGCGGCTCCGGCACCGACTTCACCCTGACCATCAGC
AGCCTGCAGCCCGAGGACGTGGCCACCTACTACTGCCAGCGGTACAACAGAGCCCCTTACACCTTCGGCCAGGGCA
CCAAGGTGGAGATCAAGCGTACGGTGGCCGCCCCCAGCGTGTTCATCTTCCCCCCCAGCGATGAGCAGCTCAAGAG
CGGCACCGCCAGCGTGGTGTGTCTGCTGAACAACTTCTACCCCCGGGAGGCCAAAGTGCAGTGGAAAGTGGACAA
CGCCCTGCAGAGCGGCAACAGCCAGGAGAGCGTGACCGAGCAGGACAGCAAGGACTCCACCTACAGCCTGAGCAG
CACCCTGACCCTGAGCAAGGCCGACTACGAGAAGCACAAAGTGTACGCCTGCGAAGTGACCCACCAGGGCCTGTCC
AGCCCCGTGACCAAGAGCTTCAACCGGGGCGAGTGC

**SEQ. ID N0.174 DMS4000 light chain (amino acid sequence)**

DIQMTQSPSSLSASVGDRVTITCRASQGIRNYLAWYQQKPGKAPKLLIYAASTLQSGVPSRFSGSGSGTDFTLTISSLQ
PEDVATYYCQRYNRAPYTFGQGTKVEIKRTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKSFNRGEC

**SEQ. ID NO.175 Hybrid antibody control heavy chain (DNA sequence)**

CAGGTCCAATTAGTGCAATCTGGGTCTGAGTTGAAGAAGCCTGGGGCCTCAGTGAAGGTTTCCTGCAAGGCCTCTG
GATACACCTTCACTAACTATGGAATGAACTGGGTGCGACAGGCCCCTGGACAAGGGCTCGAGTGGATGGGATGGA
TAAACACCAGAAATGGAAAGTCAACATATGTTGATGACTTCAAGGGGCGGTTTGTCTTCTCCTTGGACACCTCTGT
CAGCACGGCATATCTACAGATCAGCAGCCTAAAGGCTGACGACACTGCAGTGTATTACTGTGCGAGAGAAGGGAAT
ATGGATGGTTACTTCCCTTTTACTTACTGGGGCCAGGGTACACTAGTGACCGTGTCCAGCGCCAGCACCAAGGGCC
CCAGCGTGTTCCCCCTGGCCCCCAGCAGCAAGAGCACCAGCGGCGGCACAGCCGCCCTGGGCTGCCTGGTGAAGG
ACTACTTCCCCGAACCGGTGACCGTGTCCTGGAACAGCGGAGCCCTGACCAGCGGCGTGCACACCTTCCCCGCCGT
GCTGCAGAGCAGCGGCCTGTACAGCCTGAGCAGCGTGGTGACCGTGCCCAGCAGCAGCCTGGGCACCCAGACCTA
CATCTGTAACGTGAACCACAAGCCCAGCAACACCAAGGTGGACAAGAAGGTGGAGCCCAAGAGCTGTGACAAGACC
CACACCTGCCCCCCCTGCCCTGCCCCCGAGCTGCTGGGAGGCCCCAGCGTGTTCCTGTTCCCCCCCAAGCCTAAGG
ACACCCTGATGATCAGCAGAACCCCCGAGGTGACCTGTGTGGTGGTGGATGTGAGCCACGAGGACCCTGAGGTGA
AGTTCAACTGGTACGTGGACGGCGTGGAGGTGCACAATGCCAAGACCAAGCCCAGGGAGGAGCAGTACAACAGCA
CCTACCGGGTGGTGTCCGTGCTGACCGTGCTGCACCAGGATTGGCTGAACGGCAAGGAGTACAAGTGTAAGGTGT
CCAACAAGGCCCTGCCTGCCCCTATCGAGAAAACCATCAGCAAGGCCAAGGGCCAGCCCAGAGAGCCCCAGGTGTA
CACCCTGCCCCCTAGCAGAGATGAGCTGACCAAGAACCAGGTGTCCCTGACCTGCCTGGTGAAGGGCTTCTACCCC
AGCGACATCGCCGTGGAGTGGGAGAGCAACGGCCAGCCCGAGAACAACTACAAGACCACCCCCCCTGTGCTGGAC

AGCGATGGCAGCTTCTTCCTGTACAGCAAGCTGACCGTGGACAAGAGCAGATGGCAGCAGGGCAACGTGTTCAGC
TGCTCCGTGATGCACGAGGCCCTGCACAATCACTACACCCAGAAGAGCCTGAGCCTGTCCCCTGGCAAG

**SEQ. ID NO.176 Hybrid Antibody control heavy chain (amino acid sequence)**

QVQLVQSGSELKKPGASVKVSCKASGYTFTNYGMNWVRQAPGQGLEWMGWINTRNGKSTYVDDFKGRFVFSLDTSV
STAYLQISSLKADDTAVYYCAREGNMDGYFPFTYWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPSNTKVDKKVEPKSCDKTHTCPPCP
APELLGGPSVFLFPPKPKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSPGK

**SEQ. ID NO.177 Hybrid antibody control light chain (DNA sequence)**

GATATTGTCATGACTCAGTCTCCATCATCCCTGTCCGCATCAGTAGGAGACAGGGTCACCATCACCTGCAAGGCTT
CTCAGAATGTGGGTACTAATGTAGCCTGGTATCAACAGAAACCAGGGAAAGCTCCTAAAGCACTGATTTACTCGGC
ATCCTATCGGTACAGTGGAGTCCCTGATCGCTTCTCAGGCAGTGGATCCGGGACAGATTTCACTCTCACCATCAGC
AGTCTGCAGCCTGAAGACTTCGCAACGTATTACTGTCAGCAATATAACAGCTATCCTCTCACGTTCGGTGGTGGTA
CCAAGGTGGAAATAAAACGTACGGTGGCCGCCCCCAGCGTGTTCATCTTCCCCCCCAGCGATGAGCAGCTGAAGAG
CGGCACCGCCAGCGTGGTGTGTCTGCTGAACAACTTCTACCCCCGGGAGGCCAAGGTGCAGTGGAAGGTGGACAA
TGCCCTGCAGAGCGGCAACAGCCAGGAGAGCGTGACCGAGCAGGACAGCAAGGACTCCACCTACAGCCTGAGCAG
CACCCTGACCCTGAGCAAGGCCGACTACGAGAAGCACAAGGTGTACGCCTGTGAGGTGACCCACCAGGGCCTGTCC
AGCCCCGTGACCAAGAGCTTCAACCGGGGCGAGTGC

**SEQ. ID NO.178 Hybrid antibody control light chain (amino acid sequence)**

DIVMTQSPSSLSASVGDRVTITCKASQNVGTNVAWYQQKPGKAPKALIYSASYRYSGVPDRFSGSGSGTDFTLTISSLQ
PEDFATYYCQQYNSYPLTFGGGTKVEIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRGEC

**SEQ. ID NO 179 Anti-VEGF Y0317 humanized antibody fragment VH region**

EVQLVESGGGLVQPGGSLRLSCAASGYDFTHYGMNWVRQAPGKGLEWVGWINTYTGEPTYAADFKRRFTFSLDTSKS
TAYLQMNSLRAEDTAVYYCAKYPYYYGTSHWYFDVWGQGTL

**SEQ. ID NO 180 Anti-VEGF Y0317 humanized antibody fragment VL region**

DIQLTQSPSSLSASVGDRVTITCSASQDISNYLNWYQQKPGKAPKVLIYFTSSLHSGVPS RFSGSGSGTDFTLTISSLQPEDFATYYCQQYSTVPWTFGQGTKVEIKRTV

**SEQ ID NO: 181 (anti-VEGF Anticalin)**

DGGGIRRSMSGTWYLKAMTVDREFPEMNLESVTPMTLTLLKGHNLEAKVTMLISGRCQEVKAVLGRTKERKKYTADG GKHVAYIIPSAVRDHVIFYSEGQLHGKPVRGVKLVGRDPKNNLEALEDFEKAAGARGLSTESILIPRQSETCSPG

**SEQ ID NO: 182 (anti-VEGFR2 adnectin)**

EVVAATPTSLLISWRHPHFPTRYYRITYGETGGNSPVQEFTVPLQPPTATISGLKPGVDYTITVYAVTDGRNGRLLSIPIS INYRT

**SEQ ID NO: 183 (Anti-HGF nanobody HGF13)**

EVQLVESGGGLVQAGGSLRLSCAASGRTFRSYPMGWFRQAPGKEREFVASITGSGGSTYYADSVKGRFTISRDNAKNT VYLQMNSLRPEDTAVYSCAAYIRPDTYLSRDYRKYDYWGQGTQVTVSS

**SEQ ID NO: 184 (Humanised anti-HGF nanobody HGP13hum5)**

EVQLVESGGGLVQPGGSLRLSCAASGFTFSSYPMGWFRQAPGKGREFVSSITGSGGSTYYADSVKGRFTISRDNAKNT LYLQMNSLRPEDTAVYYCAAYIRPDTYLSRDYRKYDYWGQGTLVTVSS

**SEQ ID NO: 185 (Avastin Variable light chain)**

DIQMTQSPSSLSASVGDRVTITCSASQDISNYLNWYQQKPGKAPKVLIYFTSSLHSGVPSRFSGSGSGTDFTLTISSLQP EDFATYYCQQYSTVPWTFGQGTKVEIKR

**SEQ ID NO: 186 (Avastin Variable heavy chain)**

EVQLVESGGGLVQPGGSLRLSCAASGYTFTNYGMNWVRQAPGKGLEWVGWINTYTGEPTYAADFKRRFTFSLDTSKS TAYLQMNSLRAEDTAVYYCAKYPHYYGSSHWYFDVWGQGTLVTVSS

**SEQ ID NO: 187**
DDNPNLPRLVRPE
**SEQ ID NO: 188**
DEMPADLPSLAADF
**SEQ ID NO: 189**
HKDDNPNLPRLVRPEVDVM
**SEQ ID NO: 190**
ENDEMPADLPSLAADFVESKD
**SEQ ID NO.191 (Anti-VEGF Vk dAb DT02-K-044)**

DIQMTQSPSSLSASVGDRVTITCRASQWIGPELSWYQQKPGKAPKLLIYHGSILQSGVPSRFSGSGSGTDFTLTISSLQ PEDFATYYCQQYMYYPHTFGQGTKVEIKR

**SEQ ID NO:192 (Anti-VEGF Vk dAb DT02-K-044-251)**

DIQMTQSPSSLSASVGDRVTITCRASQWIGPELKWYQQKPGKAPKLLIYHGSILQSGVPSRFSGSGSGTDFTLTISSLQPEDFATYYCQQYMYYPETFGQGTKVEIKR

**SEQ ID NO:193 (Anti-VEGF Vk dAb DT02-K-044-255)**

DIQMTQSPSSLSASVGDRVTITCRASQWIGPELKWYQQKPGKAPKLLIYHGSILQSGVPSRFSGSGSGTDFTLTISSLQPEDFATYYCQQYMYYPKTFGQGTKVEIKR

**SEQ ID NO:194: DT02-K-044-085**

DIQMTQSPSSLSASVGDRVTITCRASQWIGPELKWYQQKPGKAPKLLIYHGSILQSGVPSRFSGSGSGTDFTLTISSLQPEDFATYYCQQYMYYPHTFGQGTKVEIKR

**SEQ ID NO:195 (TGLDSP)3 linker (amino acid sequence)**
TGLDSPTGLDSPTGLDSP
**SEQ ID NO:196 (TGLDSP)4 linker (amino acid sequence)**
TGLDSPTGLDSPTGLDSPTGLDSP

**Claims**

1. An antigen binding protein comprising an epitope-binding domain which is capable of binding to VEGF, and wherein the epitope binding domain comprises an amino acid sequence that has at least 95% sequence identity to the amino acid sequence of SEQ ID NO: 194.

2. An antigen binding protein according to claim 1, wherein the epitope binding domain comprises an amino acid sequence that has at least 99% sequence identity to the amino acid sequence of SEQ ID NO: 194.

3. An antigen binding protein according to claim 1 or 2, wherein the epitope binding domain comprises an amino acid sequence that is identical to the amino acid sequence of SEQ ID NO: 194.

4. A pharmaceutical composition comprising an antigen binding protein of any of claims 1 to 3 and a pharmaceutically acceptable carrier.

5. An isolated nucleic acid molecule which encodes an antigen binding protein according to any of claims 1 to 3.

6. An expression vector comprising a nucleic acid molecule according to claim 5.

7. A host cell comprising an expression vector according to claim 6.

8. An antigen binding protein as produced by the host cell of claim 7.

9. An antigen binding protein as defined in any of claims 1 to 3 for use in therapy.

10. An antigen binding protein as defined in any of claims 1 to 3 for use in the treatment of a disease associated with over production of VEGF.

11. An antigen binding protein for use according to claim 10, wherein the disease is age-related macular degeneration or diabetic retinopathy.

| | |
|---|---|
| Claim 1 | Page 13, last paragraph to page 14, line 3 |
| Claim 2 | Page 13, second last paragraph |
| Claim 3 | Page 13, second last paragraph |
| Claim 4 | Page 38, lines 15 to 17 |
| Claim 5 | Page 44, lines 17 to 22 |
| Claim 6 | Page 35, line 8 to page 38, line 4 |
| Claim 7 | Page 35, line 8 to page 38, line 4 |
| Claim 8 | Page 35, line 8 to page 38, line 4 |
| Claim 9 | Page 31, lines 18 to 24 |
| Claim 10 | Page 31, lines 18 to 24 |
| Claim 11 | Page 31, lines 18 to 24 |

**FIGURE 1**

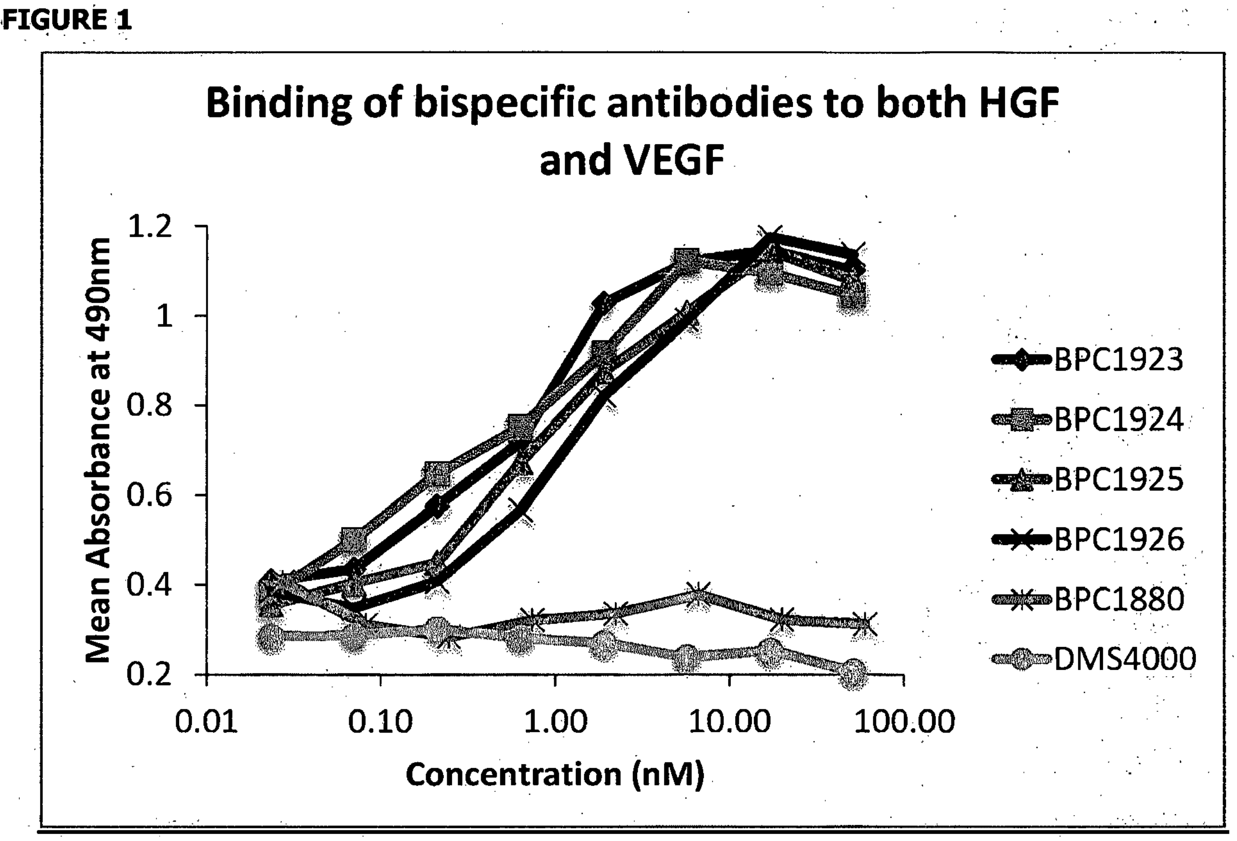
Binding of bispecific antibodies to both HGF and VEGF
Mean Absorbance at 490nm
1.2
1
0.8
0.6
0.4
0.2
0.01
0.10
1.00
10.00
100.00
Concentration (nM)
BPC1923
BPC1924
BPC1925
BPC1926
BPC1880
DMS4000

**FIGURE 2**

**A)**

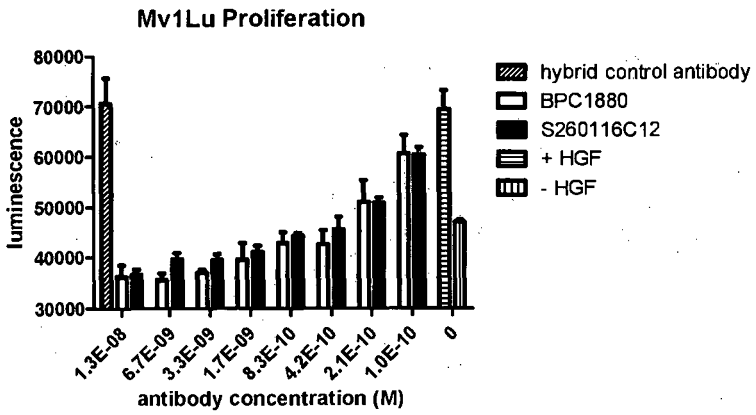
Mv1Lu Proliferation
luminescence
80000
70000
60000
50000
40000
30000
1.3E-08
6.7E-09
3.3E-09
1.7E-09
8.3E-10
4.2E-10
2.1E-10
1.0E-10
0
antibody concentration (M)
hybrid control antibody
BPC1880
S260116C12
+ HGF
- HGF

**B)**

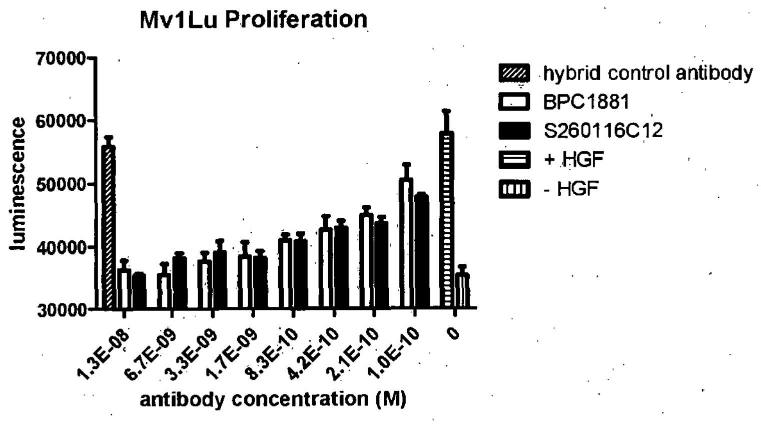
Mv1Lu Proliferation
luminescence
70000
60000
50000
40000
30000
1.3E-08
6.7E-09
3.3E-09
1.7E-09
8.3E-10
4.2E-10
2.1E-10
1.0E-10
0
antibody concentration (M)
hybrid control antibody
BPC1881
S260116C12
+ HGF
- HGF

**C)**

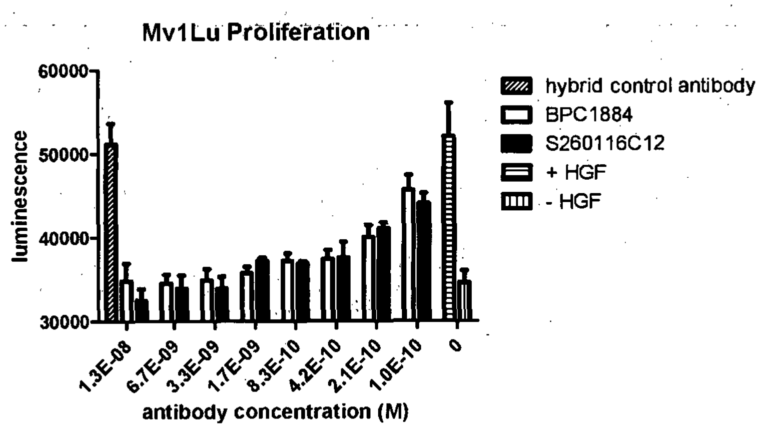
Mv1Lu Proliferation
luminescence
60000
50000
40000
30000
1.3E-08
6.7E-09
3.3E-09
1.7E-09
8.3E-10
4.2E-10
2.1E-10
1.0E-10
0
antibody concentration (M)
hybrid control antibody
BPC1884
S260116C12
+ HGF
- HGF

**FIGURE 2**

**D)**

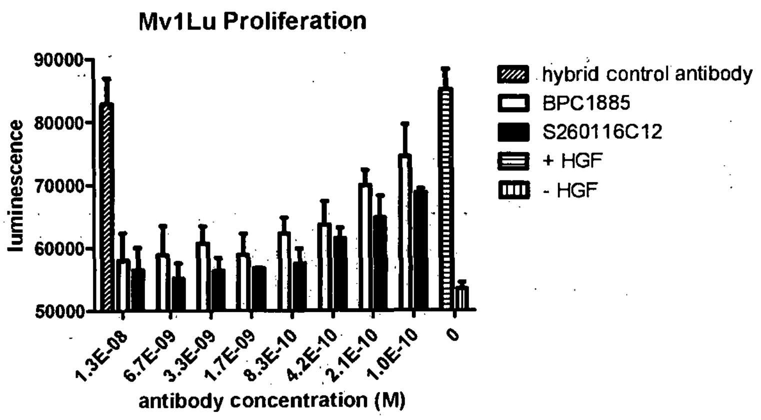
Mv1Lu Proliferation
luminescence
90000
80000
70000
60000
50000
1.3E-08
6.7E-09
3.3E-09
1.7E-09
8.3E-10
4.2E-10
2.1E-10
1.0E-10
0
antibody concentration (M)
hybrid control antibody
BPC1885
S260116C12
+ HGF
- HGF

**E)**

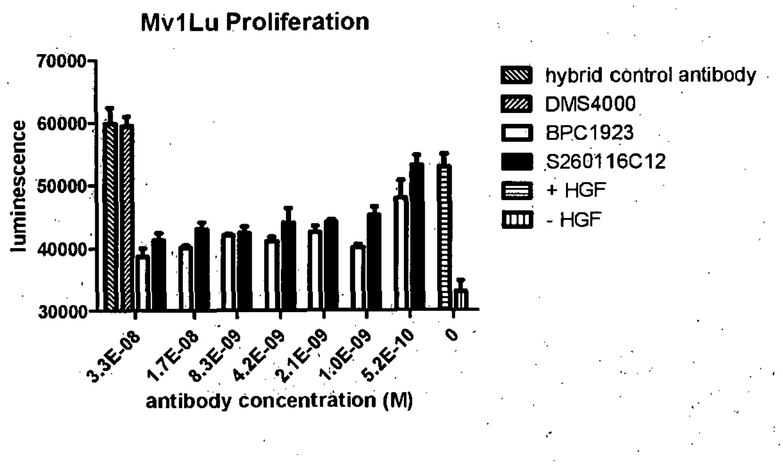
Mv1Lu Proliferation
luminescence
70000
60000
50000
40000
30000
3.3E-08
1.7E-08
8.3E-09
4.2E-09
2.1E-09
1.0E-09
5.2E-10
0
antibody concentration (M)
hybrid control antibody
DMS4000
BPC1923
S260116C12
+ HGF
- HGF

**F)**

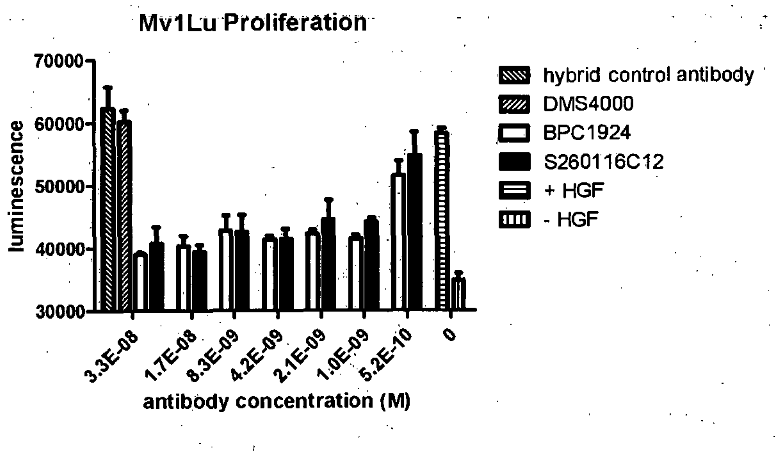
Mv1Lu Proliferation
luminescence
70000
60000
50000
40000
30000
3.3E-08
1.7E-08
8.3E-09
4.2E-09
2.1E-09
1.0E-09
5.2E-10
0
antibody concentration (M)
hybrid control antibody
DMS4000
BPC1924
S260116C12
+ HGF
- HGF

**FIGURE 3**

**A)**

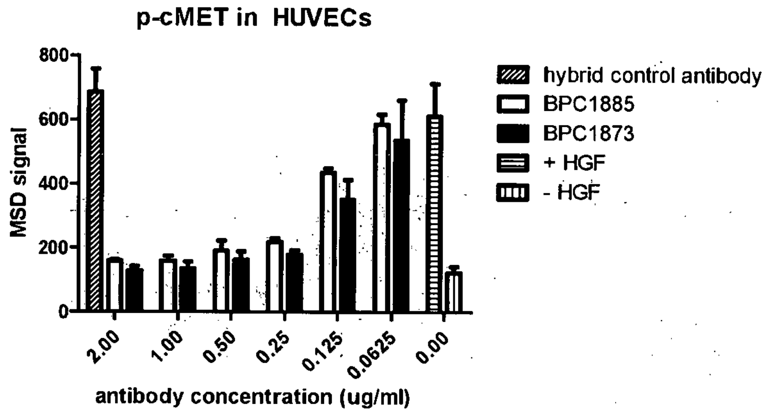
p-cMET in HUVECs
MSD signal
800
600
400
200
0
2.00
1.00
0.50
0.25
0.125
0.0625
0.00
antibody concentration (ug/ml)
hybrid control antibody
BPC1885
BPC1873
+ HGF
- HGF

**B)**

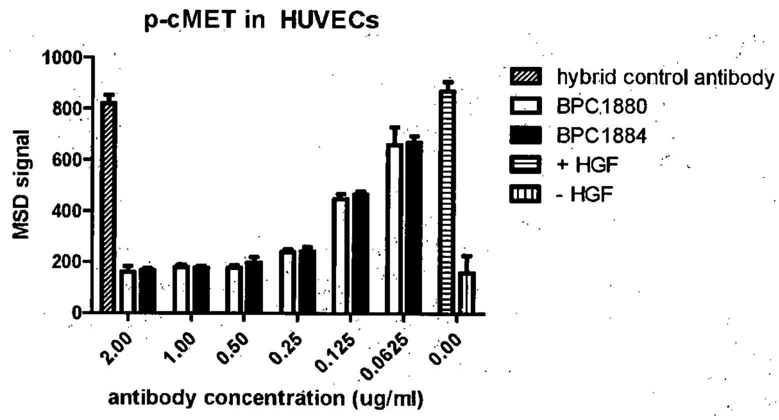
p-cMET in HUVECs
MSD signal
1000
800
600
400
200
0
2.00
1.00
0.50
0.25
0.125
0.0625
0.00
antibody concentration (ug/ml)
hybrid control antibody
BPC1880
BPC1884
+ HGF
- HGF

**C)**

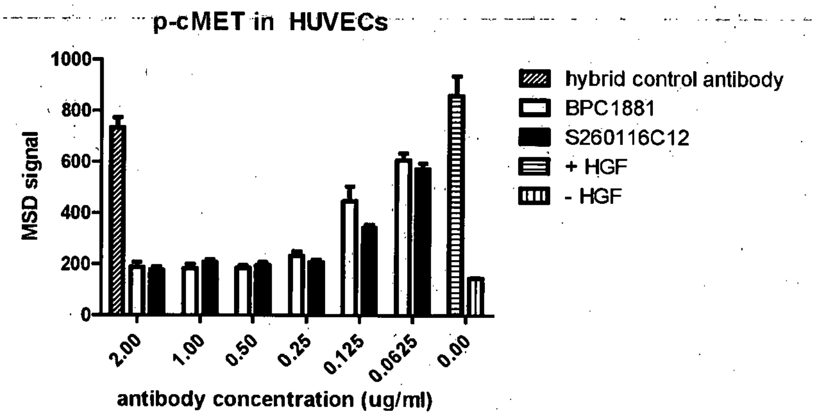
p-cMET in HUVECs
MSD signal
1000
800
600
400
200
0
2.00
1.00
0.50
0.25
0.125
0.0625
0.00
antibody concentration (ug/ml)
hybrid control antibody
BPC1881
S260116C12
+ HGF
- HGF

**FIGURE 3**

**D)**

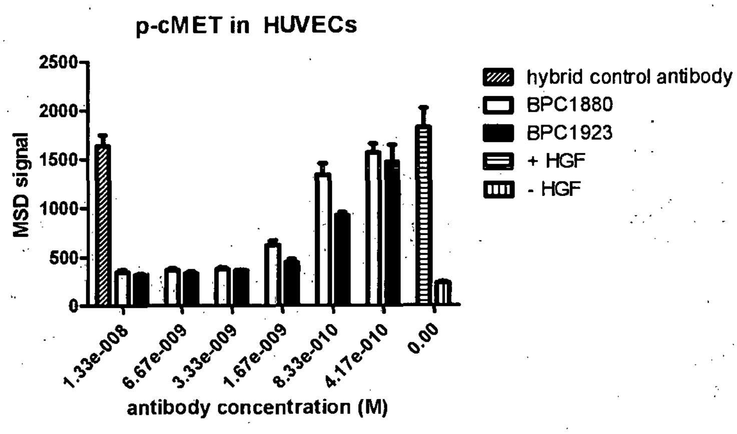
p-cMET in HUVECs
MSD signal
2500
2000
1500
1000
500
0
1.33e-008
6.67e-009
3.33e-009
1.67e-009
8.33e-010
4.17e-010
0.00
antibody concentration (M)
hybrid control antibody
BPC1880
BPC1923
+ HGF
- HGF

**E)**

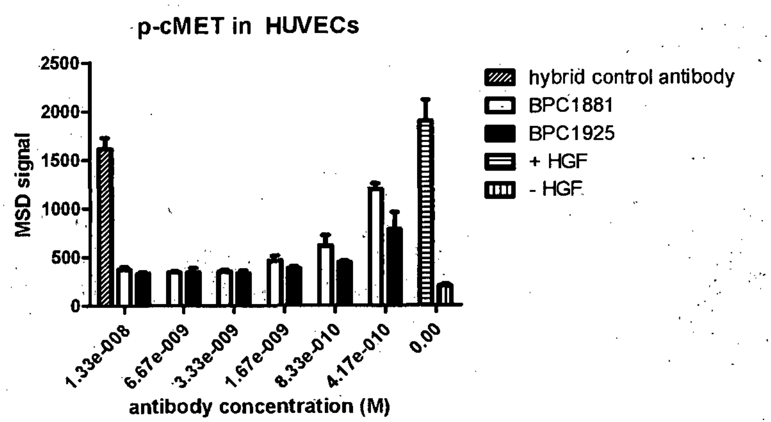
p-cMET in HUVECs
MSD signal
2500
2000
1500
1000
500
0
1.33e-008
6.67e-009
3.33e-009
1.67e-009
8.33e-010
4.17e-010
0.00
antibody concentration (M)
hybrid control antibody
BPC1881
BPC1925
+ HGF
- HGF

**FIGURE 4**

**A)**

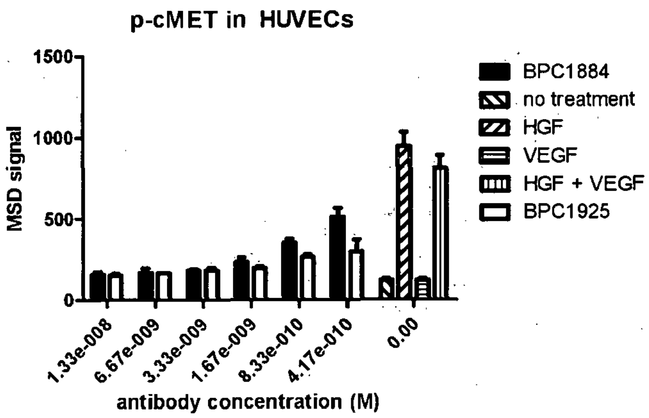
p-cMET in HUVECs
MSD signal
1500
1000
500
0
1.33e-008
6.67e-009
3.33e-009
1.67e-009
8.33e-010
4.17e-010
0.00
antibody concentration (M)
BPC1884
no treatment
HGF
VEGF
HGF + VEGF
BPC1925

**B)**

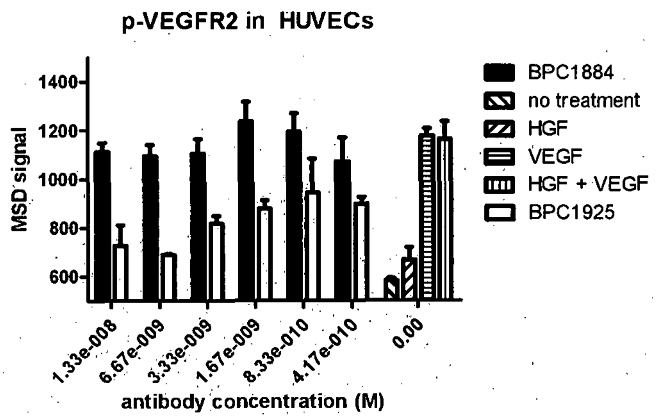
p-VEGFR2 in HUVECs
MSD signal
1400
1200
1000
800
600
1.33e-008
6.67e-009
3.33e-009
1.67e-009
8.33e-010
4.17e-010
0.00
antibody concentration (M)
BPC1884
no treatment
HGF
VEGF
HGF + VEGF
BPC1925

FIGURE 5

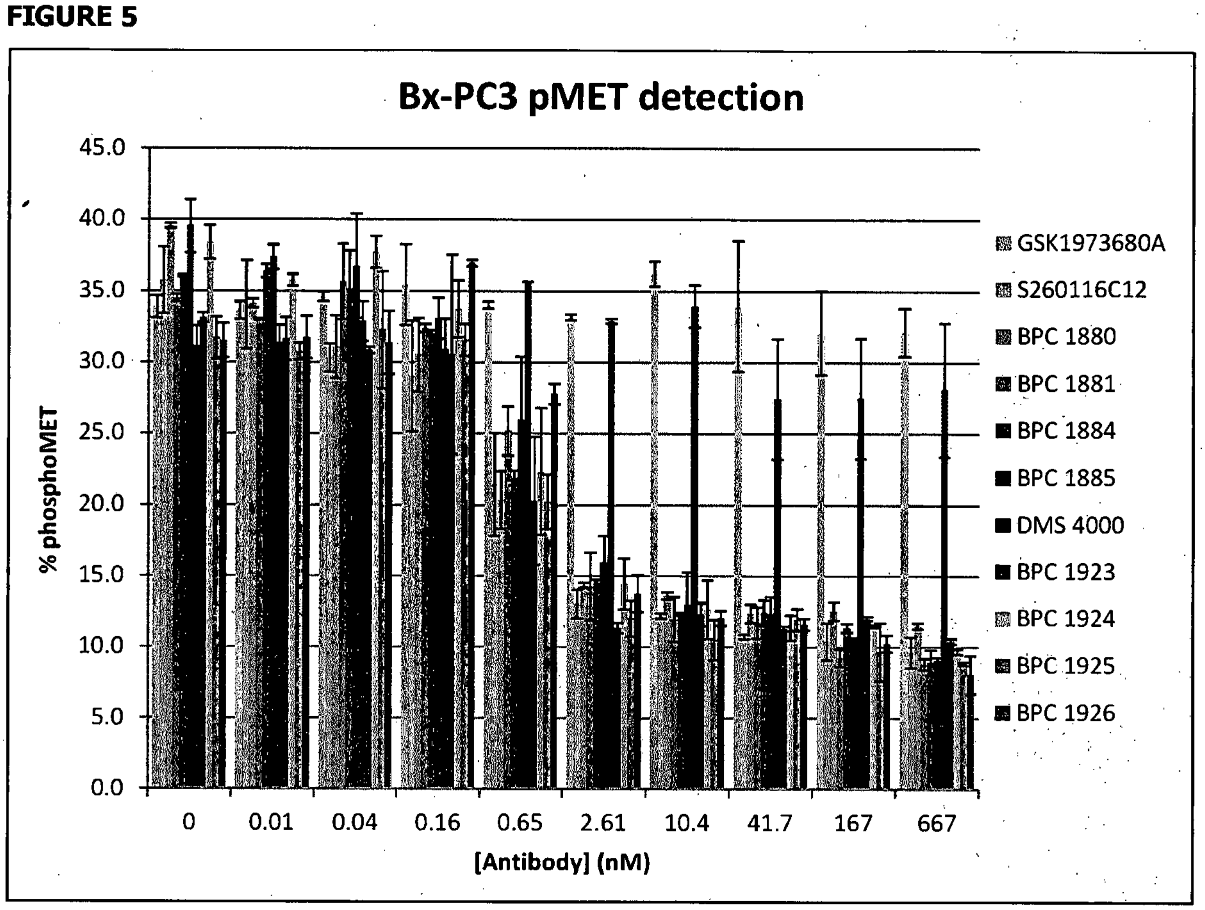
Bx-PC3 pMET detection
45.0
40.0
35.0
30.0
25.0
20.0
15.0
10.0
5.0
0.0
% phosphoMET
0
0.01
0.04
0.16
0.65
2.61
10.4
41.7
167
667
[Antibody] (nM)
GSK1973680A
S260116C12
BPC 1880
BPC 1881
BPC 1884
BPC 1885
DMS 4000
BPC 1923
BPC 1924
BPC 1925
BPC 1926

**FIGURE 6**

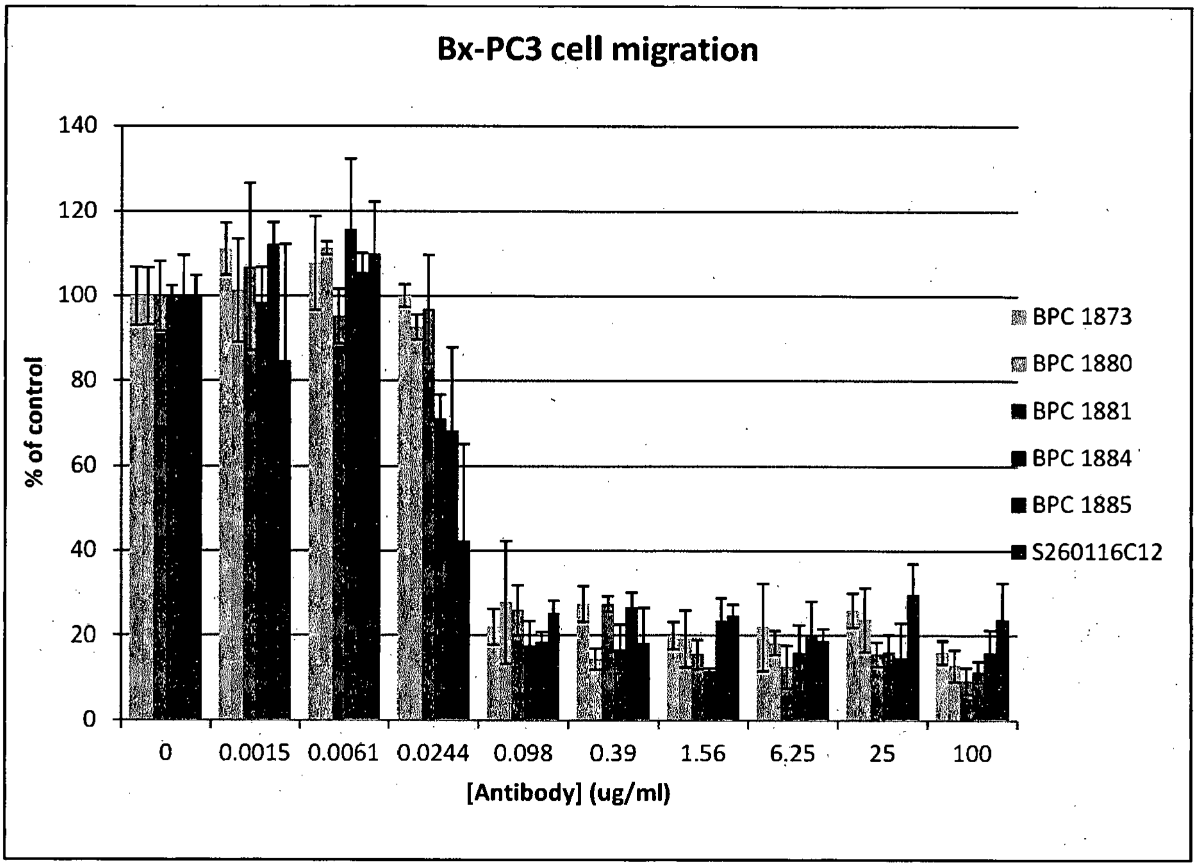
Bx-PC3 cell migration
140
120
100
80
60
40
20
0
% of control
0
0.0015
0.0061
0.0244
0.098
0.39
1.56
6.25
25
100
[Antibody] (ug/ml)
BPC 1873
BPC 1880
BPC 1881
BPC 1884
BPC 1885
S260116C12

Europäisches Patentamt
European Patent Office
Office européen des brevets

# EUROPEAN SEARCH REPORT

Application Number
EP 14 19 3184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/003388 A2 (DOMANTIS LTD [GB]; IGNATOVICH OLGA [GB]; DE WILDT RUDOLF [GB]; WOOLVEN) 12 January 2006 (2006-01-12) * page 100, paragraph 5 - page 101, paragraph 4 * * page 102, paragraph 5 * * page 118, paragraph 5 * * page 162, paragraph 3 - page 163, paragraph 2 * * page 245, last paragraph * * sequence 200 * ----- | 1-11 | INV. C07K16/22 |
| X | WO 2007/066106 A1 (DOMANTIS LTD [GB]; IGNATOVICH OLGA [GB]; HOLMES STEVE [GB]; BECKMANN R) 14 June 2007 (2007-06-14) * page 3, line 20 - line 26 * * page 120, line 25 - page 121, line 29 * * page 125, line 24 - page 126, line 1 * * page 129, line 22 - page 131, line 19 * * page 149, line 18 - line 24 * * sequence 108 * ----- | 1-11 | |
| | | | TECHNICAL FIELDS SEARCHED (IPC) |
| E | WO 2013/014208 A2 (GLAXO GROUP LTD [GB]; ASHMAN CLAIRE [GB]; CATCHPOLE IAN RICHARD [GB];) 31 January 2013 (2013-01-31) * claims 1,8-10,40-51 * * sequence 105 * ----- -/-- | 1-11 | C07K |

The present search report has been drawn up for all claims

1

| Place of search | Date of completion of the search | Examiner |
|---|---|---|
| Munich | 24 February 2015 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 3184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AVERY R L ET AL: "Intravitreal Bevacizumab (Avastin) for Neovascular Age-Related Macular Degeneration", OPHTHALMOLOGY, J. B. LIPPINCOTT CO., PHILADELPHIA, PA, US, vol. 113, no. 3, 1 March 2006 (2006-03-01), pages 363-372.e5, XP027899949, ISSN: 0161-6420 [retrieved on 2006-03-01] * the whole document * ----- | 1-11 | |
| A | GOKHAN GULKILIK ET AL: "Intravitreal bevacizumab for persistent macular edema with proliferative diabetic retinopathy", INTERNATIONAL OPHTHALMOLOGY, KLUWER ACADEMIC PUBLISHERS, DO, vol. 30, no. 6, 10 October 2010 (2010-10-10), pages 697-702, XP019865335, ISSN: 1573-2630, DOI: 10.1007/S10792-010-9403-Y * the whole document * ----- | 1-11 | |

TECHNICAL FIELDS SEARCHED (IPC)

The present search report has been drawn up for all claims

1

| Place of search | Date of completion of the search | Examiner |
|---|---|---|
| Munich | 24 February 2015 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 3184

This annex lists the patent family members relating to the patent documents cited in the above-mentioned European search report. The members are as contained in the European Patent Office EDP file on
The European Patent Office is in no way liable for these particulars which are merely given for the purpose of information.

24-02-2015

| Patent document cited in search report | Publication date | Patent family member(s) | Publication date |
|---|---|---|---|
| WO 2006003388 A2 | 12-01-2006 | AU 2005259006 A1 | 12-01-2006 |
| | | AU 2010201856 A1 | 27-05-2010 |
| | | CA 2571536 A1 | 12-01-2006 |
| | | EA 200700175 A1 | 31-08-2007 |
| | | EA 201070623 A1 | 30-12-2010 |
| | | EP 1761565 A2 | 14-03-2007 |
| | | EP 1777235 A1 | 25-04-2007 |
| | | EP 2322554 A1 | 18-05-2011 |
| | | IL 180187 A | 28-02-2011 |
| | | JP 2013018785 A | 31-01-2013 |
| | | JP 2013056890 A | 28-03-2013 |
| | | KR 20070050912 A | 16-05-2007 |
| | | KR 20100049700 A | 12-05-2010 |
| | | US 2006073141 A1 | 06-04-2006 |
| | | WO 2006003388 A2 | 12-01-2006 |
| WO 2007066106 A1 | 14-06-2007 | AU 2006323412 A1 | 14-06-2007 |
| | | BR PI0619463 A2 | 08-01-2013 |
| | | CA 2632417 A1 | 14-06-2007 |
| | | CR 10024 A | 22-09-2008 |
| | | EA 200801172 A1 | 30-12-2008 |
| | | EP 1966242 A1 | 10-09-2008 |
| | | JP 2009518024 A | 07-05-2009 |
| | | KR 20080077261 A | 21-08-2008 |
| | | MA 30021 B1 | 01-12-2008 |
| | | TW 200804425 A | 16-01-2008 |
| | | US 2010056439 A1 | 04-03-2010 |
| | | US 2013041136 A1 | 14-02-2013 |
| | | WO 2007066106 A1 | 14-06-2007 |
| WO 2013014208 A2 | 31-01-2013 | AU 2012288846 A1 | 06-02-2014 |
| | | CA 2842099 A1 | 31-01-2013 |
| | | CL 2014000204 A1 | 25-07-2014 |
| | | CN 103842380 A | 04-06-2014 |
| | | CO 7020847 A2 | 11-08-2014 |
| | | CR 20140047 A | 24-03-2014 |
| | | DO P2014000017 A | 30-04-2014 |
| | | EA 201391812 A1 | 30-06-2014 |
| | | EP 2736925 A2 | 04-06-2014 |
| | | JP 2014523746 A | 18-09-2014 |
| | | KR 20140041908 A | 04-04-2014 |
| | | MA 35352 B1 | 01-08-2014 |
| | | PE 16592014 A1 | 21-11-2014 |
| | | US 2014205604 A1 | 24-07-2014 |
| | | WO 2013014208 A2 | 31-01-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9308829 A
- WO 9404690 A
- WO 2007095338 A
- WO 0190192 A
- WO 2009068649 A
- WO 2007024715 A
- WO 2009040562 A
- WO 0029004 A
- US 20050043519 A
- US 7250297 B1
- US 20070224633 A
- EP 1641818 A1
- US 20040132028 A1
- US 20080139791 A
- WO 2005056764 A
- US 6818418 B1
- WO 2008098796 A
- WO 2009007427 A
- EP 0307434 A
- WO 2003011878 A
- WO 2006014679 A
- EP 1229125 A
- US 7214775 B
- US 6946292 B
- WO 0061739 A
- WO 0231240 A
- WO 2007011041 A
- US 20070148165 A
- WO 0042072 A
- WO 02060919A2 A
- WO 9743316 A
- US 5869046 A
- US 5747035 A
- WO 9632478 A
- WO 9114438 A
- WO 9943713 A
- WO 0009560 A
- US 4703039 A
- US 6165745 A
- WO 2007059782 A
- WO 2006015371 A
- WO 9958679 A
- WO 9616990 A
- US 4873316 A
- US 6555313 B1, Griffiths
- US 5733743 A, Johnson
- US 5969108 A, McCafferty
- US 5702892 A, Mulligan-Kehoe
- WO 9920749 A, Tomlinson
- WO 0157065 A
- WO 9958655 A
- WO 9708320 A
- WO 9902671 A
- WO 0040712 A
- US 6297053 B
- WO 9935146 A
- WO 0202552 A
- US 5747498 A
- GB 9600961 W
- WO 9633980 A
- US SN61512143 A


### Non-patent literature cited in the description


- **MILLSTEIN et al.** *Nature,* 1983, vol. 305, 537-539
- **TRAUNECKER et al.** *EMBO,* 1991, vol. 10, 3655-3659
- **SURESH et al.** *Methods in Enzymology,* 1986, vol. 121, 210
- *Mol. Immunol.,* 2006, vol. 44, 656-665
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Bibl. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- Non-Antibody Scaffolds from Handbook of Therapeutic Antibodies. 2007
- *Protein Science,* 2006, vol. 15, 14-27
- **SHIELDS et al.** *The Journal of Biological Chemistry,* 2001, vol. 276, 6591-6604

- **CHAPPEL et al.** *The Journal of Biological Chemistry,* 1993, vol. 268, 25124-25131
- **LAZAR et al.** *PNAS,* 2006, vol. 103, 4005-4010
- *J Imm Meth,* 1995, vol. 184, 29-38
- **DUNCAN et al.** *Nature,* 1988, vol. 332, 563-564
- **LUND et al.** *J. Immunol.,* 1991, vol. 147, 2657-2662
- **CHAPPEL et al.** *PNAS,* 1991, vol. 88, 9036-9040
- **BURTON ; WOOF.** *Adv. Immunol.,* 1992, vol. 51, 1-84
- **MORGAN et al.** *Immunology,* 1995, vol. 86, 319-324
- **HEZAREH et al.** *J. Virol.,* 2001, vol. 75 (24), 12161-12168
- **SHIELDS et al.** *J Biol Chem,* 2001, vol. 276, 6591-6604
- **NECHANSKY et al.** *Mol Immunol,* 2007, vol. 44, 1815-1817
- **JUNGHANS R.P.** *Immunol.Res,* 1997, vol. 16, 29-57
- **GHETIE et al.** *Annu.Rev.Immunol.,* 2000, vol. 18, 739-766
- **SHIELDS et al.** *J Biol Chem,* 2001, vol. 276, 6591-604
- **DALL'ACQUA et al.** *J Immunol.,* 2002, vol. 169, 5171-80
- **FIRAN et al.** *International immunology,* 2001, vol. 13, 993
- **REITER et al.** *J Mol Biol,* 1999, vol. 290, 685-698
- **EWERT et al.** *J Mol Biol,* 2003, vol. 325, 531-553
- **JESPERS et al.** *J Mol Biol,* 2004, vol. 337, 893-903
- **JESPERS et al.** *Nat Biotechnol,* 2004, vol. 22, 1161-1165
- **MARTIN et al.** *Protein Eng.,* 1997, vol. 10, 607-614
- **SEPULVADA et al.** *J Mol Biol,* 2003, vol. 333, 355-365
- **EPP et al.** *Biochemistry,* 1975, vol. 14, 4943-4952
- **HUAN et al.** *Biochemistry,* 1994, vol. 33, 14848-14857
- **HUANG et al.** *Mol immunol,* 1997, vol. 34, 1291-1301
- **JAMES et al.** *J Mol Biol.,* 2007, vol. 367, 603-8
- **PLÜCKTHUN, A.** *Immunol. Rev.,* 1992, vol. 130, 151-188
- **MILLER et al.** Genetic Engineering. Plenum Press, 1986, vol. 8, 277-298
- Remington's Pharmaceutical Science. Mack Publishing Company
- **LASMAR U ; PARKINS D.** The formulation of Biopharmaceutical products. *Pharma. Sci.Tech.today,* 03 April 2000, vol. 3, 129-137
- **WANG, W.** Instability, stabilisation and formulation of liquid protein pharmaceuticals. *Int. J. Pharm,* 1999, vol. 185, 129-188
- Stability of Protein Pharmaceuticals Part A and B. Plenum Press, 1992
- **AKERS,M.J.** Excipient-Drug interactions in Parenteral Formulations. *J.Pharm Sci,* 2002, vol. 91, 2283-2300
- **IMAMURA, K et al.** Effects of types of sugar on stabilization of Protein in the dried state. *J Pharm Sci,* 2003, vol. 92, 266-274
- **IZUTSU, KKOJIMA, S.** Excipient crystalinity and its protein-structure-stabilizing effect during freeze-drying. *J Pharm. Pharmacol,* 2002, vol. 54, 1033-1039
- **JOHNSON, R.** Mannitol-sucrose mixtures-versatile formulations for protein lyophilization. *J. Pharm. Sci,* 2002, vol. 91, 914-922
- **HA,E ; WANG W ; WANG Y.J.** Peroxide formation in polysorbate 80 and protein stability. *J. Pharm Sci,* 2002, vol. 91, 2252-2264
- **KABAT.** Sequences of Proteins of Immunological Interest. US Department of Health and Human Services
- **CHOTHIA ; LESK.** *J. Mol. Biol.,* 1987, vol. 196, 910-917
- **TATUSOVA, T. A. et al.** *FEMS Microbiol Lett,* 1999, vol. 174, 187-188
- **WINTER, G. et al.** *Annu. Rev. Immunol.,* 1994, vol. 12, 433-455
- **SOUMILLION, P. et al.** *Appl. Biochem. Biotechnol.,* 1994, vol. 47 (2-3), 175-189
- **CASTAGNOLI, L. et al.** *Comb. Chem. High Throughput Screen,* 2001, vol. 4 (2), 121-133
- **DENG et al.** *J. Biol. Chem.,* 1994, vol. 269, 9533
- **LOW et al.** *J. Mol. Biol.,* 1996, vol. 260, 359
- **CRAMERI et al.** *Nature Med.,* 1996, vol. 2, 100
- **RIECHMANN et al.** *Bio/Technology,* 1995, vol. 13, 475
- **LANDT, O. et al.** *Gene,* 1990, vol. 96, 125-128
- **HORTON, R.M. et al.** *Gene,* 1989, vol. 77, 61-68
- **GOLDMAN, L.A. et al.** *Biotechniques,* 1996, vol. 21, 1013-1015
- **MIZUSHIMA, S. et al.** *Nucleic Acids Res.,* 1990, vol. 18, 5322
- **TAWFIK ; GRIFFITHS.** *Nature Biotechnol,* 1998, vol. 16 (7), 652-6
- **WINTER et al.** *Ann. Rev. Immunology,* 1994, vol. 12, 433-55
- **TOMLINSON et al.** *J. Mol. Biol.,* 1992, vol. 227, 776
- **STEMMER.** *Nature,* 1994, vol. 370, 389-391

- **CHOTHIA ; LESK.** *J. Mol. Biol.,* 1987, vol. 196, 901
- **CHOTHIA et al.** *Nature,* 1989, vol. 342, 877
- **CHOTHIA et al.** *J. Mol. Biol.,* 1992, vol. 227, 799
- **TOMLINSON et al.** *EMBOJ.,* 1995, vol. 14, 4628
- **WILLIAMS et al.** *J. Mol. Biol.,* 1996, vol. 264, 220
- **MARTIN et al.** *J. Mol. Biol.,* 1996, vol. 263, 800
- **SHIRAI et al.** *FEBS Letters,* 1996, vol. 399, 1
- **HOOD et al.** *Cold Spring Harbor Symp. Quant. Biol.,* 1967, vol. 48, 133
- **KABAT et al.** Sequences of proteins of immunological interest. U.S. Department of Health and Human Services, 1991
- **HAWKINS et al.** *J. Mol. Biol.,* 1992, vol. 226, 889
- **LOW et al.** *. Mol. Biol.,* 1996, vol. 260, 359
- **BARBAS et al.** *Proc. Natl. Acad. Sci. USA,* 1992, vol. 89, 4457
- **HOOGENBOOM ; WINTER.** *J. Mol. Biol.,* 1992, vol. 227, 381
- **NISSIM et al.** *EMBO J.,* 1994, vol. 13, 692
- **GRIFFITHS et al.** *EMBO J.,* 1994, vol. 13, 3245
- **DE KRUIF et al.** *J. Mol. Biol.,* 1995, vol. 248, 97
- **TOMLINSON et al.** *J. Mol. Biol.,* 1996, vol. 256, 813
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- New guidelines to evaluate the response to treatment in solid tumors. **THERASSE P ; ARBUCK SG ; EISENHAUER EA ; WANDERS J ; KAPLAN RS ; RUBINSTEIN L et al.** J Natl Cancer Inst. European Organization for Research and Treatment of Cancer, 2000, vol. 92, 205-16
- **SEMBA et al.** *Proc. Natl. Acad. Sci. USA,* 1985, vol. 82, 6497-6501
- **YOKOTA et al.** *Oncogene,* 1988, vol. 2, 283-287
- **ZHOU et al.** *Cancer Res.,* 1987, vol. 47, 6123-6125
- **KING et al.** *Science,* 1985, vol. 229, 974-976
- **KRAUS et al.** *EMBO J.,* 1987, vol. 6, 605-610
- **VAN DE VIJVER et al.** *Mol. Cell. Biol.,* 1987, vol. 7, 2019-2023
- **YAMAMOTO et al.** *Nature,* 1986, vol. 319, 230-234
- Cancer Principles and Practice of Oncology. Lippincott Williams & Wilkins Publishers, 15 February 2001
- **WANI et al.** *J. Am. Chem, Soc.,* 1971, vol. 93, 2325
- **SCHIFF et al.** *Proc. Natl, Acad, Sci. USA,* 1980, vol. 77, 1561-1565
- **SCHIFF et al.** *Nature,* 1979, vol. 277, 665-667
- **KUMAR.** *J. Biol, Chem,* 1981, vol. 256, 10435-10441
- New trends in Natural Products Chemistry 1986. **D. G. I. KINGSTON et al.** Studies in Organic Chemistry. Elsevier, 1986, vol. 26, 219-235
- **MARKMAN et al.** *Yale Journal of Biology and Medicine,* 1991, vol. 64, 583
- **MCGUIRE et al.** *Ann. Intem, Med.,* 1989, vol. 111, 273
- **HOLMES et al.** *J. Nat. Cancer Inst.,* 1991, vol. 83, 1797
- **EINZIG.** *Proc. Am. Soc. Clin. Oncol.,* vol. 20, 46
- **FORASTIRE.** *Sem. Oncol.,* 1990, vol. 20, 56
- **WOO.** *Nature,* 1994, vol. 368, 750
- multiple cell lineages. **IGNOFF, R.J.** Cancer Chemotherapy Pocket Guide. 1998
- **KEARNS, C.M.** *Seminars in Oncology,* 1995, vol. 3 (6), 16-23
- **JONES ; BENDIG.** *Bio/Technology,* 1991, vol. 9, 88
- *J. Immunol Methods,* 16 January 1996, vol. 189 (1), 59-64